# EUROPEAN PATENT APPLICATION

(11) **EP 4 736 871 A1**
(43) Date of publication of application: **06.05.2026**
(21) Application number: 24825333.8
(22) Date of filing: 21.06.2024
(51) Int. Cl.: A61K 39/155, A61K 39/12, C07K 14/005, C12N 15/45

(54) **RESPIRATORY SYNCYTIAL VIRUS (RSV) VACCINE**

(30) Priority: 21.06.2023 WO PCT/CN2023/101796; 19.02.2024 WO PCT/CN2024/077620
(71) Applicant: Shenzhen Shenxin Biotechnology Co., Ltd., Shenzhen, Guangdong 518000 (CN)
(72) Inventor: LI, Linxian, Shenzhen, Guangdong 518000 (CN); LI, Hui, Shenzhen, Guangdong 518000 (CN)
(74) Representative: Dentons Patent Solutions Rechtsanwaltsgesellschaft mbH
(86) International application number: PCT/CN2024/100631
(87) International publication number: WO 2024/260446

(57) **Abstract**

The present invention relates to a respiratory syncytial virus (RSV) vaccine, the nucleic acid comprises a polynucleotide for encoding a mutant of RSV F protein comprising, as compared to a wild-type RSV F protein, one or more of the following mutations: disulfide bond mutations, a cavity filling mutation and an electrostatic mutation.

## Description

### Technical Field

The present invention belongs to the field of biotechnology, and relates to a respiratory syncytial virus (RSV) vaccine.

### Background

Respiratory syncytial virus (RSV) is a single-stranded RNA virus of Orthopneumovirus genus, Pneumoviridae. The genome of RSV includes 10 genes, which encode 11 proteins, including 9 structural proteins and 2 non-structural proteins, among which the structural proteins include 3 transmembrane surface glycoproteins: Attachment glycoprotein (G protein), Fusion protein (F protein) and small hydrophobic protein (SH protein).

Human respiratory syncytial virus (human orthopneumovirus, hRSV) has two subtypes: subtype A and subtype B, which differ from each other mainly in G protein. The infection of hRSV may cause upper and lower respiratory tract diseases, has a high incidence in infants, immunocompromised people and elder people, is the second most common cause of infant death, and is also the main cause of illness and death in the elder people (> 60 years old).

The fusion protein (F protein) is activated upon the cleavage into two fragments F1 and F2 by the host protease, which can cause the fusion of the virus capsule membrane and the host cell membrane, plays a role in the immunopathology caused by RSV infection, and is a popular protein in the research and development of antibodies, vaccines and other therapeutics.

During the entry of RSV into cells, F protein is rearranged from a pre-fusion conformation to a post-fusion conformation through an intermediate extension structure. During the rearrangement, the C-terminal coiling helix of the pre-fusion molecule is disassociated into the three chains constituting the same, which are then coiled around the spherical head and conjugated to three additional helices to form a post-fusion six-helix bundle. To prevent the entry of virus, it is speculated that F-specific neutralizing antibodies have to bind to F on virions with the pre-fusion conformation, or potentially extended intermediates, before the viral envelope is fused to the cell membrane. Thus, the pre-fusion form of F protein (i.e., "pre-F protein") is considered as a preferred conformation for vaccine antigens. However, during the preparation of the pre-F protein, the pre-F protein is apt to convert into a fused form. Therefore, it is still a challenge to use the pre-F protein as a vaccine antigen.

### Summary

The present disclosure provides a nucleic acid encoding a protein or polypeptide capable of inducing a specific immune response to prevent or at least reduce the infection of respiratory syncytial virus (RSV).

In addition, the present disclosure also provides a delivery carrier, a pharmaceutical composition, an RSV vaccine and use thereof in the preparation of a medicament.

A nucleic acid comprising a polynucleotide encoding a mutant of RSV F protein comprising an F1 polypeptide and an F2 polypeptide, and comprising, as compared to a wild-type RSV F protein, one or more of the following mutations: disulfide bond mutations, a cavity filling mutation and an electrostatic mutation.

In some embodiments, the disulfide bond mutations comprise one or more of the follows: S46C and T311C, A47C and I309C, L48C and V308C, S55C and L188C, V56C and T189C, V56C and V300C, I57C and S190C, I57C and Y299C, T58C and K191C, I59C and L193C, I59C and L297C, E60C and D194C, P101C and I152C, A102C and I148C, A102C and I152C, T103C and I146C, T103C and I148C, N104C and G145C, N104C and S146C, N105C and G145C, N105C and S146C, N105C and A147C, V144C and V406C, and S146C and N460C.

In some embodiments, the disulfide bond mutations comprise one or more of the follows: A47C and I309C, I57C and Y299C, P101C and I152C, A102C and I148C, A102C and I152C, T103C and I146C,N104C and G145C, N104C and S146C, N105C and G145C, N105C and S146C, and N105C and A147C.

In some embodiments, the cavity filling mutation includes one or more of the following (1) to (3):
(1) the substitution of T at position 54 by F, W, V, C, A, H, I, Y, L or M; preferably, the substitution of T at position 54 by F, W or V;
(2) the substitution of S at position 190 by V, I, M, F, L, W, Y, H, R, E, A, Q, G, P, T, C, D, N, or K; and
(3) the substitution of V at position 296 by I, L, Y, H, F, or M.

In some embodiments, the electrostatic mutation includes one or more of the follows:
(1) the substitution of D at position 486 by Q, S, H, N, T, P, C, L, I, F, or W; and
(2) the substitution of V at position 487 by Q, T, N, I, K, P, R, S, Y, D, F, L, H, or C.

In some embodiments, the mutations of the mutant include disulfide bond mutations and at least one of cavity filling mutation and electrostatic mutation; preferably, the mutations of the mutant include cavity filling mutation and disulfide bond mutations.

In some embodiments, the mutations of the mutant are selected from one group of the following (1) to (20): (1)A102C, I152C, T54V, S190M and V296L; (2)A102C, I152C, T54F, S190M and V296L; (3)A102C, I152C, T54C, S190M and V296L; (4)A102C, I152C, T54A, S190M and V296L; (5)A102C, I152C, T54V, S190F and V296L; (6)A102C, I152C, T54F, S190F and V296L; (7)A102C, I152C, T54C, S190F and V296L; (8)A102C, I152C, T54A, S190F and V296L; (9)P101C, I152C, T54V, S190M and V296L; (10)P101C, I152C, T54F, S190M and V296L; (11)P101C, I152C, T54C, S190M and V296L; (12)P101C, I152C, T54A, S190M and V296L; (13)P101C, I152C, T54V, S190F and V296L; (14)P101C, I152C, T54F, S190F and V296L; (15)P101C, I152C, T54C, S190F and V296L; (16)P101C, I152C, T54A, S190F and V296L; (17)A102C, I152C, T54V, S190I and V296L; (18)A102C, I152C, T54F, S190I and V296L; (19)A102C, I152C, T54C, S190I and V296L; and (20)A102C, I152C, T54A, S190I and V296L.

In some embodiments, the mutant is selected from one of the following (1) to (40): (1) a mutant comprising the amino acid sequence as shown in SEQ ID NO: 61 or 62; (2) a mutant comprising mutations A102C, I152C, T54V, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 61 or 62; (3) a mutant comprising the amino acid sequence as shown in SEQ ID NO: 63 or 64; (4) a mutant comprising mutations A102C, I152C, T54F, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 63 or 64; (5) a mutant comprising the amino acid sequence as shown in SEQ ID NO: 65 or 66; (6) a mutant comprising mutations A102C, I152C, T54C, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 65 or 66; (7) a mutant comprising the amino acid sequence as shown in SEQ ID NO: 67 or 68; (8) a mutant comprising mutations A102C, I152C, T54A, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 67 or 68; (9) a mutant comprising the amino acid sequence as shown in SEQ ID NO: 69 or 70; (10) a mutant comprising mutations A102C, I152C, T54V, S190F, and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 69 or 70; (11) a mutant comprising the amino acid sequence as shown in SEQ ID NO: 71 or 72; (12) a mutant comprising mutation A102C, I152C, T54F, S190F, and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 71 or 72; (13) a mutant comprising the amino acid sequence as shown in SEQ ID NO: 73 or 74; (14) a mutant comprising mutations A102C, I152C, T54C, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 73 or 74; (15) a mutant comprising the amino acid sequence as shown in SEQ ID NO: 75 or 76; (16) a mutant comprising mutations A102C, I152C, T54A, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 75 or 76; (17) a mutant comprising the amino acid sequence as shown in SEQ ID NO: 77 or 78; (18) a mutant comprising mutations P101C, I152C, T54V, S190M, and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 77 or 78; (19) a mutant comprising the amino acid sequence as shown in SEQ ID NO: 79 or 80; (20) a mutant comprising mutations P101C, I152C, T54F, S190M, and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 79 or 80; (21) a mutant comprising the amino acid sequence as shown in SEQ ID NO: 81 or 82; (22) a mutant comprising mutations P101C, I152C, T54C, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 81 or 82; (23) a mutant comprising the amino acid sequence as shown in SEQ ID NO: 83 or 84; (24) a mutant comprising mutations P101C, I152C, T54A, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 83 or 84; (25) a mutant comprising the amino acid sequence as shown in SEQ ID NO: 85 or 86; (26) a mutant comprising mutations P101C, I152C, T54V, S190F, and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 85 or 86 ; (27) a mutant comprising the amino acid sequence as shown in SEQ ID NO: 87 or 88; (28) a mutant comprising mutations P101C, I152C, T54F, S190F, and V296L and comprising at least 85%, 88%, 90%, 91%, 92%, 93%, 94%, 95%, 95.5%, 96%, 96.5% identical to the amino acid sequence as shown in SEQ ID NO: 87 or 88; (29) a mutant comprising the amino acid sequence as shown in SEQ ID NO: 89 or 90; (30) a mutant comprising mutations P101C, I152C, T54C, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 89 or 90; (31) a mutant comprising the amino acid sequence as shown in SEQ ID NO: 91 or 92; (32) a mutant comprising mutations P101C, I152C, T54A, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 91 or 92; (33) a mutant comprising the amino acid sequence as shown in SEQ ID NO: 93 or 94; (34) a mutant comprising mutations A102C, I152C, T54V, S190I, and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 93 or 94; (35) a mutant comprising the amino acid sequence as shown in SEQ ID NO: 95 or 96; (36) a mutant comprising mutations A102C, I152C, T54F, S190I and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 95 or 96; (37) a mutant comprising the amino acid sequence as shown in SEQ ID NO: 97 or 98; (38) a mutant comprising mutations A102C, I152C, T54C, S190I and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 97 or 98; (39) a mutant comprising the amino acid sequence as shown in SEQ ID NO: 99 or 100; and (40) a mutant comprising mutations A102C, I152C, T54A, S190I and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 99 or 100.

Another nucleic acid comprising a polynucleotide for encoding a mutant of RSV F protein comprising an F1 polypeptide and an F2 polypeptide, and comprising, as compared to a wild-type RSV F protein, one or more mutations of the following (1) to (8): (1) the substitution of K or E at position 66 by I, L, M, F or V; (2) the substitution of N at position 67 by I; (3) the substitution of S at position 213 by P; (4) the substitution of S at position 215 by P; (5) the substitution of N at position 216 by P; (6) the substitution of I at position 217 by P, F, Y or W; (7) the substitution of D at position 486 by Q, S, H, N, T, P, C, L, I, F, or W; and (8) the substitution of V at position 487 by Q, T, N, I, K, P, R, S, Y, D, F, L, H, or C.

In some embodiments, the mutations in the mutant comprise the following (1) to (3): (1) one of the following mutations: the substitution of S at position 213 by P, the substitution of S at position 215 by P, the substitution of N at position 216 by P and the substitution of I at position 217 by P, F, Y or W; (2) one of the following mutations: the substitution of K or E at position 66 by I, L, M, F or V and the substitution of N at position 67 by I; and (3) one of the following mutations: the substitution of D at position 486 by Q, S, H, N, T, P, C, L, I, F or W and the substitution of V at position 487 by Q, T, N, I, K, P, R, S, Y, D, F, L, H or C.

In some embodiments, the mutations in the mutant are selected from one group of the following (1) to (13): (1)I217P, E66I and D486Q; (2)I217P, N67I and E487Q; (3)I217P, E66I and E487T; (4)S215P, N67I and E487Q; (5)S213P, N67I and E487Q; (6)S213P, E661 and E487T; (7)S213P, E661 and D486Q; (8)N216P, N67I and E487Q; (9)N216P, E66I and E487T; (10)N216P, E66I and D486Q; (11)A102C, I152C, S213P and E66I; (12)P101C, I152C, S213P and E661; and (13)S213P and E66I.

In some embodiments, the mutant is selected from one of the following (1) to (30): (1) a mutant comprising the amino acid sequence as shown in SEQ ID NO: 31 or 32; (2) a mutant comprising mutations N216P, E66I and D486Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 31 or 32; (3) a mutant comprising the amino acid sequence as shown in SEQ ID NO: 33 or 34; (4) a mutant comprising mutations N216P, E66I and E487T and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 33 or 34; (5) a mutant comprising the amino acid sequence as shown in SEQ ID NO: 35 or 36; (6) a mutant comprising mutations N216P, N67I and E487Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 35 or 36; (7) a mutant comprising the amino acid sequence as shown in SEQ ID NO: 37 or 38; (8) a mutant comprising mutations S213P, E66I and D486Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 37 or 38; (9) a mutant comprising the amino acid sequence as shown in SEQ ID NO: 39 or 40; (10) a mutant comprising mutations S213P, E66I and E487T and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 39 or 40; (11) a mutant comprising the amino acid sequence as shown in SEQ ID NO: 41 or 42; (12) a mutant comprising mutation S213P, N67I and E487Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 41 or 42 (13) a mutant comprising the amino acid sequence as shown in SEQ ID NO: 43 or 44; (14) a mutant comprising mutations S215P, N67I and E487Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 43 or 44; (15) a mutant comprising the amino acid sequence as shown in SEQ ID NO: 45 or 46; (16) a mutant comprising mutations I217P, E66I and E487T and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 45 or 46; (17) a mutant comprising the amino acid sequence as shown in SEQ ID NO: 47 or 48; (18) a mutant comprising mutations I217P, N67I and E487Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 47 or 48; (19) a mutant comprising the amino acid sequence as shown in SEQ ID NO: 49 or 50; (20) a mutant comprising mutations I217P, E66I and D486Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 49 or 50; (21) a mutant comprising the amino acid sequence as shown in SEQ ID NO: 51 or 52; (22) a mutant comprising mutations S213P, E66I and E487T and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 51 or 52; (23) a mutant comprising the amino acid sequence as shown in SEQ ID NO: 53 or 54; (24) a mutant comprising mutations S213P, E66I and D486Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 53 or 54; (25) a mutant comprising the amino acid sequence as shown in SEQ ID NO: 55 or 56; (26) a mutant comprising mutations A102C, I152C, S213P and E66I and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 55 or 56 ; (27) a mutant comprising the amino acid sequence as shown in SEQ ID NO: 57 or 58; (28) a mutant comprising mutations P101C, I152C, S213P and E66I and comprising at least 85%, 88%, 90%, 91%, 92%, 93%, 94%, 95%, 95.5%, 96%, 96.5% identical to the amino acid sequence as shown in SEQ ID NO: 57 or 58; (29) a mutant comprising the amino acid sequence as shown in SEQ ID NO: 59 or 60; (30) a mutant comprising mutations S213P and E66I and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 59 or 60.

In some embodiments, the wild-type RSV is subtype A or subtype B.

In some embodiments, the wild-type RSV is wild-type hRSV.

In some embodiments, the wild-type hRSV is strain A2, strain Ontario or strain Buenos Aires.

In some embodiments, the nucleic acid is RNA. In some embodiments, the RNA is mRNA; more preferably, the mRNA comprises at least one of a 5' -cap structure, 5' -UTR, 3' -UTR, and a poly (A) tail.

In some embodiments, the nucleic acid is DNA. In some embodiments, the DNA can be transcribed into RNA.

In some embodiments, the DNA sequence corresponding to the 5' -UTR is shown in SEQ ID NO: 209;
and/or, the DNA sequence corresponding to the 3' -UTR is shown in SEQ ID NO: 210;
and/or, the poly (A) tail consists of nucleotides comprising at least 20, at least 40, at least 80, at least 100 or at least 120 A nucleotides; preferably, the poly (A) tail consists of nucleotides comprising at least 20, at least 40, at least 80, at least 100 or at least 120 consecutive A nucleotides; preferably, the poly (A) tail consists of nucleotides comprising one or more nucleotides other than the A nucleotides; more preferably, the DNA sequence corresponding to the poly (A) tail is shown in SEQ ID NO: 211.

In some embodiments, the nucleic acid contains a modified nucleotide.

In some embodiments, the nucleic acid comprises a modified nucleoside; preferably, the modified nucleoside comprises at least one of a modified uridine, a modified cytidine, a modified adenosine, and a modified guanosine.

In some embodiments, the nucleic acid comprises a polynucleotide encoding one or more mutants of RSV F protein.

In some embodiments, the nucleic acid comprises a polynucleotide encoding a protein or polypeptide other than RSV F protein.

In some embodiments, the nucleic acid further comprises one or more of a polynucleotide encoding the transmembrane domain of RSV F protein, a polynucleotide encoding a pep27 polypeptide, a polynucleotide encoding a signal peptide, and a polynucleotide encoding a trimerization domain.

A genetic engineering vector, comprising the above nucleic acid, or comprising a polynucleotide capable of being transcribed into the above nucleic acid.

A host cell comprising the above genetic engineering vector.

A mutant of RSV F protein encoded by the above nucleic acid.

In some embodiments, the mutant is a trimer.

A delivery carrier comprising a nucleic acid composition, the nucleic acid composition comprises a first nucleic acid or a first genetic engineering vector, the first nucleic acid is the above nucleic acid, and the first genetic engineering vector is the abovegenetic engineering vector;
or, the nucleic acid composition comprises a plurality of first nucleic acids or a plurality of first genetic engineering vectors, the first nucleic acids are the above nucleic acids, the first genetic engineering vectors are the above genetic engineering vectors, and the plurality of first nucleic acids are independent from each other or the plurality of first genetic engineering vectors are independent from each other.

In some embodiments, the nucleic acid composition further comprises a second nucleic acid or a second vector, the second nucleic acid comprises a polynucleotide encoding a protein or polypeptide other than the mutant of RSV F protein, and the second vector comprises a polynucleotide encoding a protein or polypeptide other than the mutant of RSV F protein; and/or the first nucleic acid and the second nucleic acid are RNAs.

In some embodiments, the first nucleic acid and the second nucleic acid are mRNAs.

In some embodiments, the delivery carrier is a lipid nanoparticle (LNP), a cationic liposome, a cationic protein, or a lipopolyplex (LPP).

In some embodiments, the delivery carrier is a lipid nanoparticle comprising a cationic lipid comprising the following compound (IV), or a pharmaceutically acceptable salt or stereoisomer thereof: 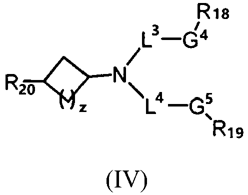 where:
L³ and L⁴ are the same or different, and are each independently C1-C12 alkylene, C2-C12 alkenylene or C2-C12 alkynylene; preferably, L³ and L⁴ are the same or different, and are each independently C3-C10 alkylene, C3-C10 alkenylene or C3-C10 alkynylene; further preferably, L³ and L⁴ are the same or different, and are each independently C3-C10 alkylene; most preferably, L³ and L⁴ are the same or different, and are each independently C5-C8 alkylene;
G⁴ and G⁵ are the same or different, and are each independently -O- (C=O)-, - (C=O)-O-, - C(=O)-, -O-, -C(=O)-S-or-S-C(=O)-; preferably, G⁴ and G⁵ are the same or different, and are each independently -O- (C=O)-, - (C=O)-O-, -C(=O)- or -O-; most preferably, G⁴ and G⁵ are the same or different, and are each independently selected from -O- (C=O)- or - (C=O)-O-;
R₁₈ and R₁₉ are the same or different, and are each independently C5-C27 alkyl or C5-C27 alkenyl containing one or more double bonds; preferably, R₁₈ and R₁₉ are the same or different, and are each independently C8-C20 alkyl or C8-C20 alkenyl containing one or more double bonds; further preferably, R₁₈ and R₁₉ are the same or different, and are each independently C9-C17 alkyl or C9-C18 alkenyl containing one or two double bonds; most preferably, R₁₈ and R₁₉ are the same or different, and are each independently 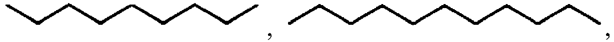 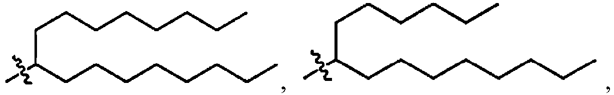 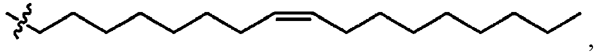 or 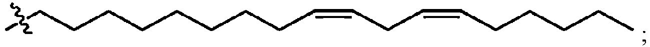
R₂₀ is halogen, hydroxyl, cyano, C1-C6 alkyl, nitro, C1-C6 alkoxy, C1-C6 alkylcarbonyloxy, C1-C6 alkoxycarbonyl, C1-C6 alkylaminocarbonyl or C1-C6 alkylcarbonylamino; preferably, R₂₀ is halogen, hydroxyl, cyano, C1-C6 alkoxy, C1-C6 alkylcarbonyloxy, C1-C6 alkoxycarbonyl, C1-C6 alkylaminocarbonyl or C1-C6 alkylcarbonylamino; further preferably, R₂₀ is halogen, hydroxyl, cyano, C1-C4 alkoxy, C1-C4 alkylcarbonyloxy, C1-C4 alkoxycarbonyl, C1-C4 alkylaminocarbonyl or C1-C4 alkylcarbonylamino; most preferably, R₂₀ is fluorine, hydroxyl, cyano, methoxy, acetoxy, methoxycarbonyl, butylaminocarbonyl or acetamido;
z is 1, 2 or 3.

In some embodiments, the cationic lipid is the following compound (IV-1), or a pharmaceutically acceptable salt or stereoisomer thereof: 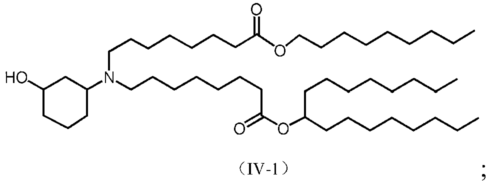 or, the cationic lipid is the following compound, or a pharmaceutically acceptable salt thereof: 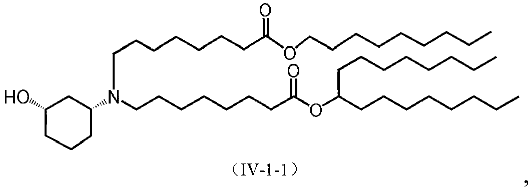 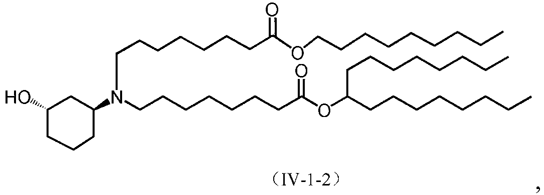 or

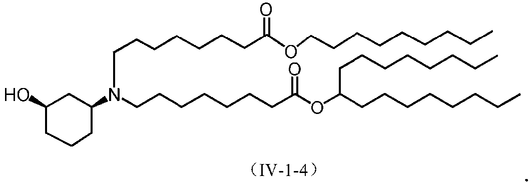

A pharmaceutical composition comprising the above nucleic acid, the above genetic engineering vector, the above host cell, the above mutant of RSV F protein or the above delivery carrier, and a pharmaceutically acceptable carrier.

In some embodiments, the pharmaceutical composition comprises a plurality of the delivery carriers;
or, the pharmaceutical composition comprises a plurality of the nucleic acids.

Use of the above nucleic acid, the above genetic engineering vector, the above host cell, the above mutant of RSV F protein, the above delivery carrier or the above pharmaceutical composition in the preparation of a medicament.

In some embodiments, the medicament is used to prevent or treat RSV infection or diseases caused by RSV infection.

In some embodiments, the medicament is a vaccine.

A vaccine comprising the above nucleic acid, the above genetic engineering vector, the above mutant of RSV F protein or the above delivery carrier.

### Brief Description of Drawings

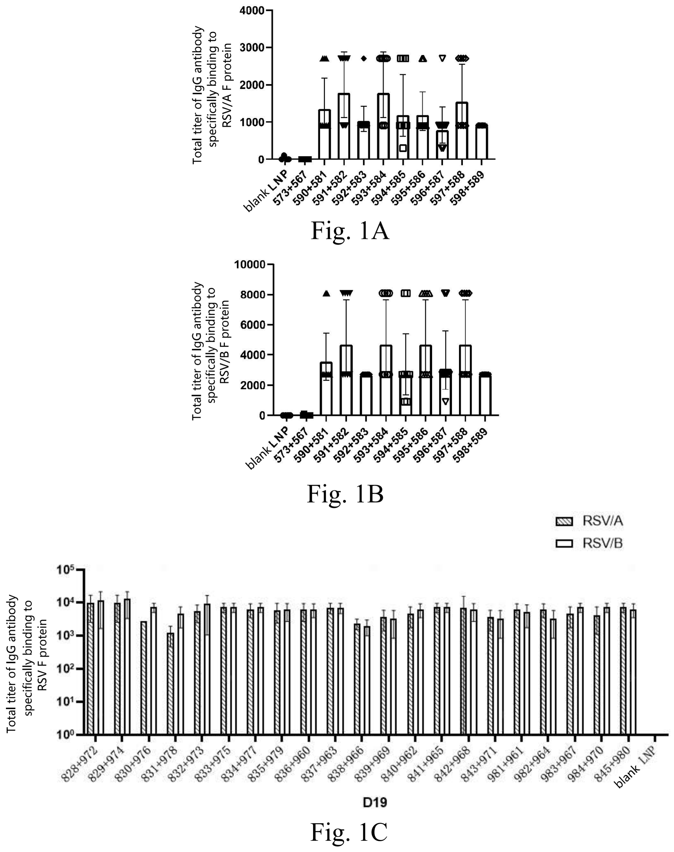

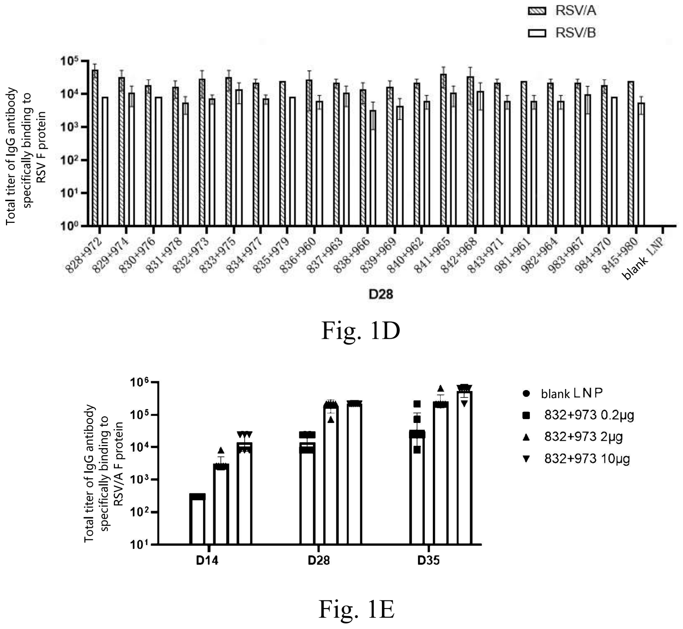

FIG. 1A to FIG. 1B show the detection of the induced total IgG antibody titers on day 35 after single immunization of mice with different mRNA vaccines (GMT, 95% CI), where FIG. 1A is a total IgG antibody titer specifically binding to RSV/A F protein, and FIG. 1B is a total IgG antibody titer specifically binding to RSV/B F protein. In FIG. 1A to FIG. 1B, "RSV/A F protein" represents an F protein of subtype A RSV (the same applies to "RSV/A F protein" occurring in other figures), "RSV/B F protein" represents an F protein of subtype B RSV (the same applies to "RSV/B F protein" occurring in other figures), "598+589" represents a bivalent RSV mRNA vaccine, which shows that an LNP preparation with mRNA numbered as 598 encapsulated therein and an LNP preparation with mRNA numbered as 589 encapsulated therein are mixed and injected into mice, and the same applies to bivalent RSV mRNA vaccines represented by other groups, and other figures not specifically described.
FIG. 1C-FIG. 1D show the detection of the induced total IgG antibody titers on days 19 and 28 after single immunization of mice with different mRNA vaccines (GMT, 95% CI), RSV/A represents subtype A RSV, RSV/B represents subtype B RSV, FIG. 1C is the total IgG antibody titer on day 19 after single immunization of mice, and FIG. 1D is the total IgG antibody titer on day 28 after single immunization of mice.
FIG. 1E shows the detection of the induced total IgG antibody titer on D14, D28 and D35 after immunization of mice with different doses of mRNA vaccines (GMT, 95% CI).

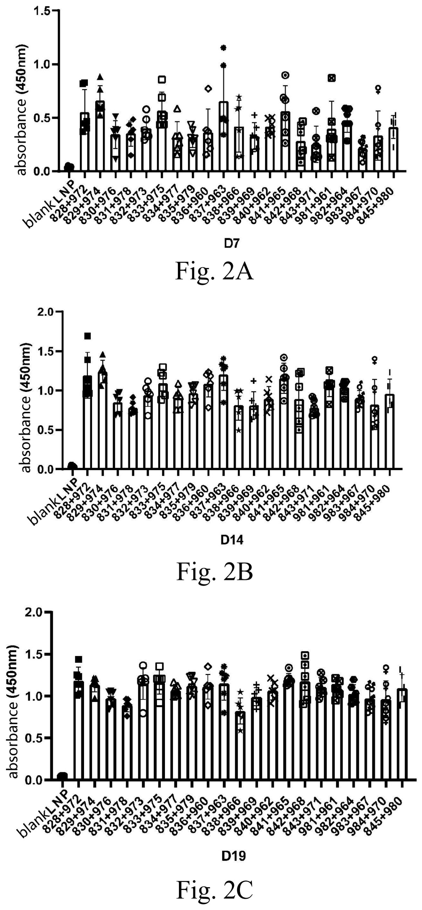

FIG. 2A to FIG. 2C show the detection of total IgG antibodies specifically binding to RSV/A F protein in the serum of mice single-immunized with different mRNA vaccines, where the serum was diluted at a ratio of 1:900, FIG. 2A shows the detection of total IgG antibodies specifically binding to RSV/A F protein in serum on day 7 after immunization, FIG. 2B shows the detection of total IgG antibodies specifically binding to RSV/A F protein in serum on day 14 after immunization, and FIG. 2C shows the detection of total IgG antibodies specifically binding to RSV/A F protein in serum on day 19 after immunization.

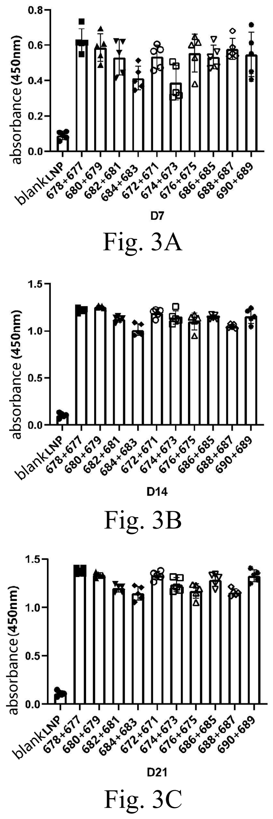

FIG. 3A to FIG. 3C show the detection of IgG total antibody specifically binding to RSV/A F protein in serum of mice single-immunized with different mRNA vaccines, where the serum was diluted at a ratio of 1:100, FIG. 3A shows the detection of IgG total antibody specifically binding to RSV/A F protein in serum on day 7 after immunization, FIG. 3B shows the detection of IgG total antibody specifically binding to RSV/A F protein in serum on day 14 after immunization, and FIG. 3C shows the detection of IgG total antibody specifically binding to RSV/A F protein in serum on day 21 after immunization.
FIG. 4A to FIG. 4C show the detection of IgG total antibody specifically binding to RSV/B F protein in serum of mice immunized with different mRNA vaccines, where the serum dilution is 1:100, FIG. 4A shows the detection of IgG total antibody specifically binding to RSV/B F protein in serum on day 7 after immunization, FIG. 4B shows the detection of IgG total antibody specifically binding to RSV/B F protein in serum on day 14 after immunization, and FIG. 4C shows the detection of IgG total antibody specifically binding to RSV/B F protein in serum on day 21 after immunization.

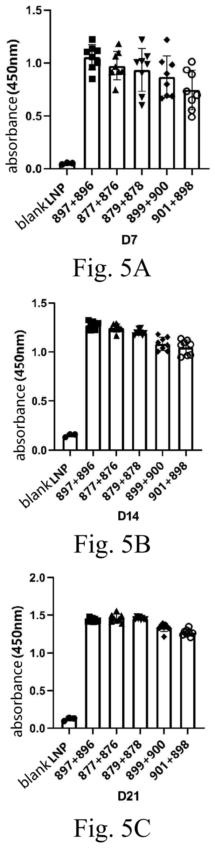

FIG. 5A to FIG. 5C show the detection of IgG total antibody specifically binding to RSV/A F protein in serum of mice single-immunized with different mRNA vaccines, where the serum was diluted at a ratio of 1:100, FIG. 5A shows the detection of IgG total antibody specifically binding to RSV/A F protein in serum on day 7 after immunization; FIG. 5B shows the detection of IgG total antibody specifically binding to RSV/A F protein in serum on day 14 after immunization, and FIG. 5C shows the detection of IgG total antibody specifically binding to RSV/A F protein in serum on day 21 after immunization.
FIG. 6A to FIG. 6B show the detection of IgG total antibody specifically binding to RSV/B F protein in serum of mice single-immunized with different mRNA vaccines, where the serum was diluted at a ratio of 1:100, FIG. 6A shows the detection of IgG total antibody specifically binding to RSV/B F protein in serum on day 7 after immunization, and FIG. 6B shows the detection of IgG total antibody specifically binding to RSV/B F protein in serum on day 14 after immunization.
FIG. 7 shows the detection of the induced neutralizing antibody against RSV/A pseudovirus on day 21 after single immunization of mice with different mRNA vaccines (GMT, 95% CI), wherein the antibody titer is NT50, wherein RSV/A represents subtype A RSV.
FIG. 8 shows the detection of the induced neutralizing antibody titer against RSV/A pseudovirus on day 19 after single immunization of mice with different mRNA vaccines (GMT, 95% CI), wherein the antibody titer is NT50, wherein RSV/A represents subtype A RSV.
FIG. 9 shows the detection of the induced neutralizing antibody against RSV/A pseudovirus on day 31 after single immunization of mice with different mRNA vaccines (GMT, 95% CI), the antibody titer is NT50, wherein RSV/A represents subtype A RSV.
FIGS. 10A-10C show the ELISpot detection of mouse splenocytes on day 35 after single immunization of mice with different mRNA vaccines, wherein FIG. 10A shows the ELISpot detection of IFN-γ, FIG. 10B shows the ELISpot detection of IL-4, and FIG. 10C shows the results of IFN-γ/IL4.
FIGS. 11A-11C show the ELISpot detection of mouse splenocytes on day 28 after single immunization of mice with different mRNA vaccines, wherein FIG. 11A shows the ELISpot detection of IFN-γ, FIG. 11B shows the ELISpot detection of IL-4, and FIG. 11C shows the results of IFN-γ/IL4.
FIG. 12 shows the binding of F protein expressed in vitro by different mRNAs to different antibodies against RSV F proteins, where 591 represents the F protein expressed by the engineered plasmid encoding the mRNA shown in 591 (without the Cap1 cap structure).
FIG. 13 shows the detection of the pulmonary RSV viral load in mice, which were immunized with RSV mRNA vaccine and RSV inactivated vaccine, 5 days after RSV challenge (GMT, 95% CI), wherein FI-RSV represents formalin inactivated RSV.
FIG. 14 shows the histopathological analysis of lung from mice, which were immunized with RSV mRNA vaccine and RSV inactivated vaccine, 5 days after RSV challenge, where FI-RSV represents formalin inactivated RSV; Vs PBS, P < 0.05, *; P < 0.01, **; P < 0.001, ***; P < 0.0001, ****; Vs FI-RSV, P < 0.05, #; P < 0.01, ##; P < 0.001, ###; P < 0.0001, ####.
FIG. 15 shows the detection of the induced neutralizing antibody titer against RSV/A pseudovirus after immunization of rats with different doses of mRNA vaccine (GMT, 95% CI).
FIG. 16 shows the ELISpot detection of mouse splenocytes on day 7 (D28) after the second immunization of mice (D21) with different doses of mRNA vaccines.

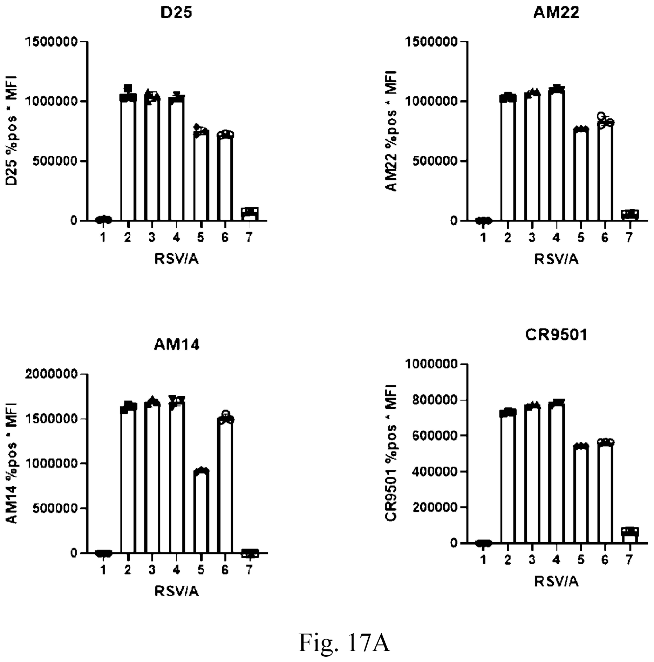

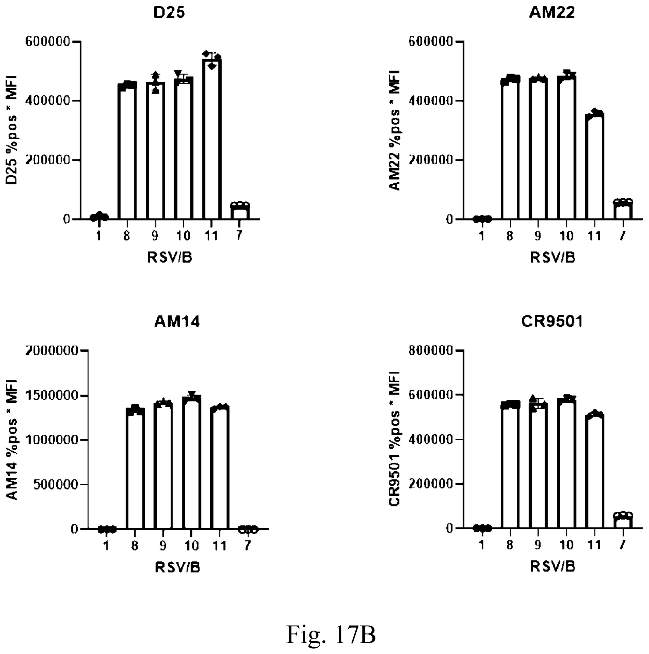

FIG. 17A to FIG. 17B show the binding of F protein expressed in vitro by different mRNAs to different antibodies against RSV F protein, wherein FIG. 17A shows the binding of F protein of subtype A RSV expressed in vitro by different mRNAs to different antibodies against RSV F protein, FIG. 17B shows the binding of F protein of subtype B RSV expressed in vitro by different mRNAs to different antibodies against RSV F protein, group 1 is a control group without mRNA transfection, groups 2-4 are different batches of mRNA shown by 832, group 5 represents mRNA shown by 1119, group 6 represents mRNA shown by 1902, group 7 is mRNA expressing Post F, group 8-10 are different batches of mRNA shown by 973, and group 11 represents mRNA shown by 1903.
FIGS. 18A-18B show the detection of RSV viral load in lung and nasal tissues of cotton mice, which were immunized with RSV mRNA vaccine, 5 days after RSV challenge, wherein FI-RSV represents formalin inactivated RSV. RSV A represents subtype A RSV virus, and RSV B represents subtype B RSV virus.
FIG. 19A to FIG. 19B show the detection of the induced neutralizing antibodies against RSV/A and RSV/B virus after immunization of cotton mice with RSV mRNA vaccine (GMT, 95% CI), the antibody titer is PRNT60, wherein RSV/A represents subtype A RSV, and RSV/B represents subtype B RSV.
FIG. 20 shows the detection of the induced neutralizing antibody against RSV/A pseudovirus on day 28 after single immunization of mice with different mRNA vaccines (GMT, 95% CI). The antibody titer is NT50, where RSV/A represents subtype A RSV.

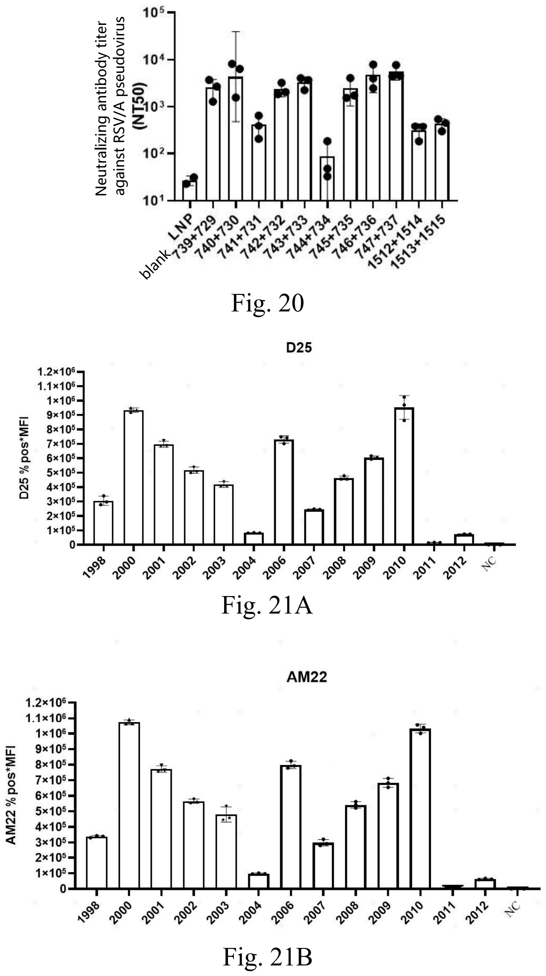

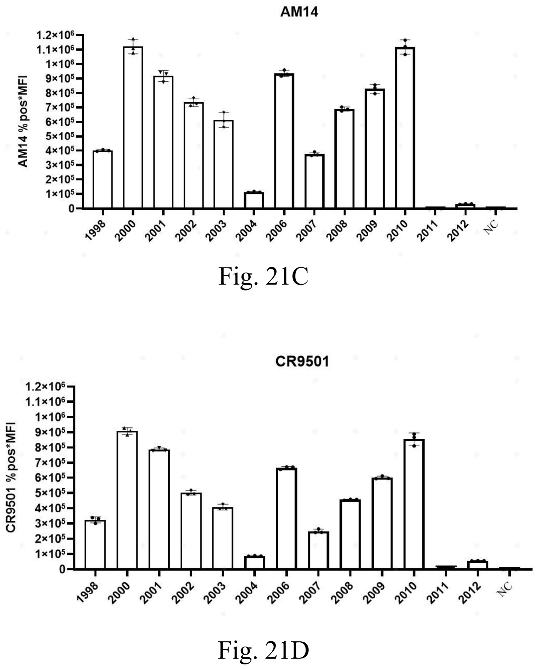

FIGs. 21A to 21D show the binding of F protein expressed in vitro by different mRNAs to different antibodies against RSV F protein. FIG. 21A shows the binding of F protein of subtype A RSV expressed in vitro by different mRNAs to antibody D25, FIG. 21B shows the binding of F protein of subtype A RSV expressed in vitro by different mRNAs to antibody AM22, FIG 21C shows the binding of F protein of subtype A RSV expressed in vitro by different mRNAs to antibody AM14, and FIG. 21D shows the binding of F protein of subtype A RSV expressed in vitro by different mRNAs to antibody CR9501. In FIGs. 21A to 21D, "NC" represents the control group without transfection with the engineered plasmid, "1988" represents the F protein expressed by the engineered plasmid encoding the mRNA numbered as 1988 (excluding the Cap1 cap structure), "2000" represents the group of the F protein expressed by the engineered plasmid encoding the mRNA numbered as 2000 (excluding the Cap1 cap structure), and the same applies to the other numbers representing groups.

### Detailed description of the invention

### I. Definition

All patents, patent applications, scientific publications, manufacturers' instructions and guidelines, etc., cited herein, whether supra or infra, are incorporated herein by reference in their entirety. Any content herein should not be understood as an admission that the present disclosure is not entitled to antedate such disclosure.

Unless otherwise indicated, scientific and technical terms used herein have meanings commonly understood by those skilled in the art. Moreover, the terms related to protein and nucleic acid chemistry, molecular biology, cell and tissue culture and microbiology used herein are all terms widely used in the corresponding fields (see, for example, Molecular Cloning: A Laboratory Manual, 2nd Edition, J. Sambrook et al. eds., Cold Spring Harbor Laboratory Press, Cold Spring Harbor 1989). In addition, to understand this application better, the following definitions and explanations of related terms are provided.

As used herein, the expressions "comprising", "including", "containing" and "having" are openended, meaning including the recited elements, steps, or components but not excluding other element, step, or component that is not recited. The expression "consisting of" excludes any element, step, or component that is not specified. The expression "consisting essentially of" means that the scope is limited to the specified elements, steps, or components, plus optional elements, steps, or components that do not materially affect the basic and novel nature of the claimed subject matter. It should be understood that the expressions "consisting essentially of" and "consisting of" are encompassed within the meaning of the expression "comprising".

As used herein, "a" and "an" and "the" and similar referents used in the context describing the application (especially in the context of the claims) should be understood to cover both the singular and the plural, unless the context indicates otherwise. The term "one or more" or "at least one" encompasses 1, 2, 3, 4, 5, 6, 7, 8, 9 or more. The term "at least one" or "one or more" encompasses 1, 2, 3, 4, 5, 6, 7, 8, 9 or more.

Numerical ranges recited herein should be understood to encompass any and all sub-ranges comprised therein. For example, a range of "1 to 10" should be understood to include not only the explicitly recited values of 1 and 10, but also any single value (e.g., 2, 3, 4, 5, 6, 7, 8 and 9) and sub-range within the range of 1 to 10 (e.g., 1 to 2, 1.5 to 2.5, 1 to 3, 1.5 to 3.5, 2.5 to 4, 3 to 4.5, etc.). This principle also applies to ranges using only one numerical value as the minimum or maximum value.

As used herein, the terms "and/or", "any combination thereof," and grammatical equivalents thereof are used interchangeably. These terms may express, specifically refer to any combination. For example, the following phrases "A, B, and/or C" or "A, B, C, or any combination thereof" may mean "A alone; B alone; C alone; A and B; B and C; A and C; and A, B, and C".

As used herein, the term "naturally occurred" or "naturally occurring" refers to the fact that a substance can be found in nature. For example, peptides, amino acids, proteins, or nucleic acids, which are present in organisms, including viruses, and can be isolated from natural sources and are not artificially modified in experiments, are naturally occurring.

As used herein, the term "non-naturally occurring" as used herein in describing a nucleic acid is intended to mean that the nucleic acid is not found in nature. For example, a non-naturally occurring nucleic acid encoding a viral peptide or protein has at least one genetic change or chemical modification which is generally not found in the wild-type strain of the mentioned virus. Such genetic alterations include, for example, the introduction of expressible nucleic acid sequences encoding peptide fragments or polypeptides heterologous to the mentioned virus, other nucleic acid additions, nucleic acid deletions, nucleic acid substitutions, and/or other functional disruptions to the genetic material of the virus. Chemical modifications include, for example, one or more functional nucleotide analogs as described herein.

All methods described herein can be performed in any suitable order unless otherwise indicated.

As used herein, the term "wild-type" means that the sequence is naturally occurring and not artificially modified, including naturally occurring mutants.

As used herein with respect to the term "% identical" or "% identity" refers to the percentage of nucleotides or amino acids that are identical in the optimal alignment between the sequences to be compared, the difference between the two sequences may be distributed over a local region (segment) or the entire length of the sequences to be compared. The identity between two sequences is typically determined after the optimal alignment to a segment or "comparison window." Optimal alignment may be performed manually or by means of algorithms known in the art. Algorithms known in the art include, but are not limited to, the local homology algorithms described by Smith and Waterman, 1981, Ads App. Math. 2, 482 and Neddleman and Wunsch, 1970, J. Mol. Biol. 48, 443, the similarity search methods described by Pearson and Lipman, 1988, Proc. Natl Acad. Sci. USA 88, 2444, or using computer programs such as Wisconsin Genetics Software Package, Genetics Computer Group, 575 Science Drive, Madison, GAP in the Wis., BESTFIT, FASTA, BLAST P, BLAST N and TFASTA. For example, the percentage identity of the two sequences may be determined using publicly available algorithms BLASTN or BLASTP on the website of National Center for Biotechnology Information (NCBI).

"% identical" or "% identity" can be obtained by determining the number of identical positions corresponding to the sequences to be compared, dividing this number by the number of compared positions (e.g., the number of positions in the reference sequence), and multiplying this result by 100 to obtain % identity. In some embodiments, a degree of identity is given to a region of at least about 50%, at least about 55%, at least about 60%, at least about 65%, at least about 70%, at least about 75%, at least about 80%, at least about 85%, at least about 90%, at least about 95%, or about 100%. In some embodiments, the degree of identity is given for the entire length of the reference sequence. Alignment for determining sequence identity can be performed with tools known in the art, preferably using optimal sequence alignment, for example, using Align with standard settings, preferably EMBOSS::needle, Matrix: Blosum62, Gap Open 10.0, Gap Extend 0.5.

As used herein, "nucleotide" includes deoxyribonucleotides, deoxyribonucleotides, deoxyribonucleotide derivatives, and ribonucleotide derivatives. As used herein, "ribonucleotide" is a substance constituting ribonucleic acid (RNA), which consists of a molecule of base, a molecule of pentose and a molecule of phosphate, which refers to a nucleotide having a hydroxyl group at the 2' position of the β-D-ribofuranosyl group, and "deoxyribonucleotide" is a substance constituting deoxyribonucleic acid (DNA), which also consists of a molecule of base, a molecule of pentose and a molecule of phosphate, which refers to a nucleotide having the hydroxyl group at the 2' position of the β-D-ribofuranosyl group substituted by hydrogen, and is the main chemical component of a chromosome.

"Nucleotide" is generally referred to by a single letter representing the base therein, "A" or "A nucleotide" refers to an adenine deoxyribonucleotide or an adenine ribonucleotide containing adenine, "C" or "C nucleotide" refers to a cytosine deoxyribonucleotide or a cytosine ribonucleotide containing cytosine, "G" or "G nucleotide" refers to a guanine deoxyribonucleotide or a guanine ribonucleotide containing guanine, "U" or "U nucleotide" refers to a uracil ribonucleotide containing uracil, and "T" or "T nucleotide" refers to a thymine deoxyribonucleotide containing thymine.

As used herein, the term "nucleic acid" generally refers to any compound comprising a polymer of deoxyribonucleotides (deoxyribonucleic acid, abbreviated as DNA) or a polymer of ribonucleotides (ribonucleic acid, abbreviated as RNA), or a combination thereof. In addition, nucleic acids herein also include derivatives of nucleic acids. The term "derivative of a nucleic acid" includes chemical derivatization of a nucleic acid on the base, the saccharide, or the phosphate of a nucleotide, as well as nucleic acids containing non-native nucleotides and nucleotide analogs. In addition, nucleic acids herein can be single or double stranded in the form of linear or covalent closed circular molecules.

"Polynucleotide sequence", "nucleic acid sequence" and "nucleotide sequence" can be used interchangeably to indicate the ordering of nucleotides in a polynucleotide. It should be understood by those skilled in the art that the coding strand (sense strand) of DNA and the RNA encoded thereby can be regarded as having the same nucleotide sequence, and the deoxythymidylate in the sequence of the coding strand of DNA corresponds to the uridylate in RNA sequence encoded thereby. RNA corresponding to DNA refers to a polynucleotide with T in DNA is completely replaced with U.

A polynucleotide can comprise one or more segments (nucleic acid fragments) (e.g., 1, 2, 3, 4, 5, 6, 7, 8 segments). For example, a polynucleotide may comprise a segment encoding a polypeptide of interest. In particular embodiments, a polynucleotide may comprise a segment encoding a polypeptide of interest as well as regulatory segments including, but not limited to, segments for the regulation of transcription and the regulation of translation. In one embodiment, the regulatory segment comprises a polynucleotide corresponding to one or more of the following regulatory elements: a promoter, a 5' untranslated region (5'-UTR), a 3' untranslated region (3'-UTR), and a poly(A) tail.

As used herein, the term "promoter" refers to a polynucleotide located upstream of the 5' end of the coding region of a gene, which contains conserved sequences specific binded by RNA polymerase and required for the initiation of transcription, and can activate RNA polymerase, so that RNA polymerase can accurately bind to template DNA and has the specificity for the initiation of transcription. The promoter may be derived from viruses, bacteria, fungi, plants, insects, and animals. Representative examples of promoters include the phage T7 promoter, the phage T3 promoter, the SP6 promoter, the lac operon-promoter, the tac promoter, the SV40 late promoter, the SV40 early promoter, the RSV-LTR promoter, the CMV IE promoter, the SV40 early promoter, or the SV40 late promoter and the CMV IE promoter.

As used herein, the term "5' untranslated region" or "5' -UTR" may be an RNA sequence in mRNA that is upstream of the coding sequence and is not translated into a protein. The 5' -UTR in a gene typically starts at the transcription initiation site and ends at the nucleotide upstream of the translation initiation codon of the coding sequence. The 5' -UTR may comprise elements that control gene expression, such as a ribosome binding site, a 5'-terminal oligo pyrimidine bundle, and a translation initiation signal such as a Kozak sequence. mRNA can be post-transcriptional modified by adding a 5' cap. Thus, 5' -UTR in mature mRNA can also refer to the RNA sequence between the 5' cap and the start codon.

As used herein, the term "3' untranslated region" or "3' -UTR" may be an RNA sequence in mRNA that is downstream of the coding sequence and is not translated into a protein. The 3' -UTR in the mRNA is located between the stop codon of the coding sequence and the poly (A) sequence, e.g., starting at the nucleotide downstream of the stop codon and ending at the nucleotide upstream of the poly (A) sequence.

As used herein, the terms "polyadenylic acid," "poly (A) sequence," and "poly (A) tail" are used interchangeably, and the naturally occurring poly (A) sequences typically consists of adenine ribonucleotides. According to the present application, the term "modified poly (A) sequence" refers to a poly (A) sequence comprising a nucleotide or a segment of nucleotides other than adenine ribonucleotide. The poly (A) sequence is typically located at the 3' end of mRNA, e.g., the 3' end (downstream) of 3' -UTR .

As used herein, the term "5'-cap structure": 5'-cap structure is typically located at the 5' end of a mature mRNA. In some embodiments, the 5'-cap structure is linked to the 5'-end of the mRNA by a 5'-5'-triphosphate linkage. The 5'-cap structure is typically formed from modified (e.g., methylated) ribonucleotides, especially from guanine nucleotide derivatives. For example, m7GpppN (cap 0 or "cap0", which is a cap structure formed by the reaction of the 5' phosphate group of hnRNA with the 5' -phosphate group of m7GTP under the action of guanylyltransferase to form a 5',5'-phosphodiester bond), wherein N is the 5'-end nucleotide of the nucleic acid carrying the 5'-cap structure. In some embodiments, the 5'-cap structure includes, but is not limited to, cap 0, cap 1 (a cap structure formed by further methylation at 2' -OH of glycosyl in the nucleotide at position 1 of hnRNA on the basis of cap 0, or referred to as "cap1"), cap 2 (a cap structure formed by further methylation at 2' -OH of glycosyl in the nucleotide at position 2 of hnRNA on the basis of cap 1, or referred to as "cap2"), cap 4, cap 0 analog, cap 1 analog, cap 2 analog, or cap 4 analog.

As used herein, the term "expression" includes transcription and/or translation of a nucleotide sequence. Thus, expression may involve the production of transcripts and/or polypeptides. The term "transcription" refers to the process of transcribing the genetic codons in a DNA sequence into an RNA (transcript). The term "in vitro transcription" refers to the in vitro synthesis of RNA, particularly mRNA, in a cell-free system (e.g., in an appropriate cell extract) (see, e.g., Pardi N., Muramatsu H., Weissman D., Karikó K. (2013). In: Rabinovich P. (eds) Synthetic Messenger RNA and Cell Metabolism Modulation. Methods in Molecular Biology (Methods and Protocols), vol 969. Humana Press, Totowa, NJ.). A vector that can be used to produce a transcript are also referred to as "transcription vector" which contains regulatory sequences required for transcription. The term "transcription" encompasses "in vitro transcription."

As used herein, the term "host cell" refers to a cell for receiving, holding, replicating, expressing a polynucleotide or vector. The term "host cell" includes prokaryotic cells (e.g., E. coli) or eukaryotic cells (e.g., yeast cells and insect cells). For example, cells from human, mouse, hamster, pig, goat, primates. Cells can be derived from a variety types of tissues and include primary cells and cell lines. Some specific examples include keratinocytes, peripheral blood leukocytes, bone marrow stem cells, and embryonic stem cells. In other embodiments, the host cell is an antigen presenting cell, particularly a dendritic cell, a monocyte, or a macrophage. Nucleic acids can be present in a host cell as a single copy or as several copies. In some embodiments, the host cell may be a cell in which the polypeptide of the present application is expressed.

In the context of the present application, the term "plasmid" generally refers to a circular DNA molecule, but the term may also encompass linearized DNA molecules. Specifically, the term "plasmid" also encompasses molecules obtained by linearizing a circular plasmid by, for example, digesting the circular plasmid with a restriction enzyme, thereby converting the circular plasmid molecule into a linear molecule. Plasmids can be replicated, i.e., amplified in cells independently from the genetic information stored as chromosomal DNA, and can be used for cloning, i.e., for amplifying genetic information in bacterial cells. In an alternatively particular example, the DNA plasmid is a medium-copy or high-copy plasmid. In another alternatively particular example, the DNA plasmid is a high-copy plasmid. Examples of such high-copy plasmids include, for example, pUC and pTZ plasmids or any other plasmid (e.g., pMB1, p CoIE1) that includes an origin of replication that supports the high copy of the plasmid.

The term "vaccine" is typically understood to be a prophylactic or therapeutic material that provides at least one antigen or has an antigenic function, which can stimulate the adaptive immune system in the body to provide an adaptive immune response.

The term "treating" or the like is used herein to generally refer to obtaining a desired pharmacological and/or physiological effect. Thus, the treatment in the present application may involve the treatment of the condition of a certain disease, but may also involve prophylactic treatment in terms of the complete or partial prevention of the disease or the symptoms thereof. In some embodiments, the term "treating" should be understood to be therapeutic in partially or completely curing a disease and/or adverse effects and/or symptoms attributed to the disease. The treatment may also be a prophylactic or preventive treatment, i.e., a measure conducted to prevent a disease, e.g., to prevent an infection and/or the onset of a disease.

As used herein, the terms "subject" and "patient" may be used interchangeably. In certain embodiments, the subject is a mammal, such as human, non-human primates (e.g., simian, chimpanzee, monkey, and chimpanzee), domestic animals (including dog and cat and livestocks (e.g., horse, cow, pig, sheep, and goat)), or other mammals. Other mammals include, but are not limited to, mouse, rat, guinea pig, rabbit, hamster, etc. In particular embodiments, the subject is human. In one embodiment, the subject is a mammal (e.g., human) suffering from an infectious disease or a neoplastic disease . In another embodiment, the subject is a mammal (e.g., human) at risk of developing an infectious or neoplastic disease.

As used herein, the term "administering" refers to providing or administering an agent to a subject by any effective route. Exemplary routes of administration include, but are not limited to, one or more of the follows: injection (e.g., subcutaneous, intramuscular, intradermal, intraperitoneal, intrathecal, intracerebroventricular, or intravenous), oral, intrabile, sublingual, rectal, transdermal, intranasal, vaginal, and inhalation. When used to treat a disease, disorder, condition, or symptom thereof, the administration of the substance is typically performed after the onset of the disease, disorder, condition, or symptom thereof. When used to prevent a disease, disorder, condition or symptom, the administration of the substance is typically performed prior to the onset of the disease, disorder, condition or symptom.

"Antibody D25" or "monoclonal antibody D25" is a neutralizing monoclonal antibody that specifically binds to the pre-fusion conformation of the RSV F protein but not to the post-fusion conformation of the RSV F protein. The protein and nucleic acid sequences of antibody D25 are known, e.g., the amino acid sequences of the heavy and light chains of the D25 antibody are set forth in U.S. Patent Application Publication No. 2010/0239593, incorporated herein by reference in its entirety; see also Kwakenebs et al., Nat. Med., 16:123-128, 2009. D25 specifically binds to quaternary epitopes (included on antigenic sites) present in the pre-fusion conformation of the RSVF protein rather than the post-fusion conformation, which epitope is included in RSVF positions 62-69 and 196-209, and are located at the distal apex from the membrane in the RSVF protein in the pre-fusion conformation. Antibody D25 specifically binds to preF protein.

"Antibody AM14" or "monoclonal antibody AM14" is a neutralizing monoclonal antibody that specifically binds to the pre-fusion conformation of the RSV F protein but not to the post-fusion conformation of the RSVF protein. The proteins and nucleic acid sequences of AM14 are known, for example, as described in WO 2008/147196 A2.

"Antibody AM22" or "monoclonal antibody AM22" is a neutralizing monoclonal antibody that specifically binds to the pre-fusion conformation of the RSV F protein but not to the post-fusion conformation of the RSV F protein. The protein and nucleic acid sequences of AM22 are known, for example, the heavy and light chain amino acid sequences of AM22 antibodies are set forth in U.S. Patent Application Publication No. 2012/0070446, which is incorporated herein by reference in its entirety. AM22 specifically binds to an epitope (included on antigenic sites) including positions present in the pre-fusion conformation rather than post-fusion conformation of the RSVF protein. Such epitopes are included within positions 62-69 and 196-209 of RSV F and are located at the distal apex from the membrane in the RSV F protein in the pre-fusion conformation. Antibody AM22 specifically binds to the preF protein disclosed herein.

"Antibody clone 131-2A" or "monoclonal antibody 131-2 A" specifically binds to the site I epitope of the RSV F protein, Antibody clone 131-2A is a monoclonal antibody that preferentially binds to the post-fusion conformation of the RSV F protein but not to the pre-fusion conformation of the RSV F protein.

The term "mutation" refers to the deletion, addition or substitution of an amino acid residue in the amino acid sequence of a protein or polypeptide as compared to the amino acid sequence of a reference protein or polypeptide. Throughout the specification and claims, the amino acid substitution at a particular position in a protein sequence is referred to with the designation "(amino acid residue in wild-type protein) (amino acid position) (amino acid residue in engineered protein)". For example, the designation P101C refers to the substitution of the proline (P) residue at position 101 of the amino acid sequence of the reference protein with a cysteine (C) residue (in the mutant of the reference protein).

Herein, some elements of the present application will be described. These elements are listed together with specific embodiments, however it should be understood that they can be combined in any manner and in any number to generate additional embodiments. The different described examples and preferred embodiments should not be construed as limiting the application to the explicitly described embodiments only. This specification should be understood to support and include embodiments that combine the explicitly described embodiments with any number of the disclosed and/or preferred elements. In addition, any arrangement and combination of all described elements in the present application should be construed as disclosed in the description of the present application unless indicated otherwise in the context. For example, in one embodiment, the mutations of the mutant comprise: P101C and I152C, and in another embodiment, the mutations of the mutant comprise: the substitution of T at position 54 by F, W or V, the substitution of S at position 190 by I, and the substitution of V at position 296 by L, then the following solution is also an embodiment claimed in the present application: the mutations of the mutant comprise: P101C and I152C, the substitution of T at position 54 by F, W or V, the substitution of S at position 190 by I, and the substitution of V at position 296 by L.

### II. Nucleic Acid

F0 is the precursor of RSV F protein, and F0 is a polypeptide with a length of 574 amino acids translated from the mRNA of RSV F protein. F0 contains a signal peptide (amino acid 1-25) located at the N-terminus, after translation, the signal peptide is removed from F0 by the signal peptidase, the remaining F0 (i.e., residue 26-574) is further cleaved at two cleavage sites (amino acids 109/110 and 136/137) by a cellular protease (e.g., furin), 27 amino acids (amino acid 110-136, i.e., pep27 polypeptide) are removed to form an F1 polypeptide (C-terminal moiety; amino acid 137-574) and an F2 polypeptide (N-terminal moiety; amino acid 26-109), and the F1 polypeptide and the F2 polypeptide are linked by a disulfide bond.

The RSV F protein complex in trimeric form mediates fusion between the virion membrane and the host cell membrane and also promotes the formation of a syncytium. The pre-fusion RSV F protein can be recognized by monoclonal antibodies D25, AM22 and MPE8. The pre-fusion F protein trimer can be specifically recognized by the monoclonal antibody AM14.

### 1. Nucleic Acid 1

One embodiment of the present disclosure provides a nucleic acid comprising a polynucleotide encoding a mutant of RSV F protein, the mutant of RSV F protein (hereinafter referred to as "mutant" for short) comprises or is one or more of the following mutations as compared to wild-type RSV F protein: a disulfide bond mutations, a cavity filling mutation and an electrostatic mutation. The RSV F protein can be stably a pre-F protein by disulfide bond mutations, cavity filling mutation and/or electrostatic mutation. It should be noted that, in this specification, "a plurality of" means at least two, for example, two, three, four, five, or more.

In some embodiments, the disulfide bond mutations comprise or are one or more of the follows: S46C and T311C, A47C and I309C, L48C and V308C, S55C and L188C, V56C and T189C, V56C and V300C, I57C and S190C, I57C and Y299C, T58C and K191C, I59C and L193C, I59C and L297C, E60C and D194C, P101C and I152C, A102C and I148C, A102C and I152C, T103C and I146C, T103C and I148C, N104C and G145C, N104C and S146C, N105C and G145C, N105C and S146C, N105C and A147C, V144C and V406C, and S146C and N460C. In some embodiments, the disulfide bond mutations comprise or are one or more of the follows: A47C and I309C, I57C and Y299C, P101C and I152C, A102C and I148C, A102C and I152C, T103C and I146C, N104C and G145C, N104C and S146C, N105C and G145C, N105C and S146C, and N105C and A147C. In some embodiments, the disulfide bond mutations comprise or are one or more of P101C and I152C, A102C and I148C, A102C and I152C, N104C and S146C, and N105C and A147C.

Given the substantial conservation of RSV F sequences, one of ordinary skill in the art can readily compare amino acid positions between different native RSVF sequences to identify corresponding RSV F amino acid positions between different RSV strains and subtypes. For example, among almost all identified natural RSV F0 precursor proteins, the furin cleavage sites fall in the same amino acid positions. Thus, the conservation of the native RSV F protein sequence between strains and subtypes permits the use of a reference RSV F sequence to compare amino acids at specific positions in the RSV F protein. For purposes of this disclosure (unless indicated otherwise in the context), the amino acids of the RSV F protein are positioned by reference to the sequence of F0 shown in SEQ ID NO: 216 (the amino acid sequence of the full-length native F0 of strain Ontario). However, it should be noted, and one skilled in the art will understand, that different RSV F0 sequences may have different numbering systems, e.g., adding or removing additional amino acid residues as compared to SEQ ID NOs: 216. Thus, it should be understood that when a particular amino acid residue is referred to by a number, the description is not limited to the amino acid located exactly at that numbered position when counted from a given amino acid sequence, but also refers to the equivalent/corresponding amino acid residue in any and all RSV F sequences, even if the residue is not at the same exact numbered position, e.g., if the RSV sequence is shorter or longer than SEQ ID NO: 216, or has an insertion or deletion compared to SEQ ID NO: 216.

In some embodiments, the disulfide bond mutations comprise or are one of the follows: A47C and I309C, I57C and Y299C, P101C and I152C, A102C and I148C, A102C and I152C, T103C and I146C, N104C and G145C, N104C and S146C, N105C and G145C, N105C and S146C, and N105C and A147C. In some embodiments, the disulfide bond mutations comprise or are one of the follows: P101C and I152C, A102C and I148C, A102C and I152C, N104C and S146C, and N105C andA147C.

In some embodiments, the disulfide bond mutations comprise or are: (i) at least one of A47C and I309C, I57C and Y299C, P101C and I152C, A102C and I148C, A102C and I152C, T103C and I146C, N104C and G145C, N104C and S146C, N105C and G145C, N105C and S146C, and N105C and A147C, and (ii) one of V144C and V406C, and S146C and N460C.

In some embodiments, the disulfide bond mutations are selected from: (i) one of A47C and I309C, I57C and Y299C, P101C and 1152C, A102C and I148C, A102C and I152C, T103C and 1146C, N104C and G145C, N104C and S146C, N105C and G145C, N105C and S146C, and N105C and A147C, and (ii) one of V144C and V406C, and S146C and N460C. In some embodiments, the disulfide bond mutations are selected from: (i) one of P101C and I152C, A102C and I148C, A102C and I152C, N104C and S146C, and N105C and A147C, and (ii) one of V144C and V406C, and S146C and N460C.

In some embodiments, the cavity filling mutation includes one or more of the following (1) to (3):
(1) the substitution of T at position 54 by F, W, V, C, A, H, I, Y, L or M; preferably, the substitution of T at position 54 by F, W, V, C or A;
(2) the substitution of S at position 190 by V, I, M, F, L, W, Y, H, R, E, A, Q, G, P, T, C, D, N, or K; and
(3) the substitution of V at position 296 by I, L, Y, H, F, or M, for example, the substitution of V at position 296 by L, F or M.

In some embodiments, the mutant of RSV F protein comprises a cavity filling mutation comprising the substitution of T at position 54 by A.

In some embodiments, the mutant of RSV F protein comprises a cavity filling mutation comprising the substitution of T at position 54 by A, and the mutant of RSV F protein does not comprise the mutation at position 185.

In some embodiments, the mutant of RSV F protein comprises a cavity filling mutation comprising the substitution of T at position 54 by A, and the mutant of RSV F protein does not comprise the mutation V185F.

In some embodiments, the mutant RSV F protein comprises a cavity filling mutation comprising the substitution of V at position 296 by L.

In some embodiments, the mutant of RSV F protein comprises a cavity filling mutation comprising the substitution of V at position 296 by L, and the mutant of RSV F protein does not comprise the mutation at position 291.

In some embodiments, the mutant of RSV F protein comprises a cavity filling mutation comprising the substitution of V at position 296 by L, and the mutant of RSV F protein does not comprise the mutation I291V.

In some embodiments, the cavity filling mutation comprises or is the substitution of T at position 54 by F, W, V, C, A, H, I, Y, L, or M, and the substitution of S at position 190 by an amino acid other than S. In some embodiments, the cavity filling mutation is the substitution of T at position 54 by F, W, C, A or V, and the substitution of S at position 190 by V, I, M, F, L, W, Y, H, R, E, A, Q, G, P, T, C, D, N or K.

In some embodiments, the cavity filling mutation comprises or is the substitution of T at position 54 by F, W, V, C, A, H, I, Y, L, or M, and the substitution of V at position 296 by I, L, Y, H, F, or M. In some embodiments, the cavity filling mutation is the substitution of T at position 54 by F, W, C, A or V, and the substitution of V at position 296 by I, L, Y, H, F or M. Alternatively, the cavity filling mutation includes the substitution of T at position 54 by F, W, V, C, A, H, I, Y, L or M, and the substitution of V at position 296 by L, F or M. In some embodiments, the cavity filling mutation is the substitution of T at position 54 by F, W or V, and the substitution of V at position 296 by L, F or M.

In some embodiments, the cavity filling mutation comprises or is: the substitution of S at position 190 by V, I, M, F, L, W, Y, H, R, E, A, Q, G, P, T, C, D, N, or K, and the substitution of V at position 296 by I, L, Y, H, F, or M. In some embodiments, the cavity filling mutation is the substitution of S at position 190 by V, I, M, F, L, W, Y, H, R, E, A, Q, G, P, T, C, D, N or K, and the substitution of V at position 296 by L, F or M.

In some embodiments, the cavity filling mutation comprises or is: the substitution of T at position 54 by F, W, V, C, A, H, I, Y, L or M, the substitution of S at position 190 by an amino acid other than S, and the substitution of V at position 296 by I, L, Y, H, F or M. In some embodiments, the cavity filling mutation is the substitution of T at position 54 by F, W, C, A or V, the substitution of S at position 190 by V, I, M, F, L, W, Y, H, R, E, A, Q, G, P, T, C, D, N or K, and the substitution of V at position 296 by I, L, Y, H, F or M. Alternatively, the cavity filling mutation comprises the substitution of T at position 54 by F, W, V, C, A, H, I, Y, L or M, the substitution of S at position 190 by V, I, M, F, L, W, Y, H, R, E, A, Q, G, P, T, C, D, N or K, and the substitution of V at position 296 by L, F or M. In some embodiments, the cavity filling mutation is the substitution of T at position 54 by F, W, C, A or V, the substitution of S at position 190 by V, I, M, F, L, W, Y, H, R, E, A, Q, G, P, T, C, D, N or K, and the substitution of V at position 296 by L, F or M. In some embodiments, the cavity filling mutation is the substitution of T at position 54 by F, W or V, the substitution of S at position 190 by V, I, M, F, L, W, Y, H, R, E, A, Q, G, P, T, C, D, N or K, and the substitution of V at position 296 by L.

In some embodiments, the cavity filling mutation comprises or is: the substitution of T at position 54 by F, W, V, C, A or H, the substitution of S at position 190 by an amino acids other than S, and the substitution of V at position 296 by I or L. In some embodiments, the cavity filling mutation comprises or is one group of: (1)T54V, S190M and V296L; (2)T54F, S190M and V296L; (3)T54C, S190M and V296L; (4)T54A, S190M and V296L; (5)T54V, S190F and V296L; (6)T54F, S190F and V296L; (7)T54C, S190F and V296L; (8)T54A, S190F and V296L; (9)T54V, S190M and V296L; (10)T54F, S190M and V296L; (11)T54C, S190M and V296L; (12)T54A, S190M and V296L; (13)T54V, S190F and V296L; (14)T54F, S190F and V296L; (15)T54C, S190F and V296L; (16)T54A, S190F and V296L; (17)T54V, S190I and V296L; (18)T54F, S190I and V296L; (19)T54C, S190I and V296L; and (20)T54A, S190I and V296L.

In some embodiments, the mutant has the following (i) and (ii): (i) comprising one group of the following mutations: (1)T54V, S190M and V296L; (2)T54F, S190M and V296L; (3)T54C, S190M and V296L; (4)T54A, S190M and V296L; (5)T54V, S190F and V296L; (6)T54F, S190F and V296L; (7)T54C, S190F and V296L; (8)T54A, S190F and V296L; (9)T54V, S190M and V296L; (10)T54F, S190M and V296L; (11)T54C, S190M and V296L; (12)T54A, S190M and V296L; (13)T54V, S190F and V296L; (14)T54F, S190F and V296L; (15)T54C, S190F and V296L; (16)T54A, S190F and V296L; (17)T54V, S190I and V296L; (18)T54F, S190I and V296L; (19)T54C, S190I and V296L; and (20)T54A, S190I and V296L; and (ii) the amino acid sequence of the mutant is at least 85%, 88%, 90%, 91%, 92%, 93%, 94%, 95%, 95.5%, 96%, 96.5%, 97%, 97.5%, 98%, 98.5% or 99% identical to the corresponding wild-type RSV F protein.

In some embodiments, the electrostatic mutation comprises or is one or both of: (1) the substitution D at position 486 by Q, S, H, N, T, P, C, L, I, F, or W; and (2) the substitution of V at position 487 by Q, T, N, I, K, P, R, S, Y, D, F, L, H, or C. In some embodiments, the electrostatic mutation is the substitution of D at position 486 by Q, L or I. In some embodiments, the electrostatic mutation is the substitution of D position 486 by Q.

In some embodiments, the mutations of the mutant comprise or are disulfide bond mutations.

In some embodiments, the disulfide bond mutations comprise one or more of the follows: S46C and T311C, A47C and I309C, L48C and V308C, S55C and L188C, V56C and T189C, V56C and V300C, I57C and S190C, I57C and Y299C, T58C and K191C, I59C and L193C, I59C and L297C, E60C and D194C, P101C and I152C, A102C and I148C, A102C and I152C, T103C and I146C, T103C and I148C, N104C and G145C, N104C and S146C, N105C and G145C, N105C and S146C, N105C and A147C, V144C and V406C, and S146C and N460C. In some embodiments, the disulfide bond mutations comprise one or more of the follows: A47C and I309C, 157C and Y299C, P101C and I152C, A102C and I148C, A102C and I152C, T103C and I146C, N104C and G145C, N104C and S146C, N105C and G145C, N105C and S146C, and N105C and A147C. In some embodiments, the disulfide bond mutations comprise or are one or more of the follows: P101C and I152C, A102C and I148C, A102C and I152C, N104C and S146C, and N105C and A147C.

In some embodiments, the disulfide bond mutations comprise one or more of the follows: A47C and I309C, I57C and Y299C, P101C and I152C, A102C and I148C, A102C and I152C, T103C and I146C, N104C and G145C, N104C and S146C, N105C and G145C, N105C and S146C, and N105C and A147C. In some embodiments, the disulfide bond mutations comprise or are one of the follows: P101C and I152C, A102C and I148C, A102C and I152C, N104C and S146C, and N105C and A147C.

In some embodiments, the disulfide bond mutations comprise or are: (i) at least one of A47C and I309C, I57C and Y299C, P101C and I152C, A102C and I148C, A102C and I152C, T103C and I146C, N104C and G145C, N104C and S146C, N105C and G145C, N105C and S146C, and N105C and A147C, and (ii) one of V144C and V406C, and S146C and N460C.

In some embodiments, the disulfide bond mutations are selected from: (i) one of the follows: A47C and I309C, I57C and Y299C, P101C and I152C, A102C and 1148C, A102C and I152C, T103C and I146C, N104C and G145C, N104C and S146C, N105C and G145C, N105C and S146C, and N105C and A147C, and (ii) one ofV144C and V406C, and S146C and N460C. In some embodiments, the disulfide bond mutations are selected from: (i) one of the follows: P101C and I152C, A102C and I148C, A102C and I152C, N104C and S146C, and N105C and A147C, and (ii) one of V144C and V406C, and S146C and N460C.

In some embodiments, the mutant has the following (i) and (ii): (i) comprising one of the following mutations: A47C and I309C, I57C and Y299C, P101C and I152C, A102C and I148C, A102C and I152C, T103C and I146C, N104C and G145C, N104C and S146C, N105C and G145C, N105C and S146C, and N105C and A147C; (ii) the amino acid sequence of the mutant is at least 85%, 88%, 90%, 91%, 92%, 93%, 94%, 95%, 95.5%, 96%, 96.5%, 97%, 97.5%, 98%, 98.5%, 99%, or 99.5% identical to the corresponding wild-type RSV F protein. In some embodiments, the mutations of the mutant comprise or are: a cavity filling mutation and an electrostatic mutation.

In some embodiments, the cavity filling mutation comprises or is one or more of the following (1) to (3): (1) the substitution of T at position 54 by F, W, V, C, A, H, I, Y, L or M; preferably, the substitution of T at position 54 by F, W, C, A or V; (2) the substitution of S at position 190 by V, I, M, F, L, W, Y, H, R, E, A, Q, G, P, T, C, D, N or K; and (3) the substitution of V at position 296 by I, L, Y, H, F or M; the electrostatic mutation comprises or is one or both of the following (1) to (2): (1) the substitution of D at position 486 by Q, S, H, N, T, P, C, L, I, F or W; and (2) the substitution of V at position 487 by Q, T, N, I, K, P, R, S, Y, D, F, L, H or C.

In some embodiments, the cavity filling mutation comprised in the mutant is the substitution of T at position 54 by F, W, V, C, A, H, I, Y, L or M, and the electrostatic mutation is the substitution of D at position 486 by Q, S, H, N, T, P, C, L, I, F or W. In some embodiments, the cavity filling mutation comprised in the mutant is the substitution of T at position 54 by F, W, C, A or V, and the electrostatic mutation is the substitution of D at position 486 by Q, S, H, N, T, P, C, L, I, F or W. Alternatively, the cavity filling mutation comprised in the mutant is the substitution of T at position 54 by F, W, V, C, A, H, I, Y, L or M, and the electrostatic mutation is the substitution of D at position 486 by Q, L or I. In some embodiments, the cavity filling mutation comprised in the mutant is the substitution of T at position 54 by F, W, V, C, A, H, I, Y, L or M, and the electrostatic mutation is the substitution of D at position 486 by Q. In some embodiments, the cavity filling mutation comprised in the mutant is the substitution of T at position 54 by F, W or V, and the electrostatic mutation is the substitution of D at position 486 by Q.

In some embodiments, the cavity filling mutation comprised in the mutant is the substitution of S at position 190 by V, I, M, F, L, W, Y, H, R, E, A, Q, G, P, T, C, D, N or K, and the electrostatic mutation is the substitution of D at position 486 by Q, S, H, N, T, P, C, L, I, F or W. In some embodiments, the cavity filling mutation comprised in the mutant is the substitution of S at position 190 by V, I, M, F, L, W, Y, H, R, E, A, Q, G, P, T, C, D, N or K, and the electrostatic mutation is the substitution of D at position 486 by Q, L or I. In some embodiments, the cavity filling mutation comprised in the mutant is the substitution of S at position 190 by V, I, M, F, L, W, Y, H, R, E, A, Q, G, P, T, C, D, N or K, and the electrostatic mutation is the substitution of D at position 486 by Q.

In some embodiments, the cavity filling mutation comprised in the mutant is the substitution of V at position 296 by I, L, Y, H, F or M, and the electrostatic mutation is the substitution of D at position 486 by Q, S, H, N, T, P, C, L, I, F or W. In some embodiments, the cavity filling mutation comprised in the mutant is the substitution of V at position 296 by I, L, Y, H, F or M, and the electrostatic mutation is the substitution of D at position 486 by Q, L or I. Alternatively, the mutation of the mutant comprises the substitution of V at position 296 by L, F or M, and the substitution of D at position 486 by Q, S, H, N, T, P, C, L, I, F or W. In some embodiments, the cavity filling mutation comprised in the mutant is the substitution of V at position 296 by I, L, Y, H, F or M, and the electrostatic mutation is the substitution of D at position 486 by Q. In some embodiments, the cavity filling mutation comprised in the mutant is the substitution of V at position 296 by L, F or M, and the electrostatic mutation is the substitution of D at position 486 by Q.

In some embodiments, the cavity filling mutations comprised in the mutant are the substitution of T at position 54 by F, W, V, C, A, H, I, Y, L or M, and the substitution of S at position 190 by F, W, V, C, A, H, I, Y, L or M, and the electrostatic mutation is the substitution of D at position 486 by Q, S, H, N, T, P, C, L, I, F or W. In some embodiments, the cavity filling mutations comprised in the mutant are the substitution of T at position 54 by F, W, C, A or V, and the substitution of S at position 190 by V, I, M, F, L, W, Y, H, R, E, A, Q, G, P, T, C, D, N or K, and the electrostatic mutation is the substitution of D at position 486 by Q, S, H, N, T, P, C, L, I, F or W. Alternatively, the cavity filling mutations comprised in the mutant are the substitution of T at position 54 by F, W, V, C, A, H, I, Y, L or M, and the substitution of S at position 190 by V, I, M, F, L, W, Y, H, R, E, A, Q, G, P, T, C, D, N or K, and the electrostatic mutation is the substitution of D at position 486 by Q, L or I. In some embodiments, the cavity filling mutation comprised in the mutant is the substitution of T at position 54 by F, W, C, A or V, and the substitution of S at position 190 by V, I, M, F, L, W, Y, H, R, E, A, Q, G, P, T, C, D, N or K, and the electrostatic mutation is the substitution of D at position 486 by Q, L or I. In some embodiments, the cavity filling mutation comprised in the mutant is the substitution of T at position 54 by F, W or V, and the substitution of S at position 190 by V, I, M, F, L, W, Y, H, R, E, A, Q, G, P, T, C, D, N or K, and the electrostatic mutation is the substitution of D at position 486 by Q.

In some embodiments, the cavity filling mutations comprised in the mutant are the substitution of S at position 190 by V, I, M, F, L, W, Y, H, R, E, A, Q, G, P, T, C, D, N or K, and the substitution of V at position 296 by I, L, Y, H, F or M, and the electrostatic mutation is the substitution of D at position 486 by Q, S, H, N, T, P, C, L, I, F or W. In some embodiments, the cavity filling mutations comprised in the mutant are the substitution of S at position 190 by V, I, M, F, L, W, Y, H, R, E, A, Q, G, P, T, C, D, N or K, and the substitution of V at position 296 by L, F or M, and the electrostatic mutation is the substitution of D at position 486 by Q, S, H, N, T, P, C, L, I, F or W. Alternatively, the cavity filling mutations comprised in the mutant are the substitution of S at position 190 by V, I, M, F, L, W, Y, H, R, E, A, Q, G, P, T, C, D, N or K, and the substitution of V at position 296 by I, L, Y, H, F or M, and the electrostatic mutation is the substitution of D at position 486 by Q, L or I. In some embodiments, the cavity filling mutations comprised in the mutant are the substitution of S at position 190 by V, I, M, F, L, W, Y, H, R, E, A, Q, G, P, T, C, D, N or K, and the substitution of V at position 296 by I, L, Y, H, F or M, and the electrostatic mutation is the substitution of D at position 486 by Q. In some embodiments, the cavity filling mutations comprised in the mutant are the substitution of S at position 190 by V, I, M, F, L, W, Y, H, R, E, A, Q, G, P, T, C, D, N or K, and the substitution of V at position 296 by L, F or M, and the substitution of D at position 486 by Q.

In some embodiments, the cavity filling mutations comprised in the mutant are the substitution of T at position 54 by F, W, V, C, A, H, I, Y, L or M, the substitution of S at position 190 by an amino acid other than S, and the substitution of V at position 296 by L, F or M, and the electrostatic mutation is the substitution of D at position 486 by Q, S, H, N, T, P, C, L, I, F or W. In some embodiments, the cavity filling mutations comprised in the mutant are the substitution of T at position 54 by F, W, C, A or V, the substitution of S at position 190 by V, I, M, F, L, W, Y, H, R, E, A, Q, G, P, T, C, D, N or K, and the substitution of V at position 296 by I, L, Y, H, F or M, and the electrostatic mutation is the substitution of D at position 486 by Q, S, H, N, T, P, C, L, I, F or W. Alternatively, the cavity filling mutations comprised in the mutant are the substitution of T at position 54 by F, W, V, C, A, H, I, Y, L or M, the substitution of S at position 190 by V, I, M, F, L, W, Y, H, R, E, A, Q, G, P, T, C, D, N or K, and the substitution of V at position 296 by L, F or M, and the electrostatic mutation is the substitution of D at position 486 by Q, S, H, N, T, P, C, L, I, F or W. Alternatively, the cavity filling mutations comprised in the mutant are the substitution of T at position 54 by F, W, V, C, A, H, I, Y, L or M, the substitution of S at position 190 by an amino acid other than S, and the substitution of V at position 296 by I, L, Y, H, F or M, and the electrostatic mutation is the substitution of D at position 486 by Q, L or I. Alternatively, the cavity filling mutations comprised in the mutant are the substitution of T at position 54 by F, W, V, C, A, H, I, Y, L or M, the substitution of S at position 190 by an amino acid other than S, and the substitution of V at position 296 by I, L, Y, H, F or M, and the electrostatic mutation is the substitution of D at position 486 by Q.

In some embodiments, the cavity filling mutations comprised in the mutant are the substitution of T at position 54 by F, W, C, A or V, the substitution of S at position 190 by V, I, M, F, L, W, Y, H, R, E, A, Q, G, P, T, C, D, N or K, and the substitution of V at position 296 by L, F or M, and the electrostatic mutation is the substitution of D at position 486 by Q, S, H, N, T, P, C, L, I, F or W. Alternatively, the cavity filling mutations comprised in the mutant are the substitution of T at position 54 by F, W, V, C, A, H, I, Y, L or M, the substitution of S at position 190 by V, I, M, F, L, W, Y, H, R, E, A, Q, G, P, T, C, D, N or K, and the substitution of V at position 296 by L, F or M, and the electrostatic mutation is the substitution of D at position 486 by Q, L or I. Alternatively, the cavity filling mutations comprised in the mutant are the substitution of T at position 54 by F, W, C, A or V, the substitution of S at position 190 by an amino acid other than S, and the substitution of V at position 296 by I, L, Y, H, F or M, and the electrostatic mutation is the substitution of D at position 486 by Q, L or I.

In some embodiments, the cavity filling mutations comprised in the mutant are the substitution of T at position 54 by F, W or V, the substitution of S at position 190 by V, I, M, F, L, W, Y, H, R, E, A, Q, G, P, T, C, D, N or K, and the substitution of V at position 296 by L, F or M, and the electrostatic mutation is the substitution of D at position 486 by Q, L or I. In some embodiments, the cavity filling mutations comprised in the mutant are the substitution of T at position 54 by F, W or V, the substitution of S at position 190 by V, I, M, F, L, W, Y, H, R, E, A, Q, G, P, T, C, D, N or K, and the substitution of V at position 296 by L, F or M, and the substitution of D at position 486 by Q.

In some embodiments, the mutations of the mutant comprise or are disulfide bond mutations and one of a cavity filling mutation and an electrostatic mutation.

In some embodiments, the mutations of the mutant comprise or are: (i) one or more of the following disulfide bond mutations: S46C and T311C, A47C and I309C, L48C and V308C, S55C and L188C, V56C and T189C, V56C and V300C, I57C and S190C, I57C and Y299C, T58C and K191C, I59C and L193C, I59C and L297C, E60C and D194C, P101C and I152C, A102C and I148C, A102C and I152C, T103C and I146C, T103C and I148C, N104C and G145C, N104C and S146C, N105C and G145C, N105C and S146C, N105C and A147C, V144C and V406C, and S146C and N460C; and (ii) one or more of the following cavity filling mutations: (1)the substitution of T at position 54 by F, W, V, C, A, H, I, Y, L or M, (2)the substitution of S at position 190 by V, I, M, F, L, W, Y, H, R, E, A, Q, G, P, T, C, D, N or K, and (3)the substitution of V at position 296 by I, L, Y, H, F or M. In some embodiments, the cavity filling mutation of (ii) is the substitution of S at position 190 by V, I, M, F, L, W, Y, H, R, E, A, Q, G, P, T, C, D, N or K.

In some embodiments, the mutation of the mutant comprises or is: (i) one or more of the following disulfide bond mutations: A47C and I309C, I57C and Y299C, P101C and I152C, A102C and I148C, A102C and I152C, T103C and I146C, N104C and G145C, N104C and S146C, N105C and G145C, N105C and S146C, and N105C and A147C; and (ii) one or more of the following cavity-filling mutations: (1)the substitution of T at position 54 by F, W, V, C, A, H, I, Y, L or M, (2)the substitution of S at position 190 by V, I, M, F, L, W, Y, H, R, E, A, Q, G, P, T, C, D, N or K, and (3)the substitution of V at position 296 by I, L, Y, H, F or M. In some embodiments, the cavity filling mutation of (ii) is the substitution of S at position 190 by V, I, M, F, L, W, Y, H, R, E, A, Q, G, P, T, C, D, N or K.

In some embodiments, the mutation of the mutant comprises or is: (i) one or more of the following disulfide bond mutations: P101C and I152C, A102C and I148C, A102C and I152C, N104C and S146C, and N105C and A147C; and (ii) one or more of the following cavity filling mutations: (1)the substitution of T at position 54 by F, W, V, C, A, H, I, Y, L or M, (2)the substitution of S at position 190 by V, I, M, F, L, W, Y, H, R, E, A, Q, G, P, T, C, D, N or K, and (3)the substitution of V at position 296 by I, L, Y, H, F or M. In some embodiments, the cavity filling mutation of (ii) is the substitution of S at position 190 by V, I, M, F, L, W, Y, H, R, E, A, Q, G, P, T, C, D, N or K.

In some embodiments, the mutation of the mutant comprises or is: (i) one of the following disulfide bond mutations: one of A47C and I309C, I57C and Y299C, P101C and I152C, A102C and I148C, A102C and I152C, T103C and I146C, N104C and G145C, N104C and S146C, N105C and G145C, N105C and S146C, and N105C and A147C; and (ii) one or more of the following cavity-filling mutations: (1)the substitution of T at position 54 by F, W, C, A or V, (2)the substitution of S at position 190 by V, I, M, F, L, W, Y, H, R, E, A, Q, G, P, T, C, D, N or K, and (3)the substitution of V at position 296 by I, L, Y, H, F or M.

In some embodiments, the mutation of the mutant comprises or is: (i) one of the following disulfide bond mutations: one of A47C and I309C, I57C and Y299C, P101C and I152C, A102C and I148C, A102C and I152C, T103C and I146C, N104C and G145C, N104C and S146C, N105C and G145C, N105C and S146C, and N105C and A147C; and (ii) one or more of the following cavity-filling mutations: (1)the substitution of T at position 54 by F, W, C, A or V, (2)the substitution of S at position 190 by V, I, M, F, L, W, Y, H, R, E, A, Q, G, P, T, C, D, N or K, and (3)the substitution of V at position 296 by L, F or M.

In some embodiments, the mutations of the mutant are selected from one group of the following (1) to (20): (1)A102C, I152C, T54V, S190M and V296L; (2)A102C, I152C, T54F, S190M and V296L; (3)A102C, I152C, T54C, S190M and V296L; (4)A102C, I152C, T54A, S190M and V296L; (5)A102C, I152C, T54V, S190F and V296L; (6)A102C, I152C, T54F, S190F and V296L; (7)A102C, I152C, T54C, S190F and V296L; (8)A102C, I152C, T54A, S190F and V296L; (9)P101C, I152C, T54V, S190M and V296L; (10)P101C, I152C, T54F, S190M and V296L; (11)P101C, I152C, T54C, S190M and V296L; (12)P101C, I152C, T54A, S190M and V296L; (13)P101C, I152C, T54V, S190F and V296L; (14)P101C, I152C, T54F, S190F and V296L; (15)P101C, I152C, T54C, S190F and V296L; (16)P101C, I152C, T54A, S190F and V296L; (17)A102C, I152C, T54V, S190I and V296L; (18)A102C, I152C, T54F, S190I and V296L; (19)A102C, I152C, T54C, S190I and V296L; and (20)A102C, I152C, T54A, S190I and V296L.

In some embodiments, the mutant is selected from one of the following (1) to (40): (1) a mutant comprising the amino acid sequence as shown in SEQ ID NO: 61 or 62; (2) a mutant comprising mutations A102C, I152C, T54V, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 61 or 62; (3) a mutant comprising the amino acid sequence as shown in SEQ ID NO: 63 or 64; (4) a mutant comprising mutations A102C, I152C, T54F, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 63 or 64; (5) a mutant comprising the amino acid sequence as shown in SEQ ID NO: 65 or 66; (6) a mutant comprising mutations A102C, I152C, T54C, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 65 or 66; (7) a mutant comprising the amino acid sequence as shown in SEQ ID NO: 67 or 68; (8) a mutant comprising mutations A102C, I152C, T54A, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 67 or 68; (9) a mutant comprising the amino acid sequence as shown in SEQ ID NO: 69 or 70; (10) a mutant comprising mutations A102C, I152C, T54V, S190F, and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 69 or 70; (11) a mutant comprising the amino acid sequence as shown in SEQ ID NO: 71 or 72; (12) a mutant comprising mutation A102C, I152C, T54F, S190F, and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 71 or 72; (13) a mutant comprising the amino acid sequence as shown in SEQ ID NO: 73 or 74; (14) a mutant comprising mutations A102C, I152C, T54C, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 73 or 74; (15) a mutant comprising the amino acid sequence as shown in SEQ ID NO: 75 or 76; (16) a mutant comprising mutations A102C, I152C, T54A, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 75 or 76; (17) a mutant comprising the amino acid sequence as shown in SEQ ID NO: 77 or 78; (18) a mutant comprising mutations P101C, I152C, T54V, S190M, and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 77 or 78; (19) a mutant comprising the amino acid sequence as shown in SEQ ID NO: 79 or 80; (20) a mutant comprising mutations P101C, I152C, T54F, S190M, and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 79 or 80; (21) a mutant comprising the amino acid sequence as shown in SEQ ID NO: 81 or 82; (22) a mutant comprising mutations P101C, I152C, T54C, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 81 or 82; (23) a mutant comprising the amino acid sequence as shown in SEQ ID NO: 83 or 84; (24) a mutant comprising mutations P101C, I152C, T54A, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 83 or 84; (25) a mutant comprising the amino acid sequence as shown in SEQ ID NO: 85 or 86; (26) a mutant comprising mutations P101C, I152C, T54V, S190F, and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 85 or 86 ; (27) a mutant comprising the amino acid sequence as shown in SEQ ID NO: 87 or 88; (28) a mutant comprising mutations P101C, I152C, T54F, S190F, and V296L and comprising at least 85%, 88%, 90%, 91%, 92%, 93%, 94%, 95%, 95.5%, 96%, 96.5% identical to the amino acid sequence as shown in SEQ ID NO: 87 or 88; (29) a mutant comprising the amino acid sequence as shown in SEQ ID NO: 89 or 90; (30) a mutant comprising mutations P101C, I152C, T54C, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 89 or 90; (31) a mutant comprising the amino acid sequence as shown in SEQ ID NO: 91 or 92; (32) a mutant comprising mutations P101C, I152C, T54A, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 91 or 92; (33) a mutant comprising the amino acid sequence as shown in SEQ ID NO: 93 or 94; (34) a mutant comprising mutations A102C, I152C, T54V, S190I, and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 93 or 94; (35) a mutant comprising the amino acid sequence as shown in SEQ ID NO: 95 or 96; (36) a mutant comprising mutations A102C, I152C, T54F, S190I and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 95 or 96; (37) a mutant comprising the amino acid sequence as shown in SEQ ID NO: 97 or 98; (38) a mutant comprising mutations A102C, I152C, T54C, S190I and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 97 or 98; (39) a mutant comprising the amino acid sequence as shown in SEQ ID NO: 99 or 100; and (40) a mutant comprising mutations A102C, I152C, T54A, S190I and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 99 or 100.

In some embodiments, the mutations of the mutant comprise or are one group of the following (1) to (7):
(1)P101C, I152C, T54A, S190F and V296L; (2)P101C, I152C, T54C, S190F and V296L; (3)P101C, I152C, T54A, S190M and V296L; (4)P101C, I152C, T54C, S190M and V296L; (5)P101C, I152C, T54V, S190M and V296L; (6)A102C, I152C, T54A, S190F and V296; and (7)A102C, I152C, T54C, S190M and V296L.

In some embodiments, the mutations of the mutant comprise or are one group of the following (1)- (3): (1) P101C, I152C and S190M; (2) P101C, I152C and S190F; and (3) P101C, I152C and S190I.

In some embodiments, the mutations of the mutant comprise or are: (i) one or more of the following disulfide bond mutations: S46C and T311C, A47C and I309C, L48C and V308C, S55C and L188C, V56C and T189C, V56C and V300C, I57C and S190C, I57C and Y299C, T58C and K191C, I59C and L193C, I59C and L297C, E60C and D194C, P101C and I152C, A102C and I148C, A102C and I152C, T103C and I146C, T103C and I148C, N104C and G145C, N104C and S146C, N105C and G145C, N105C and S146C, N105C and A147C, V144C and V406C, and S146C and N460C; and (ii) one or more of the following electrostatic mutations: the substitution of D at position 486 by Q, S, H, N, T, P, C, L, I, F or W; and the substitution of V at position 487 by Q, T, N, I, K, P, R, S, Y, D, F, L, H or C.

In some embodiments, the mutation of the mutant comprises or is: (i) one or more of the following disulfide bond mutations: A47C and I309C, I57C and Y299C, P101C and I152C, A102C and I148C, A102C and I152C, T103C and I146C, N104C and G145C, N104C and S146C, N105C and G145C, N105C and S146C, and N105C and A147C; and (ii) one or more of the following electrostatic mutations: the substitution of D at position 486 by Q, S, H, N, T, P, C, L, I, F or W; and the substitution of V at position 487 by Q, T, N, I, K, P, R, S, Y, D, F, L, H or C.

In some embodiments, the mutations of the mutant comprise or are: (i) one of the following disulfide bond mutations: one of A47C and I309C, I57C and Y299C, P101C and I152C, A102C and I148C, A102C and I152C, T103C and I146C, N104C and G145C, N104C and S146C, N105C and G145C, N105C and S146C, and N105C and A147C; and (ii) the following electrostatic mutation: the substitution of D at position 486 by Q, L, or I. In some embodiments, the electrostatic mutation of (ii) is the substitution of D at position 486 by Q.

In some embodiments, the mutations of the mutant comprise or are disulfide bond mutations, a cavity filling mutation, and an electrostatic mutation.

In some embodiments, the mutations of the mutant comprise or are: (i) one or more of the following disulfide bond mutations: S46C and T311C, A47C and 1309C, L48C and V308C, S55C and L188C, V56C and T189C, V56C and V300C, I57C and S190C, I57C and Y299C, T58C and K191C, I59C and L193C, I59C and L297C, E60C and D194C, P101C and I152C, A102C and I148C, A102C and I152C, T103C and I146C, T103C and I148C, N104C and G145C, N104C and S146C, N105C and G145C, N105C and S146C, N105C and A147C, V144C and V406C, and S146C and N460C; (ii) one or more of the following cavity filling mutations: (1) the substitution of T at position 154 by F, W, V, C, A, H, I, Y, L or M, (2) the substitution of S at position 190 by V, I, M, F, L, W, Y, H, R, E, A, Q, G, P, T, C, D, N or K, and (3) the substitution of V at position 296 by I, L, Y, H, F or M; and (iii) one or both of the following electrostatic mutations: the substitution of D at position 486 by Q, S, H, N, T, P, C, L, I, F or W, and the substitution of V at position 487 by Q, T, N, I, K, P, R, S, Y, D, F, L, H or C.

In some embodiments, the disulfide bond mutations in (i) are selected from one or more of the following disulfide bond mutations: A47C and I309C, I57C and Y299C, P101C and I152C, A102C and I148C, A102C and I152C, T103C and I146C, N104C and G145C, N104C and S146C, N105C and G145C, N105C and S146C, and N105C and A147C. In some embodiments, the disulfide bond mutations in (i) are selected from one or more of the following disulfide bond mutations: P101C and I152C, A102C and I148C, A102C and I152C, N104C and S146C, and N105C and A147C.

In some embodiments, the cavity filling mutation in (ii) is the substitution of S at position 190 by an amino acids other than S, and at least one of the substitution of T at position 54 by F, W, V, C, A, H, I, Y, L or M and the substitution of V at position 296 by I, L, Y, H, F or M. Alternatively, the cavity filling mutation in (ii) is one or both of the follows: the substitution of T at position 54 by F, W, V, C, A, H, I, Y, L or M, and the substitution of V at position 296 by I, L, Y, H, F or M. In some embodiments, the cavity filling mutation in (ii) is: the substitution of T at position 54 by F, W or V, and at least one of the substitution of S at position 190 by V, I, M, F, L, W, Y, H, R, E, A, Q, G, P, T, C, D, N or K and the substitution of V at position 296 by I, L, Y, H, F or M; or the cavity filling mutation in (ii) is the substitution of S at position 190 by an amino acid other than S, and at least one of the substitution of T at position 54 by F, W or V and the substitution of V at position 296 by L, F or M; or the cavity filling mutation in (ii) is one or both of the substitution of T at position 54 by F, W or V and the substitution of V at position 296 by L, F or M.

In some embodiments, the electrostatic mutation in (iii) comprises or is the substitution of D at position 486 by Q, or L. In some embodiments, the electrostatic mutation in (iii) comprises or is the substitution of D at position 486 by Q.

In some embodiments, the mutations of the mutant comprise or are: (i) one or more of the following disulfide bond mutations: A47C and I309C, I57C and Y299C, P101C and I152C, A102C and I148C, A102C and I152C, T103C and I146C, N104C and G145C, N104C and S146C, N105C and G145C, N105C and S146C, and N105C and A147C; (ii) one or more of the following cavity filling mutations: (1) the substitution of T at position 54 by F, W, C, A or V; (2) the substitution of S at position 190 by V, I, M, F, L, W, Y, H, R, E, A, Q, G, P, T, C, D, N or K; and (3) the substitution of V at position 296 by I, L, Y, H, F or M; and (iii) one or more of the following electrostatic mutations: the substitution of D at position 486 by Q, L or I, and the substitution of V at position 487 by Q, T, N, I, K, P, R, S, Y, D, F, L, H or C. In some embodiments, the disulfide bond mutations in (i) are one of the follows: P101C and I152C, A102C and I148C, A102C and I152C, N104C and S146C, and N105C and A147C; and/or V at position 296 in (ii) is substituted by L, F or M, and/or T at position 54 in (ii) is substituted by F, W or V; and/or the electrostatic mutation in (iii) is the substitution of D at position 486 by Q.

In some embodiments, the mutations of the mutant comprise or are: (i) one of the following disulfide bond mutations: one of A47C and I309C, I57C and Y299C, P101C and I152C, A102C and I148C, A102C and I152C, T103C and I146C, N104C and G145C, N104C and S146C, N105C and G145C, N105C and S146C, and N105C and A147C; (ii) one or more of the following cavity-filling mutations: (1) the substitution of T at position 54 by F, W, C, A or V; (2) the substitution of S at position 190 by V, I, M, F, L, W, Y, H, R, E, A, Q, G, P, T, C, D, N or K; and (3) the substitution of V at position 296 by L, F or M; and (iii) the following electrostatic mutation: the substitution of D at position 486 by Q, L or I. In some embodiments, the disulfide bond mutations in (i) are selected from one or more of P101C and I152C, A102C and I148C, A102C and I152C, N104C and S146C, and N105C and A147C. and/or, the electrostatic mutation in (iii) is the substitution of D at position 486 by Q.

In some embodiments, the mutations of the mutant is at least one of the substitution of T at position 54 by F, W or V, and the substitution of D at position 486 by Q, and one of the following disulfide bond mutations: A47C and I309C , I57C and Y299C, P101C and I152C , A102C and I148C, A102C and I152C, T103C and I146C, N104C and G145C, N104C and S146C, N105C and G145C, N105C and S146C, N105C and A147C. In some embodiments, the disulfide bond mutations are selected from one or more of P101C and I152C, A102C and I148C, A102C and I152C, N104C and S146C, and N105C and A147C.

In some embodiments, the mutation of the mutant comprises or is: (i) one of the following disulfide bond mutations: one of P101C and I152C, A102C and I148C, A102C and I152C, N104C and S146C, and N105C and A147C; (ii) one or more of the following cavity filling mutations: (1) the substitution of T at position 54 by F, W, V, C, A, H, I, Y, L or M; (2) the substitution of S at position 190 by V, I, M, F, L, W, Y, H, R, E, A, Q, G, P, T, C, D, N or K; and (3) the substitution of V at position 296 by I, L, Y, H, F or M; and (iii) one or both of the the following electrostatic mutations: the substitution of D at position 486 by Q, S, H, N, T, P, C, L, I, F or W, and the substitution of V at position 487 by Q, T, N, I, K, P, R, S, Y, D, F, L, H or C.

In some embodiments, the mutations of the mutant comprise or are: (i) one of the following disulfide bond mutations: one of P101C and I152C, A102C and I148C, A102C and I152C, N104C and S146C, and N105C and A147C; (ii) the following cavity-filling mutations: (1) the substitution of T at position 54 by F, W, V, C, A, H, I, Y, L or M; (2) the substitution of S at position 190 by V, I, M, F, L, W, Y, H, R, E, A, Q, G, P, T, C, D, N or K; and (3) the substitution of V at position 296 by I, L, Y, H, F or M; and (iii) the following electrostatic mutation: the substitution of D at position 486 by Q, S, H, N, T, P, C, L, I, F or W.

In some embodiments, the mutations of the mutant comprise or are: (i) one of the following disulfide bond mutations: one of P101C and I152C, A102C and I148C, A102C and I152C, N104C and S146C, and N105C and A147C; (ii) one or more of the following cavity-filling mutations: (1) the substitution of T at position 54 by H, F, W or V; (2) the substitution of S at position 190 by I, M, F, L, W, Y, H, R, E, A, Q, G, P, T, C, D, N or K; and (3) the substitution of V at position 296 by I, L, F or M; and (iii) the electrostatic mutation which is the substitution of D at position 486 by S. In some embodiments, the mutations of the mutant comprise or are: (i) one of the following disulfide bond mutations: one of P101C and I152C, A102C and I148C, A102C and I152C, N104C and S146C, and N105C and A147C; (ii) one or more of the following cavity-filling mutations: (1) the substitution of T at position 54 by H, F, W or V; (2) the substitution of S at position 190 by I; and (3) the substitution of V at position 296 by I, L, F or M; and (iii) the electrostatic mutation which is the substitution of D at position 486 by S.

In some embodiments, the mutations of the mutant comprise or are one group of the following (1) to (15): (1)T103C, S146C, T54H, S190I, V296I and D486S; (2)P101C, I152C, T54H, S190I, V296I and D486S; (3)N105C, S146C, T54H, S190I, V296I and D486S; (4)N105C, G145C, T54H, S190I, V296I and D486S ; (5)N105C, A147C, T54H, S190I, V296I and D486S ; (6)N104C, S146C, T54H, S190I, V296I and D486S; (7)N104C, G145C, T54H, S190I, V296I and D486S; (8)A102C, I152C, T54H, S190I, V296I and D486S; (9)A102C, I148C, T54H, S190I, V296I and D486S; (10)A47C, I309C, T54H, V144C, V406C and D486S; (11)S46C, T311C, T54H and D486S; (12)S46C, T311C, T54H, V144C, V406C and D486S; (13)L48C, V308C, T54H and D486S; (14)L48C, V308C, T54H, V144C, V406C and D486S; and (15)A47C, I309C, T54H and D486S.

In some embodiments, the mutant is selected from one of the following (1) to (34): (1) a mutant comprising the amino acid sequence as shown in SEQ ID NO: 1 or 2; (2) a mutant comprising A47C, I309C, T54H, V144C, V406C and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 1 or 2; (3) a mutant comprising the amino acid sequence as shown in SEQ ID NO: 3 or 4; (4) a mutant comprising mutations A47C, I309C, T54H and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 3 or 4; (5) a mutant comprising the amino acid sequence as shown in SEQ ID NO: 5 or 6; (6) a mutant comprising L48C, V308C, T54H, V144C, V406C and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 5 or 6; (7) a mutant comprising the amino acid sequence as shown in SEQ ID NO: 7 or 8; (8) a mutant comprising L48C, V308C, T54H and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 7 or 8; (9) a mutant comprising the amino acid sequence as shown in SEQ ID NO: 9 or 10; (10) a mutant comprising mutations S46C, T311C, T54H, V144C, V406C, and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 9 or 10; (11) a mutant comprising the amino acid sequence as shown in SEQ ID NO: 11 or 12; (12) a mutant comprising mutations S46C, T311C, T54H and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 11 or 12; (13) a mutant comprising the amino acid sequence as shown in SEQ ID NO: 13 or 14; (14) a mutant comprising mutations A102C, I148C, T54H, S190I, V296I and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 13 or 14; (15) a mutant comprising the amino acid sequence as shown in SEQ ID NO: 15 or 16; (16) a mutant comprising mutations A102C, I152C, T54H, S190I, V296I, and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 15 or 16; (17) a mutant comprising the amino acid sequence as shown in SEQ ID NO: 17 or 18; (18) a mutant comprising mutations N104C, G145C, T54H, S190I, V296I and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 17 or 18; (19) a mutant comprising the amino acid sequence as shown in SEQ ID NO: 19 or 20; (20) a mutant comprising mutations N104C, S146C, T54H, S190I, V296I and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 19 or 20; (21) a mutant comprising the amino acid sequence as shown in SEQ ID NO: 21 or 22; (22) a mutant comprising mutations N105C, A147C, T54H, S190I, V296I, and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 21 or 22; (23) a mutant comprising the amino acid sequence as shown in SEQ ID NO: 23 or 24; (24) a mutant comprising mutations N105C, G145C, T54H, S190I, V296I and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 23 or 24; (25) a mutant comprising the amino acid sequence as shown in SEQ ID NO: 25 or 26; (26) a mutant comprising mutations N105C, S146C, T54H, S190I, V296I and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 25 or 26; (27) a mutant comprising the amino acid sequence as shown in SEQ ID NO: 27 or 28; (28) a mutant comprising mutations P101C, I152C, T54H, S190I, V296I, and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 27 or 28; (29) a mutant comprising the amino acid sequence as shown in SEQ ID NO: 29 or 30; (30) a mutant comprising mutations T103C, S146C, T54H, S190I, V296I and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 29 or 30; (31) a mutant comprising the amino acid sequence as shown in SEQ ID NO: 101 or 102; (32) a mutant comprising mutations T103C, I148C, T54H, S190I, V296I and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 101 or 102; (33) a mutant comprising the amino acid sequence as shown in SEQ ID NO: 103 or 104; and (34) a mutant comprising mutations T103C, I148C, T54H, S190I, V296I and D486S and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 101 or 102.

In some embodiments, the mutant of any of the above embodiments further comprises one or more of the following mutations:
(1) the substitution of K or E at position 66 by I, L, M, F or V;
(2) the substitution of N at position 67 by I;
(3) the substitution of S at position 213 by P, G;
(4) the substitution of S at position 215 by P, G;
(5) the substitution of N at position 216 by P;
(6) the substitution of I at position 217 by P, C, F, Y or W; and
(7) the substitution of V at position 487 by Q, T, N, I, K, P, R, S, Y, D, F, L, H, or C.

In some embodiments, the wild-type RSV is subtype A or subtype B.

In some embodiments, the wild-type RSV is wild-type hRSV. In some embodiments, the wild-type hRSV is strain A2, strain Ontario or strain Buenos Aires. It can be understood that in other embodiments, the wild-type RSV is not limited to those listed above, and may also be others.

In some embodiments, the mutant comprises an F1 polypeptide and an F2 polypeptide. The F1 and F2 polypeptides of the mutant of RSV protein, into which one or more mutations are introduced, may be derived from any wild-type RSV F protein known in the art or discovered in the future, including but not limited to F proteins of subtype A RSV and subtype B strains (e.g., A2, Ontario, Buenos Aires) or any other subtype. In addition, the F1 polypeptide and the F2 polypeptide may be from F proteins of RSV from the same subtype , or may be from F proteins of RSV from different subtypes. For example, F1 polypeptide is from F protein of subtype A RSV, F2 polypeptide is from F protein of subtype B RSV; or F1 polypeptide is from F protein of subtype B RSV, F2 polypeptide is from F protein of subtype A RSV.

The amino acid sequence of F protein of strain Ontario is shown in SEQ ID NO: 216; and the amino acid sequence of F protein of strain Buenos Aires is shown in SEQ ID NO: 217.

In some embodiments, the mutant further comprises one or more of the follows: a pep27 polypeptide, a transmembrane domain (TM) of an RSV F protein (e.g., a transmembrane domain of a wild-type RSV F protein), a signal peptide, and a trimerization domain (e.g., a foldon). In some embodiments, the mutant further comprises a pep27 polypeptide. In some embodiments, the mutant further comprises a trimeric domain (such as foldon). In some embodiments, the mutant further comprises the transmembrane domain (TM) of RSV F protein (such as the transmembrane domain of wild-type hRSV F protein). In some embodiments, the mutant further comprises a signal peptide.In some embodiments, the mutant further comprises one or more of the follows: a pep27 polypeptide, a signal peptide, and a trimerization domain (e.g., a foldon). Trimerization domains are used to promote the formation of trimers of three F1/F2 heterodimers. The trimerization domain is not particularly limited, and may be, for example, a GCN4 leucine zipper, a trimerization motif from a pulmonary surfactant protein, a phage T4 fibritin foldon, etc. In some embodiments, the foldon is located at the C-terminus of the F1 polypeptide. In an optional specific example, the amino acid sequence of the foldon is: GYIPEAPRDGQAYVRKDGEWVLLSTFL (SEQ ID NO: 212). In some embodiments, the mutant comprises a pep27 polypeptide, a signal peptide, and a trimerization domain. In other embodiments, the mutant comprises a pep27 polypeptide, a signal peptide, and a transmembrane domain.

In some embodiments, the signal peptide is derived from RSV F protein.

In some embodiments, the amino acid sequence of the signal peptide is positions 1-26 of SEQ ID NO: 1 or 2.

In some embodiments, the nucleotide sequence of the signal peptide is positions 1 to 78 of SEQ ID NO: 105 or 106. In an optional specific example, the mutant of RSV F protein is derived from the F protein of the Ontario strain, and the amino acid sequence of the signal peptide is positions 1-26 of SEQ ID NO: 1. In an optional specific example, the mutant of RSV F protein is derived from the F protein of the Buenos Aires strain, and the amino acid sequence of the signal peptide is positions 1-26 of SEQ ID NO: 2.

In some embodiments, the signal peptide is heterologous to the RSV F protein. As used herein, the term "heterologous" refers to two parts of a nucleic acid, such as a polynucleotide encoding RSV F protein and a polynucleotide encoding a signal peptide, which do not occur in such a combination in nature (in their natural state). They are usually recombinant. Preferably, the polynucleotide encoding the signal peptide is derived from a different gene from the polynucleotide encoding the RSV F protein, i.e., the source gene of the signal peptide is different from the source gene of the polynucleotide encoding the RSV F protein. For example, the source gene of the polynucleotide encoding RSV F protein and the source gene of the signal peptide are genes encoding different proteins. For example, the source gene of the polynucleotide encoding RSV F protein and the source gene encoding the signal peptide are genes that encode the same protein but belong to different subtypes. For example, the source gene of the polynucleotide encoding the signal peptide is the gene encoding the F protein of subtype A hRSV, and the source gene of the polynucleotide encoding the RSV F protein is the gene encoding the F protein of subtype B hRSV. In some embodiments, the signal peptide is an IgE signal peptide or an IgG kappa signal peptide. In some embodiments, the IgE signal peptide is the IgE HC (Ig heavy chain ε -1) signal peptide.

In some embodiments, the F1 polypeptide of the mutant has the same length as the full-length F1 polypeptide of the corresponding wild-type RSV F protein. The full-length F1 polypeptide of the mutant of RSV F protein corresponds to amino acid position 137-574 of the native RSV F0 precursor and includes (from N-terminal to C-terminal) an extracellular region (residue 137-524), a transmembrane domain (residue 525-550), and a cytoplasmic domain (residue 551-574).

In other embodiments, the mutant F1 polypeptide has a deletion as compared to the full-length F1 polypeptide (or natural F1 polypeptide) of the corresponding wild-type RSV F protein. For example, an optional sequence in front of amino acid residue 482 of a native F1 polypeptide may not be present in a mutant F1 polypeptide. For example, the optional sequence in front of the amino acid residue 514 of the native F1 polypeptide may not be present in the mutant F1 polypeptide, that is, the mutant F1 polypeptide has the deletion of 1-60 amino acid residues at the C-terminus of the natural F1 polypeptide. For example, a mutant F1 polypeptide lacks the entire cytoplasmic domain. For another example, a mutant F1 polypeptide lacks the cytoplasmic domain and a part or all of the transmembrane domain. Further, for example, the mutant F1 polypeptide lacks the amino acid residue at positions 510, 511, 512, 513, 514, 515, 520, 525, or 530 to 574 as compared to the native F1 polypeptide. Typically, amino acids 482 to 574 are absent or amino acids 514 to 574 are absent for mutants to which a trimerization domain, such as a foldon, is attached. Thus, in some embodiments, amino acid residues 482 to 574 or 514 to 574 are not present in the F1 polypeptide of the mutant. In other embodiments, the mutant F1 polypeptide comprises or consists of amino acid residues 137-513 of the native F0 polypeptide.

In some embodiments, the F2 polypeptide of the mutant may have the same length as the full-length F2 polypeptide of the corresponding wild-type RSV F protein, or may have a deletion, e.g., deletion of 1, 2, 3, 4, 5, 6, 7, 8, 9 or 10 amino acid residues from the N-terminus or C-terminus of the F2 polypeptide.

In addition, the nucleic acid further comprises one or more of the follows: a polynucleotide encoding a protease cleavage site (e.g., a thrombin cleavage site (LVPRGS, SEQ ID NO: 213)), a polynucleotide encoding a protein tag (e.g., 6× His-tag (HHHHHH, SEQ ID NO: 214) and a streptomycin tag II (WSHPGFEK, SEQ ID NO: 215)), a polynucleotide encoding an linker sequence (e.g., GG and GS). Polypeptides encoded by these sequences are not necessary for the function of the RSV F protein (e.g., inducing an immune response). Such sequences are well known to those skilled in the art, and it is understood that these sequences are not limited to the above.

In some embodiments, the above nucleic acid is an isolated nucleic acid.

In some embodiments, the above nucleic acid is a non-naturally occurring nucleic acid.

In some embodiments, the above nucleic acid is a codon-optimized polynucleotide. Codon optimization methods are known in the art and may be used as provided herein. In some embodiments, the codon optimization can be used to: match the codon frequencies of the target and host organisms to ensure proper folding; bias GC content to increase mRNA stability or reduce secondary structure; minimize tandem repeat codons or base extension that can impair gene construction or expression; customize transcription and translation control regions; insert or remove protein transport sequences; remove/add post-translation modification sites in the encoded protein (e.g., glycosylation site); add, remove, or shuffle protein domains; insert or delete restriction sites; modify ribosome binding sites and mRNA degradation sites; modulate translation rates so that various domains of the protein can be folded properly; or reduce or eliminate problematic secondary structures within the polynucleotide. Codon optimization tools, algorithms, and services are known in the art, non-limiting examples include services from GeneArt (Life Technologies), DNA2.0(Menlo Park CA), and/or proprietary methods. In some embodiments, the open reading frame (ORF) sequence is optimized using an optimization algorithm. In some embodiments, the codon-optimized nucleic acid is at least 50%, 55%, 60%, 65%, 70%, 75%, 80%, 85%, 90%, 95%, or 99% identical to the nucleic acid prior to codon-optimization.

In some embodiments, the above nucleic acid is RNA. In some embodiments, the above nucleic acid is RNA, and the RNA contains an open reading frame (ORF).

In some embodiments, the above nucleic acid is RNA, and the RNA is mRNA.

In some embodiments, the above nucleic acid is non-self-replicating mRNA.

In some embodiments, the above nucleic acid further comprises at least one of a 5' -cap structure, a 5' -UTR, a 3' -UTR, and a poly (A) tail. In an optional specific example, the nucleic acid further comprises a 5' -cap structure, a 5' -UTR, a 3' -UTR and a poly (A) tail.

In some embodiments, the 5' -cap structure is selected from at least one of m⁷GpppG, m₂^{7,3'}-^{O}GpppG, m⁷G ppp (5')N1 and m⁷Gppp(m^{2'}- ^{O})N 1; wherein "m⁷G" represents a 7-methyl guanosine cap nucleoside, "ppp" represents a triphosphate bond between the 5' carbon of the cap nucleoside and the first nucleotide of the primary RNA transcript, N1 is the 5'-most nucleotide, "G" represents a guanosine, "7" represents a methyl group at the 7-position of guanosine, and "m^{2'}- ^{O}" represents a methyl group at the 2' -O position of nucleotide. In some embodiments, the 5' -cap structure is m⁷G ppp (5')N1 or m⁷Gppp(m^{2'}- ^{O})N 1. It can be understood that in other embodiments, the 5' -cap structure is not limited to the above.

In some embodiments, the DNA sequence corresponding to 5' -UTR is shown in SEQ ID NO: 209; and/or, the DNA sequence corresponding to 3' -UTR is shown in SEQ ID NO: 210. It should be noted that the "DNA sequence corresponding to 5' -UTR" refers to the nucleotide sequence of 5' - UTR in DNA form, and the same applies to the "DNA sequence corresponding to 3' -UTR" and the "DNA sequence corresponding to poly (A) tail" below.

It may be understood that, in another embodiment, the 5' -UTR and the 3' -UTR are not limited to the above, and may alternatively be other 5' -UTR and 3' -UTR described in patents such as CN108291230A, CN104321432A, and CN107849574A.

In some embodiments, the nucleotides constituting the poly (A) tail comprise at least 20, at least 40, at least 80, at least 100, or at least 120 A nucleotides. In some embodiments, the nucleotides constituting the poly (A) tail comprise at least 20, at least 40, at least 80, at least 100, or at least 120 A consecutive nucleotides.

In some embodiments, the nucleotides constituting the poly (A) tail comprise one or more nucleotides other than the A nucleotide. In some embodiments, the poly (A) tail comprises two or more contiguous nucleotides other than A nucleotide. In an optional specific example, the nucleotide DNA sequence corresponding to the poly (A) tail is shown in SEQ ID NO: 211. It can be understood that the poly (A) tail contained in the nucleic acid of the present disclosure is not limited to the above, and may also be other poly (A) tails, such as the poly (A) tail described in patents such as US20170166905A1 and WO2020074642A1.

In some embodiments, the above nucleic acid does not contain a modified nucleotide.

In some embodiments, the above nucleic acid contains a modified nucleotide.

In some embodiments, the above nucleic acid contains a modified nucleoside. In some embodiments, the modified nucleoside comprised in the above nucleic acid comprises at least one of a modified uridine, a modified cytidine, a modified adenosine, and a modified guanosine.

In some embodiments, the modified nucleoside in the above nucleic acid is a modified uridine. In some embodiments, 0.1%~100% of the uridines in the above nucleic acid are modified. In some embodiments, 80%~100% of the uridines are modified. In some embodiments, 100% of the uridines are modified. Exemplary modified uridine comprises pseudouridine (ψ), N1-methylpseudouridine, pyridin-4-one ribonucleoside, 5-aza-uridine, 6-aza-uridine, 2-thio-5-aza-uridine, 2-thio-uridine (s2U), 4-thio-uridine (s4U), 4-thio-uridine, 2-thio-pseudouridine, 5-hydroxy-uridine (ho5U), 5-aminoallyl-uridine, 5-halo-uridine (e.g., 5-iodo-uridine or 5-bromo-uridine), 3-methyl-uridine (m3U), 5-methoxy-uridine (mo5U), uridine-5-oxyacetic acid (cmo5U), uridine-5-oxyacetate methyl ester (mcmo5U), 5-carboxymethyl-uridine (cm5U), 1-carboxymethyl-pseudouridine, 5-carboxyhydroxymethyl-uridine (chm5U), 5-carboxyhydroxymethyl-uridine methyl ester (mchm5U), 5-methoxycarbonylmethyl-uridine (mcm5U), 5-methoxycarbonylmethyl-2-thio-uridine (mcm5s2U), 5-aminomethyl-2-thio-uridine (nm5s2U), 5-methylaminomethyl-uridine (mnm5U), 5-methylaminomethyl-2-thio-uridine (mnm5s2U), 5-methylaminomethyl-2-seleno-uridine (mnm5se2U), 5-carbamoylmethyl-uridine (ncm5U), 5-carboxymethylaminomethyl-uridine (cmnm5U), 5-carboxymethylaminomethyl-2-thio-uridine (cmnm5s2U), 5-propynyl-uridine, 1-propynyl-pseudouridine, 5-taurinomethyl-uridine (τm5U), 1-Taurine methyl-pseudouridine, 5-Taurine methyl-2-thio-uridine (τm5s2U), 1-Taurine methyl-4-thio-uridine, 5-methyl-uridine (m5U, i.e., deoxythymine with nucleobase), 1-methyl-pseudouridine (m1Ψ), 5-methyl-2-thio-uridine (m5s2U), 1-Methyl-4-thio-pseudouridine (m1s4Ψ), 4-thio-1-methyl-pseudouridine, 3-methyl-pseudouridine (m3Ψ), 2-thio-1-methyl-pseudouridine, 1-methyl-1-denitride-pseudouridine, 2-thio-1-methyl-1-denitride-pseudouridine, dihydrouridine (D), dihydropseudouridine, 5,6-dihydrouridine, 5-methyl-dihydrouridine (m5D), 2-thio-dihydrouridine, 2-thio-dihydrouridine, 2-methoxy-uridine, 2-methoxy-4-thio-uridine, 4-methoxy-uridine, 4-methoxy-2-thio-uridine, N1-methyl-uridine, 3-(3-amino-3-carboxypropyl) uridine (acp3U), 1-methyl-3-(3-amino-3-carboxypropyl) pseudouridine (acp3Ψ), 5- (isopentenylaminomethyl) uridine (inm5U), 5- (isopentenylaminomethyl)-2-thio-uridine (inm5s2U), α-thio-uridine, 2'-O-methyl-uridine (Um), 5,2'-O- dimethyl-uridine (m5U m), 2'-O-methyl-pseudouridine (ψm), 2-thio-2'-O-methyl-uridine (s2Um), 5-methoxycarbonylmethyl-2'-O-methyl-uridine (mcm5Um), 5-carbamoylmethyl-2'-0-methyl-uridine (ncm5Um), 5-carboxymethylaminomethyl-2'-O-methyl-uridine (cmnm5Um), 3,2'-O-dimethyl-uridine (m3Um), 5-(isopentenylaminomethyl)-2'-O- methyl-uridine (inm5Um), 1-thio-uridine, deoxythymidine, 2'-F-ara-uridine, 2'-F-uridine, 2'-OH-ara-uridine, 5-(2-carbomethoxyvinyl) uridine, and 5-[3-(1-E-propenylamino) uridine.

In some embodiments, the modified nucleoside in the above nucleic acid is a modified cytidine. In some embodiments, 0.1%~100% of the cytidines in the above nucleic acid are modified. In some embodiments, 80%~100% of the cytidine are modified. In some embodiments, 100% of the cytidine are modified. Exemplary modified cytidine comprises 5-aza-cytidine, 6-aza-cytidine, pseudoisocytidine, 3-methyl-cytidine (m3C), N4-acetyl-cytidine (ac4C), 5-formyl-cytidine (f5C), N4-methyl-cytidine (m4C), 5-methyl-cytidine (m5C), 5-halo-cytidine (e.g., 5-iodo-cytidine), 5-hydroxymethyl-cytidine (hm5C), 1-methyl-pseudoisocytidine, pyrrolo-cytidine, pyrrolo-pseudoisocytidine, 2-thio-cytidine (s2C), 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-ethoxy-1-methyl-pseudoisocytidine, Lysidine (K2C), α-thio-cytidine, 2'-O-methyl-cytidine (Cm), 5,2'-O-dimethyl-cytidine (m5Cm), N4-Acetyl-2'-O-methyl-cytidine (ac4Cm), N4,2'-O-dimethyl-cytidine (m4Cm), 5-formyl-2'-O-methyl-cytidine (f5Cm), N4,N4,2'-O-trimethyl-cytidine (m42Cm), 1-thio-cytidine, 2'-F-arabino-cytidine, 2'-F-cytidine, and 2'-OH-arabino-cytidine.

In some embodiments, the modified nucleoside in the above nucleic acid is a modified adenosine. In some embodiments, 0.1%~100% of the adenosines in above the nucleic acid are modified. In some embodiments, 80%~100% of the adenosines are modified. In some embodiments, 100% of the adenosines are modified. Exemplary modified adenosine comprises 2-amino-purine, 2,6-diaminopurine, 2-amino-6-halo-purine (e.g., 2-amino-6-chloro-purine), 6-halo-purine (e.g., 6-chloropurine), 2-amino-6-methyl-purine, 8-azido-adenosine, 7-deaza-adenine, 7-deaza-8-aza-adenine, 7-deaza-2-amino-purine, 7-deaza-8-aza-2-amino-purine, 7-deaza-2,6-diaminopurine, 7-deaza-8-aza-2,6-diaminopurine, 1-methyl-adenosine (m1A), 2-methyl-adenine (m2A), N6-methyl-adenosine (m6A), 2-methylthio-N6-methyl-adenosine (ms2m6A), N6-isopentenyl-adenosine (i6A), 2-methylthio-N6-isopentenyl-adenosine (ms2i6A), N6- (cis-hydroxyisopentenyl) adenosine (io6A), 2-methylthio -N6- (cis-hydroxyisopentenyl) adenosine (ms2io6A), N6-glycinylcarbamoyl-adenosine (g6A), N6-threonyl-carbamoyl-adenosine (t6A), N6-methyl -N6- threonyl-carbamoyl-adenosine (m6t6A), 2-methylthio -N6-threonyl-carbamoyl-adenosine (ms2g6A), N6,N6-dimethyl-adenosine (m62A), N6-hydroxynorvalylcarbamoyl-adenosine (hn6A), 2-methylthio-N6-hydroxynorvalylcarbamoyl-adenosine (ms2hn6A), N6-acetyl-adenosine (ac6A), 7-methyl-adenine, 2-methylthio-adenine, 2-methoxy-adenine, α-thio-adenosine, 2'-O-methyl-adenosine (Am), N6,2'-O-dimethyl-adenosine (m6Am), N6,N6,2'-O- trimethyl-adenosine (m62Am), 1,2'-O-dimethyl-adenosine (m1Am), 2'-O-ribosyl-adenosine (phosphate) (Ar(p)), 2-amino-N6-methyl-purine, 1-thio-adenosine, 8-azido-adenosine, 2'-F-arabine-adenosine, 2'-F-adenosine, 2'-OH-arabine-adenosine, and N6-(19-amino-pentaoxa-nonadecyl)-adenosine.

In some embodiments, the modified nucleoside in the above nucleic acid is a modified guanosine. In some embodiments, 0.1%~100% of the guanosines in the above nucleic acid are modified. In some embodiments, 80%~100% of the guanosines are modified. In some embodiments, 100% of the guanosines are modified. Exemplary modified guanosine comprises inosine (I), 1-methyl-inosine (m1I), wyosine (imG), methyl wyosine (mimG), 4-demethyl-wyosine (imG-14), isowyosine (imG2), wybutosine (yW), peroxywybutosine (o2yW), hydroxywybutosine (OHyW), undermodified hydroxywybutosine (OHyW*), 7-deaza-guanosine, queuosine (Q), epoxy-queuosine (oQ), galactosyl-queuosine (galQ), mannosyl-queuosine (manQ), 7-cyano-7-deaza-guanosine (preQ0), 7-aminomethyl-7-deaza-guanosine (preQ1), archaeosine (G+), 7-deaza-8-aza-guanosine, 6-thio-guanosine, 6-thio-7-deaza-guanosine, 6-thio-7-deaza--8-aza-guanosine, 7-methyl-guanosine (m7G), 6-thio-7-methyl-guanosine, 7-methyl-inosine, 6-methoxy-guanosine, 1-methyl-guanosine (mlG), N2-methyl-guanosine (m2G), N2,N2-dimethyl-guanosine (m22G), N2,7-dimethyl-guanosine (m2,7G), N2,N2,7-trimethyl-guanosine (m2,2,7G), 8-oxo-guanosine, 7-methyl-8-oxo-guanosine, 1-methyl-6-thio-guanosine, N2-methyl-6-thio-guanosine, N2,N2-dimethyl-6-thioguanosine, α-thio-guanosine, 2'-O-methyl-guanosine (Gm), N2-methyl-2'-O-methylguanosine (m2Gm), N2,N2-dimethyl-2'-O-methyl-guanosine (m22Gm), 1-methyl-2'-O-methyl-guanosine (mlGm), N2,7-dimethyl-2'-O-methyl-guanosine (M2,7Gm), 2'-O-methyl-inosine (Im), 1,2'-O-dimethyl-inosine (m1Im), 2'-O-ribosyl-guanosine (phosphate) (Gr(p)), 1-thio-guanosine, O6-methyl-guanosine, 2'-F-arabino-guanosine, and 2'-F-guanosine.

In some embodiments, the modified nucleotide in the above nucleic acid comprises a nucleotide containing an isotope.

In some embodiments, the above nucleic acid comprises a nucleotide containing an isotope of hydrogen. The isotope of hydrogen is not limited to deuterium, tritium. In addition, in some embodiments, the above nucleic acid further comprises or is a nucleotide containing an isotope of other elements than hydrogen, wherein the other elements include, but are not limited to, carbon, oxygen, nitrogen and phosphorus.

In some embodiments, the above nucleic acid comprises RNA corresponding to a polynucleotide having a nucleotide sequence as shown in any one of SEQ ID NOs: 105-134 and 165-208, and the uridines in the nucleic acid are all replaced with N1-methylpseudouridine.

In some embodiments, the above nucleic acid is DNA.

In some embodiments, the DNA can be transcribed into RNA in vitro.

In some embodiments, the above nucleic acid comprises a polynucleotide having a nucleotide sequence as shown in any one of SEQ ID NOs: 105-134 and 165-208.

In some embodiments, the above nucleic acid comprises a polynucleotide encoding one or more mutants of RSV F protein. It should be noted that "a mutant of RSV F protein" herein and below refers to any one of the plurality of mutants of RSV F proteins mentioned above.

In some embodiments, the nucleic acid comprises a polynucleotide encoding a plurality of mutants of RSV F protein. At this moment, the coding strands for the plurality of mutants of RSV F protein are located on the same strand of the nucleic acid, and the respective polynucleotides encoding the plurality of mutants of RSV F protein are directly linked or indirectly linked through a linking element (e.g., a polynucleotide encoding a 2A peptide or a polynucleotide encoding a flexible linker peptide). A flexible linker peptide comprises "GS" linker peptides consisting of Gly and Ser residues, e.g., (Gly-Gly-Gly-Gly-Ser)n, the length of the "GS" linker peptide can be changed by adjusting the value of n (number of repeats), allowing for optimal separation or interaction between two linked proteins. It should be noted that the "plurality of mutants of RSV F protein" herein and below refers to any of (e.g., two, three, four or more) the plurality of mutants of RSV F protein mentioned above. For example, the plurality of mutants of RSV F protein are different RSV F proteins from the same subtype strain but with different mutations. Further for example, the plurality of mutants of RSV F protein are two types, the first type of mutants are RSV F proteins derived from the strain Ontario and having mutations P101C, I152C and S190M, and the second type of mutants are RSV F proteins derived from the strain Ontario and having mutations P101C, I152C and S190F. Correspondingly, the above nucleic acid comprises a polynucleotide encoding an RSV F protein derived from the strain Ontario and having mutations P101C, I152C and S190M and a polynucleotide encoding an RSV F protein derived from the strain Ontario and having mutations P101C, I152C and S190F.

For another example, the plurality of mutants of RSV F protein are different RSV F proteins from different subtype strains but with the same mutation. Further, for example, the plurality of mutants of RSV F protein are two types, the first types of mutants are RSV F proteins derived from the strain Ontario and having mutations P101C, I152C and S190M, and the second type of mutants are RSV F proteins derived from the strain Buenos Aires and having mutations P101C, I152C and S190M. Correspondingly, the above nucleic acid comprises a polynucleotide encoding an RSV F protein derived from the strain Ontario and having mutations P101C, I152C and S190M and a polynucleotide encoding an RSV F protein derived from the strain Buenos Aires and having mutations P101C, I152C and S190M.

For another example, the plurality of mutants of RSV F protein are different RSV F proteins from different subtype strains and with different mutations. Further, for example, the plurality of mutants of RSV F protein are two types, the first type of mutants are RSV F proteins derived from the strain Ontario and having mutations P101C, I152C and S190M, and the second type of mutants are RSV F proteins derived from the strain Buenos Aires and having mutations P101C, I152C and S190F. Correspondingly, the above nucleic acid comprises a polynucleotide encoding the RSV F protein derived from the strain Ontario and having mutations P101C, I152C and S190M and a polynucleotide encoding the RSV F protein derived from the strain Buenos Aires and having mutations P101C, I152C and S190F.

For another example, the plurality of mutants of RSV F protein are three types, the first type of mutants are polynucleotides of RSV F proteins derived from the strain Ontario and having mutations P101C, I152C and S190M, the second type of mutants are polynucleotides of RSV F proteins derived from the strain Buenos Aires and having mutations P101C, I152C and S190M, and the third type of mutants are polynucleotides of RSV F protein derived from the strain Ontario and having mutations P101C, I152C and S190F. Correspondingly, the nucleic acid comprises a polynucleotide encoding an RSV F protein derived from the strain Ontario and having mutations P101C, I152C and S190M, a polynucleotide encoding an RSV F protein derived from the strain Buenos Aires and having mutations P101C, I152C and S190M, and a polynucleotide encoding an RSV F protein derived from the strain Ontario and having mutationsP101C, I152C and S190F.

In some embodiments, the nucleic acid comprises a polynucleotide encoding a protein or polypeptide other than RSV F protein. It can be understood that other proteins or polypeptides in addition to the mutant of RSV F protein refer to proteins or polypeptides with biological significance (e.g., immunogenicity), such as other proteins or polypeptides of RSV, or mutants thereof (e.g., G protein of RSV or mutants thereof), or proteins or polypeptides of other viruses, or mutants thereof (e.g., HA protein of influenza virus or mutants thereof, or S protein of SARS-CoV-2 or mutants thereof, etc.).

In addition, it can be understood that in the above nucleic acids, whether the polynucleotide encoding one mutant of RSV F protein or the polynucleotides encoding a plurality of mutants of RSV F protein can have a plurality of (e.g., two or three) repeated units on the same strand. For example, for a polynucleotide encoding one mutant of RSV F protein, the polynucleotide encoding one of the mutants of RSV F protein (e.g., the mutant having mutations P101C, I152C, T54A, S190M and V296L) is referred to as "A fragment" for short, and the same nucleic acid above may contain a plurality of A fragments. For another example, a polynucleotide encoding a plurality of mutants of RSV F protein, a polynucleotide encoding one of the mutants of RSV F protein (e.g., the mutant having mutations P101C, I152C, T54A, S190M and V296L)is referred to as "A fragment" for short, and a polynucleotide encoding another mutant of RSV F protein (e.g., the mutant having mutations P101C, I152C, T54C, S190M and V296L) is referred to as "B fragment" for short, then the same nucleic acid above may contain a plurality of A fragments and a plurality of B fragments, may contain one A fragment and a plurality of B fragments, or may contain a plurality of A fragments and one B fragment.

In some embodiments, the RSV F protein produced by the above nucleic acid is a pre-F protein.

In some embodiments, the RSV F protein produced by the above nucleic acid can bind to at least one of the following antibodies: AM14, D25, AM22, and CR9501. In some embodiments, the RSV F protein produced by the above nucleic acid can bind to the following antibodies: AM14, D25, AM22, and CR9501.

In some embodiments, as compared to Post-F protein, the RSV F protein produced by the above nucleic acid has a higher affinity to antibodies AM14, D25, AM22, or CR9501. In some embodiments, the affinity of the RSV F protein produced by the above nucleic acid to antibodies AM14, D25, AM22, or CR9501 is at least 1.1 times, 1.3 times, 1.5 times, 2 times, 5 times, 10 times, 15 times, 20 times, 25 times, 30 times, 35 times, 40 times, 45 times, 50 times, 55 times, 60 times, 65 times, 70 times, 80 times, 90 times, 100 times, 110 times, 120 times, 130 times, 140 times, 150 times, 160 times, 170 times, 180 times, 190 times, 200 times, 250 times, 300 times, 350 times, 400 times, 450 times, or 500 times greater than the affinity of Post-F protein to antibodies AM14, D25, AM22, or CR9501.

In some embodiments, as compared with Post-F protein, the RSV F protein produced by the above nucleic acid has a lower affinity to antibody clone 131-2A. In some embodiments, the affinity of Post-F protein to the antibody clone 131-2A is at least 1.1 times, 1.3 times, 1.5 times, 2 times, 5 times, 10 times, 15 times, 20 times, 25 times, 30 times, 35 times, 40 times, 45 times, 50 times, 55 times, 60 times, 65 times, 70 times, 80 times, 90 times, 100 times, 110 times, 120 times, 130 times, 140 times, 150 times, 160 times, 170 times, 180 times, 190 times, 200 times, 250 times, 300 times, 350 times, 400 times, 450 times or 500 times greater than the affinity of the RSV F protein produced by the above nucleic acid to the antibody clone 131-2A..

In some embodiments, the above nucleic acid can induce the production of neutralizing antibodies against subtype A hRSV.

In some embodiments, the above nucleic acid can induce the production of neutralizing antibodies against subtype B hRSV.

In some embodiments, the above nucleic acid can induce the production of neutralizing antibodies against subtypes A and B hRSV.

The above nucleic acid comprises a polynucleotide encoding a mutant of RSV F protein having one or more of the following mutations: disulfide bond mutations, a cavity filling mutation and an electrostatic mutation, and the RSV F protein can be stably a pre-F protein in vitro through the disulfide bond mutations, cavity filling mutation and/or electrostatic mutation of the RSV F protein. It has been verified that the above nucleic acid encoding produced protein or polypeptide of the present disclosure is capable of inducing a specific immune response, thereby preventing or at least reducing the infection of respiratory syncytial virus, and that the nucleic acid of the present disclosure induces an immune response which is at least the same as an inactivated RSV-based vaccine consisting of whole viruses.

### 2, Nucleic Acid 2

Another embodiment of the present disclosure further provides another nucleic acid, which differs from nucleic acid 1 in that the mutant of RSV F protein corresponding to the nucleic acid is at least partially different from the mutant of RSV F protein corresponding to nucleic acid 1. Specifically, as compared to the wild-type RSV F protein, the mutant of RSV F protein encoded by the nucleic acid comprises or is one or more mutations of the following (1) to (8):
(1) the substitution of K or E at position 66 by I, L, M, F or V;
(2) the substitution of N at position 67 by I;
(3) the substitution of S at position 213 by P or G;
(4) the substitution of S at position 215 by P or G;
(5) the substitution of N at position 216 by P;
(6) the substitution of I at position 217 by P, C, F, Y or W;
(7) the substitution of D at position 486 by Q, S, H, N, T, P, C, L, I, F, or W; and
(8) the substitution of V at position 487 by Q, T, N, I, K, P, R, S, Y, D, F, L, H, or C.

In some embodiments, the mutant of RSV F protein encoded by the above nucleic acid comprises or is one or more mutations of the following (1) to (8): (1) the substitution of K or E at position 66 by I, L, M, F or V; (2) the substitution of N at position 67 by I; (3) the substitution of S at position 213 by P; (4) the substitution of S at position 215 by P; (5) the substitution of N at position 216 by P; (6) the substitution of I at position 217 by P, F, Y or W; (7) the substitution of D at position 486 by Q, S, H, N, T, P, C, L, I, F, or W; and (8) the substitution of V at position 487 by Q, T, N, I, K, P, R, S, Y, D, F, L, H, or C.

In some embodiments, the mutations of the mutant encoded by the above nucleic acid comprise or are: (i) the substitution of K or E at position 66 by I, L, M, F or V; (ii) one of the following mutations: (1) the substitution of S at position 213 by P or G, (2) the substitution of S at position 215 by P or G, (3) the substitution of N at position 216 by P, and (4) the substitution of I at position 217 by P, C, F, Y or W; and (iii) one or both of the following mutations: (1) the substitution of D at position 486 by Q, S, H, N, T, P, C, L, I, F, or W, and (2) the substitution of V at position 487 by Q, T, N, I, K, P, R, S, Y, D, F, L, H, or C.

Alternatively, the mutations of the mutant encoded by the above nucleic acid comprise or are: (i) the substitution of N at position 67 by I; (ii) one of the following mutations: (1) the substitution of S at position 213 by P or G, (2) the substitution of S at position 215 by P or G, (3) the substitution of N at position 216 by P, and (4) the substitution of I at position 217 by P, C, F, Y or W; and (iii) one or both of the following mutations: (1) the substitution of D at position 486 by Q, S, H, N, T, P, C, L, I, F, or W, and (2) the substitution of V at position 487 by Q, T, N, I, K, P, R, S, Y, D, F, L, H, or C.

In some embodiments, the mutations of the mutant encoded by the above nucleic acid comprise or are: (i) the substitution of K or E at position 66 by I, L, M, F or V; (ii) one of the following mutations: (1) the substitution of S at position 213 by P, (2) the substitution of S at position 215 by P or G, (3) the substitution of N at position 216 by P, and (4) the substitution of I at position 217 by P, C, F, Y or W; and (iii) one or both of the following mutations: (1) the substitution of D at position 486 by Q, L, or I, and (2) the substitution of V at position 487 by Q, T, N, I, K, P, R, S, Y, D, F, L, H, or C.

Alternatively, the mutations of the mutant encoded by the above nucleic acid comprise or are: (i) the substitution of N at position 67 by I; (ii) one of the following mutations: (1) the substitution of S at position 213 by P, (2) the substitution of S at position 215 by P or G, (3) the substitution of N at position 216 by P, and (4) the substitution of I at position 217 by P, C, F, Y or W; and (iii) one or both of the following mutations: (1) the substitution of D at position 486 by Q, L, or I, and (2) the substitution of V at position 487 by Q, T, N, I, K, P, R, S, Y, D, F, L, H, or C.

In some embodiments, the mutation of (ii) is one of the follows: (1) the substitution of S at position 213 by P or G, (2) the substitution of N at position 216 by P, and (3) the substitution of I at position 217 by P, C, F, Y or W.

In some embodiments, the mutations of the mutant encoded by the above nucleic acid comprise or are: the substitution of K or E at position 66 by I, L, M, F or V, the substitution of S at position 213 by P, and one of the following mutations: (1) the substitution of D at position 486 by Q, L or I, and (2) the substitution of V at position 487 by T.

In some embodiments, the mutations of the mutant encoded by the above nucleic acid comprise or are one group of the following (1) to (11): (1)I217P, E66I and D486Q; (2)I217P, N67I and E487Q; (3)I217P, E66I and E487T; (4)S215P, N67I and E487Q; (5)S213P, N67I and E487Q; (6)S213P, E661 and E487T; (7)S213P, E661 and D486Q; (8)N216P, N67I and E487Q; (9)N216P, E661 and E487T; (10)N216P, E66I and D486Q; and (11)S213P and E66I.

In some embodiments, the mutant is selected from one of the following (1) to (26): (1) a mutant comprising the amino acid sequence as shown in SEQ ID NO: 31 or 32; (2) a mutant comprising mutations N216P, E66I and D486Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 31 or 32; (3) a mutant comprising the amino acid sequence as shown in SEQ ID NO: 33 or 34; (4) a mutant comprising mutations N216P, E66I and E487T and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 33 or 34; (5) a mutant comprising the amino acid sequence as shown in SEQ ID NO: 35 or 36; (6) a mutant comprising mutations N216P, N67I and E487Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 35 or 36; (7) a mutant comprising the amino acid sequence as shown in SEQ ID NO: 37 or 38; (8) a mutant comprising mutations S213P, E66I and D486Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 37 or 38; (9) a mutant comprising the amino acid sequence as shown in SEQ ID NO: 39 or 40; (10) a mutant comprising mutations S213P, E66I and E487T and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 39 or 40; (11) a mutant comprising the amino acid sequence as shown in SEQ ID NO: 41 or 42; (12) a mutant comprising mutation S213P, N67I and E487Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 41 or 42 (13) a mutant comprising the amino acid sequence as shown in SEQ ID NO: 43 or 44; (14) a mutant comprising mutations S215P, N67I and E487Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 43 or 44; (15) a mutant comprising the amino acid sequence as shown in SEQ ID NO: 45 or 46; (16) a mutant comprising mutations I217P, E661 and E487T and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 45 or 46; (17) a mutant comprising the amino acid sequence as shown in SEQ ID NO: 47 or 48; (18) a mutant comprising mutations I217P, N67I and E487Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 47 or 48; (19) a mutant comprising the amino acid sequence as shown in SEQ ID NO: 49 or 50; (20) a mutant comprising mutations I217P, E66I and D486Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 49 or 50; (21) a mutant comprising the amino acid sequence as shown in SEQ ID NO: 51 or 52; (22) a mutant comprising mutations S213P, E66I and E487T and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 51 or 52; (23) a mutant comprising the amino acid sequence as shown in SEQ ID NO: 53 or 54; (24) a mutant comprising mutations S213P, E661 and D486Q and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 53 or 54; (25) a mutant comprising the amino acid sequence as shown in SEQ ID NO: 59 or 60; (26) a mutant comprising mutations S213P and E66I and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 59 or 60.

In some embodiments, the mutant encoded by the nucleic acid of any of the above embodiments further comprises disulfide bond mutations. In some embodiments, the disulfide bond mutations are selected from one or more of the follows: S46C and T311C, A47C and I309C, L48C and V308C, S55C and L188C, V56C and T189C, V56C and V300C, I57C and S190C, I57C and Y299C, T58C and K191C, I59C and L193C, I59C and L297C, E60C and D194C, P101C and I152C, A102C and I148C, A102C and I152C, T103C and I146C, T103C and I148C, N104C and G145C, N104C and S146C, N105C and G145C, N105C and S146C, N105C and A147C, V144C and V406C, and S146C and N460C. In some embodiments, the disulfide bond mutations are selected from one or more of the follows: A47C and I309C, I57C and Y299C, P101C and I152C, A102C and I148C, A102C and I152C, T103C and I146C,N104C and G145C, N104C and S146C, N105C and G145C, N105C and S146C, and N105C and A147C. In some embodiments, the disulfide bond mutations are selected from one or more of the follows: P101C and I152C, A102C and I148C, A102C and I152C, N104C and S146C, and N105C and A147C.

In some embodiments, the mutations of the mutant encoded by the above nucleic acid comprise or are one group of the following (1) to (2): (1) A102C, I152C, S213P and E66I; and (2) P101C, I152C, S213P and E66I.

In some embodiments, the mutant is selected from one of the following (1) to (4): (1) a mutant comprising the amino acid sequence as shown in SEQ ID NO: 55 or 56; (2) a mutant comprising mutations A102C, I152C, S213P and E66I and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 55 or 56; (3) a mutant comprising the amino acid sequence as shown in SEQ ID NO: 57 or 58; and (4) a mutant comprising mutations P101C, I152C, S213P and E66I and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 57 or 58.

In some embodiments, the amino acid sequence of the mutant encoded by the above nucleic acid is at least 80%, 85%, 90%, 91%, 92%, 93%, 94%, 95%, 95.5%, 96%, 96.5%, 97%, 97.5%, 98%, 98.5%, 99% or 99.5% identical to the amino acid sequence of the F protein of the corresponding wild-type RSV. Please refer to the corresponding description of nucleic acid 1 for specific types of wild-type RSV.

In some embodiments, the mutant encoded by the above nucleic acid comprises an F1 polypeptide and an F2 polypeptide. Please refer to corresponding descriptions of the nucleic acid 1 for specific descriptions of the F1 polypeptide and the F2 polypeptide.

In some embodiments, the mutant encoded by the above nucleic acid also comprises one or more of the follows: a pep27 polypeptide, a transmembrane domain (TM) of an RSV F protein (e.g., a transmembrane domain of a wild-type RSV F protein), a signal peptide, and a trimerization domain (e.g., a foldon). Trimerization domains are used to promote the formation of trimers of 3 F1/F2 heterodimers. The trimerization domain is not particularly limited, and may be, for example, a GCN4 leucine zipper, a trimerization motif from a pulmonary surfactant protein, a phage T4 fibritin foldon, etc. In some embodiments, the foldon is at the C-terminus of the F1 polypeptide. In an optional specific example, the amino acid sequence of the foldon is: GYIPEAPRDGQAYVRKDGEWVLLSTFL (SEQ ID NO: 212). In some embodiments, the mutant encoded by the above nucleic acid comprises a pep27 polypeptide, a signal peptide, and a trimerization domain. In other embodiments, the mutant encoded by the above nucleic acid comprises a pep27 polypeptide, a signal peptide, and a transmembrane domain.

In addition, the nucleic acid further comprises one or more of the followis: a polynucleotide encoding a protease cleavage site (e.g., a thrombin cleavage site (LVPRGS, SEQ ID NO: 213)), a polynucleotide encoding a protein tag (e.g., a 6× His-tag (HHHHHH, SEQ ID NO: 214) and a streptomycin tag II (WSHPGFEK, SEQ ID NO: 215)), a polynucleotide encoding a linker sequence (e.g., GG and GS)). The polypeptides encoded by the above sequences are not necessary for the function of the RSV F protein (e.g., inducing an immune response). Such sequences are well known to those skilled in the art, and it can be understood that these sequences are not limited to the above.

In some embodiments, the above nucleic acid is an isolated nucleic acid.

In some embodiments, the above nucleic acid is a non-naturally occurring nucleic acid.

In some embodiments, the above nucleic acid is a codon-optimized polynucleotide. In some embodiments, the codon-optimized nucleic acid is at least 50%, 55%, 60%, 65%, 70%, 75%, 80%, 85%, 90%, 95%, or 99% identical to the nucleic acid prior to the codon-optimization.

In some embodiments, the above nucleic acid is RNA.

In some embodiments, the above nucleic acid is RNA comprising an open reading frame (ORF).

In some embodiments, the above nucleic acid is RNA, and the RNA is mRNA.

In some embodiments, the above nucleic acid is non-self-replicating mRNA.In some embodiments, the nucleic acid further comprises at least one of a 5'-cap structure, a 5'-UTR, a 3'-UTR, and a poly(A) tail. In some embodiments, please refer to the related descriptions of the nucleic acid 1 for the 5'-cap structure, the 5' -UTR, the 3'-UTR and the poly(A) tail in the above nucleic acid, which is not repeated here.

In some embodiments, the above nucleic acid does not contain a modified nucleotide.

In some embodiments, the above nucleic acid contains a modified nucleotide. In some embodiments, the above nucleic acid contains a modified nucleoside. In some embodiments, the modified nucleoside comprised in the above nucleic acid comprises at least one of a modified uridine, a modified cytidine, a modified adenosine, and a modified guanosine. , Please refer to the related descriptions of the modified nucleotide, the modified uridine, the modified cytidine, the modified adenosine and the modified guanosine of the nucleic acid 1 for the modified nucleotide, the modified uridine, the modified cytidine, the modified adenosine and the modified guanosine of the nucleic acid, which are not repeated here.

In some embodiments, the above nucleic acid comprises RNA corresponding to a polynucleotide having a nucleotide sequence as shown in any one of SEQ ID NOs: 135-164, and the uridines in the nucleic acid are all replaced with N1-methylpseudouridine.

In some embodiments, the nucleic acid is DNA.

In some embodiments, the DNA can be transcribed into RNA in vitro.

In some embodiments, the nucleic acid comprises a polynucleotide having a nucleotide sequence as shown in any one of SEQ ID NOs: 135-164.

In some embodiments, the nucleic acid comprises a polynucleotide encoding one mutant of RSV F protein.

In other embodiments, the nucleic acid comprises a polynucleotide encoding a plurality of mutants of RSV F protein. At this moment, the coding strands of the plurality of mutants of RSV F protein are located on the same nucleic acid strand, and the respective polynucleotides encoding the plurality of mutants of RSV F protein are directly linked or indirectly linked through a linking element (e.g., a polynucleotide encoding a 2A peptide or a polynucleotide encoding a flexible linker peptide). A flexible linker peptide comprises "GS" linker peptides consisting of Gly and Ser residues, e.g., (Gly-Gly-Gly-Gly-Ser)n, the length of the "GS" linker peptide can be changed by adjusting the value of n (number of repeats), allowing for optimal separation or interaction between two linked proteins.

In some embodiments, the above nucleic acid comprises a polynucleotide encoding a protein or polypeptide other than RSV F protein. It can be understood that other proteins or polypeptides in addition to the mutant of RSV F protein refer to proteins or polypeptides with biological significance (such as immunogenicity), such as other proteins or polypeptides of RSV, or mutants thereof (such as the G protein of RSV or mutants thereof), or proteins or polypeptides of other viruses, or mutants thereof (such as the HA protein of influenza virus or mutants thereof, or the S protein of SARS-CoV-2 or mutants thereof).

In addition, similarly, in the above nucleic acids, both the polynucleotide encoding one mutant of RSV F protein and the polynucleotides encoding the plurality of mutants of RSV F protein can have a plurality of (e.g., two or three) repeat units on the same strand.

In some embodiments, the RSV F protein produced by the above nucleic acid is a pre-F protein.

In some embodiments, the RSV F protein produced by the above nucleic acid can bind to at least one of the following antibodies: AM14, D25, AM22, and CR9501. In some embodiments, the RSV F protein produced by the above nucleic acid can bind to the following antibodies: AM14, D25, AM22, and CR9501.

In some embodiments, as compared to Post-F protein, the RSV F protein produced by the above nucleic acid has a higher affinity to antibodies AM14, D25, AM22, or CR9501. In some embodiments, the affinity of the RSV F protein produced by the above nucleic acid to antibodies AM14, D25, AM22, or CR9501 is at least 1.1 times, 1.3 times, 1.5 times, 2 times, 5 times, 10 times, 15 times, 20 times, 25 times, 30 times, 35 times, 40 times, 45 times, 50 times, 55 times, 60 times, 65 times, 70 times, 80 times, 90 times, 100 times, 110 times, 120 times, 130 times, 140 times, 150 times, 160 times, 170 times, 180 times, 190 times, 200 times, 250 times, 300 times, 350 times, 400 times, 450 times, or 500 times greater than the affinity of Post-F protein to antibodies AM14, D25, AM22, or CR9501.

In some embodiments, as compared with Post-F protein, the RSV F protein produced by the above nucleic acid has a lower affinity to antibody clone 131-2A. In some embodiments, the affinity of Post-F protein to the antibody clone 131-2A is at least 1.1 times, 1.3 times, 1.5 times, 2 times, 5 times, 10 times, 15 times, 20 times, 25 times, 30 times, 35 times, 40 times, 45 times, 50 times, 55 times, 60 times, 65 times, 70 times, 80 times, 90 times, 100 times, 110 times, 120 times, 130 times, 140 times, 150 times, 160 times, 170 times, 180 times, 190 times, 200 times, 250 times, 300 times, 350 times, 400 times, 450 times or 500 times greater than the affinity of the RSV F protein produced by the above nucleic acid to the antibody clone 131-2A..

In some embodiments, the above nucleic acid can induce the production of neutralizing antibodies against subtype A hRSV.

In some embodiments, the above nucleic acid can induce the production of neutralizing antibodies against subtype B hRSV.

In some embodiments, the above nucleic acid can induce the production of neutralizing antibodies against subtypes A and B hRSV.

In some embodiments, the above nucleic acid is any mRNA in Table 1. The mRNA in Table 1 is the mRNAs encoding the mutants of RSV F protein comprising a Cap1 type cap structure, 5'-UTR corresponding to the sequence shown in SEQ ID NO: 209, 3'-UTR corresponding to the sequence shown in SEQ ID NO: 210, a poly(A) tail corresponding to the sequence shown in SEQ ID NO: 211, and an F protein coding region corresponding to one sequence of SEQ ID NOs: 105-208, wherein the F protein coding region is RNA, and the uridines in all of the mRNAs in Table 1 are replaced with N1-methylpseudouridine. For example, the mRNA shown in No. 832 comprises a Cap1 cap structure, a 5'-UTR corresponding to the sequence shown in SEQ ID NO: 209, a 3'-UTR corresponding to the sequence shown in SEQ ID NO: 210, a poly(A) tail corresponding to the sequence shown in SEQ ID NO: 211, and an F protein coding region corresponding to the sequence shown in SEQ ID NO: 187, and all uridines in the mRNA shown in No. 832 are replaced with N1-methylpseudouridine. In addition, in Table 1, all mRNAs in G1-G25 and G51 groups contain a polynucleotide encoding a foldon derived from T4 phage fibrin as shown in SEQ ID NO: 212 at the C-terminus. For example, positions 1552-1632 of SEQ ID NO: 105 of G1 group encode the foldon derived from T4 phage fibrin shown in SEQ ID NO: 212 (i.e., positions 518-544 of SEQ ID NO: 1).

**Table 1**

| Groups | mRNA number | Mutations contained in the mutant of RSV F protein corresponding to the mRNA | Amino acid sequence of the mutant of RSV F protein (SEQ ID NO: ) | DNA sequence corresponding to the coding region for RSV F protein (SEQ ID NO: ) |
|---|---|---|---|---|
| G1 | 573 | A47C, I309C, T54H, V144C, V406C and D486S | 1 | 105 |
| | 567 | A47C, I309C, T54H, V144C, V406C and D486S | 2 | 106 |
| G2 | 574 | A47C, I309C, T54H and D486S | 3 | 107 |
| | 568 | A47C, I309C, T54H and D486S | 4 | 108 |
| G3 | 575 | L48C, V308C, T54H, V144C, V406C and D486S | 5 | 109 |
| | 569 | L48C, V308C, T54H, V144C, V406C and D486S | 6 | 110 |
| G4 | 576 | L48C, V308C, T54H and D486S | 7 | 111 |
| | 570 | L48C, V308C, T54H and D486S | 8 | 112 |
| G5 | 577 | S46C, T311C, T54H, V144C, V406C and D486S | 9 | 113 |
| | 571 | S46C, T311C, T54H, V144C, V406C and D486S | 10 | 114 |
| G6 | 578 | S46C, T311C, T54H and D486S | 11 | 115 |
| | 572 | S46C, T311C, T54H and D486S | 12 | 116 |
| G7 | 590 | A102C, I148C, T54H, S190I, V296I and D486S | 13 | 117 |
| | 581 | A102C, I148C, T54H, S190I, V296I and D486S | 14 | 118 |
| G8 | 591 | A102C, I152C, T54H, S190I, V296I and D486S | 15 | 119 |
| | 582 | A102C, I152C, T54H, S190I, V296I and D486S | 16 | 120 |
| G9 | 592 | N104C, G145C, T54H, S190I, V296I and D486S | 17 | 121 |
| | 583 | N104C, G145C, T54H, S190I, V296I and D486S | 18 | 122 |
| G10 | 593 | N104C, S146C, T54H, S190I, V296I and D486S | 19 | 123 |
| | 584 | N104C, S146C, T54H, S190I, V296I and D486S | 20 | 124 |
| G11 | 594 | N105C, A147C, T54H, S190I, V296I and D486S | 21 | 125 |
| | 585 | N105C, A147C, T54H, S190I, V296I and D486S | 22 | 126 |
| G12 | 595 | N105C, G145C, T54H, S190I, V296I and D486S | 23 | 127 |
| | 586 | N105C, G145C, T54H, S190I, V296I and D486S | 24 | 128 |
| G13 | 596 | N105C, S146C, T54H, S190I, V296I and D486S | 25 | 129 |
| | 587 | N105C, S146C, T54H, S190I, V296I and D486S | 26 | 130 |
| G14 | 597 | P101C, I152C, T54H, S190I, V296I and D486S | 27 | 131 |
| | 588 | P101C, I152C, T54H, S190I, V296I and D486S | 28 | 132 |
| G15 | 598 | T103C, S146C, T54H, S190I, V296I and D486S | 29 | 133 |
| | 589 | T103C, S146C, T54H, S190I, V296I and D486S | 30 | 134 |
| G16 | 672 | N216P, E66I and D486Q | 31 | 135 |
| | 671 | N216P, E66I and D486Q | 32 | 136 |
| G17 | 674 | N216P, E661 and E487T | 33 | 137 |
| | 673 | N216P, E661 and E487T | 34 | 138 |
| G18 | 676 | N216P, N67I and E487Q | 35 | 139 |
| | 675 | N216P, N67I and E487Q | 36 | 140 |
| G19 | 678 | S213P, E661 and D486Q | 37 | 141 |
| | 677 | S213P, E661 and D486Q | 38 | 142 |
| G20 | 680 | S213P, E661 and E487T | 39 | 143 |
| | 679 | S213P, E661 and E487T | 40 | 144 |
| G21 | 682 | S213P, N67I and E487Q | 41 | 145 |
| | 681 | S213P, N67I and E487Q | 42 | 146 |
| G22 | 684 | S215P, N67I and E487Q | 43 | 147 |
| | 683 | S215P, N67I and E487Q | 44 | 148 |
| G23 | 685 | I217P, E66I and E487T | 45 | 149 |
| | 686 | I217P, E66I and E487T | 46 | 150 |
| G24 | 688 | I217P, N67I and E487Q | 47 | 151 |
| | 687 | I217P, N67I and E487Q | 48 | 152 |
| G25 | 690 | I217P, E661 and D486Q | 49 | 153 |
| | 689 | I217P, E661 and D486Q | 50 | 154 |
| G26 | 879 | S213P, E661 and E487T | 51 | 155 |
| | 878 | S213P, E661 and E487T | 52 | 156 |
| G27 | 877 | S213P, E661 and D486Q | 53 | 157 |
| | 876 | S213P, E661 and D486Q | 54 | 158 |
| G28 | 901 | A102C, I152C, S213P and E661 | 55 | 159 |
| | 898 | A102C, I152C, S213P and E661 | 56 | 160 |
| G29 | 899 | P101C, I152C, S213P and E661 | 57 | 161 |
| | 900 | P101C, I152C, S213P and E661 | 58 | 162 |
| G30 | 897 | S213P and E661 | 59 | 163 |
| | 896 | S213P and E661 | 60 | 164 |
| G31 | 843 | A102C, I152C, T54V, S190M and V296L | 61 | 165 |
| | 971 | A102C, I152C, T54V, S190M and V296L | 62 | 166 |
| G32 | 842 | A102C, I152C, T54F, S190M and V296L | 63 | 167 |
| | 968 | A102C, I152C, T54F, S190M and V296L | 64 | 168 |
| G33 | 841 | A102C, I152C, T54C, S190M and V296L | 65 | 169 |
| | 965 | A102C, I152C, T54C, S190M and V296L | 66 | 170 |
| G34 | 840 | A102C, I152C, T54A, S190M and V296L | 67 | 171 |
| | 962 | A102C, I152C, T54A, S190M and V296L | 68 | 172 |
| G35 | 839 | A102C, I152C, T54V, S190F and V296L | 69 | 173 |
| | 969 | A102C, I152C, T54V, S190F and V296L | 70 | 174 |
| G36 | 838 | A102C, I152C, T54F, S190F and V296L | 71 | 175 |
| | 966 | A102C, I152C, T54F, S190F and V296L | 72 | 176 |
| G37 | 837 | A102C, I152C, T54C, S190F and V296L | 73 | 177 |
| | 963 | A102C, I152C, T54C, S190F and V296L | 74 | 178 |
| G38 | 836 | A102C, I152C, T54A, S190F and V296L | 75 | 179 |
| | 960 | A102C, I152C, T54A, S190F and V296L | 76 | 180 |
| G39 | 835 | P101C, I152C, T54V, S190M and V296L | 77 | 181 |
| | 979 | P101C, I152C, T54V, S190M and V296L | 78 | 182 |
| G40 | 834 | P101C, I152C, T54F, S190M and V296L | 79 | 183 |
| | 977 | P101C, I152C, T54F, S190M and V296L | 80 | 184 |
| G41 | 833 | P101C, I152C, T54C, S190M and V296L | 81 | 185 |
| | 975 | P101C, I152C, T54C, S190M and V296L | 82 | 186 |
| G42 | 832 | P101C, I152C, T54A, S190M and V296L | 83 | 187 |
| | 973 | P101C, I152C, T54A, S190M and V296L | 84 | 188 |
| G43 | 831 | P101C, I152C, T54V, S190F and V296L | 85 | 189 |
| | 978 | P101C, I152C, T54V, S190F and V296L | 86 | 190 |
| G44 | 830 | P101C, I152C, T54F, S190F and V296L | 87 | 191 |
| | 976 | P101C, I152C, T54F, S190F and V296L | 88 | 192 |
| G45 | 829 | P101C, I152C, T54C, S190F and V296L | 89 | 193 |
| | 974 | P101C, I152C, T54C, S190F and V296L | 90 | 194 |
| G46 | 828 | P101C, I152C, T54A, S190F and V296L | 91 | 195 |
| | 972 | P101C, I152C, T54A, S190F and V296L | 92 | 196 |
| G47 | 984 | A102C, I152C, T54V, S190I and V296L | 93 | 197 |
| | 970 | A102C, I152C, T54V, S190I and V296L | 94 | 198 |
| G48 | 983 | A102C, I152C, T54F, S190I and V296L | 95 | 199 |
| | 967 | A102C, I152C, T54F, S190I and V296L | 96 | 200 |
| G49 | 982 | A102C, I152C, T54C, S1901 and V296L | 97 | 201 |
| | 964 | A102C, I152C, T54C, S190I and V296L | 98 | 202 |
| G50 | 981 | A102C, I152C, T54A, S190I and V296L | 99 | 203 |
| | 961 | A102C, I152C, T54A, S190I and V296L | 100 | 204 |
| G51 | 986 | T103C, I148C, T54H, S190I, V296I and D486S | 101 | 205 |
| | 985 | T103C, I148C, T54H, S190I, V296I and D486S | 102 | 206 |
| G52 | 845 | T103C, I148C, T54H, S190I, V296I and D486S | 103 | 207 |
| | 980 | T103C, I148C, T54H, S190I, V296I and D486S | 104 | 208 |
| G53 | 1119 | A149C, Y458C, S155C, S290C, S190F and V207L | 218 | 221 |
| G54 | 1902 | T103C, I148C, S190I, V296I and D486S | 219 | 222 |
| | 1903 | T103C, I148C, S190I, V296I and D486S | 220 | 223 |
| G55 | 739 | A102C, I148C, T54H, S190I, V296I and D486S | 224 | 259 |
| | 729 | A102C, I148C, T54H, S190I, V296I and D486S | 225 | 260 |
| G56 | 740 | A102C, I152C, T54H, S190I, V296I and D486S | 226 | 261 |
| | 730 | A102C, I152C, T54H, S190I, V296I and D486S | 227 | 262 |
| G57 | 741 | N104C, G145C, T54H, S190I, V296I and D486S | 228 | 263 |
| | 731 | N104C, G145C, T54H, S190I, V296I and D486S | 229 | 264 |
| G58 | 742 | N104C, S146C, T54H, S190I, V296I and D486S | 230 | 265 |
| | 732 | N104C, S146C, T54H, S190I, V296I and D486S | 231 | 266 |
| G59 | 743 | N105C, A147C, T54H, S190I, V296I and D486S | 232 | 267 |
| | 733 | N105C, A147C, T54H, S190I, V296I and D486S | 233 | 268 |
| G60 | 744 | N105C, G145C, T54H, S190I, V296I and D486S | 234 | 269 |
| | 734 | N105C, G145C, T54H, S190I, V296I and D486S | 235 | 270 |
| G61 | 745 | N105C, S146C, T54H, S190I, V296I and D486S | 236 | 271 |
| | 735 | N105C, S146C, T54H, S190I, V296I and D486S | 237 | 272 |
| G62 | 746 | P101C, I152C, T54H, S190I, V296I and D486S | 238 | 273 |
| | 736 | P101C, I152C, T54H, S190I, V296I and D486S | 239 | 274 |
| G63 | 747 | T103C, S146C, T54H, S190I, V296I and D486S | 240 | 275 |
| | 737 | T103C, S146C, T54H, S190I, V296I and D486S | 241 | 276 |
| G64 | 1512 | A102C, A153C, T54H, S190I, V296I and D486S | 242 | 277 |
| | 1514 | A102C, A153C, T54H, S190I, V296I and D486S | 243 | 278 |
| G65 | 1513 | A102C, S150C, T54H, S190I, V296I and D486S | 244 | 279 |
| | 1515 | A102C, S150C, T54H, S190I, V296I and D486S | 245 | 280 |
| G66 | 1998 | T54A | 246 | 281 |
| G67 | 2000 | P101C and I152C | 247 | 282 |
| G68 | 2001 | T103C and I148C | 248 | 283 |
| G69 | 2002 | S190M | 249 | 284 |
| G70 | 2003 | S190I | 250 | 285 |
| G71 | 2004 | V296L | 251 | 286 |
| G72 | 2006 | T54A and S190M | 252 | 287 |
| G73 | 2007 | T54A and V296L | 253 | 288 |
| G74 | 2008 | S190M and V296L | 254 | 289 |
| G75 | 2009 | T54A, S190M and V296L | 255 | 290 |
| G76 | 2010 | A102C and I152C | 256 | 291 |
| G77 | 2011 | A102C and A153C | 257 | 292 |
| G78 | 2012 | A102C and S150C | 258 | 293 |

### III. Genetic Engineering Vector, Preparation Method of Nucleic Acid, Host Cell and Protein Mutant

The present disclosure also provides a genetic engineering vector comprising the nucleic acid of any of the above embodiments, or a polynucleotide capable of being transcribed into the nucleic acid of any of the above embodiments. In some embodiments, the genetic engineering vector is a plasmid, cosmid, virus, phage, or another vector conventionally used in genetic engineering. In an optional specific example, the genetic engineering vector is a plasmid. In some embodiments, the genetic engineering vector further comprises at least one or more of the follows: origin of replication (ORI), a marker gene or fragment thereof, a reporter gene or fragment thereof, and a restriction site allowing insertion of a DNA element. Restriction sites in the form of multiple cloning sites (MCS) are preferred.

In some embodiments, the genetic engineering vector is an expression vector. In some embodiments, the genetic engineering vector comprises a promoter, a 5'-UTR, a coding region of the mutant of RSV F protein of any of the above embodiments (referred to as "mutant coding region"), a 3'-UTR, and a poly(A) tail, wherein the poly(A) tail, the promoter, the 5'-UTR, the mutant coding region, and the 3'-UTR are operably linked to each other.

In other embodiments, the genetic engineering vector is a cloning vector. It can be understood that the above nucleic acid contained in the genetic engineering vector does not contain a 5'-cap structure.

The present disclosure also provides a method of preparing the nucleic acid of any one of the above embodiments, which comprises a step of introducing (e.g., introducing in the form of a plasmid) the genetic engineering vector of any one of the above embodiments into a host cell (e.g., E. coli) and then culturing the host cell containing the nucleic acid.

In addition, the present disclosure also provides another preparation method of the above nucleic acid, which includes a step of preparing the nucleic acid according to the nucleotide sequence of the nucleic acid of any one of the above embodiments by chemical synthesis. It can be understood that the chemical synthesis method may be a method known in the art, such as a solid phase phosphoramidite method.

It can be understood that the preparation method of the nucleic acid of any of the above embodiments is not limited to the above, and may also be other methods.

In addition, the present disclosure also provides a host cell comprising the nucleic acid of any of the above embodiments or the genetic engineering vector of any of the above embodiments.

In some embodiments, the above host cell is an isolated cell.

In some embodiments, the host cell is used to store and/or amplify the above nucleic acid.

In some embodiments, the host cell is a bacterial cell. Bacterial host cells include E. coli cells which is well known to those skilled in the art.

The host cell of the present disclosure can be prepared by transforming the genetic engineering vector of any of the above embodiments into a competent host cell. Competent host cells are cells that have the ability to uptake free extracellular genetic material (e.g., DNA plasmid) independent of sequence. A variety of bacterial cells known to those skilled in the art are naturally capable of uptaking exogenous DNA from the environment and thus can serve as bacterial host cells according to the present disclosure. In addition, it is known to those skilled in the art that competent bacterial host cells can be obtained from natural non-competent bacterial cells using, for example, electroporation or chemicals (e.g., treatment with calcium ions with high temperature exposure). Upon uptake, the exogenous DNA is preferably neither degraded nor integrated in the genome of the bacterial host cell.

In addition, the present disclosure also provides a mutant of RSV F protein, which is encoded by the nucleic acid of any of the above embodiments.

In some embodiments, the mutant of RSV F protein is a single molecule or a multi-molecule complex (e.g., trimer).

In addition, the present disclosure also provides an RSV immunogen comprising a mutant of RSV F protein encoded by the nucleic acid of any of the above embodiments.

In some embodiments, the above RSV immunogen is a trimer. In some embodiments, the mutations of the monomers of the trimer are identical. In other embodiments, the mutations of the three monomers of the trimer are partially the same or completely different.

In addition, the present disclosure also provides a method of preparing a mutant of RSVF protein, comprising: transcribing a nucleic acid comprising a polynucleotide encoding the mutant of RSV F protein into mRNA; and translating the transcribed mRNA into a polypeptide or protein.

In some embodiments, the method of preparing the mutant of RSV F protein is performed completely or partially in vitro.

### IV. Methods for Preparing RNA and RNA

The present disclosure also provides a method of preparing RNA, comprising a step of transcription using the genetic engineering vector of any of the above embodiments of the present disclosure.

In some embodiments, the method of preparing RNA is an in vitro method. In some embodiments, the method of preparing RNA comprises contacting the genetic engineering vector (e.g., plasmid) of any of the above embodiments with an RNA polymerase. In some embodiments, the method of preparing RNA further comprises the step of linearizing the genetic engineering vector (e.g., plasmid). In some embodiments, prior to the linearization, the superhelical rate of the genetic engineering vector (e.g., plasmid) is at least about 85% (e.g., 85%, 86%, 87%, 88%, 89%, 90%, 91%, 92%, 93%, etc.). In some embodiments, the method of preparing RNA further comprises a step of purifying the linearized genetic engineering vector. In some embodiments, the method of preparing RNA further comprises a step of purifying RNA.

The present disclosure further provides another method of preparing RNA, comprising a step of preparing the RNA according to the nucleotide sequence of the RNA or DNA corresponding to any of the above embodiments by chemical synthesis. It can be understood that the chemical synthesis method may be a method known in the art, such as a solid phase phosphoramidite method.

In some embodiments, the nucleic acid is RNA.

In some embodiments, any of the above methods of preparing RNA further comprises a step of capping and optionally purifying the capping product. In some embodiments, the cap is a Cap1 cap. The Cap1 cap structure is as follows:
cap G¹G² = m⁷G-5'-ppp-5'-Gm2'-3'-p-[m7 = 7-CH₃; m2' = 2'-O-CH₃; -ppp- = -PO₂H-O-PO₂H-O-PO₂H)-; -p- = -PO₂H-].

The capping reaction is as follows:
pppNl(p)Nx-OH(3') → ppN1(pN)x-OH(3') + Pi
ppN1(pN)x-OH(3') + GTP → G(5')ppp(5')N1(pN)x-OH(3') + PPi
G(5')ppp(5')N1(pN)x-OH(3') + AdoMet → m7G(5')ppp(5')N1(pN)x-OH(3') + AdoHyc
m7GpppN1(pN)x-OH(3') + AdoMet → m7Gppp[m2'-O]N1(pN)x-OH(3') + AdoHyc.

In other embodiments, the method of preparing RNA is partially an in vitro method. The method of preparing RNA, at this moment, comprises the following steps: preparing the genetic engineering vector of any of the above embodiments in vitro; and introducing the genetic engineering vector into an organism (e.g., in the form of a plasmid). In some embodiments, the genetic engineering vector is encapsulated in a delivery carrier. At this moment, the genetic engineering vector is delivered into the organism via the delivery carrier.

In some embodiments, the RNA prepared in the method of preparing RNA according to any one of the above embodiments comprises a modified nucleoside or a modified nucleotide. Correspondingly, the raw material for preparing the RNA comprises one or more modified nucleosides or nucleotides. It can be understood that the amount and type of modified nucleosides or nucleotides correspond to the RNA to be prepared.

In addition, the present disclosure further provides an RNA prepared by the method of preparing an RNA according to any one of the above embodiments. In some embodiments, the RNA is mRNA.

### V. Nucleic Acid Composition

The present disclosure also provides a nucleic acid composition comprising a first nucleic acid or a first genetic engineering vector, the first nucleic acid is the nucleic acid of any of the above embodiments, and the first genetic engineering vector is the genetic engineering vector of any of the above embodiments.

In some embodiments, the nucleic acid is RNA. In some embodiments, the nucleic acid is mRNA.

In some embodiments, the nucleic acid composition comprises a first nucleic acid or a first genetic engineering vector, the first nucleic acid is a nucleic acid comprising a polynucleotide encoding any one or more of the plurality of mutants of RSV F protein, and the first genetic engineering vector is a genetic engineering vector comprising a polynucleotide encoding any one or more of the plurality of mutants of RSV F protein.

In some embodiments, the nucleic acid composition comprises a first nucleic acid or a first genetic engineering vector, the first nucleic acid is a nucleic acid comprising a polynucleotide encoding one of the plurality of mutants of RSV F protein, and the first genetic engineering vector is a genetic engineering vector comprising a polynucleotide encoding one of the plurality of mutants of RSV F protein.

In other embodiments, the nucleic acid composition comprises a first nucleic acid or a first genetic engineering vector, the first nucleic acid is a nucleic acid comprising polynucleotides encoding a plurality of mutants of the plurality of mutants of RSV F protein, and the first genetic engineering vector is a genetic engineering vector comprising polynucleotides encoding a plurality of mutants of the plurality of mutants of RSV F protein, wherein the polynucleotides encoding the plurality of mutants of RSV F protein are located on the same nucleic acid strand.

In some embodiments, the nucleic acid composition comprises a plurality of first nucleic acids or a plurality of first genetic engineering vectors, the plurality of first nucleic acids are independent of each other or the plurality of first genetic engineering vectors are independent of each other, the plurality of first nucleic acids are used for encoding a plurality of mutants of RSV F protein, and the plurality of first genetic engineering vectors are used for cloning or expressing a plurality of polynucleotides encoding the RSV F protein. It should be noted that "independent of each other" herein refers to that there is not a linking element to link the plurality of first nucleic acids, or there is not a linking element to link the plurality of first genetic engineering vectors. At this moment, the respective polynucleotides encoding the plurality of mutants of RSV F protein are not on the same nucleic acid strand.

In some embodiments, the plurality of mutants of RSVF proteins corresponding to the plurality of first nucleic acids are RSV F proteins derived from different subtype strains and having the same mutation, RSV F proteins derived from different subtype strains and having different mutations, and/or RSV F proteins derived from the same subtype strain and having different mutations.

For example, the plurality of first nucleic acids are nucleic acids (or referred to as first first nucleic acids) comprising polynucleotides encoding RSV F proteins derived from the strain Ontario and having mutations P101C, I152C and S190M, and nucleic acids (or referred to as second first nucleic acids) comprising polynucleotides encoding RSV F proteins derived from the strain Buenos Aires and having mutations P101C, I152C and S190M. In other words, the nucleic acid composition comprises two first nucleic acids, the first first nucleic acid is a nucleic acid comprising a polynucleotide encoding an RSV F protein derived from the strain Ontario and having mutations P101C, I152C, and S190M, and the second first nucleic acid is a nucleic acid comprising a polynucleotide encoding an RSV F protein derived from the strain Buenos Aires and having mutations P101C, I152C, and S190M. In some embodiments, the nucleic acid composition comprises two first nucleic acids, the first first nucleic acid comprises a polynucleotide encoding an RSV F protein derived from the Ontario strain having mutations P101C, 1152C, T54A, S190M, and V296L, and the second first nucleic acid comprises a polynucleotide encoding an RSV F protein derived from the Buenos Aires strain having mutations P101C, I152C, T54A, S190M, and V296L.

In some embodiments, the nucleic acid composition comprises two first nucleic acids, the first first nucleic acid comprises a polynucleotide encoding a mutant of RSV F protein comprising mutations P101C, I152C, T54A, S190M, and V296L and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence shown in SEQ ID NO: 83; the second first nucleic acid comprises a polynucleotide encoding a mutant of RSV F protein comprising mutations P101C, I152C, T54A, S190M, and V296L and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence shown in SEQ ID NO: 84.

In some embodiments, the nucleic acid composition comprises two first nucleic acids, the first first nucleic acid comprises a polynucleotide encoding a mutant of RSV F protein comprising the amino acid sequence as shown in SEQ ID NO: 83; the second first nucleic acid comprises a polynucleotide encoding a mutant of RSV F protein comprising the amino acid sequence shown in SEQ ID NO: 84.

In some embodiments, the nucleic acid composition comprises two first nucleic acids, the first first nucleic acid comprises a DNA encoding an RSV F protein with the amino acid sequence as shown in SEQ ID NO: 83 and at least 85%, 88%, 90%, 91%, 92%, 93%, 94%, 95%, 95.5%, 96%, 96.5%, 97%, 97.5%, 98%, 98.5%, 99% or 99.5% identical to the DNA as shown in SEQ ID NO: 187, or the corresponding RNA thereof; the second first nucleic acid comprises a DNA encoding an RSV F protein with the amino acid sequence as shown in SEQ ID NO: 84 and at least 85%, 88%, 90%, 91%, 92%, 93%, 94%, 95%, 95.5%, 96%, 96.5%, 97%, 97.5%, 98%, 98.5%, 99% or 99.5% identical to the DNA as shown in SEQ ID NO: 188, or the corresponding RNA thereof.

In some embodiments, the nucleic acid composition comprises two first nucleic acids, the first first nucleic acid comprises a DNA as shown in SEQ ID NO: 187, or the corresponding RNA thereof; the second first nucleic acid comprises a DNA as shown in SEQ ID NO: 188, or the corresponding RNA thereof. For example, the plurality of first nucleic acids are a nucleic acid comprising the polynucleotide encoding the RSV F protein derived from the strain Ontario and having mutations P101C, I152C, and S190M (first first nucleic acids), and a nucleic acid comprising the polynucleotide encoding the RSV F protein derived from the strain Buenos Aires and having mutations P101C, I152C, and S190F (second first nucleic acids). In other words, the nucleic acid composition comprises two first nucleic acids, the first first nucleic acid is a nucleic acid comprising a polynucleotide encoding an RSV F protein derived from the strain Ontario and having mutations P101C, I152C, and S190M, and a second first nucleic acid is a nucleic acid comprising a polynucleotide comprising an RSV F protein derived from the strain Buenos Aires and having mutations P101C, I152C, and S190F.

For example, the plurality of first nucleic acids are a nucleic acid comprising the polynucleotide encoding RSV F protein derived from the strain Ontario and having mutations P101C, I152C, and S190M (first first nucleic acids), and a nucleic acid comprising the polynucleotide encoding RSV F protein derived from the strain Ontario and having mutations P101C, I152C, and S190F (second first nucleic acids). In other words, the nucleic acid composition comprises two first nucleic acids, the first first nucleic acid is a nucleic acid comprising the polynucleotide encoding RSV F protein derived from the strain Ontario and having mutations P101C, I152C, and S190M, and the second first nucleic acid is a nucleic acid comprising the polynucleotide of RSVF protein derived from the strain Ontario and having mutations P101C, 1152C, and S190F.

For another example, the plurality of first nucleic acids are a nucleic acid comprising the polynucleotide encoding RSV F protein derived from the strain Ontario and having mutations P101C, I152C, and S190M, a nucleic acid comprising the polynucleotide encoding RSV F protein derived from the strain Buenos Aires and having mutations P101C, I152C, and S190M, and a nucleic acid comprising the polynucleotide encoding RSV F protein derived from the strain Ontario and having mutations P101C, 1152C, and S190F. In other words, the nucleic acid composition comprises three first nucleic acids, the first first nucleic acid is a nucleic acid comprising a polynucleotide encoding RSV F protein derived from the strain Ontario and mutations having P101C, I152C, and S190M, the second first nucleic acid is a nucleic acid comprising the polynucleotide encoding RSV F protein derived from the strain Buenos Aires and mutations having P101C, I152C, and S190M, and the third first nucleic acid is a nucleic acid comprising the polynucleotide comprising RSV F protein derived from the strain Ontario and having mutations P101C, I152C, and S190F.

It can be understood that the nucleic acid composition is not limited to include one or more first nucleic acids or first genetic engineering vectors, but also includes other nucleic acids (such as nucleic acids encoding other immunogens) and/or other substances (such as buffers or lyoprotectants).

In some embodiments, the nucleic acid composition further comprises a second nucleic acid or a second vector, the second nucleic acid comprises a polynucleotide encoding a protein or polypeptide other than the mutant of RSV F protein, and the second vector comprises a polynucleotide encoding a protein or polypeptide other than the mutant of RSVF protein; and/or the first nucleic acid and the second nucleic acid are RNAs. Other proteins or polypeptides in addition to the mutation of the RSV F protein are as described above. In some embodiments, the nucleic acid is RNA. In some embodiments, the nucleic acid is mRNA. It can be understood that the second nucleic acid contained in the above nucleic acid composition is not limited to one but also be more. For example, the nucleic acid composition may further comprises a second nucleic acid comprising the polynucleotide encoding an additional protein or polypeptide other than the mutant of RSV F protein; the nucleic acid composition may also be a nucleic acid further comprising a plurality of second nucleic acids that are independent of each other and are a plurality of nucleic acids comprising polynucleotides encoding additional proteins or polypeptides other than the mutant of RSV F protein (e.g., nucleic acids comprising polynucleotides encoding HA of an influenza virus, or variants thereof, and nucleic acids comprising polynucleotides encoding S protein of SARS-Cov-2, or variants thereof). The second vector is also not limited to one but also more.

### VI. Delivery carrier, pharmaceutical composition and use thereof

The present disclosure also provides a delivery carrier comprising the nucleic acid of any of the above embodiments, the RNA of any of the above embodiments, the genetic engineering vector of any of the above embodiments, or the nucleic acid composition of any of the above embodiments.

In some embodiments, the nucleic acid is RNA. In some embodiments, the nucleic acid is mRNA.

In some embodiments, the delivery carrier is selected from one or a complex of several of the follows: lipid nanoparticles (LNPs), liposomes, cationic proteins, vesicles, microparticles, polymers, and micelles. In some embodiments, the delivery carrier is selected from one of lipid nanoparticles, liposomes, cationic proteins, vesicles, microparticles, polymers, and micelles.

In some embodiments, the delivery carrier is a lipid nanoparticle (LNP) comprising the nucleic acid of any of the above embodiments, the RNA of any of the above embodiments, the genetic engineering vector of any of the above embodiments, or the nucleic acid composition of any of the above embodiments.

In some embodiments, lipid nanoparticles refer to particles at the order of nanometers (e.g., 1nm-1000 nm) that include one or more lipids.

In some embodiments, the lipid nanoparticles have an average diameter of 20nm~800nm, 20nm~500nm, 20nm~400nm, 20nm~300nm, 20nm~200nm, 20nm~100nm, 30nm~700nm, 30nm~500nm, 30nm~300nm, 30nm~200nm, 30nm~100nm, 40nm~800nm, 40nm~600nm, 40nm~500nm, 40nm~300nm, 40nm~200nm, 40nm~100nm, 50nm~800nm, 50nm~600nm, 50nm~500nm, 50nm~500nm, 50nm~400nm, 50nm~500nm, 50nm~400nm, 50nm~300nm, 50nm~200nm, 50nm~100nm, 60nm~800nm, 60nm~600nm, 60nm~500nm, 60nm~400nm, 60nm~300nm, 60nm~200nm or 60nm~100nm. In some alternative specific examples, the lipid nanoparticles have an average diameter of 26nm, 31nm, 36nm, 41nm, 46nm, 51nm, 56nm, 61nm, 66nm, 71nm, 76nm, 81nm, 86nm, 91nm, 96nm, 101nm, 106nm, 111nm, 116nm, 121nm, 126nm, 131nm, 136nm, 141nm, 146nm, 151nm, 156nm, 161nm, 166nm, 171nm, 176nm, 181nm, 186nm, 191nm, 196nm, 201nm, 206nm, 211nm, 216nm, 221nm, 226nm, 231nm, 236nm, 241nm, 246nm or 249nm. Herein, the average diameter of the lipid nanoparticles may be expressed as a z-average determined by dynamic light scattering.

In some embodiments, the lipid nanoparticles comprise one of the follows: cationic lipid nanoparticles, solid lipid nanoparticles (SLN), nanostructured lipid carriers (NLC), and non-lamellar lipid nanoparticles. In an optional specific example, the lipid nanoparticles are cationic lipid nanoparticles.

In some embodiments, the lipid nanoparticles contain one or more of the following substances: cationic lipids, helper lipids, structural lipids, and polymer-lipids.

The term "cationic lipid" refers to a lipid that becomes positively charged by lowering the pH below the pKa of the ionizable group of the lipid, but becomes gradually neutral at higher pH values, where the positively charged lipid is able to bind to negatively charged nucleic acids. In certain embodiments, the cationic lipid comprises a zwitterionic lipid.

In some embodiments, the cationic lipid comprises the following compound (I), N-oxide thereof, salt thereof or isomer thereof: 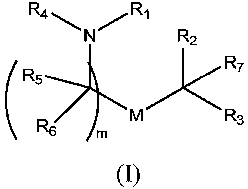 where:
R₁ is selected from the group consisting of C5-C30 alkyl, C5-C20 alkenyl, -R*YR", -YR", and -R"M'R';
R₂ and R₃ are independently selected from the group consisting of H, C1-C14 alkyl, C2-C14 alkenyl, -R*YR", -YR", and -R*OR", or R₂ and R₃ together with the atom to which they are attached form a heterocyclic or carbocyclic ring;
R₄ is selected from the group consisting of hydrogen, C3-C6 carbocycle, -(CH₂)ₙQ, - (CH₂)ₙCHQR, -(CH₂)ₒC(R₁₀)₂(CH₂)ₙ₋ₒQ, -CHQR, -CQ(R)₂, and unsubstituted C1-C6 alkyl, wherein Q is selected from carbocycle, heterocycle, -OR, -O(CH₂)ₙN(R)₂, -C(O)OR, -OC(O)R, - CX₃, -CX₂H, -CXH₂, -CN, -N(R)₂, -C(O)N(R)₂, -N(R)C(O)R, -N(R)S(O)₂R, -N(R)C(O)N(R)₂, - N(R)C(S)N(R)₂, N(R)R₈, -N(R)S(O)₂R₈, -O(CH₂)ₙOR, -N(R)C( = NR₉)N(R)₂, -N(R)C( = CHR₉)N(R)₂, -OC(O)N(R)₂, -N(R)C(O)OR, -N(OR)C(O)R, -N(OR)S(O)₂R, -N(OR)C(O)OR, - N(OR)C(O)N(R)₂, -N(OR)C(S)N(R)₂, -N(OR)C(=NR₉)N(R)₂, -N(OR)C(=CHR₉)N(R)₂, -C(= NR₉)N(R)₂, -C( = NR₉)R, -C(O)N(R)OR and -C(R)N(R)₂C(O)OR, each o is independently selected from 1, 2, 3 and 4, and each n is independently selected from 1, 2, 3, 4 and 5;
each R₅ is independently selected from the group consisting of OH, C1-C3 alkyl, C2-C3 alkenyl, and H;
each R₆ is independently selected from the group consisting of OH, C1-C3 alkyl, C2-C3 alkenyl, and H;
M and M ' are independently selected from the group consisting of -C(O)O-, -OC(O)-, -OC(O)-M"-C(O)O-, -C(O)N(R')-, -N(R')C(O)-, -C(O)-, -C(S)-, -C(S)S-, -SC(S)-, -CH(OH)-, - P(O)(OR')O-, -S(O)₂-, -S-S-, aryl groups, and heteroaryl groups, wherein M " is a bond, C1-C13 alkyl, or C2--C13 alkenyl;
R₇ is selected from the group consisting of C1-3 alkyl, C2-C3 alkenyl, and H;
R₈ is selected from the group consisting of C3-6 carbocyclic and heterocyclic;
R₉ is selected from the group consisting of H, CN, NO₂, C1-C6 alkyl, -OR, -S(O)₂R, - S(O)₂N(R)₂, C2-C6 alkenyl, C3-C6 carbocyclic, and heterocyclic;
R₁₀ is selected from the group consisting of H, C1-C3 alkyl, and C2-C3 alkenyl;
each R is independently selected from the group consisting of C1-C3 alkyl, C2-C3 alkenyl, (CH₂)_{q}OR*, and H,
and each q is independently selected from 1, 2 and 3;
each R' is independently selected from the group consisting of C1-C18 alkyl, C2-C18 alkenyl, -R*YR", -YR", H and 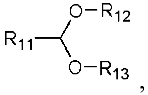 and R₁₁, R₁₂ and R₁₃ are each independently selected from the group consisting of C1-C12 alkyl and C2-Cl₂ alkenyl;
each R" is independently selected from the group consisting of C3-C15 alkyl and C3-C15 alkenyl;
each R* is independently selected from the group consisting of absent, C1-C12 alkyl, and C2-C12 alkenyl;
each Y is independently a C3-C6 carbocyclic ring;
each X is independently selected from the group consisting of F, Cl, Br, and I; and
m is selected from 5, 6, 7, 8, 9, 10, 11, 12 and 13; and wherein when R₄ is -(CH₂)ₙQ, - (CH₂)ₙCHQR, -CHQR, or -CQ(R)₂, then (i) when n is 1, 2, 3, 4 or 5, Q is not -N(R)₂; or (ii) when n is 1 or 2, Q is not 5, 6 or 7-membered heterocycloalkyl.

In an optional specific example, the cationic lipid is the following compound (I), N-oxide thereof, salt thereof or isomer thereof:

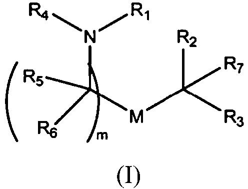

R₁-R₇, M and m are as defined above.

In some embodiments, the cationic lipid comprises the following compound (II), N-oxide thereof, salt thereof or isomer thereof: 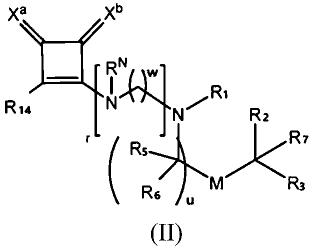 where:
R₁, R₂, R₃, R₅, R₆, M and R₇ are as described above,
R^{N} is H or C₁-C₃ alkyl;
X^{a} and X^{b} are each independently O or S;
R₁₄ is selected from the group consisting of H, halogen,-OH, R^{b}, -N(R^{b})₂, -CN, -N₃, -C(O)OH, -C(O)OR^{b}, -OC(O)R^{b}, -OR^{b}, -SR^{b}, -S(O)R^{b}, -S(O)OR^{b}, -S(O)₂OR^{b}, -NO₂, -S(O)₂N(R^{b})₂, - N(R^{b})S(O)₂R^{b}, -NH(CH₂)ₜ₁N(R^{b})₂, -NH(CH₂)ₚ₁O(CH₂)_{q1}N(R^{b})₂, -NH(CH₂)ₛ₁OR^{b}, -(R^{b})-carbocycle, -N(R^{b})-heterocycle, N(R^{b})-aryl, -N(R^{b})-heteroaryl, -N(R^{b})(CH₂)ₜ₁-carbocycle, -N(R^{b})(CH₂)ₜ₁ - heterocycle, -N(R^{b})(CH₂)ₜ₁-aryl, -N(R^{b})(CH₂)ₜ₁-heteroaryl, carbocycle, heterocycle, aryl and heteroaryl;
each R^{b} is independently selected from the group consisting of C1-C3 alkyl, C2-C3 alkenyl, and H;
u is 5, 6, 7, 8, 9, 10, 11, 12 or 13;
w is 1, 2, 3, 4, 5, 6, 7, 8, 9 or 10;
r is 0 or 1;
t¹ is 1, 2, 3, 4 or 5;
p¹ is 1, 2, 5, 4 or 5;
q¹ is 1, 2, 5, 4 or 5; and
s¹ is 1, 2, 3, 4 or 5.

In an optional specific example, the cationic lipid is the following compound (II), its N-oxide, its salt or its isomer:

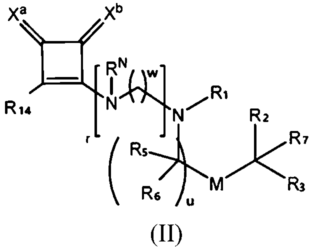

R₁-R₃, R₅-R₇, R₁₄, X^{a}, X^{b}, R^{N}, M, u, w and r are as defined above.

In some embodiments, the cationic lipid comprises the following compound (III), N-oxide thereof, salt thereof or isomer thereof: 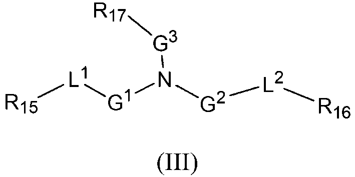 where:
one of L¹ or L² is -O(C=O)-, -(C=O)O-, -C(=O)-, -O-, -S(O)ₓ-, -S-S-, -C(=O)S-, -SC(= O)-, -NR^{a}C(=O)-, -C(=O)NR^{a}-, NR^{a}C(=O)NR^{a}-, -OC(=O)NR^{a}- or -NR^{a}C(=O)O-, and the other of L¹ or L² is -O(C=O)-, -(C=O)O-, -C(=O)-, -O-, -S(O)ₓ-, -S-S-, -C(=O)S-, SC(=O)-, - NR^{a}C(=O)-, -C(=O)NR^{a}-, NR^{a}C(=O)NR^{a}-, -OC(=O)NR^{a}- or -NR^{a}C(=O)O-or a bond;
G¹ and G² are each independently unsubstituted C1-C12 alkylene or C1-C12 alkenylene;
G³ is C1-C24 alkylene, C1-C24 alkenylene, C3-C8 cycloalkylene, C3-C8 cycloalkenylene;
R^{a} is H or C1-C12 hydrocarbyl;
R₁₅ and R₁₆ are each independently C6-C24 alkyl or C6-C24 alkenyl;
R₁₇ is H, OR₁₈, CN, -C(=O)OR₁₉, -OC(=O)R₁₉ or -NR₁₈C(=O)R₁₉;
R₁₉ is a C1-C12 hydrocarbyl group;
R₁₈ is H or C1-C6 hydrocarbyl; and
x is 0, 1 or 2.

In an optional specific example, the cationic lipid is the following compound (III), its N-oxide, its salt or its isomer:

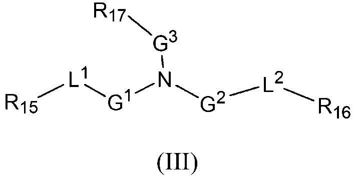

R₁₅-R₁₇, G¹-G³, L¹-L² are as defined above.

In some embodiments, the cationic lipid comprises the following compound (IV), or a pharmaceutically acceptable salt or stereoisomer thereof: 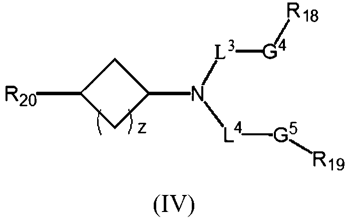 where:
L³ and L⁴ are the same or different, and are each independently C1-C12 alkylene, C2-C12 alkenylene or C2-C12 alkynylene; in some embodiments, L³ and L⁴ are the same or different, and are each independently C3-C10 alkylene, C3-C10 alkenylene or C3-C10 alkynylene; in some embodiments, L³ and L⁴ are the same or different, and are each independently C3-C10 alkylene; in some embodiments, L³ and L⁴ are the same or different, and are each independently C5-C8 alkylene;
G⁴ and G⁵ are the same or different, and are each independently -O-(C=O)-, -(C=O)-O-, - C(=O)-, -O-, -C(=O)-S-or-S-C(=O)-; in some embodiments, G⁴ and G⁵ are the same or different, and are each independently -O-(C=O)-, -(C=O)-O-, -C(=O)- or -O-; in some embodiments, G⁴ and G⁵ are the same or different, and are each independently selected from -O- (C=O)- or - (C=O)-O-;
R₁₈ and R₁₉ are the same or different, and are each independently C5-C27 alkyl or C5-C27 alkenyl containing one or more double bonds; in some embodiments, R₁₈ and R₁₉ are the same or different, and are each independently C8-C20 alkyl or C8-C20 alkenyl containing one or more double bonds; in some embodiments, R₁₈ and R₁₉ are the same or different, and are each independently C9-C17 alkyl or C9-C18 alkenyl containing one or two double bonds; in some embodiments, R₁₈ and R₁₉ are the same or different, and are each independently 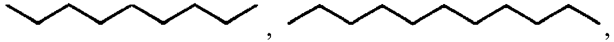 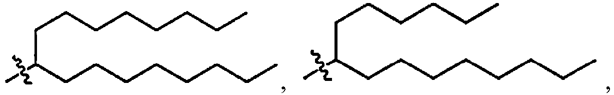 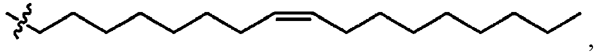 or 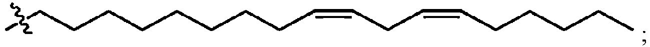
R₂₀ is halogen, hydroxyl, cyano, C1-C6 alkyl, nitro, C1-C6 alkoxy, C1-C6 alkylcarbonyloxy, C1-C6 alkoxycarbonyl, C1-C6 alkylaminocarbonyl or C1-C6 alkylcarbonylamino; in some embodiments, R₂₀ is halogen, hydroxyl, cyano, C1-C6 alkoxy, C1-C6 alkylcarbonyloxy, C1-C6 alkoxycarbonyl, C1-C6 alkylaminocarbonyl or C1-C6 alkylcarbonylamino; in some embodiments, R₂₀ is halogen, hydroxyl, cyano, C1-C4 alkoxy, C1-C4 alkylcarbonyloxy, C1-C4 alkoxycarbonyl, C1-C4 alkylaminocarbonyl or C1-C4 alkylcarbonylamino; in some embodiments, R₂₀ is fluorine, hydroxyl, cyano, methoxy, acetoxy, methoxycarbonyl, butylaminocarbonyl or acetamido;
z is 1, 2 or 3.

In some embodiments, the cationic lipid is the following compound (IV), or a pharmaceutically acceptable salt or stereoisomer thereof:

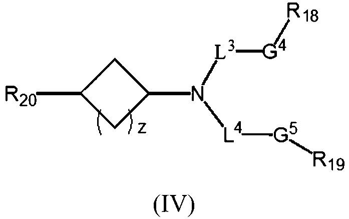

R₁₈-R₂₀, G⁴-G⁵, L³-L⁴ and z are as defined above.

In some embodiments, the cationic lipid comprises the following compound (IV-1), or a pharmaceutically acceptable salt or stereoisomer thereof:

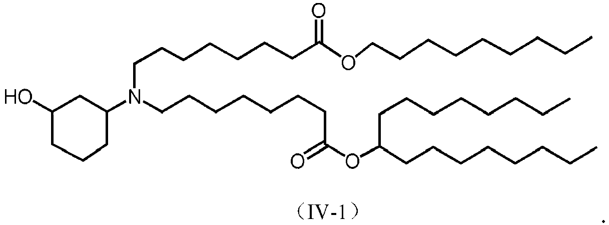

In some embodiments, the cationic lipid includes the following compounds, or pharmaceutically acceptable salts thereof: 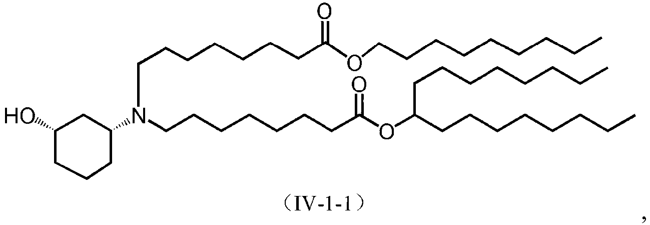 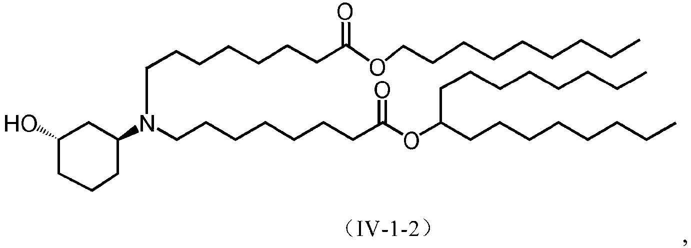 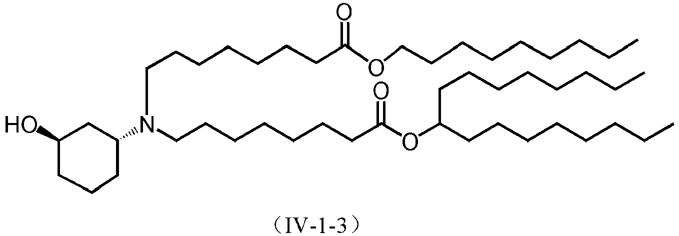 or

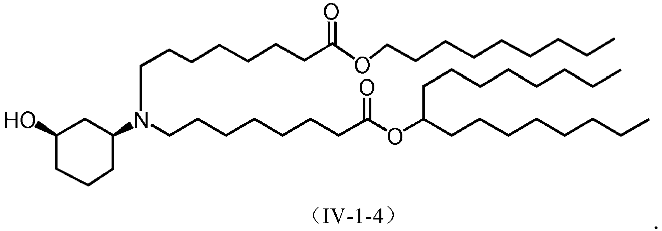

In some embodiments, the cationic lipid comprises one or more of ALC-0315 (CAS No. 2036272-55-4), SM-102 (CAS No. 2089251-47-6), and

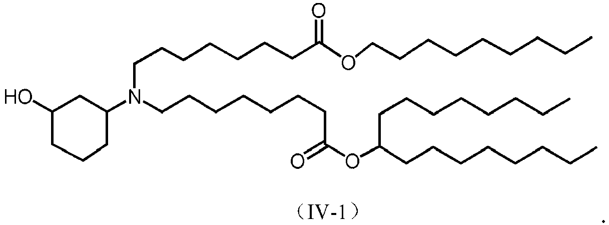

In some embodiments, the auxiliary lipid of the lipid nanoparticle comprises a phospholipid. A phospholipid is generally semi-synthetic and may be naturally derived or chemically modified. In an optional specific example, the auxiliary lipid of the lipid nanoparticle is a phospholipid. In some embodiments, the phospholipid of the lipid nanoparticles includes one or more of the following: DSPC (distearoyl phosphatidylcholine), DOPE (dioleoyl phosphatidylethanolamine), DOPC (dioleoyl phosphatidylcholine), DOPS (dioleoyl phosphatidylserine), DSPG (1,2-dioctadecanoyl-sn-glycero-3-phospho-(1'-rac-glycerol)), DPPG (dipalmitoyl phosphatidylglycerol), DPPC (dipalmitoyl phosphatidylcholine), DGTS (1,2-dipalmitoyl-sn-glycero-3-O-4'-(N,N,N-trimethyl) homoserine), and lysophospholipids. In some embodiments, the auxiliary lipid of the lipid nanoparticle is one or more selected from the group consisting of DSPC, DOPE, DOPC, and DOPS. In some embodiments, the auxiliary lipid of the lipid nanoparticles is DSPC and/or DOPE.

In some embodiments, the structural lipids of the lipid nanoparticles include a sterol. In an optional specific example, the structural lipid of the lipid nanoparticle is a sterol. In some embodiments, the sterol of the lipid nanoparticles includes one or more of the follows: 20α-hydroxycholesterol, cholesterol, cholesterol esters, sterols hormones, sterols vitamins, bile acids, cholesterol, ergosterol, β-sitosterol, and oxidized cholesterol derivatives. In some embodiments, the structural lipid of the lipid nanoparticle comprises at least one of cholesterol, cholesterol ester, sterol hormone, sterol vitamin, and bile acid. In some embodiments, the structural lipid of the lipid nanoparticles is cholesterol. In an alternative specific example, the structural lipid of the lipid nanoparticles is high purity cholesterol, in particular injection-grade high purity cholesterol, such as CHO-HP (produced by AVT). In other embodiments, the structural lipid is 20α-hydroxycholesterol.

A polymer-lipid refers to a conjugate comprising a polymer and a lipid coupled to the polymer. The polymer-lipid (e.g., polyethylene glycol-lipid) in the lipid nanoparticles can improve the stability of the lipid nanoparticles in vivo.

In some embodiments, the lipids of the polymer-lipid used to form the lipid nanoparticles include one or more of 1,2-dimyristoyl-sn-glycerol (DMG), distearoyl-phosphatidyl-ethanolamine (DSPE), diacylglycerol (DAG), dialkyloxypropyl (DAA), phospholipids, ceramide (Cer), 1,2-distearoyl-rac-glycerol (DSG), and 1,2-dipalmitoyl-rac-glycero (DPG).

In some embodiments, the polymer-lipid polymer used to form the lipid nanoparticles includes one or both of a hydrophilic polymer and a zwitterionic polymer.

In some embodiments, the polymer-lipid polymer used to form the lipid nanoparticles is a hydrophilic polymer. In other embodiments, the polymer-lipid polymer used to form the lipid nanoparticles is a zwitterionic polymer.

In some embodiments, the hydrophilic polymer includes one or more of the following: polyethylene glycol (PEG), poly (oxazolines)(POX), poly (glycerols) (PGs), poly (hydroxypropyl methacrylate) (PHPMA), poly (2-hydroxy ethyl methacrylate) (PHEMA), poly (N- (2-hydroxy propyl) methacrylamide) (HPMA), poly (vinylpyrrolidone) (PVP), poly(N,N-dimethyl acrylamide) (PDMA), poly(N-acryloyl morpholine) (PAcM), polyaminoacids, glycosaminoglycans (GAGs), heparin, hyaluronic acid (HA), polysialic acid (PSA), elastin-like polypeptides (ELPs), and serum albumin and CD47.

Correspondingly, polymer-lipids include one or more of the follows: polyethylene glycol-lipids (PEG-lipids), polyoxazoline-lipids, polyglycerol-lipids, polyhydroxypropyl methacrylate-lipids, poly(2-hydroxyethyl methacrylate)-lipids, poly(N-(2-hydroxypropyl) methacrylamide)-lipids, polyvinylpyrrolidone-lipids, poly(N,N-dimethylacrylamide)-lipids, poly(N-acryloylmorpholine)-lipids, glycosaminoglycan-lipids, heparin-lipids, hyaluronic acid-lipids, polysialyl-lipids, elastin-like lipids, serum albumin-lipids, and CD47-lipids. It should be noted that "PEG-lipid" is a conjugate of polyethylene glycol and lipid, "polyoxazoline-lipid" refers to a conjugate formed by coupling polyoxazoline with lipid, "polyglycerol-lipid" refers to a conjugate formed by coupling polyglycerol with lipid, and the same applies to other polymer-lipids. In an optional specific example, the hydrophilic polymer comprises polyethylene glycol.

In some embodiments, the polymer-lipid comprises a PEG-lipid. In an alternative specific example, the polymer-lipid is a PEG-lipid. In some embodiments, PEG- lipids include one or more of PEG-myristoyl diglyceride (PEG-DMG), PEG-distearoyl phosphatidylethanolamine (PEG-DSPE), PEG-diacylglycerol (PEG-DAG), PEG-dialkyloxypropyl (PEG-DAA), PEG-phospholipids, PEG-ceramide (PEG-Cer), PEG-1,2-distearoyl-rac-glycerol (PEG-DSG), and PEG-1,2-dipalmitoyl-rac-glycerol (PEG-DPG). The PEG-lipid is preferably PEG-DMG, PEG-DSG, PEG-DPG. PEG-DMG is a polyethylene glycol derivative of glycerol 1,2-dimyristate. In some embodiments, the average molecular weight of the PEG in the PEG-lipid is about 2000-5000. In an alternative specific example, the average molecular weight of PEG in PEG-lipid is about 2000. In some embodiments, the zwitterionic polymer includes one or more of poly(carboxybetaine) (pCB), poly(sulfobetaine) (pSB), phosphobetaine-base polymers, and phosphorylcholine polymers. In some embodiments, the zwitterionic polymer includes one or more of the following: polycarboxybetaine acrylamide, (pCBAA), poly(carboxybetaine methacrylate), poly(sulfobetaine methacrylate), poly(methacryloyloxyethyl phosphorylcholine), poly(vinyl-pyridinio propanesulfonate), poly(carboxybetaine) based on vinylimidazole, poly(sulfobetaine) based on vinylimidazole, poly(sulfobetaine) based on vinylpyridine.

Correspondingly, the polymer-lipid comprises one or more of the following: polyhydroxybetaine-lipid, polysulfobetaine-lipid, phosphobetaine-based polymer-lipid, and phosphocholine polymer-lipid. In some embodiments, the polymer-lipid comprises one or more of poly (carboxybetaine acrylamide)-lipid, poly (carboxybetaine methacrylate)-lipid, poly (sulfobetaine methacrylate)-lipid, poly (methacryloxyethyl phosphorylcholine)-lipid, poly (vinyl pyridyl propanesulfonate)-lipid, polyvinylimidazolyl betaine-lipid, polyvinylimidazolyl sulfobetaine-lipid, polyvinylpyridyl sulfobetaine-lipid.

In addition, in some embodiments, the polymers applied to nanoparticles in Hoang Thi, Thai Thanh, et al., "The Importance of Poly (ethylene glycol) Alternatives for Overcoming PEG Immunogenicity in Drug Delivery and Bioconjugate." Polymers vol. 12,2298, are also incorporated herein.

In some embodiments, the lipid nanoparticle contains a cationic lipid, a helper lipid, a structural lipid, and a polymer-lipid. In some embodiments, the lipid nanoparticles comprise about 25.0%~75.0%, such as about 25.0%~28.0%, 28.0%~32.0%, 32.0%~35.0%, 35.0%~40.0%, 4.0% to 42.0%, 42.0% to 45.0%, 45.0% to 46.3%, 46.3% to 48.0%, 48.0% to 49.5%, 49.5% to 50.0%, 50.0% to 55.0%, 55.0% to 60.0%, 60.0% to 65.0%, or 65.0% to 75.0%, based on the total amount of cationic lipid, auxiliary lipid, structural lipid, and polymer-lipid.

In some embodiments, the lipid nanoparticle comprises a cationic lipid, a helper lipid, a structural lipid, and a polymer-lipid, the cationic lipid accounts for 25 mol% to 75 mol% of the total lipids present in lipid nanoparticle, the auxiliary lipid accounts for 0 mol% to 45 mol% of the total lipids present in lipid nanoparticle, the structural lipid accounts for 0 mol% to 60 mol% of the total lipids present in lipid nanoparticle, and the polymer lipid accounts for 0.5 mol% to 5 mol% of the total lipids present in lipid nanoparticle.

In some embodiments, the cationic lipid in the above lipid nanoparticle accounts for 25 mol% to 75 mol% of the total lipids present in the lipid nanoparticle, e.g., 25 mol%, 28mol%, 30mol%, 31mol%, 32mol%, 33mol%, 34mol%, 35mol%, 36mol%, 37mol%, 38mol%, 39mol%, 40mol%, 41mol%, 42mol%, 43mol%, 44mol%, 45mol%, 45.5mol%, 46mol%, 46.5mol%, 47mol%, 47.5mol%, 48mol%, 48.5mol%, 49mol%, 49.5mol%, 50mol%, 50.5mol%, 51mol%, 52.5mol%, 53mol%, 53.5mol%, 54mol%, 54.5mol%, 55mol%, 55.5mol%, 56mol%, 56.5mol%, 57mol%, 57.5mol%, 58mol%, 58.5mol%, 59mol%, 59.5mol%, 60mol%, 60.5mol%, 61mol%, 61.5mol%, 62mol%, 62.5mol%, 63mol%, 63.5mol%, 64mol%, 64.5mol%, 65mol%, 68mol%, 70mol% Or 75mol%. In some embodiments, the cationic lipid in the above lipid nanoparticle accounts for 30mol%~65mol%, 30mol%~60mol%, 35mol%~60mol%, 40mol%~60mol%, 45mol%~55mol%, or 50mol%~55mol% of the total lipids present in the lipid nanoparticle.

In some embodiments, the auxiliary lipid (such as DSPC) in the above lipid nanoparticle accounts for 0mol% to 45mol% of the total lipids present in the lipid nanoparticle, e.g., 0.5 mol%, 1mol%, 3mol%, 5mol%, 7.5mol%, 8mol%, 8.5mol%, 9mol%, 9.5mol%, 10mol%, 10.5mol%, 11mol%, 11.5mol%, 12mol%, 12.5mol%, 13mol%, 13.5mol%, 14mol%, 14.5mol%, 15mol%, 15.5mol%, 16mol%, 16.5mol%, 17mol%, 17.5mol%, 18mol%, 18.5mol%, 19mol%, 19.5mol%, 20mol%, 20.5mol%, 21mol%, 21.5mol%, 22mol%, 22.5mol%, 23mol%, 23.5mol%, 24mol%, 24.5mol%, 25mol%, 25.5mol%, 26mol%, 26.5mol%, 27mol%, 27.5mol%, 28mol%, 28.5mol%, 29mol%, 29.5mol%, 30mol%, 34mol%, 35mol%, 36mol%, 38mol%, 40mol%, 42mol%, 44mol% Or 45mol%. In some embodiments, the auxiliary lipid (such as DSPC) in the above lipid nanoparticle accounts for 1mol%~40mol%, 5mol%~40mol%, 5mol%~35mol%, 5mol%~30mol%, or 5mol%~25mol% of the total lipids present in the lipid nanoparticle.

In some embodiments, the structural lipid (such as cholesterol) in the above lipid nanoparticle accounts for 0mol% to 60mol% of the total lipids present in the lipid nanoparticle, e.g., 0 mol%, 1mol%, 5mol%, 8mol%, 10mol%, 12mol%, 14mol%, 15mol%, 16mol%, 18mol%, 20mol%, 22mol%, 24mol%, 25mol%, 27mol%, 27.5mol%, 28mol%, 28.5mol%, 29mol%, 29.5mol%, 30mol%, 30.5mol%, 31mol%, 31.5mol%, 32mol%, 32.5mol%, 33mol%, 33.5mol%, 34mol%, 34.5mol%, 35mol%, 35.5mol%, 36mol%, 36.5mol%, 37mol%, 37.5mol%, 38mol%, 38.5mol%, 39mol%, 39.5mol%, 40mol%, 40.5mol%, 41mol%, 41.5mol%, 42mol%, 42.5mol%, 43mol%, 44mol%, 45mol%, 46mol%, 47mol%, 48mol%, 49mol%, 50mol%, 51mol%, 52mol%, 53mol%, 54mol%, 55mol%, 56mol%, 57mol%, 58mol%, 59mol% Or 60mol%. In some embodiments, the structural lipid (such as cholesterol) in the above-mentioned lipid nanoparticle accounts for 1 mol%~60mol%, 1mol%~55mol%, 5mol%~55mol%, 10mol%~50mol%, 15mol%~50mol%, 15mol%~45mol%, 20mol%~45mol%, 25mol%~40mol% of the total lipids present in the lipid nanoparticle.

In some embodiments, the polymer lipid (such as a PEG lipid) in the above lipid nanoparticle accounts for 0.5 mol% to 5 mol% of the total lipids present in the lipid nanoparticle, e.g., 0.5mol%, 1mol%, 1.5mol%, 2mol%, 2.5mol%, 3mol%, 3.5mol%, 4mol%, 4.5mol%, or 5mol%. In some embodiments, the polymer lipid (such as a PEG lipid) in the above lipid nanoparticle accounts for 0.5 mol% to 4.5 mol%, 1 mol% to 4.5 mol%, 1 mol% to 4 mol%, 1.5 mol% to 4 mol%, 1.5 mol% to 3.5 mol%, or 1.5 mol% to 3 mol% of the total lipids present in the lipid nanoparticle.

In some embodiments, the non-lamellar lipid nanoparticles are selected from one of: ethosomes and echogenic liposomes.

In some embodiments, the above delivery carrier is a liposome comprising the nucleic acid of any of the above embodiments, the RNA of any of above the embodiments, the genetic engineering vector of any of the above embodiments, or the nucleic acid composition of any of the above embodiments. The liposome encapsulates the nucleic acid, RNA, vector or nucleic acid composition of any of the above embodiments with a vesicle formed by a phospholipid bilayer membrane. In some embodiments, the composition of the liposome comprises a phospholipid and cholesterol.

In some embodiments, the above delivery carrier is a cationic protein loaded with the nucleic acid of any of the above embodiments, the RNA of any of the above embodiments, the genetic engineering vector of any of the above embodiments, or the nucleic acid composition of any of the above embodiments. In some embodiments, the cationic protein includes, but is not limited to, protamine.

In some embodiments, the above delivery carrier is a polymer comprising the nucleic acid of any of the above embodiments, the RNA of any of the above embodiments, the genetic engineering vector of any of the above embodiments, or the nucleic acid composition of any of the above embodiments. In some embodiments, the polymer is a lipopolyplex (LPP) and/or a hyaluronic acid polymer (e.g., a hyaluronic acid gel) comprising the nucleic acid, RNA, genetic engineering vector or nucleic acid composition of any of the above embodiments. In an optional specific example, the polymer is a lipopolyplex or a hyaluronic acid gel. A lipopolyplex is a bilayer structure with a polymer-encapsulated nucleic acid (e.g., mRNA) as the core and a lipid (e.g., phospholipid) encapsulated as the shell.

It can be understood that the delivery carrier suitable for the present disclosure is not limited to the above, and may also be other substances capable of transporting the nucleic acid of any of the above embodiments, the RNA of any of the above embodiments, the genetic engineering vector of any of the above embodiments or the nucleic acid composition of any of the above embodiments into an organism. For example, vesicles (e.g., exosomes) and the like.

In addition, the present disclosure further provides a preparation method for the above compound (IV), and the reaction of the preparation method is as follows: 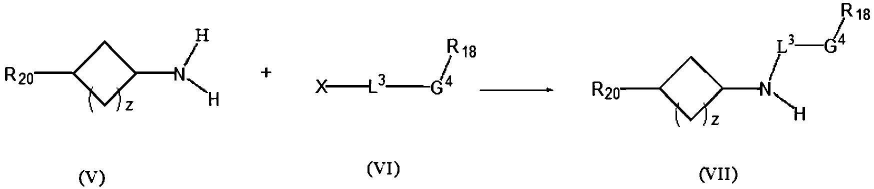 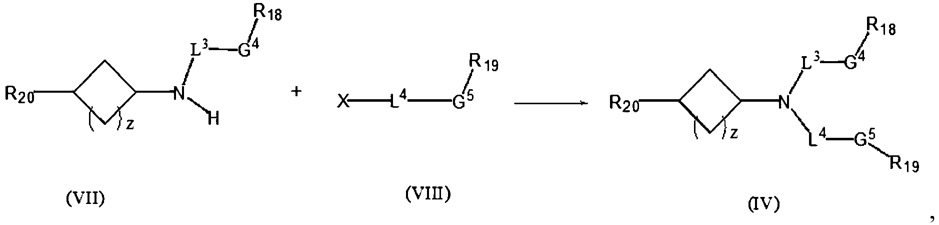 where, R₁₈~R₂₀, G⁴-G⁵, L³-L⁴ and z are as defined above, and X is halogen, preferably bromine.

The preparation method includes step S11 and step S12.

Step S11: subjecting the intermediate compound (V) and the intermediate compound (VI) to a substitution reaction at room temperature (16° C to 30° C, the same applies below) in an organic solvent in the presence of an acid-binding agent to obtain an intermediate compound (VII). The organic solvent in step S1 is selected from one or more of nitrile organic solvents, alcohol organic solvents, halogenated hydrocarbon organic solvents, amide organic solvents, and aromatic hydrocarbon organic solvents, for example, the organic solvent in step S1 is selected from one or more of acetonitrile, methanol, ethanol, dichloromethane, and dichloroethane (DCE). The acid-binding agent in step S1 is selected from one or more of an organic base and an inorganic base. For example, the acid-binding agent in step S1 is selected from one or more of sodium hydroxide, potassium hydroxide, sodium carbonate, potassium carbonate, triethylamine, and DIPEA.

Step S12: subjecting the intermediate compound (VII) and the intermediate compound (VIII) to a substitution reaction at room temperature in an organic solvent in the presence or absence of an acid-binding agent and an iodide to obtain the compound of formula (IV). The organic solvent in step S2 is selected from one or more of nitrile organic solvents, alcohol organic solvents, halogenated hydrocarbon organic solvents, amide organic solvents and aromatic hydrocarbon organic solvents. For example, the organic solvent in step S2 is selected from one or more of acetonitrile, methanol, ethanol, dichloromethane and dichloroethane. The acid-binding agent is selected from one or more of organic bases and inorganic bases. For example, the acid-binding agent is selected from one or more of sodium hydroxide, potassium hydroxide, sodium carbonate, potassium carbonate, triethylamine and DIPEA. Iodides are common iodides, such as potassium iodide.

In addition, the present disclosure also provides a preparation method for the optical isomer (IV-1- i) of the above compound (IV-1), and the reaction of the preparation method is as follows: 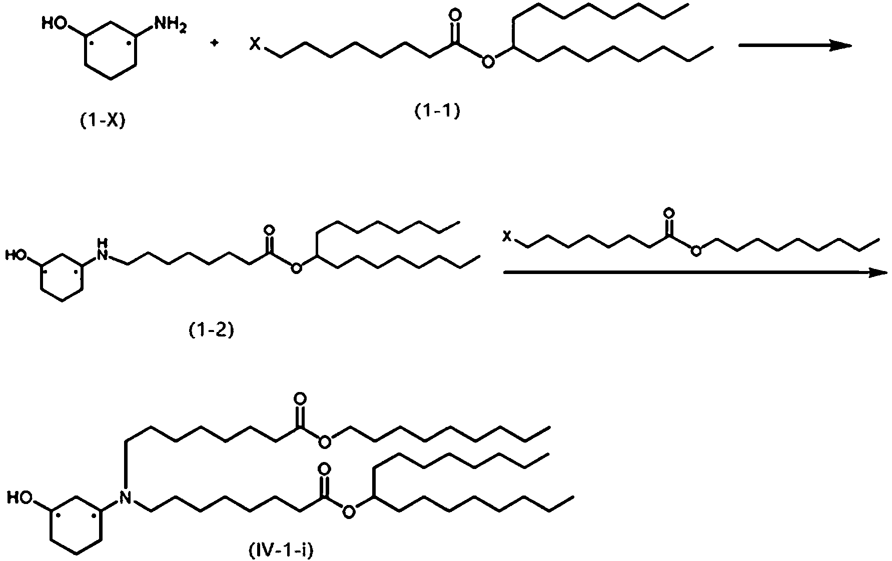 where X is halogen, preferably bromine.

Specifically, the preparation steps for the optical isomer (IV-1- i) of compound (IV-1) of the present disclosure include step S21 and step S22.

Step S21: subjecting compound (1-X) and compound (1-1) to an N-alkylation reaction at 30° C to 50° C in a solvent (such as nitrile, alcohol, halogenated hydrocarbon, amide, aromatic hydrocarbon solvent, specifically acetonitrile, methanol, ethanol, dichloromethane, dichloroethane (DCE), etc.) to prepare compound (1-2);
Step S22: subjecting the compound (1-2) and nonyl 8-halooctanoate to an N-alkylation reaction at 60° C to 110° C in a solvent (such as nitriles, alcohols, halogenated hydrocarbons, amides, aromatic hydrocarbons, ether solvents, specifically acetonitrile, methanol, ethanol, dichloromethane, dichloroethane (DCE), cyclopentylether, methyl tert-butyl ether, etc.), in the presence or absence of an acid binding agent and a catalyst to prepare the compound (IV-1-i), wherein the acid binding agent is selected from one or more of organic bases and inorganic bases. For example, one or more selected from the group consisting of sodium hydroxide, potassium hydroxide, sodium carbonate, potassium carbonate, triethylamine and DIPEA; the catalyst is an iodide, preferably KI.

In some embodiments, according to the above method, when the compound (1-X) as used is (1S,3R)-3-aminocyclohexanol, the compound (1-2) is as shown in structural formula (1-2-1): 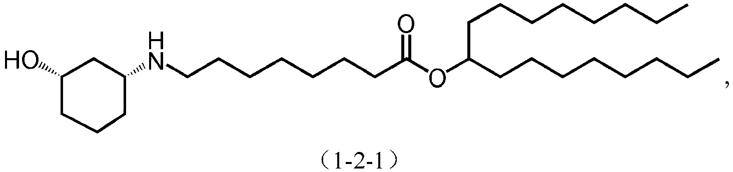 the optical isomer compound (IV-1-1) of the compound (IV-1) was obtained:

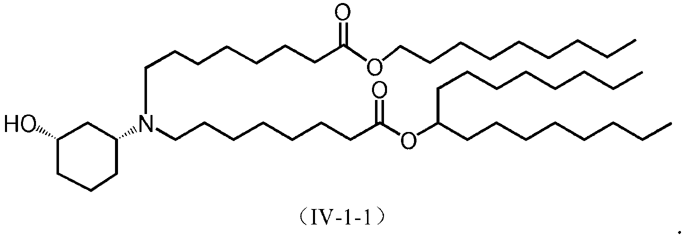

When the compound (1-X) is (1S,3S)-3-aminocyclohexanol, and the compound (1-2) is shown in the structural formula (1-2-2): 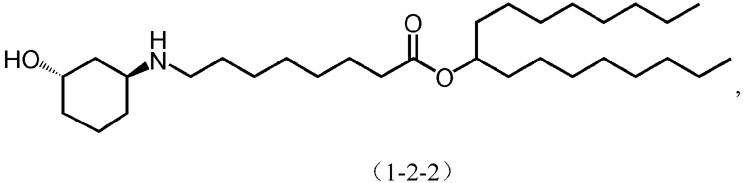 the optical isomer compound (IV-1-2) of the compound (IV-1) was obtained:

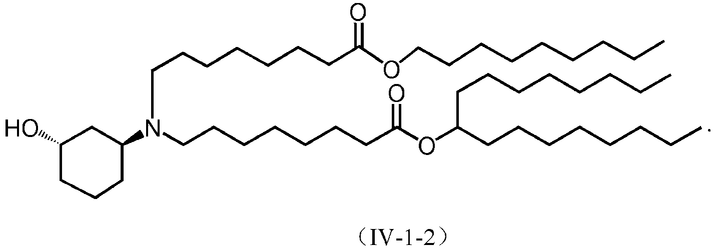

When the compound (1-X) is (1R,3R)-3-aminocyclohexanol, and the compound (1-2) is shown in the structural formula (1-2-3): 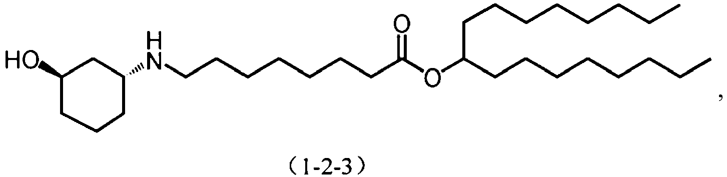 the optical isomer compound (IV-1-3) of the compound (IV-1) was obtained:

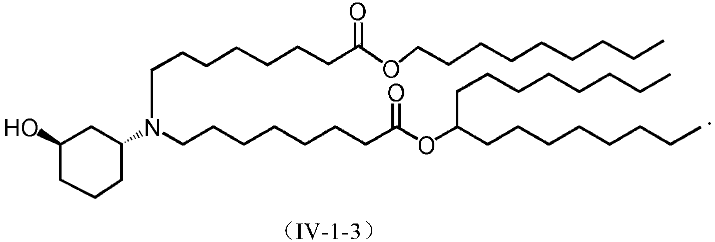

When the compound (1-X) is (1R,3S)-3-aminocyclohexanol, and the compound (1-2) is shown in the structural formula (1-2-4): 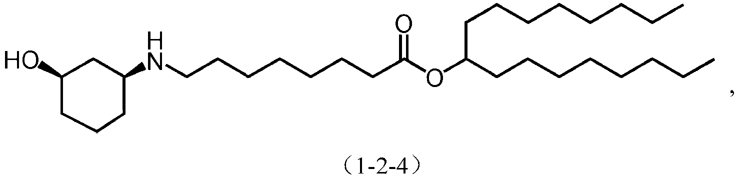 the optical isomer compound (IV-1-4) of the compound (IV-1) was obtained:

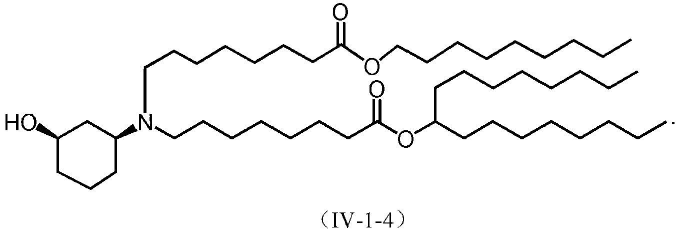

In addition, the present disclosure also provides a pharmaceutical composition comprising the nucleic acid of any of the above embodiments, the RNA of any of the above embodiments, the genetic engineering vector of any of the above embodiments, the host cell of any of the above embodiments, the nucleic acid composition of any of the above embodiments, the immunogen of any of the above embodiments, the mutant of RSV F protein of any of the above embodiments or the delivery carrier of any of the above embodiments, and a pharmaceutically acceptable carrier.

In some embodiments, the above pharmaceutical composition comprises a plurality of nucleic acids.

In some embodiments, the above pharmaceutical composition comprises a plurality of nucleic acids, which are comprised in the same delivery carrier (e.g. LNPs) or different delivery carriers (e.g. LNPs).

In some embodiments, the above pharmaceutical composition comprises one or more delivery carriers.

In some embodiments, the above pharmaceutical composition comprises a delivery carrier comprising a nucleic acid or a nucleic acid composition of any one of the above embodiments.

In some embodiments, the above pharmaceutical composition comprises a delivery carrier comprising a first nucleic acid and a second nucleic acid, the first nucleic acid comprises a polynucleotide encoding a mutant of RSV F protein comprising mutations P101C, I152C, T54A, S190M, and V296L and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence shown in SEQ ID NO: 83; the second nucleic acid comprises a polynucleotide encoding a mutant of RSV F protein, which is a mutant of RSV F protein comprising mutations P101C, I152C, T54A, S190M, and V296L and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence shown in SEQ ID NO: 84.

In some embodiments, the above pharmaceutical composition comprises a delivery carrier comprising two first nucleic acids, the first first nucleic acid comprises a polynucleotide encoding a mutant of RSV F protein comprising the amino acid sequence as shown in SEQ ID NO: 83; the second first nucleic acid comprises a polynucleotide encoding a mutant of RSV F protein comprising the amino acid sequence shown in SEQ ID NO: 84.

In some embodiments, the above pharmaceutical composition comprises a delivery carrier comprising two first nucleic acids, the first first nucleic acid comprises a DNA encoding an RSV F protein with the amino acid sequence as shown in SEQ ID NO: 83 and at least 85%, 88%, 90%, 91%, 92%, 93%, 94%, 95%, 95.5%, 96%, 96.5%, 97%, 97.5%, 98%, 98.5%, 99% or 99.5% identical to the DNA as shown in SEQ ID NO: 187, or the corresponding RNA thereof; the second first nucleic acid comprises a DNA encoding an RSV F protein with the amino acid sequence as shown in SEQ ID NO: 84 and at least 85%, 88%, 90%, 91%, 92%, 93%, 94%, 95%, 95.5%, 96%, 96.5%, 97%, 97.5%, 98%, 98.5%, 99% or 99.5% identical to the DNA as shown in SEQ ID NO: 188, or the corresponding RNA thereof.

In some embodiments, the above pharmaceutical composition comprises a delivery carrier comprising two first nucleic acids, the first first nucleic acid comprises a DNA as shown in SEQ ID NO: 187, or the corresponding RNA thereof; the second first nucleic acid comprises a DNA as shown in SEQ ID NO: 188, or the corresponding RNA thereof.

In some embodiments, the above pharmaceutical composition comprises a delivery carrier, which is an LNPs comprising the nucleic acid of any one of the above embodiments or the nucleic acid composition of any one of the above embodiments.

In some embodiments, the above pharmaceutical composition comprises a plurality of delivery carriers, each independently comprising a nucleic acid or nucleic acid composition of any one of the above embodiments.

In some embodiments, the pharmaceutical composition comprises a plurality of delivery carriers, and the mutants of RSV F protein encoded by the nucleic acids contained in the plurality of delivery carriers are from the same subtype strain but have different mutations, are from different subtype strains but have the same mutation, or are different subtype strains and have different mutations.

For example, the above pharmaceutical composition comprises a first delivery carrier and a second delivery carrier, the first delivery carrier comprises a first nucleic acid, the second delivery carrier comprises a second nucleic acid, the first nucleic acid is a nucleic acid comprising a polynucleotide encoding an RSV F protein derived from the strain Ontario and having mutations P101C, I152C and S190M, and the second nucleic acid is a nucleic acid comprising a polynucleotide encoding an RSV F protein derived from the strain Buenos Aires and having mutations P101C, I152C and S190M. In some embodiments, the above pharmaceutical composition comprises a first delivery carrier comprising a first nucleic acid comprising a polynucleotide encoding an RSV F protein derived from the Ontario strain and having mutations P101C, I152C, T54A, S190M, and V296L and a second delivery carrier comprising a second nucleic acid comprising a polynucleotide encoding an RSV F protein derived from the Buenos Aires strain and having mutations P101C, I152C, T54A, S190M, and V296L.

In some embodiments, the above pharmaceutical composition comprises a first delivery carrier comprising a first nucleic acid and a second delivery carrier comprising a second nucleic acid, the first nucleic acid comprising a polynucleotide encoding a mutant of RSV F protein comprising mutations P101C, I152C, T54A, S190M, and V296L and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence shown in SEQ ID NO: 83; the second nucleic acid comprises a polynucleotide encoding a mutant of RSV F protein comprising mutations P101C, I152C, T54A, S190M, and V296L and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence shown in SEQ ID NO: 84.

In some embodiments, the above pharmaceutical composition comprises a first delivery carrier comprising a first nucleic acid and a second delivery carrier comprising a second nucleic acid, the first nucleic acid comprises a polynucleotide encoding a mutant of RSV F protein comprising the amino acid sequence as shown in SEQ ID NO: 83; the second nucleic acid comprises a polynucleotide encoding a mutant of RSVF protein comprising the amino acid sequence shown in SEQ ID NO: 84.

In some embodiments, the pharmaceutical composition comprises a first delivery carrier comprising a first nucleic acid and a second delivery carrier comprising a second nucleic acid, the first nucleic acid comprises a DNA encoding an RSV F protein comprising the amino acid sequence as shown in SEQ ID NO: 83 and at least 85%, 88%, 90%, 91%, 92%, 93%, 94%, 95%, 95.5%, 96%, 96.5%, 97%, 97.5%, 98%, 98.5%, 99% or 99.5% identical to the DNA as shown in SEQ ID NO: 187, or the corresponding RNA thereof; the second nucleic acid comprises a DNA encoding an RSV F protein comprising the amino acid sequence as shown in SEQ ID NO: 84 and at least 85%, 88%, 90%, 91%, 92%, 93%, 94%, 95%, 95.5%, 96%, 96.5%, 97%, 97.5%, 98%, 98.5%, 99% or 99.5% identical to the DNA as shown in SEQ ID NO: 188, or the corresponding RNA thereof.

In some embodiments, the above pharmaceutical composition comprises a first delivery carrier comprising a first nucleic acid and a second delivery carrier comprising a second nucleic acid, the first nucleic acid comprises a DNA as shown in SEQ ID NO: 187, or the corresponding RNA thereof; the second nucleic acid comprises a DNA as shown in SEQ ID NO: 188, or the corresponding RNA thereof. For another example, the above pharmaceutical composition comprises a first delivery carrier and a second delivery carrier, the first delivery carrier comprises a first nucleic acid, the second delivery carrier comprises a second nucleic acid, the first nucleic acid is a nucleic acid comprising a polynucleotide encoding an RSV F protein derived from the strain Ontario and having mutations P101C, I152C and S190M, and the second nucleic acid is a nucleic acid comprising a polynucleotide encoding an RSV F protein derived from the strain Ontario and having mutations P101C, I152C and S190F. In some embodiments, the first delivery carrier and the second delivery carrier are LNPs comprising the nucleic acid of any one of the above embodiments or the nucleic acid composition of any one of the above embodiments.

For another example, the above pharmaceutical composition comprises a delivery carrier, the delivery carrier comprises a first nucleic acid and a second nucleic acid, the first nucleic acid is a nucleic acid comprising a polynucleotide encoding an RSV F protein derived from the strain Ontario and having mutations P101C, I152C and S190M, and the second nucleic acid is a nucleic acid comprising a polynucleotide encoding an RSV F protein derived from the strain Buenos Aires and having mutations P101C, I152C and S190M.

In some embodiments, the above pharmaceutical composition comprises a plurality of nucleic acids of any of the above embodiments, a plurality of RNAs of any of the above embodiments, a plurality of genetic engineering vectors of any of the above embodiments, or a plurality of host cells of any of the above embodiments. As used herein, the term "pharmaceutically acceptable" means approved for use in animals and/or human by a regulatory agency, such as the China Food and Drug Administration (CFDA), the United States Food and Drug Administration (FDA)), or a recognized pharmacopoeia, such as the Chinese pharmacopoeia, European Pharmacopeia. The term "pharmaceutically acceptable carrier" refers to a substance that can be administered with the nucleic acid, genetic engineering vector, nucleic acid composition, protein mutant or delivery carrier of the present disclosure, including but not limited to diluents, sweeteners, flavoring agents, wetting agents, adjuvants, glidants, preservatives, dyes/colorants, surfactants, dispersants, suspending agents, stabilizers, isotonic agents, solvents or emulsifiers.

In some embodiments, the above pharmaceutical composition does not comprise an adjuvant.

In some embodiments, the above pharmaceutical composition further comprises an adjuvant.

In some embodiments, the adjuvant comprises flagellin adjuvant. In some embodiments, the flagellin adjuvant is an mRNA encoding flagellin.In addition, the present disclosure also provides use of the nucleic acid of any of the above embodiments, the genetic engineering vector of any of the above embodiments, the host cell of any of the above embodiments, the nucleic acid composition of any of the above embodiments, the mutant of RSV F protein of any of the above embodiments, the RNA of any of the above embodiments, the immunogen of any of the above embodiments, the delivery carrier of any of the above embodiments or the pharmaceutical composition of any of the above embodiments in the preparation of a medicament.

In some embodiments, the medicament is used to prevent or treat RSV infection or diseases caused by RSV infection. In some embodiments, the medicament is a vaccine.

### VII. Vaccine

The present disclosure also provides a vaccine comprising the nucleic acid of any of the above embodiments, the genetic engineering vector of any of the above embodiments, the nucleic acid composition of any of the above embodiments, the mutant of RSV F protein of any of the above embodiments, the RNA of any of the above embodiments, the immunogen of any of the above embodiments, or the delivery carrier of any of the above embodiments.

In some embodiments, the above vaccine is an RSV vaccine.

In some embodiments, the above vaccine is a multivalent vaccine.

In some embodiments, the above vaccine comprises any plurality of the nucleic acids or any plurality of mutants of RSV F protein.

In some embodiments, the above vaccine is a combined vaccine.

In some embodiments, the above vaccine is a combined nucleic acid vaccine. In some embodiments, in addition to any one or more of the nucleic acids comprising the polynucleotide encoding the mutant of RSV F protein, the vaccine further comprises a nucleic acid encoding a protein or polypeptide of the RSV other than the F protein or a protein or polypeptide of other viruses and capable of acting as an immunogen. Of course, the other viruses may be a virus subtype, or a plurality of viruses or a plurality of virus subtypes. For example, the other viruses can be influenza A, and can also be influenza A and SARS-CoV-2, or can further be influenza A and influenza B.

In some embodiments, the above vaccine is a combined protein vaccine. In some embodiments, in addition to any one or more of the RSV F protein mutants, the vaccine further comprises a protein or polypeptide of other viruses that can be used as an immunogen. Of course, there may be one or more other viruses.

In some embodiments, the above vaccine does not comprise an adjuvant.

In some embodiments, the above vaccine further comprises an adjuvant.

In some embodiments, the adjuvant comprises flagellin adjuvant. In some embodiments, the flagellin adjuvant is an mRNA encoding flagellin. When combined with flagellin adjuvant, especially when one or more mRNAs encoding the mutant(s) of RSVF protein are combined with the mRNA encoding flagellin, the efficacy of RNA (e.g. mRNA) in the vaccine can be significantly enhanced. In some embodiments, the above vaccine comprises one or more delivery carrier.

In some embodiments, the above vaccine comprises a delivery carrier, which is an LNP comprising the nucleic acid or nucleic acid composition of any one of the above embodiments.

In some embodiments, the above vaccine comprises a delivery carrier comprising a first nucleic acid and a second nucleic acid, the first nucleic acid comprises a polynucleotide encoding a mutant of RSV F protein comprising mutations P101C, I152C, T54A, S190M, and V296L and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence shown in SEQ ID NO: 83; the second nucleic acid comprises a polynucleotide encoding a mutant of RSV F protein comprising mutations P101C, I152C, T54A, S190M, and V296L and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence shown in SEQ ID NO: 84.

In some embodiments, the above vaccine comprises a delivery carrier comprising two first nucleic acids, the first first nucleic acid comprises a polynucleotide encoding a mutant of RSV F protein comprising the amino acid sequence as shown in SEQ ID NO: 83; the second first nucleic acid comprises a polynucleotide encoding a mutant of RSV F protein comprising the amino acid sequence shown in SEQ ID NO: 84.

In some embodiments, the above vaccine comprises a delivery carrier comprising two first nucleic acids, the first first nucleic acid comprises a DNA encoding an RSV F protein with the amino acid sequence as shown in SEQ ID NO: 83 and at least 85%, 88%, 90%, 91%, 92%, 93%, 94%, 95%, 95.5%, 96%, 96.5%, 97%, 97.5%, 98%, 98.5%, 99% or 99.5% identical to the DNA as shown in SEQ ID NO: 187, or the corresponding RNA thereof; the second first nucleic acid comprises a DNA encoding an RSV F protein with the amino acid sequence as shown in SEQ ID NO: 84 and at least 85%, 88%, 90%, 91%, 92%, 93%, 94%, 95%, 95.5%, 96%, 96.5%, 97%, 97.5%, 98%, 98.5%, 99% or 99.5% identical to the DNA as shown in SEQ ID NO: 188, or the corresponding RNA thereof.

In some embodiments, the above vaccine comprises a delivery carrier comprising two first nucleic acids, the first first nucleic acid comprises a DNA as shown in SEQ ID NO: 187, or the corresponding RNA thereof; the second first nucleic acid comprises a DNA as shown in SEQ ID NO: 188, or the corresponding RNA thereof.

In some embodiments, the above vaccine comprises a plurality of delivery carriers, which are independently the LNPs comprising the nucleic acid or nucleic acid composition of any one of the above embodiments.

In some embodiments, the vaccine comprises a first delivery carrier comprising a first nucleic acid and a second delivery carrier comprising a second nucleic acid, the first nucleic acid comprises a polynucleotide encoding a mutant of RSV F protein comprising mutations P101C, I152C, T54A, S190M, and V296L and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence shown in SEQ ID NO: 83; the second nucleic acid comprises a polynucleotide encoding a mutant of RSV F protein comprising mutations P101C, I152C, T54A, S190M, and V296L and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence shown in SEQ ID NO: 84.

In some embodiments, the vaccine comprises a first delivery carrier comprising a first nucleic acid and a second delivery carrier comprising a second nucleic acid, the first nucleic acid comprises a polynucleotide encoding a mutant of RSVF protein comprising the amino acid sequence as shown in SEQ ID NO: 83; the second nucleic acid comprises a polynucleotide encoding a mutant of RSVF protein comprising the amino acid sequence shown in SEQ ID NO: 84.

In some embodiments, the vaccine comprises a first delivery carrier comprising a first nucleic acid and a second delivery carrier comprising a second nucleic acid, the first nucleic acid comprises a DNA encoding an RSV F protein with the amino acid sequence as shown in SEQ ID NO: 83 and at least 85%, 88%, 90%, 91%, 92%, 93%, 94%, 95%, 95.5%, 96%, 96.5%, 97%, 97.5%, 98%, 98.5%, 99% or 99.5% identical to the DNA as shown in SEQ ID NO: 187, or the corresponding RNA thereof; the second nucleic acid comprises a DNA encoding an RSV F protein with the amino acid sequence as shown in SEQ ID NO: 84 and at least 85%, 88%, 90%, 91%, 92%, 93%, 94%, 95%, 95.5%, 96%, 96.5%, 97%, 97.5%, 98%, 98.5%, 99% or 99.5% identical to DNA as shown in SEQ ID NO: 188, or the corresponding RNA thereof.

In some embodiments, the vaccine comprises a first delivery carrier comprising a first nucleic acid and a second delivery carrier comprising a second nucleic acid, the first nucleic acid comprises a DNA as shown in SEQ ID NO: 187, or the corresponding RNA thereof; the second nucleic acid comprises a DNA as shown in SEQ ID NO: 188, or the corresponding RNA thereof.

In some embodiments, the vaccine is an mRNA vaccine.

In some embodiments, the vaccine is an mRNA vaccine, and the dosage form of the vaccine is nasal, transtracheal or injectable (e.g., intravenous, intraocular, intravitreal, intramuscular, intradermal, intracardiac, intraperitoneal and subcutaneous).

It can be understood that the dosage form of the vaccine is not particularly limited.

### VIII. Methods for Prevention or Treatment

The present disclosure also provides a method for preventing or treating RSV viral infection, comprising administering to a subject the nucleic acid of any of the above embodiments, the genetic engineering vector of any of the above embodiments, the nucleic acid composition of any of the above embodiments, the mutant of RSV F protein of any of the above embodiments, the RNA of any of the above embodiments, the immunogen of any of the above embodiments, the delivery carrier of any of the above embodiments, the pharmaceutical composition of any of the above embodiments, or the vaccine of any of the above embodiments.

In some embodiments, the subject is a mammal (e.g., a human, a non-human primate (e.g., simian, chimpanzee, monkey, and chimpanzee)), a domestic animal (e.g., dog, cat, and livestock (e.g., horse, cow, pig, sheep, and goat)), or other mammals. Other mammals include, but are not limited to, mouse, rat, guinea pig, rabbit, hamster, etc. In an optional specific example, the subject is a human.

In some embodiments, the frequency of administrations is one, two, three, four, or more.In addition, the present disclosure also provides a method of eliciting an immune response against RSV in a subject. In some embodiments, the method comprises administering to a subject the nucleic acid of any of the above embodiments, the genetic engineering vector of any of the above embodiments, the nucleic acid composition of any of the above embodiments, the mutant of RSV F protein of any of the above embodiments, the RNA of any of the above embodiments, the immunogen of any of the above embodiments, the delivery carrier of any of the above embodiments, the pharmaceutical composition of any of the above embodiments, or the vaccine of any of the above embodiments. After vaccination with the vaccine of the present disclosure, the anti-antigen antibody titer in the subject is increased as compared to the anti-antigen antibody titer in the subject vaccinated with a prophylactically effective dose of a traditional vaccine against RSV. An "anti-antigen antibody" is a serum antibody that specifically binds to an antigen.

In some embodiments, the above method for preventing or treating viral infection of RSV or inducing an immune response against RSV in a subject comprises administering the nucleic acid, genetic engineering vector, nucleic acid composition, mutant of RSV F protein, RNA, immunogen, delivery carrier, pharmaceutical composition, or vaccine (such as mRNA vaccine) of any one of the above embodiments to the subject at a dose sufficient to deliver 0.025mg/kg to 0.250mg/kg, 0.025mg/kg to 0.500mg/kg, 0.025mg/kg to 0.750mg/kg, or 0.025mg/kg to 1.0mg/kg, based on the subject's body weight, once or twice (or more).

In some embodiments, the above method for preventing or treating viral infection of RSV or inducing an immune response against RSV in a subject comprises administering the nucleic acid, genetic engineering vector, nucleic acid composition, mutant of RSV F protein, RNA, immunogen, delivery carrier, pharmaceutical composition, or vaccine (e.g. mRNA vaccine) of any one of the above embodiments to the subject at a total dose of 0.0100mg to 1.0mg or at a dose level sufficient to deliver the total dose, once or twice (or more times).

In some embodiments, the above methods for preventing or treating viral infection of RSV or inducing immune responses against RSV in subjects comprises administering the nucleic acids, genetic engineering vectors, nucleic acid compositions, mutant of RSV F protein, RNA, immunogens, delivery carriers, pharmaceutical composition, or vaccine (such as mRNA vaccine) of any one of the above embodiments at a total dose of 0.0100 mg, 0.025mg, 0.050mg, 0.075mg, 0.100mg, 0.125mg, 0.150mg, 0.175mg, 0.200mg, 0.225mg, 0.250mg, 0.275mg, 0.300mg, 0.325mg, 0.350mg, 0.375mg, 0.400mg, 0.425mg, 0.450mg, 0.475mg, 0.500mg, 0.525mg, 0.550mg, 0.575mg, 0.600mg, 0.625mg, 0.650mg, 0.675mg, 0.700mg, 0.725mg, 0.750mg, 0.775mg, 0.800mg, 0.825mg, 0.850mg, 0.875mg, 0.900mg, 0.925mg, 0.950mg, 0.975mg, or 1.0mg or at a dose sufficient to deliver the total dose to the subject twice (e.g., on days 0 and 7, days 0 and 14, days 0 and 21, days 0 and 28, days 0 and 60, days 0 and 90, days 0 and 120, days 0 and 150, days 0 and 180, day 0 and 3 months, day 0 and 6 months, day 0 and 9 months, day 0 and 12 months, day 0 and 18 months, day 0 and 2 years, day 0 and 5 years, or day 0 and 10 years. The present disclosure covers administrations with higher and lower dosages and frequencies. For example, the above vaccine (such as mRNA vaccine) can be administered three or four times.

In addition, the present disclosure further provides use of the nucleic acid of any one of the above embodiments, the genetic engineering vector of any one of the above embodiments, the nucleic acid composition of any one of the above embodiments, the mutant of RSV F protein of any one of the above embodiments, the RNA of any one of the above embodiments, the immunogen of any one of the above embodiments, the delivery carrier of any one of the above embodiments, or the pharmaceutical composition of any one of the above embodiments in the preparation of a detection reagent or kit for an RSV F protein-binding antibody.

Moreover, the present disclosure also provides a detection reagent or kit for an RSV F protein-binding antibody, comprising the nucleic acid of any of the above embodiments, the genetic engineering vector of any of the above embodiments, the nucleic acid composition of any of the above embodiments, the mutant of RSV F protein of any of the above embodiments, the RNA of any of the above embodiments, the immunogen of any of the above embodiments, the delivery carrier of any of the above embodiments, or the composition of the drug of any of the above embodiments.

In addition, the present disclosure also provides a method for detecting or isolating an RSV F protein-binding antibody in a subject, the method comprising: providing an effective amount of the nucleic acid of any one of the above embodiments, the genetic engineering vector of any one of the above embodiments, the nucleic acid composition of any one of the above embodiments, the mutant of RSV F protein of any one of the above embodiments, the immunogen of any one of the above embodiments, or the pharmaceutical composition or vaccine containing the mutant of RSV F protein; contacting a biological sample from the subject with the mutant of RSV F protein under conditions sufficient to form an immune complex between the mutant and the RSVF binding antibody; and detecting the immune complex, thereby detecting or isolating the RSVF binding antibody in the subject.

### Examples

To make the objectives and technical solutions of the present disclosure clearer, they are described in details below in view of the particular Examples. Apparently, the described Examples are only a part of the embodiments of the present disclosure, rather than all of the embodiments. All other embodiments obtained by a person of ordinary skill in the art based on the Examples of the present disclosure without creative efforts shall fall within the protection scope of the present disclosure. Unless otherwise specified, the reagents and instruments used in the Examples are conventional choices in the art. Experimental methods without specific conditions specified in the Examples shall be implemented according to conventional conditions, such as those described in references, books, or methods recommended by manufacturers.

### Example 1

### I. Synthesis of Compound (IV-1)

### Step 1): Synthesis of nonyl 8-((3-hydroxycyclohexyl) amino)octanoate (Compound (IV-1-p1))

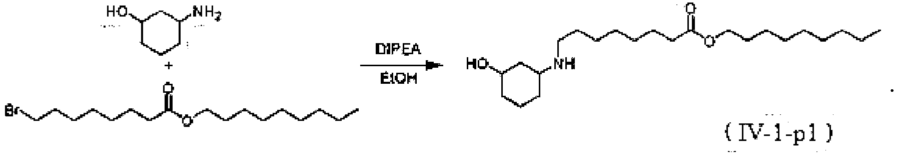

In a 100 mL reaction flask, nonyl 8-bromooctanoate (3.50 g, 10 mmol), 3-aminocyclohexanol (11.5 g, 100 mmol), and 30 mL of ethanol were added in sequence, stirred and dissolved, then N, N-diisopropylethylamine (2.58 g, 20 mmol) was added, reacted at room temperature for 24 h, 100 mL of dichloromethane was added, washed with water for 3 times, dried with anhydrous sodium sulfate, concentrated, and purified by a rapid column chromatography system (dichloromethane: methanol = 20:1 to 5:1) to obtain nonyl 8-((3-hydroxycyclohexyl)amino)octanoate.

¹H NMR (600 MHz, CDCl₃) δ 4.20-4.13 (m, 0.5H), 4.05 (t, 2H), 3.83 (m, 0.5H), 3.09 (m, 0.5H), 2.87 (m, 0.5H), 2.76-2.59 (m, 2H), 2.29 (t, 2H), 2.00 (m, 0.5H), 1.93-1.78 (m, 1.5H), 1.78-1.65 (m, 2H), 1.65-1.46 (m, 8H), 1.40-1.18 (m, 20H), 0.88 (t, 3H).

LCMS:384.3[M+H]⁺ .

### Step 2): Synthesis of Compound (IV-1)

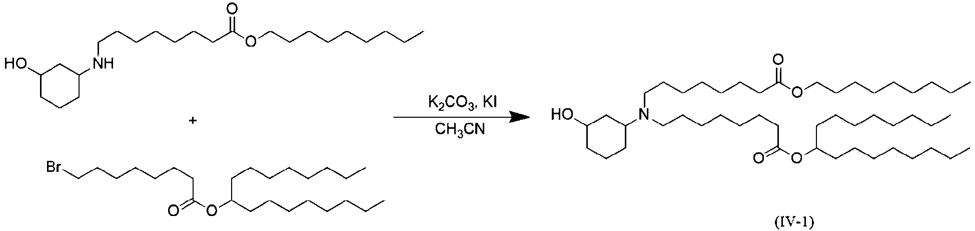

In a 100 mL reaction flask, nonyl 8- ((3-hydroxycyclohexyl) amino) octanoate (383 mg, 1 mmol), 1-octylnonyl 8-bromooctanoate (554 mg, 1.2 mmol), and 20 mL of acetonitrile were sequentially added, stirred and dissolved, then potassium carbonate (276 mg, 2 mmol) and potassium iodide (166 mg, 1 mmol) were added, reacted at room temperature for 24 h, 100 mL of dichloromethane was added, washed with water for 3 times, dried over anhydrous sodium sulfate, concentrated, and purified by a flash column chromatography system (dichloromethane: methanol = 20:1 to 5:1) to obtain compound (IV-1).

¹H NMR (600 MHz, CDCl₃) δ 4.95-4.81 (m, 1H), 4.30-4.12 (m, 0.5H), 4.07 (t, 2H), 3.72-3.61 (m, 0.5H), 2.97 (m, 0.5H), 2.64-2.52 (m, 0.5H), 2.48-2.37 (m, 4H), 2.30 (q, 4H), 1.93-1.81 (m, 2H), 1.72-1.57 (m, 8H), 1.51 (t, 4H), 1.43-1.18 (m, 56H), 0.89 (m, 9H).

LCMS:765.3[M+H]⁺.

### II. Preparation of Compound (IV-1-1)

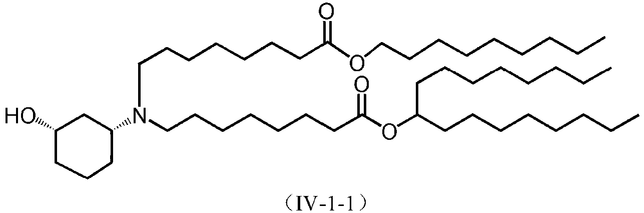

To a 250 mL single-necked flask, 8g (17.3 mmol) of 9-heptadecyl-8-bromooctanoate, 10 g (76.5 mmol) of (1S, 3R)-3-aminocyclohexanol and 100 mL of ethanol were added, and reacted at 50° C for 15 h, and the raw material 9-heptadecyl-8-bromooctanoate was completely reacted. After the solvent was removed by rotary evaporation, the obtained crude product was added with 200 mL of ethyl acetate (EA), washed twice with 100 mL of water, (1S, 3R)-3-aminocyclohexanol was completely washed off, and the EA phase was rotary dried to obtain the crude product of compound (IV-1-1-p) (i.e., 9-heptadecyl-8-(((1R, 3S)-3-hydroxycyclohexyl)amino) octanoate). The crude product was purified by column chromatography (dichloromethane: methanol = 20:1 to 5:1) to obtain compound (IV-1-1-p).

### NMR data:

¹H NMR (600 MHz, CDCl₃): δ 4.91-4.81 (m, 1H), 3.86-3.82 (m, 1H), 2.84-2.81 (m, 1H), 2.69-2.54 (qt, *J =* 11.2, 7.3 Hz, 2H), 2.29-2.26 (t, *J =* 7.5 Hz, 2H), 1.93-1.86 (m, 1H), 1.77-1.74(d, *J* = 12.0 Hz, 1H), 1.72-1.57 (m, 6H), 1.54-1.45 (m, 6H), 1.37-1.30 (m, 8H), 1.30-1.22 (m, 24H), 0.88 (t, *J =* 7.0 Hz, 6H).

LCMS:496.7[M+H]⁺ .

6g (12.1 mmol) of the above compound (IV-1-1-p) was added into a 250 mL single-necked flask, 90 mL of acetonitrile and 60 mL of cyclopentylmethyl ether were added, then 6.7 g of potassium carbonate powder (48.4 mmol), 2 g of potassium iodide (12.1 mmol) and 5 g of nonyl 8-bromooctanoate (17.5 mmol) were added, the reaction was carried out at 90° C for 24 h, the raw material compound ((IV-1-1-p)) was completely reacted, the reaction was removed from the oil bath and cooled to room temperature, the solid was removed by suction filtration, the filtrate was rotary dried to obtain a crude product, and finally purified by column chromatography (dichloromethane: methanol = 20:1 to 5:1) to obtain compound (IV-1-1).

### NMR data:

¹H NMR (600 MHz, CDCl₃): δ 4.93-4.84 (m, 1H), 4.09 (t, *J =* 6.8 Hz, 2H), 3.68-3.61 (m, 1H), 2.62-2.54 (m, 1H), 2.53-2.41 (m, 4H), 2.31-2.26 (q, *J =* 7.4 Hz, 4H), 1.97 (m, 1H), 1.91-1.78 (m, 2H), 1.71-1.61 (m, 7H), 1.54 (d, *J =* 6.0 Hz, 4H), 1.43-1.37 (m, 4H), 1.39-1.23 (m, 52H), 0.91 (m, 9H).

### Specific rotation:+4.3°

LCMS:765.2[M+H]⁺ .

### III. Preparation of Compound (IV-1-2)

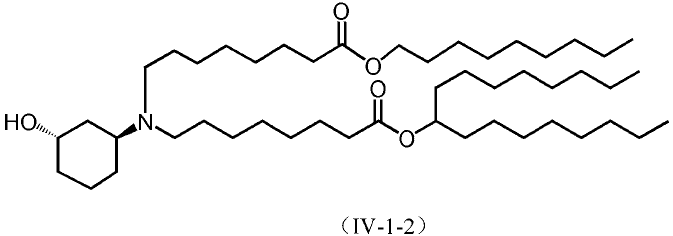

2g of 9-heptadecyl-8-bromooctanoate (4.3 mmol), 2.5 g of (1S, 3S)-3-aminocyclohexanol (20.2 mmol) and 20 mL of ethanol were added to a 100 mL single-necked flask and reacted at 50° C for 15 h, and the raw material 9-heptadecyl-8-bromooctanoate was completely reacted. After the solvent was removed by rotary evaporation, the obtained crude product was added with 50 mL of EA, washed twice with 25 mL of water, (1S, 3S)-3-aminocyclohexanol was thoroughly washed off, and the EA phase was rotary dried to obtain a crude product of the compound (IV-1-2-p) (i.e., 9-heptadecyl-8-(((1S, 3S)-3-hydroxycyclohexyl)amino) octanoate). The crude product was purified by column chromatography (dichloromethane: methanol = 20:1 to 5:1) to obtain compound (IV-1-2-p).

### NMR data:

¹H NMR (600 MHz, CDCl₃) : δ 4.91-4.82 (m, 1H), 4.16-4.10 (m, 1H), 2.96-2.87 (m, 1H), 2.65-2.55 (qt, *J =* 11.2, 7.3 Hz, 2H), 2.28-2.26 (t, *J =* 7.5 Hz, 2H), 1.93-1.77 (m, 4H), 1.67-1.55 (m, 6H), 1.54-1.45 (m, 6H), 1.33-1.31 (m, 6H), 1.30-1.22 (m, 24H), 0.88 (t, *J =* 7.0 Hz, 6H).

LCMS:496.7[M+H]⁺.

1.5g (3.0 mmol) of the above compound (IV-1-2-p) was added into a 100 mL single-necked flask, 25 mL of acetonitrile and 15 mL of cyclopentylmethyl ether were added, then 1.67 g of potassium carbonate powder (12 mmol), 0.5g of potassium iodide (3 mmol) and 1.26 g of nonyl 8-bromooctanoate (3.6 mmol) were added, the reaction was carried out at 90° C for 24 h, the raw material compound (IV-1-2-p) was completely reacted, the reaction was removed from the oil bath and cooled to room temperature, the solid was removed by suction filtration, the filtrate was rotary dried to obtain a crude product, and finally purified by column chromatography (dichloromethane: methanol = 20:1 to 5:1) to obtain compound (IV-1-2).

### NMR data:

¹H NMR (600 MHz, CDCl₃): δ 4.91-4.80 (m, 1H), 4.28-4.22 (m, 1H), 4.06-4.04 (t, *J =* 6.8 Hz, 2H), 3.03-2.97 (m, 1H), 2.47-2.41 (m, 4H), 2.33-2.25 (q, *J =* 7.5 Hz, 4H), 1.89-1.85 (d, *J =* 12.1 Hz, 1H), 1.80-1.77 (t, *J =* 12.2 Hz, 1H), 1.73-1.65 (m, 2H), 1.64-1.59 (m, 6H), 1.54-1.48 (m, 4H), 1.48-1.39 (m, 4H), 1.34-1.29 (m, 14H), 1.29-1.23 (m, 38H), 0.89-0.87 (m, 9H).

### Specific rotation: -12.9°

LCMS: 765.2 [M+H]⁺.

### IV. Preparation of Compound (IV-1-3)

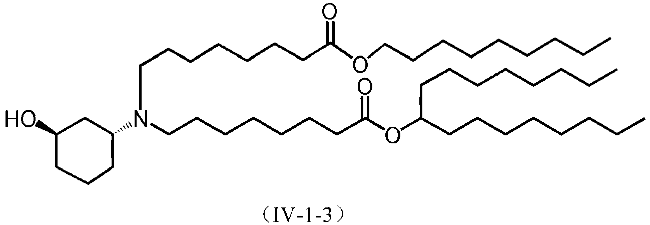

2g of 9-heptadecyl-8-bromooctanoate (4.3 mmol), 2.5 g of (1S, 3S)-3-aminocyclohexanol (20.2 mmol) and 20 mL of ethanol were added to a 100 mL single-necked flask and reacted at 50° C for 15 h, and the raw material 9-heptadecyl-8-bromooctanoate was completely reacted. After the solvent was removed by rotary evaporation, the obtained crude product was added with 50 mL of EA, washed twice with 25 mL of water, (1S, 3S)-3-aminocyclohexanol was thoroughly washed off, and the EA phase was rotary dried to obtain a crude product of the compound (IV-1-3-p) (i.e., 9-heptadecyl-8-(((1R,3R)-3-hydroxycyclohexyl)amino) octanoate). The crude product was purified by column chromatography (dichloromethane: methanol = 20:1 to 5:1) to obtain compound (IV-1-1-p).

### NMR data:

¹H NMR (600 MHz, CDCl₃): δ 4.91-4.81 (m, 1H), 4.17-4.08 (m, 1H), 2.94-2.84 (m, 1H), 2.64-2.5 (qt, *J =* 11.2, 7.3 Hz, 2H), 2.28-2.26 (t, *J =* 7.5 Hz, 2H), 1.88-1.76 (m, 2H), 1.75-1.54 (m, 8H), 1.54-1.40 (m, 6H), 1.33-1.3 (m, 6H), 1.30-1.23 (m, 24H), 0.88 (t, *J =* 7.0 Hz, 6H).

LCMS: 496.7[M+H]⁺.

1.5g (3.0 mmol) of the above compound (IV-1-3-p) was added into a 100 mL single-necked flask, 25 mL of acetonitrile and 15 mL of cyclopentylmethyl ether were added, then 1.67 g of potassium carbonate powder (12 mmol), 0.5g of potassium iodide (3 mmol) and 1.26 g of nonyl 8-bromooctanoate (3.6 mmol) were added, the reaction was carried out at 90° C for 24 h, the raw material compound (IV-1-3-p) was completely reacted, the reaction was removed from the oil bath and cooled to room temperature, the solid was removed by suction filtration, the filtrate was rotary dried to obtain a crude product, and finally purified by column chromatography (dichloromethane: methanol = 20:1 to 5:1) to obtain compound (IV-1-2).

### NMR data:

¹H NMR (600 MHz, CDCl₃): δ 4.89-4.82 (m, 1H), 4.24-4.22 (m, 1H), 4.06-4.04 (t, *J =* 6.8 Hz, 2H), 3.00-2.95 (m, 1H), 2.47-2.36 (m, 4H), 2.30-2.26 (q, *J =* 7.5 Hz, 4H), 1.86-1.84 (d, *J =* 12.1 Hz, 1H), 1.79-1.77 (t, *J =* 12.2 Hz, 1H), 1.70-1.66 (m, 2H), 1.65-1.57 (m, 6H), 1.53-1.48 (m, 4H), 1.45-1.38 (m, 4H), 1.35-1.30 (m, 14H), 1.29-1.22 (m, 38H), 0.89-0.87 (m, 9H).

### Specific rotation: +12.6°

LCMS: 765.2 [M+H]⁺.

### V. Preparation of Compound (IV-1-4)

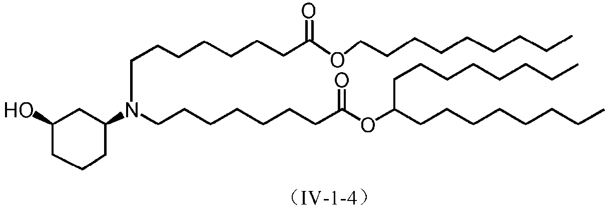

8g (17.3 mmol) of 9-heptadecyl-8-bromooctanoate, 10 g (76.5 mmol) of (1S, 3R)-3-aminocyclohexanol and 100 mL of ethanol were added to a 250 mL single-necked flask, and reacted at 50° C for 15 h, and the raw material 9-heptadecyl-8-bromooctanoate was completely reacted. After the solvent was removed by rotary evaporation, the obtained crude product was added with 50 mL of EA, washed twice with 25 mL of water, (1S,3S)-3-aminocyclohexanol was thoroughly washed off, and the EA phase was rotary dried to obtain a crude product of the compound (IV-1-4-p) (i.e., 9-heptadecyl-8-(((1S, 3R)-3-hydroxycyclohexyl)amino) octanoate).

### NMR data:

¹H NMR (600 MHz, CDCl₃) : δ 4.90-4.83 (m, 1H), 3.86-3.82 (m, 1H), 2.86-2.82 (m, 1H), 2.69-2.58 (qt, *J =* 11.2, 7.3 Hz, 2H), 2.29-2.26 (t, *J =* 7.5 Hz, 2H), 1.94-1.86 (m, 1H), 1.79-1.77 (d, *J =* 12.0 Hz, 1H), 1.74-1.64 (m, 3H), 1.64-1.59 (m, 3H), 1.51-1.46 (m, 6H), 1.36-1.30 (m, 8H), 1.30-1.22 (m, 24H), 0.88 (t, *J =* 7.0 Hz, 6H).

LCMS: 496.7[M+H]⁺ .

6g (12.1 mmol) of the above compound (IV-1-4- p) was added into a 250 mL single-necked flask, 90 mL of acetonitrile and 60 mL of cyclopentylmethyl ether were added, then 6.7 g of potassium carbonate powder (48.4 mmol), 2 g of potassium iodide (12.1 mmol) and 5 g of nonyl 8-bromooctanoate (17.4) were added, the reaction was carried out at 90° C for 24 h, the raw material compound (IV-1-4-p) was completely reacted, the reaction was removed from the oil bath and cooled to room temperature, the solid was removed by suction filtration, the filtrate was rotary dried to obtain a crude product, and finally purified by column chromatography (dichloromethane: methanol = 20:1 to 5:1) to obtain compound (IV-1-4).

### NMR data:

¹H NMR (600 MHz, CDCl₃) : δ 4.90-4.82 (m, 1H), 4.05 (t, *J =* 6.8 Hz, 2H), 3.70-3.60 (m, 1H), 2.64-2.54 (m, 1H), 2.51-2.41 (m, 4H), 2.30-2.26 (q, *J =* 7.5 Hz, 4H), 1.95 (m, 1H), 1.91-1.77 (m, 2H), 1.69-1.56 (m, 7H), 1.50 (d, *J =* 6.0 Hz, 4H), 1.41-1.37 (m, 4H), 1.34-1.18 (m, 52H), 0.88 (m, 9H).

### Specific rotation: - 4.5°

LCMS: 765.2[M+H]⁺ .

### Example 2. Design of Mutants of RSV F Protein and Coding Sequence Thereof

The mutants of RSV F protein as shown in Table 1 was designed, and the codon-optimized DNA sequence (the DNA sequence corresponding to the coding region of RSVF protein in Table 1) was further designed according to the amino acid sequence of the mutant, and the synthesis was entrusted to GenScript Biotech, and the detection showed that all DNA sequences were correct.

### Example 3. Construction of Engineered Plasmids

The method for the construction was as follows:
1. Construction of Plasmid B: The Amp resistance gene in plasmid pcDNA3.1 was replaced with the Kana resistance gene, new restriction sites HindIII, BamHI, KpnI and ApaI were further added subsequent to T7 promoter, and the NeoR/KanR sequence starting from 2136 bp to 2930 bp were deleted to obtain plasmid B, which was conducted by GENEWIZ.
2. Construction of Plasmid C: Plasmid B was linearized with ApaI, and plasmid C was obtained by the homologous recombination of the linearized Plasmid B with the nucleic acid fragment having a nucleotide sequence as shown in SEQ ID NO: 211, which was synthesized by IDT (Integrated DNA Technologies).
3. Construction of plasmid D: Plasmid C was double-digested with KpnI and ApaI, the large fragment of about 4.7 kb was recovered by agarose gel electrophoresis, and then ligated with a nucleic acid fragment having a nucleotide sequence as shown in SEQ ID NO: 210, which was synthesized by IDT, with T4 enzyme to obtain plasmid D.
4. Construction of Plasmid F: Plasmid D was double-digested with BamHI and HindIII, a large fragment of about 4.8 kb was recovered by agarose gel electrophoresis, and then ligated with a nucleic acid fragment having a nucleotide sequence as shown in SEQ ID NO: 209, which was synthesized by IDT, with T4 enzyme to obtain plasmid F.
5. Construction of engineered plasmids: homologous arm sequences were introduced at the ends of the DNA sequences corresponding to the protein coding regions of different mutants of RSV F protein (see Table 1 for details) by PCR, and then the PCR products and plasmid F were subjected to BamHI and KpnI double enzyme digestion, the large fragment of about 4.8 kb was recovered for homologous recombination, and finally a variety of engineered plasmids were obtained, which were completely correct in sequencing.

### Example 4. Preparation of mRNA Encoding Mutants of RSV F Protein

1. The engineered plasmid was extracted, ensuring that the superhelical rate of the plasmid was 85% or more.
2. Linearization of Plasmid
(1) Linearization by Enzymatic Digestion: 20µg of different engineered plasmids containing the coding sequence for the mutants of the RSV F protein as prepared in Example 3 were taken to prepare the reaction systems according to Table 2, which were blended, and placed in an incubator at 37° C for enzymatic digestion reaction for 16 h.

**Table 2**

| | |
|---|---|
| Plasmid | 20µg |
| Buffer | 8µL |
| Endonuclease (BsaI) | 1µL |
| H₂O | Supplementing to 80µL in total |

(2) Purification of Linearized Plasmid: After complete enzymatic digestion, the linearized plasmid was recovered using a DNA fragment purification kit (Takara, Cat. No. 9761).
(3) Identification: 100 ng of purified linear plasmid and original circular plasmid were subjected to 1% agarose gel electrophoresis to confirm whether the plasmid was completely linearized.
3. In Vitro Transcription of mRNA
(1) The IVT reaction system was prepared according to Table 3.

**Table 3**

| H₂O | Supplementing to 100µL in total |
|---|---|
| Linearized plasmid | 5µg |
| 10 × Reaction Buffer | 10µL |
| ATP (75mM) | 10µL |
| GTP (75mM) | 10µL |
| CTP (75mM) | 10µL |
| N1-Me-Pseudo UTP (100mM) | 7.5µL |
| T7 RNA polymerase | 10µL |

(2) The reaction system was reacted at 37° C for 2 h, 5µL of DNase was added to the reaction system at the end of the reaction, and incubated at 37° C for 15min (removing the linearized plasmid template with DNase).
(3) The above IVT product was subjected to magnetic bead purification according to the instructions of magnetic beads for purification (Hieff NGS ^{®} RNA Cleaner, YEASEN, Cat. No. 12602 ES56).
(4) Capping:
The mRNA Cap1-type capping reaction:
The structure and reaction principle of the Cap1 type cap are as follows:
pppN1(p)Nx-OH(3') → ppN1(pN)x-OH(3')+Pi
ppN1(pN)x-OH(3') + GTP → G(5')ppp(5')N1(pN)x-OH(3') + PPi
G(5')ppp(5')N1(pN)x-OH(3') + AdoMet → m7G(5')ppp(5')N1(pN)x-OH(3') + AdoHyc m7GpppN1(pN)x-OH(3') + AdoMet → m7Gppp[m2'-O]N1(pN)x-OH(3') + AdoHyc 5'-Cap1-type cap structure:
   cap G¹G² = m⁷G-5'-ppp-5'-Gm²'-3'-p-[m⁷ = 7-CH₃; m^{2'} = 2'-O-CH₃; -ppp- = -PO₂H-O-PO₂H-O-PO₂H)-; -p- = -PO₂H-].
   67µL of an aqueous solution of mRNA (25 pmol capless mRNA supplemented with water to 67µL) was preheated at 65°C for 5 min, then mixed with a mixture comprising 10µL of 10× ScriptCap Casing Buffer, 10µL of 10 mM GTP, 2.5µL of 20 mM SAM, 2.5µL of ScriptGuard RNase Inhibitor, 4µL of ScriptCap 2' -O- Methyltransferase (100 U/µL) and 4µL of ScriptCap Casing Enzyme (10 U/µL), and incubated at 37° C for 1 h. The above reagents for the capping reaction were purchased from Cellscript.

(5) Purification of Capped Product:
The capped product was purified by magnetic beads according to the instructions of magnetic beads for purification (Hieff NGS ^{®} RNA Cleaner, YEASEN, Cat. No. 12602 ES56). Characterization data: the concentration was determined by onedrop or Qubit.

Results of Experiment: mRNAs encoding different mutants of RSVF protein containing Cap1 type cap structure, 5'-UTR, 3'-UTR, poly(A) tail with all uridine replaced by N1-methylpseudouridine were obtained, and the amino acid sequence of the mutants of RSV F protein upon the translation of the coding region for protein in each mRNA was shown in Table 1.

### Example 5. Immunization Experiment on Mice with Encapsulated RSV mRNA Vaccine

(1) Encapsulation: mRNA was encapsulated by using a microsyringe and a microfluidic chip (SN. 000038) at a speed of 9 mL/min in an aqueous phase and 3 mL/min in an alcohol phase, to prepare a crude product of LNP preparations containing mRNA encoding the mutant of RSV F protein , where the LNP preparations differs from each other in the encapsulated mRNA only, the mRNA used in this Example was prepared according to the method described in Example 4, and the compositions of the aqueous phase and the alcohol phase of each LNP preparation are shown in Table 4 below.

**Table 4**

| Aqueous phase (mRNA) | Mass/mg | Concentration of mRNA sample | mRNA volume/µL | Volume of Acetic acid-sodium acetate buffer (pH5.0) /µL | Volume of DEPC water/µL | Total volume of aqueous phase /µL |
|---|---|---|---|---|---|---|
| | 1 | 2.78µg/µL | 360 | 469 | 2921 | 3750 |
| | Composition | Concentration of stock solution(in ethyl alcohol) | Molar mass% | Mass/mg | Required volume of working solution/µL | Total volume of alcohol phase /µL |
| Alcohol phase | Compound (IV-1-1 ) of Exampe 1 (MW: 764.27) | 20.0 mg/mL | 49.5% | 10 | 495 | 1250 |
| | DSPC ( MW : 790.14) | 10.0 mg/mL | 10% | 2.07 | 207 | |
| | CHO-HP (MW: 386.7) | 20.0 mg/mL | 39% | 3.9 | 197 | |
| | M-DMG2000 (MW: 2650) | 25.0 mg/mL | 1.5% | 1.04 | 42 | |
| | EtOH ( MW : 46.07 ) | | NA | NA | 309 | |

(2) Solution Exchange by Dialysis:
Preparation of Dialysis Solution: 1× PBS+8% (m/V) sucrose solution: 2 packets of 1× PBS prefabricated powder were transferred into a beaker, dissolved with 2L DEPC water and blended, 160 g sucrose was subsequently added to obtain 1× PBS+8% sucrose solution upon blending.
(b) Dialysis: the encapsulated crude product was loaded into a 100 KD dialysis bag, immersed in a beaker containing 1L of dialysis solution, which was wrapped with aluminum foil paper and the dialysis was performed at 100 rpm for 1 h at room temperature, and then the dialysis was continued for 1 h after replacing the dialysis solution. Upon dialysis, the preparation samples were sterilized and filtered using a 0.22µm disposable filter membrane to prepare various LNP preparations encapsulating mRNA encoding mutants of RSV F protein, wherein the mRNA concentration in the LNP preparation was 0.2µg/µL, the mass ratio of mRNA: Lipid was 1:10, the particle size was 80 nm- 130 nm, and the encapsulation rate was 85% or more.

(3) In vivo Immunization: 7-9 weeks old Balb/c female mice (17g-22 g, adaptive feeding in SPF environment) were divided into a blank LNP group and a test group. The test samples were intramuscularly injected at 10µg/mouse, where each test sample included an equal amount of LNP preparation encapsulating mRNA encoding a mutant of F protein of subtype A RSV and LNP preparation encapsulating mRNA encoding a mutant of F protein of subtype B RSV with the same mutation, and the blank LNP group was injected with LNP preparation without mRNA, for example, the LNP preparation encapsulating 5µg of mRNA numbered as 832 and the LNP preparation encapsulating 5µg of mRNA numbered as 973 were mixed and then injected into mice, the time of the injection was recorded as Day 0, and blood was collected on Day 7, Day 14, Day 21 (or Day 19), Day 28 (or Day 31) and Day 35 after immunization (after injection), the blood was collected using a non-anticoagulant tube, and placed on ice for 30 minutes, followed by centrifugation at 4° C, 3500 rpm for 10 minutes, and the upper pale yellow liquid was pipetted after stratification to prepare immunoserum.

The spleens of a part of test batches of mice were collected on Day 28 and Day 35, respectively, and the splenocytes were isolated for subsequent ELISpot detection (Example 8).

### Example 6. Experiment for IgG Antibody Titer

The immunoserum used in this Example was provided by the experiment in Example 5.

Reagents: Human respiratory syncytial virus (RSV) (A2) Fusion glycoprotein/RSV-F Protein (ECD, His Tag), Human respiratory syncytial virus (RSV) (B, strain 18537) Fusion glycoprotein F0/RSV- F Protein (His Tag), Goat anti-Mouse IgG (H+L) HRP Conjugate, PBS, FBS, single component TMB developing solution, stop solution.

The operation steps include:
(1) The F proteins of subtype A RSV or subtype B RSV were diluted to 1µg/mL with a coating solution and blended for standby; added to a 96-well ELISA plate at 100µL per well, sealed with a plate sealing membrane, and placed at 2-8°C overnight (16-20 hours).
(2) After incubation, the plate was subjected to machine wash, 300µL/well, for 3 times with washing solution, and drying on clean paper.
(3) The ELISA plate was added with a blocking solution at 250µL/well, sealed with a sealing membrane, and incubated at 37° C for 60 min.
(4) After blocking, the plate was subjected to machine wash, 300µL/well, for 3 times with washing solution, and drying on clean paper.
(5) The serum separated in advance was taken out and mixed by vortex for IgG titer detection.
(6) The separated serum was taken, the initial dilution factor was determined according to different immunization times, and the dilution factor was used as the first dilution factor for gradient dilution. The diluted serum was added to an ELISA plate, 100µL/well, and incubated at 37°C for 2 h.
(7) After incubation, the plate was subjected to machine wash, 300µL/well, for 3 times with washing solution, and drying on clean paper.
(8) The enzyme-labeled secondary antibody was diluted by 10,000 folds with the sample diluting solution, 100µL/well, and incubated at 37° C for 60 min.
(9) After incubation, the plate was subjected to machine wash, 300µL/well, for 3 times with washing solution, and drying on clean paper.
(10) The TMB single-component developing solution was equilibrated to room temperature in advance, 100µL was added to each well, and incubated at room temperature in the dark for 15 min.
(11) At the end of development, 50µL/well of stop solution was added.
(12) A detection wavelength of 450 nm and a reference wavelength of 630 nm were selected for reading and analysis in a microplate reader.

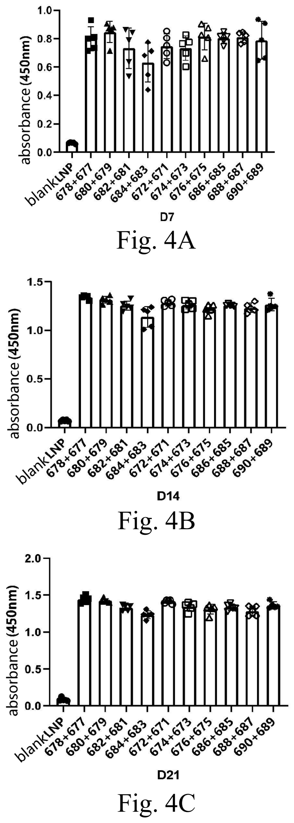

The detection of IgG antibody titers for RSV mRNA vaccines containing different mRNAs are shown in FIG. 1A to FIG. 6B, except for the RSV mRNA vaccine represented by 573+ 567, other RSV mRNA vaccines induce IgG antibodies against F protein of RSVsubtype A and/or F protein of RSV subtype B that are significantly higher than those in the blank LNP group (the LNP group without encapsulating mRNA).

In addition, according to the above steps, the LNP preparation encapsulating 0.1µg of mRNA numbered as 832 and the LNP preparation encapsulating 0.1µg of mRNA numbered as 973 were mixed to obtain a bivalent mRNA vaccine (832+ 9730.2µg), the bivalent mRNA vaccine was injected into BALB/c mice on D0 and D21, and the immune serum was collected on the 14th day (D14) after the first immunization (D0), on the 7th day (D28) and the 14th day 14 (D35) after the second immunization (D21), and the IgG antibody titer was detected. At the same time, the IgG antibody titers for other doses of the bivalent mRNA vaccine were detected, and the other doses of the mRNA vaccine were: a bivalent mRNA vaccine (832+ 9732 µg) obtained by mixing an LNP preparation encapsulating 1µg of mRNA numbered as 832 and an LNP preparation encapsulating 1µg of mRNA numbered as 973, and a bivalent mRNA vaccine (832+ 97310µg) obtained by mixing an LNP preparation encapsulating 5µg of mRNA numbered as 832 and an LNP preparation encapsulating 5µg of mRNA numbered as 973, and the results were shown in FIG. 1E.

### Example 7. RSV-Luc5 Pseudovirus Neutralization Experiment

The immunoserum required for the pseudovirus neutralization assay was provided by the experiment of Example 5.

Reagent and consumables: RSV-Luc5 pseudovirus (6.2 log₁₀TCID 50/mL), mouse serum inactivated by incubation at 56° C for 30 min, Hep2 cells, DMEM, FBS, detecting reagents for Luciferase reporter gene.

The operation steps include:
(1) Hep2 cells (3× 10⁴ cells/well) were added to 96-well white plate and cultured in DMEM medium containing 5% FBS for 24 hours.
(2) Serum samples were diluted three folds with DMEM without FBS with an initial dilution of 1:30. 310µL of serum-free DMEM medium was added to 96-well deep well plates H7-H12 as cell control (CC), and 155µL of DMEM medium was added to H1-H6 as virus control (VC).
(3) 233µL of diluted serum sample was added to A1-A12 as the initial diluent of the sample, and 155µL of DMEM serum-free medium was added to 1-12 of B, C, D, E, F, and G. 78µL of volume was taken from A1 to A12 respectively to the next row and blended, then 3 folds gradient dilution was performed, there were 7 gradients in total, and the excess 78µL of liquid in the last row was discarded.
(4) 155µL of diluted virus was added to each well except H7-H12 and mixed, the titer of the mixed virus was diluted to 1800 TCID50/mL, and incubated at 37° C for 1 h;
(5) 93µL of mixed solution was added to the 96-well white plate with Hep2 cells coated one day in advance (the cells were washed once in advance with PBS), and cultured for 24 h, three replicate wells were arranged for each dilution .
(6) The 96-well white plate was taken out, the medium was pepitted from the 96-well plate, 100µL of PBS was added for washing once, then 14µL of 1 x lysis buffer was added for incubation at room temperature for 5 minutes, and after incubation on ice for 5 minutes, 70µL of luciferase substrate was added, immediately followed by detecting the chemiluminescence value (RLU) with a microplate reader.

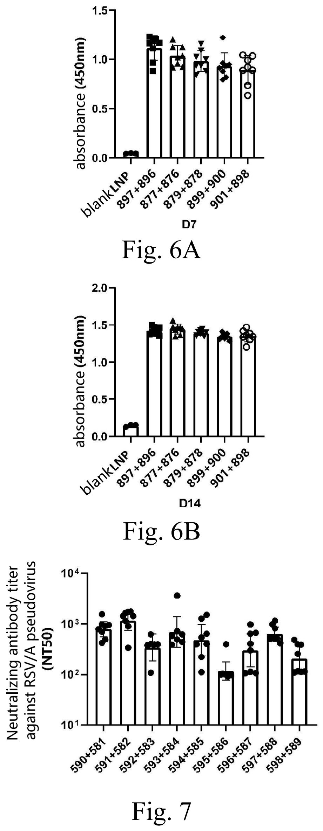

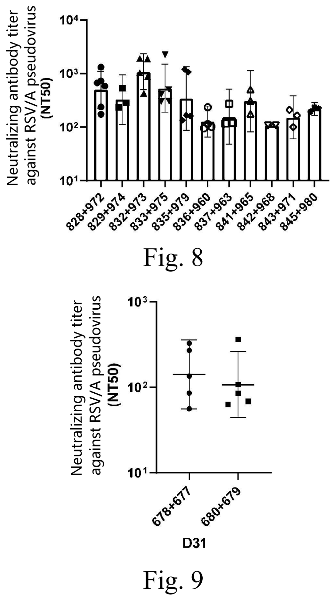

The detection of neutralizing antibody titers of RSV mRNA vaccines containing different mRNA combinations are shown in FIG. 7 to FIG. 9 and FIG. 20.

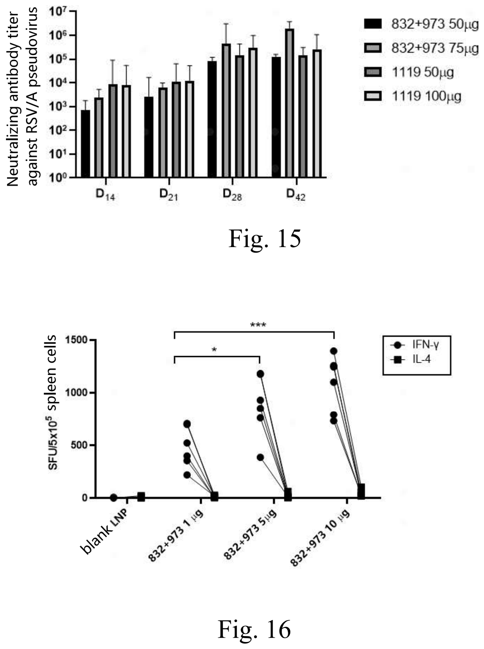

In addition, by reference to the above steps, the LNP preparation encapsulating 25µg of mRNA numbered as 832 and the LNP preparation encapsulating 25µg of mRNA numbered as 973 were mixed to obtain a bivalent mRNA vaccine (832+97350µg), the mRNA vaccine was injected into rats at D0, immune sera on day 14 (D14) and day 21 (D21) after the first immunization (D0) were collected, the mRNA vaccine was injected once more after the sampling on day 21, immune sera on day 7 (D28) and on day 21 (D42) after the second immunization (D21) were collected, and the pseudovirus neutralizing antibody titer was detected. At the same time, the IgG antibody titers of other mRNA vaccines were detected, and the other mRNA vaccines were: a divalent mRNA vaccine (832+ 97375µg) obtained by mixing an LNP preparation encapsulating 37.5µg of mRNA numbered as 832 and an LNP preparation encapsulating 37.5µg of mRNA numbered as 973, an LNP preparation encapsulating 50µg of mRNA numbered as 1119 (1119 50µg), and an LNP preparation encapsulating 100µg of mRNA numbered as 1119 (1119 100µg), and the results were shown in FIG. 15.

### Example 8. ELISpot Experiment on Mouse Splenocytes

Mouse splenocytes required for the ELIS pot experiment were provided by Example 5 experiment.

Reagents: RPMI Medium 1640 basic (1×), mouse lymphocyte isolation solution, positive reference PMA+Ionomycin, ELISpot Plus Mouse IFN-gamma (HRP), RSV F peptide library, ELISpot Plus Mouse IL-4 (HRP), erythrocyte lysis solution.

The operation steps include:
(1) The mice were sacrificed by neck breaking, soaked in 75% ethanol, and the spleens of the mice were taken out in the ultra-clean hood.
(2) In a 6-well cell culture plate, 7 mL of mouse lymphocyte separation solution was added to each well (recovered to room temperature and shaken up before use). During grinding with a syringe piston, the upward rebound force of the cell sieve was used to control the grinding force and minimize possible mechanical damage to the cells.
(3) The isolation solution with spleen cells suspended therein was immediately transferred to a 15 mL centrifugation tube, and 10 mL of RPMI 1640 medium was slowly added, keeping the liquid level boundary obvious.
(4) Centrifuging with a horizontal rotor at 800g for 30 min at room temperature.
(5) The lymphocyte layer was pepitted, and then, 10 mL of RPMI 1640 medium was added for washing by overturning the tube. Cells were collected by the centrifugation at 250g for 10 min at room temperature.
(6) The erythrocytes were lysed according to the instructions of the erythrocyte lysis solution. 2 mL of the erythrocyte lysis solution was added to each tube for resuspending. After placing at 4° C for 2 minutes, 10 mL of DPBS was added to terminate the erythrocyte lysis reaction. After centrifugation at 250g of 4° C for 10 minutes, and then cells were collected.
(7) 10 mL of RPMI 1640 medium was added to the cells collected in the above step for washing by overturning the tube. Cells were collected by centrifugation at 250g for 10 min at room temperature.
(8) Finally, the cells were resuspended with culture medium, counted and diluted.
(9) The cell suspension at an adjusted concentration was added to each test well, 1× 10⁵ cells/100µL/well.
(10) Stimulators (PMA+Ionomycin working solution and F protein peptide library (1µg/mL)) were added, 10µL/well.
(11) After all samples and irritants were added, the plate was covered a lid. The cells were incubated in a 37° C, 5% CO₂ incubator for 21 hours.
(12) The liquid was thrown out of the well, PBS buffer was added, 200µL/well, which was repeated five times with drying on absorbent paper each time.
(13) The detection antibody was diluted to 1µg/mL with the solution for diluting sample, 100µL/well. The mixture was incubated at room temperature for 2 hours.
(14) The above plate washing operation was repeated.
(15) Enzyme-labeled avidin (Streptavidin-HRP) was diluted for 1000 folds with the solution for diluting sample, 100µL/well, followed by incubation at room temperature for 1 hour.
(16) The above plate washing operation was repeated.
(17) The TMB substrate developing solution was equilibrated to room temperature in advance, 100µL was added to each well, followed by incubation at room temperature in dark for 10 min.
(18) The liquid in the wells was poured, the base of the plate was opened, and the front and back surfaces and the base were washed with deionized water for 3-5 times to terminate the development. The plate was placed in a shady and dark place at room temperature, and the base was closed after the plate was naturally dried.
(19) The plate was read with an enzyme-linked immunospot analyzer for various parameters of the spots and statistical analysis was performs.

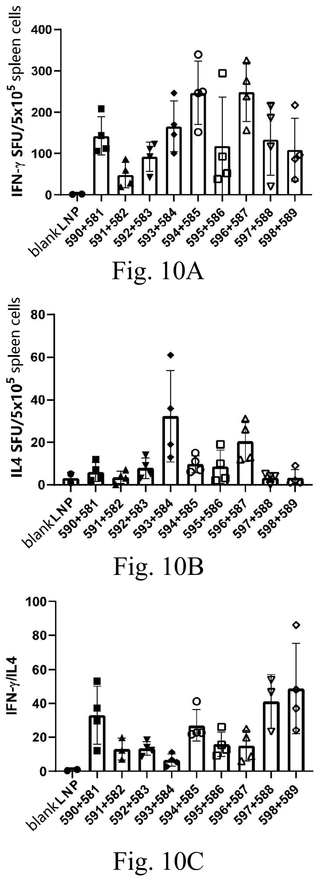

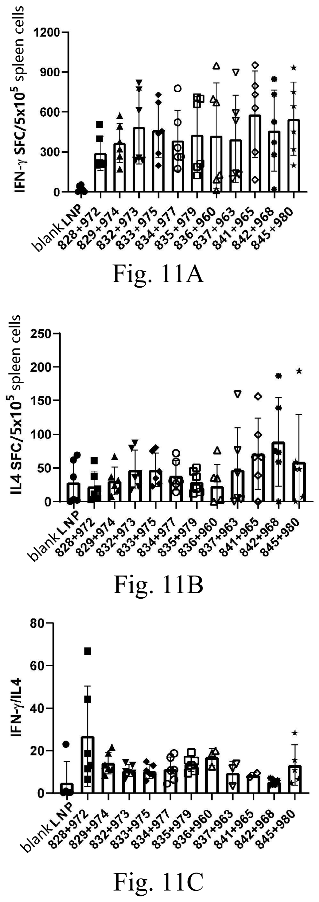

The Elispot detection of RSV mRNA vaccines containing different mRNAs was shown in FIG. 10A to FIG. 11C, the RSV mRNA vaccines containing different mRNAs could induce the production of significant T cell immune responses in the body, and the frequency of F protein-specific cells secreting IFN-γ was significantly higher than the frequency of F protein-specific cells secreting IL-4, indicating that the immune response is Th1 biased rather than Th2 biased.

In addition, by reference to the above steps, the LNP preparation encapsulating 0.5µg of mRNA numbered as 832 and the LNP preparation encapsulating 0.5µg of mRNA numbered as 973 were mixed to obtain a bivalent mRNA vaccine (832+973 1µg), the mRNA vaccine was injected into BALB/c mice on D0 and D21, spleen cells were taken on D28 for ELISpot detection, and the titer of IgG antibodies of blank LNP (i.e., the LNP preparation not encapsulating mRNA) and other doses of the bivalent mRNA vaccine was detected at the same time, the other doses of the bivalent mRNA vaccine were: a bivalent mRNA vaccine (832+973 5µg) obtained by mixing 2.5µg of mRNA numbered as 832 and 2.5 µg of LNP preparation encapsulating mRNA numbered as 973, and a bivalent mRNA vaccine (832+973 10µg) obtained by mixing LNP preparation encapsulating5µg of mRNA numbered as 832 and 5µg of LNP preparation encapsulating mRNA numbered as 973, and the results were shown in FIG. 16.

### Example 9. Detection of In Vitro Conformation of Protein

Reagents: Lipofectamine^{™} MessengerMAX^{™} transfection reagent, Opi-MEM^{™} I reduced serum medium, DMEM basic (1×), FBS, TrypLE Express, DPBS, AM14 (Cambridge Bio, Cat No.: 01-07-0119), D25 (Cambridge Bio, Cat No.: 01-07-0120), AM22 (Cambridge Bio, Cat No.: 01-07-0144), clone 131-2A (Sigma-Aldrich, Cat No.: MAB8599), PE anti-human IgG Fc Recombinant Antibody (PE-IgG), CR 9501(Cambridge Bio, Cat No.: 01-07-0145).

The operation steps include:
(1) The various engineered plasmids expressing different mutants of RSVF protein prepared in Example 3 or the various mRNAs expressing different RSV F protein mutants prepared in Example 4 were transfected into HKE293 cells with Lipofectamine^{™} MessengerMAX^{™} transfection reagent, and the transfected cells were incubated in a CO₂ incubator at 37°C for 24h.
(2) The cell supernatant was removed, 1 mL of cell digestion solution was added to each dish, the cells were digested at room temperature for 1 min, 1 mL of DPBS was added to terminate the digestion, the cells were pipetted into a 1.5 mL centrifuging tube, centrifuged at 1000 rpm for 3 min, and the supernatant was discarded.
(3) After the supernatant was discarded, 1 mL of DPBS was added for resuspension. After centrifugation at 1000 rpm for 3 min, the supernatant was discarded.
(4) 100µL of DPBS containing primary antibody (AM14, D25, AM22, clone 131-2A) was added to each well, followed by staining at 4° C for 60 min in the dark, and centrifugation at 1000 rpm for 3 min. The supernatant was discarded.
(5) The cells were washed once with 200µL of DPBS per well. After centrifugation at room temperature for 3 min at 1300 rpm, the supernatant was discarded.
(6) 100µL of DPBS containing the secondary antibody (PE-IgG) was added, followed by staining at 4°C for 30 min in the dark, and centrifugation at 1300 rpm for 3 min at room temperature. The supernatant was discarded.
(7) The cells were washed once with 200µL of DPBS per well. After centrifugation at at room temperature for 3 min at 1300 rpm, the supernatant was discarded, and the cells were resuspended with 200µL of DPBS, and detected with equipment.

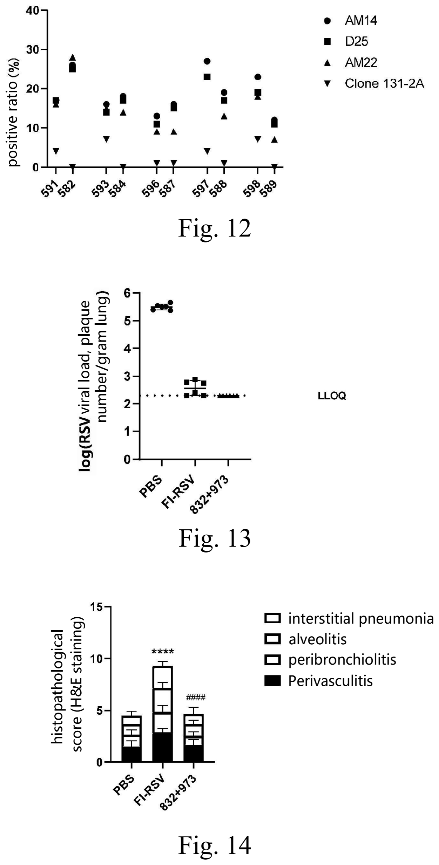

The detection of the conformation of F proteins expressed by different mRNAs were shown in FIGs. 12, 17A-17B and 21A-21D, in which group 7 was the test group expressing Post F (Jason S. McLellan, et al., J Virol., 2011 Aug; 85(15): 7788-7796.).

As shown in FIG. 12, the proportion of cells bound to the antibodies AM14, D25 and AM22 was much higher than the proportion of cells bound to the antibody Clone 131-2 A. It can be seen that the F proteins expressed by the mRNAs were mainly in the pre-fusion conformation, i.e., the F proteins expressed by the mRNA constructs were mainly the pre-F protein that can be used as the ideal vaccine antigen.

As shown in FIGs. 17A-17B both the RSV/A subtype F protein expressed by cells transfected with the mRNA numbered as 832 and the RSV/B subtype F protein expressed by cells transfected with the mRNA numbered as 973 could be recognized by the four pre-F specific antibodies D25, AM22, CR9501 and AM14, indicating that the F proteins encoded by the mRNAs numbered as 832 and 973 were in the pre-F conformation. At the same time, the F protein produced by cells transfected with the mRNA encoding the RSV/A subtype Post-F protein could not be recognized by any one of the used antibodies, which met expectation and synchronously verifies the pre-F specificity of the above four antibodies.

As shown in FIG. 21A to FIG. 21D, most of the F proteins expressed by the mRNAs (such as the mRNAs numbered as 1998, 2000, 2001, 2002, 2003, 2006, 2007, 2008, 2009, and 2010) were mainly in the pre-fusion conformation.

### Example 10. Challenge Experiment in Mice

The 5-week-old to 6-week-old Balb/c female mice (adaptively fed in SPF environment) were divided into three groups: PBS group, FI-RSV group (formalin inactivated RSV) and mRNA vaccine group, with 6 mice in each group. On day 0 and day 21 of the experiment, the mice were inoculated with PBS buffer, FI-RSV (100 ng/mouse) and mRNA vaccine (10µg/mouse) by intramuscular injection, and the mice were inoculated with RSV/A2 virus by nasal drip on day 35 at a dose of 5.0E+05 PFU/mouse. On day 40, lung tissues were collected, viral titers in the lung tissues were detected by a plaque test, and lung tissue sections were H&E stained to detect inflammatory indicators in the lung tissues.
1. Detection of RSVtiter in lung tissue samples by plaque assay
   (1) Cell plating: 1 mL of HEp2 cells at a density of 3.0×10⁵ cells/mL was added to each well of a 12-well plate and placed in a CO₂ cell incubator overnight to obtain a monolayer of cells.
   (2) Sample incubation: the lung tissue samples collected on day 40 were melted and then grounded with a tissue homogenizer, the supernatant was collected after centrifugation, which was diluted with the experimental culture medium, 2 folds, 3 gradients, 6 gradients in total for the sample of group 1, and the last three dilutions were used for detection. 0.05 mL supernatant of tissue homogenate or a diluent thereof was added to a 12-well plate with cells plated, and the viruses were fully adsorbed by incubation in a cell incubator at 37° C for 4 hours; the liquid in the 12-well plate was replaced with medium containing agarose, and after the agar was solidified, the cells were incubated in the cell incubator at 37° C for 7 days.
   (3) Fixing and staining: on day 8, the cells were fixed with paraformaldehyde at room temperature for 4h, followed by removing the paraformaldehyde, and washing with water, then 0.2 mL/well of 0.5% crystal violet solution was added, followed by shaking on a shaker for 15 min, washing with water to remove crystal violet and drying.
   (4) Plaque counting: scanning the image and reading the number of plaques therein, and calculating the viral titer in the sample, where the viral titer is expressed as Log₁₀ (the number of plaques per gram of tissue homogenate).
2. Detection of inflammatory indicators in lung tissue by H&E staining

Pathological analysis was performed by sectioning and H&E staining lung tissues, the lung tissue lesion was assessed by H&E staining, including perivasculitis (PV), peribronchitis (PB), alveolitis (A), and interstitial pneumonia (IP). The specific operation steps were as follows: the lung tissue on day 40 was fully fixed, sampled, dehydrated, paraffin embedded and sectioned, then baked at 60-65°C, dewaxed, HE stained and sealed, and finally subjected to manual semi-quantitative scoring by reference to the scoring criteria in Table 5.

**Table 5. Scoring criteria for histopathological semi-quantitative scoring**

| **Description of histological features** | **Scoring** | **Criteria** |
|---|---|---|
| Alveolar region: Inflammatory infiltration of macrophages, lymphocytes, and neutrophils, etc. observed in the alveolar tissue | 0 | No visible lesions |
| | 1 | Mild |
| | 2 | Moderate |
| | 3 | Severe |
| | 4 | Very Severe |
| Perivascular: Inflammatory infiltration of macrophages, lymphocytes, and neutrophils etc. observed around the blood vessels | 0 | No visible lesions |
| | 1 | Mild |
| | 2 | Moderate |
| | 3 | Severe |
| | 4 | Very Severe |
| Peribronchial: Inflammatory infiltration of macrophages, lymphocytes, and neutrophils, etc. observed around the bronchioles | 0 | No visible lesions |
| | 1 | Mild |
| | 2 | Moderate |
| | 3 | Severe |
| | 4 | Very Severe |
| Interstitial: Inflammatory infiltration of macrophages, lymphocytes, and neutrophils etc. observed in the interstitial | 0 | No visible lesions |
| | 1 | Mild |
| | 2 | Moderate |
| | 3 | Severe |
| | 4 | Very Severe |

The results of the challenge experiment are shown in FIGS. 13-14. It can be seen from the results of lung RSV viral titers of mice immunized with PBS, FI- RSV and mRNA vaccine that as compared with the PBS group, the mRNA vaccine containing mRNA 832+973 could significantly reduce the RSV viral load in mouse lungs after challenge. It can be seen from the lung histopathology of mice immunized with PBS, FI-RSV and mRNA vaccine respectively that the mRNA vaccination group did not exhibit the enhanced RSV disease (ERD) as exhibited by the FI-RSV vaccination group, and the pathological scores of the mRNA vaccination group with respect to interstitial pneumonia, alveolitis, peribronchitis and perivasculitis were significantly reduced as compared to the FI-RSV vaccination group.

### Example 11 Challenge Experiment in Cotton Mouse

Animals: 6-8 weeks old cotton mice, female.

On day 0, the animals were divided into groups: 4 per group (1 group in total) and 6 per group (9 groups in total). All animals were numbered with ear marks, and blood was collected before immunization. Each group of animals were immunized by intramuscular injection or intranasal infection. Grouping, injection of test articles and doses are shown in Table 6 below.

**Table 6**

| Gro ups | Num ber | Day 0 | Day 28 | Dose | Administration route | Admini stered volume (ml)* | RSV A/A2 Challe nge (5 Log₁₀ pfu/mo use) | RSV B Challe nge (5 Log₁₀ pfu/mo use) | Sampl ing after Challe nge ( day s) |
|---|---|---|---|---|---|---|---|---|---|
| A | 4 | PBS | PBS | n/a | IM/IM | 0.1 | No | No | 5 |
| B | 6 | PBS | PBS | n/a | IM/IM | 0.1 | Yes | No | 5 |
| C | 6 | FI-RSV Lot#10 0 | FI-RSV | 100-fold diluted in PBS | IM/IM | 0.1 | Yes | No | 5 |
| D | 6 | RSV A/A2 Live | - | 5.0 Log₁₀ PFU | IN | 0.1 | Yes | No | 5 |
| E | 6 | 832+973 | 832+973 | 30 µg | IM/IM | 0.12 | Yes | No | 5 |
| F | 6 | 832+973 | 832+973 | 60 µg | IM/IM | 0.24 | Yes | No | 5 |
| G | 6 | RSV B | - | 5.0 Log₁₀ PFU | IN | 0.1 | No | Yes | 5 |
| H | 6 | PBS | PBS | n/a | IM/IM | 0.1 | No | Yes | 5 |
| I | 6 | 832+973 | 832+973 | 30 µg | IM/IM | 0.12 | No | Yes | 5 |
| J | 6 | 832+973 | 832+973 | 60 µg | IM/IM | 0.24 | No | Yes | 5 |

| | | | | | | | | | |
|---|---|---|---|---|---|---|---|---|---|
| "832+973" represents a divalent RSV mRNA vaccine, which is a mixture of LNP preparation encapsulating mRNA numbered as 832 and LNP preparation encapsulating mRNA numbered as 973; "IM" is intramuscular injection; and "IN" is intranasal vaccination of virus. | | | | | | | | | |

On day 28, blood was collected from the eye socket to obtain serum, followed by boosting the animals in groups A-C, E-F and H-J, and the test articles and doses are shown in Table 6. On day 49, blood was collected from the eye socket to obtain serum. Animals of Group A were intranasally infected with 0.1 ml of PBS, pH7.4. Animals of Groups B-F were intranasally infected with RSV A/A2(Lot # 092215 SSM), 0.1 ml, 10⁵ PFU per mouse. Animals of Groups G-J were intranasally infected with RSV B 18537 (Lot # 032417 SSM) 0.1 ml, 10 ⁵ PFU per mouse. Viral dilutions for challenge were back titerated to confirm the challenge dose. On day 54, all animals were sacrificed. Nasal tissue was collected and homogenized in 3 mL of HBSS containing 10% SPG to detect viral titer. The whole lung was collected and divided into three aliquots for viral titration (left side, homogenization in 3 ml of HBSS containing 10% SPG), pathological analysis (right side, perfusion of 10% neutral formalin fixed solution) and qPCR detection (lingula , rapid freezing in liquid nitrogen) were performed respectively.

### viral titers in Lung and Nasal Tissue

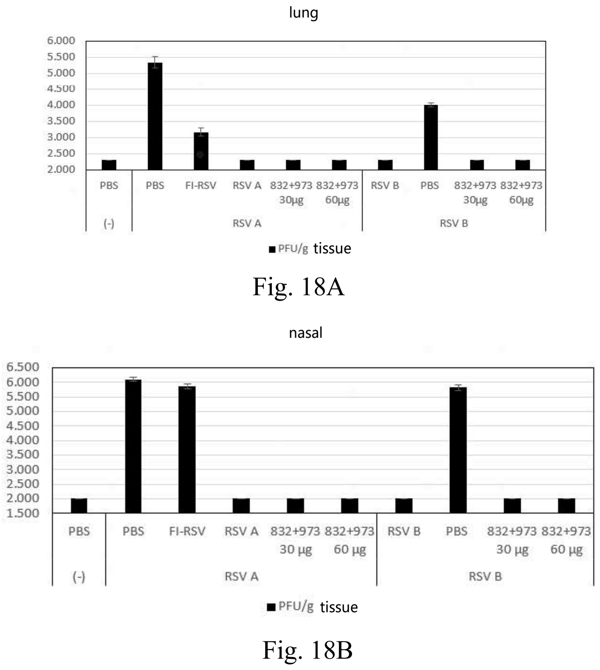

The lung and nasal tissue homogenate was centrifuged to obtain a supernatant, which was diluted with EMEM. In a 24-well plate, a monolayer of HEp-2 cells in wells were infected with diluted tissue homogenate. The cells were incubated in an incubator at 37°C, 5% CO₂, for 1 hour. The 24-well plate was covered with 0.75% methylcellulose medium. After 4 days of incubation, the methylcellulose medium was removed and the cells were fixed and stained with 0.1% crystal violet stain for 1 hour, then rinsed and air-dried. The number of plaques was read and the viral titer was expressed as the number of plaques per gram of tissue homogenate. Viral titer was calculated as geometric mean ± standard error. The results are shown in FIGS. 18A-18B.

Animals of PBS group showed the highest viral titer in lung tissues of the animals after challenge with respiratory syncytial virus RSV A/A2 (group B) or RSV B (group H) (5.3 Log₁₀PFU/g and 4 Log₁₀PFU/g for RSV A/A2 and RSV B, respectively), showing expected positive results. For the animals pre-infected with RSV A/A2 or RSV B (group D and G), after the infection with homologous virus again, the lung viral titer was lower than the lower detection limit, indicating that they were completely free of virus infection. The animals of FI-RSV Lot 100 group (group C) showed local protection in the lungs after RSV A/A2 challenge, and the viral titer decreased to about 2.1 Log₁₀PFU/g.

Animals of each 832+973 group were challenged with RSV A/A2 (groups E and F) or RSV B (groups I and J), the viral titer was lower than the lower detection limit, showing complete protection against lung viral titer, and no dose-dependence was shown.

Animals of PBS group showed the highest viral titer in the nasal tissues of the animals after challenge with respiratory syncytial virus RSV A/A2 (group B) or RSV B (group H) (6.0 Log₁₀PFU/g and 5.8 Log₁₀PFU/g for RSV A/A2 and RSV B, respectively), showing expected positive results. Animals pre-infected with RSV A/A2 or RSV B groups (D and G groups) were infected with homologous virus again, and the viral titer in nasal tissues was lower than the lower detection limit. Animals of FI-RSV Lot 100 group (group C) showed no significant protection on nasal tissues after RSV A/A2 challenge.

Animals of 832+973 groups showed complete protection of nasal tissues from viral infection after RSV A/A2(groups E and F) or RSV B (groups I and J) challenge, and there was no dose dependence.

### RSV Neutralization Antibody Experiment (60% Inhibition)

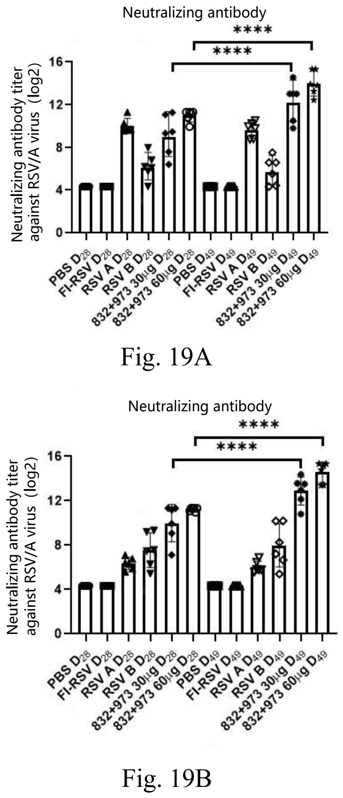

The serum samples were thermo-inactivated, diluted 10 folds with EMEM, and fold-diluted at 1:4. The diluted serum samples were mixed with RSV A/A2 or RSV B(25-50 PFU) and incubated at room temperature for 1 hour. The mixture was added to a 24-well plate plated with a monolayer of HEp-2 cells and incubated in an incubator at 37° C, 5% CO₂ for 1 hour. The 24-well plate was covered with 0.75% methyl cellulose medium. After incubation for 4 days (6 days for RSV B), the covering was removed, followed by fixing and staining with 0.1% crystal violet staining solution, and then, rinsing and air-drying. The corresponding neutralizing antibody titer was calculated using the statistical program "plqrd.manifeal.entry", which was expressed as the dilution at which 60% virus infection was inhibited. Viral titer was calculated as geometric mean ± standard error. The results are shown in FIGS. 19A-19B.

Neutralized antibody titers against two subtypes of RSV were significantly increased in the serum from animals immunized with 832+973 and were comparable to pre-infection-induced neutralizing antibody levels (anti-RSV A/A2, day 28) or better (anti-RSV B, day 28; anti-RSV A/A2 and RSV B, day 49). Within the dose range (30µg and 60µg), neutralizing antibodies induced by 832+973 exhibited slight dose dependence without significant difference (FIG. ANOVA, ns, p > 0.05). After the second immunization with 832+973, the neutralizing antibodies against RSV A/A2 and RSV B were significantly increased (as shown in FIG. ANOVA, * * * *, p < 0.0001).

### Sequence

SEQ ID NO:1 
MELPILKTNAITTILAAVTLCFASSQNITEEFYQSTCSAVSKGYLSCLRTGWYHSVITIELSNIK
ENKCNGTDAKVKLIKQELDKYKNAVTELQLLMQSTPAANSRARRELPRFMNYTLNNTKNT
NVTLSKKRKRRFLGFLLGCGSAIASGIAVSKVLHLEGEVNKIKSALLSTNKAVVSLSNGVSV
LTSKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEVLAYVVQLPLYGVCDTP
CWKLHTSPLCTTNTKEGSNICLTRTDRGWYCDNAGSVSFFPQAETCKVQSNRVFCDTMNS
LTLPSEVNLCNIDIFNPKYDCKIMTSKTDVSSSCITSLGAIVSCYGKTKCTASNKNRGIIKTFS
NGCDYVSNKGVDTVSVGNTLYYVNKQEGKSLYVKGEPIINFYDPLVFPSSEFDASISQVNEK
INQSLAFIRKSDELLSAIGGYIPEAPRDGQAYVRKDGEWVLLSTFL
SEQ ID NO:2 
MELLIHRSSAIFLTLAINALYLTSSQNITEEFYQSTCSAVSRGYFSCLRTGWYHSVITIELSNIK
ETKCNGTDTKVKLIKQELDKYKNAVTELQLLMQNTPAANNRARREAPQYMNYTINTTKNL
NVSISKKRKRRFLGFLLGCGSAIASGIAVSKVLHLEGEVNKIKNALLSTNKAVVSLSNGVSV
LTSKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEVLAYVVQLPIYGVCDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSCITSLGAIVSCYGKTKCTASNKNRGIIKTFSNGCD
YVSNKGVDTVSVGNTLYYVNKLEGKNLYVKGEPIINYYDPLVFPSSEFDASISQVNEKINQS
LAFIRRSDELLSAIGGYIPEAPRDGQAYVRKDGEWVLLSTFL
SEQ ID NO:3 
MELPILKTNAITTILAAVTLCFASSQNITEEFYQSTCSAVSKGYLSCLRTGWYHSVITIELSNIK
ENKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEVLAYVVQLPLYGVCDTP
CWKLHTSPLCTTNTKEGSNICLTRTDRGWYCDNAGSVSFFPQAETCKVQSNRVFCDTMNS
LTLPSEVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSSEFDASISQVNEK
INQSLAFIRKSDELLSAIGGYIPEAPRDGQAYVRKDGEWVLLSTFL
SEQ ID NO:4 
MELLIHRSSAIFLTLAINALYLTSSQNITEEFYQSTCSAVSRGYFSCLRTGWYHSVITIELSNIK
ETKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEVLAYVVQLPIYGVCDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSSEFDASISQVNEKINQS
LAFIRRSDELLSAIGGYIPEAPRDGQAYVRKDGEWVLLSTFL
SEQ ID NO:5 
MELPILKTNAITTILAAVTLCFASSQNITEEFYQSTCSAVSKGYLSACRTGWYHSVITIELSNI
KENKCNGTDAKVKLIKQELDKYKNAVTELQLLMQSTPAANSRARRELPRFMNYTLNNTK
NTNVTLSKKRKRRFLGFLLGCGSAIASGIAVSKVLHLEGEVNKIKSALLSTNKAVVSLSNGV
SVLTSKVLDLKNYIDKQLLPIVNKQSCSISNIETVIEFQQKNNRLLEITREFSVNAGVTTPVST
YMLTNSELLSLINDMPITNDQKKLMSSNVQIVRQQSYSIMSIIKEEVLAYVVQLPLYGCIDTP
CWKLHTSPLCTTNTKEGSNICLTRTDRGWYCDNAGSVSFFPQAETCKVQSNRVFCDTMNS
LTLPSEVNLCNIDIFNPKYDCKIMTSKTDVSSSCITSLGAIVSCYGKTKCTASNKNRGIIKTFS
NGCDYVSNKGVDTVSVGNTLYYVNKQEGKSLYVKGEPIINFYDPLVFPSSEFDASISQVNEK
INQSLAFIRKSDELLSAIGGYIPEAPRDGQAYVRKDGEWVLLSTFL
SEQ ID NO:6 
MELLIHRSSAIFLTLAINALYLTSSQNITEEFYQSTCSAVSRGYFSACRTGWYHSVITIELSNIK
ETKCNGTDTKVKLIKQELDKYKNAVTELQLLMQNTPAANNRARREAPQYMNYTINTTKNL
NVSISKKRKRRFLGFLLGCGSAIASGIAVSKVLHLEGEVNKIKNALLSTNKAVVSLSNGVSV
LTSKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEVLAYVVQLPIYGCIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSCITSLGAIVSCYGKTKCTASNKNRGIIKTFSNGCD
YVSNKGVDTVSVGNTLYYVNKLEGKNLYVKGEPIINYYDPLVFPSSEFDASISQVNEKINQS
LAFIRRSDELLSAIGGYIPEAPRDGQAYVRKDGEWVLLSTFL
SEQ ID NO:7 
MELPILKTNAITTILAAVTLCFASSQNITEEFYQSTCSAVSKGYLSACRTGWYHSVITIELSNI
KENKCNGTDAKVKLIKQELDKYKNAVTELQLLMQSTPAANSRARRELPRFMNYTLNNTK
NTNVTLSKKRKRRFLGFLLGVGSAIASGIAVSKVLHLEGEVNKIKSALLSTNKAVVSLSNGV
SVLTSKVLDLKNYIDKQLLPIVNKQSCSISNIETVIEFQQKNNRLLEITREFSVNAGVTTPVST
YMLTNSELLSLINDMPITNDQKKLMSSNVQIVRQQSYSIMSIIKEEVLAYVVQLPLYGCIDTP
CWKLHTSPLCTTNTKEGSNICLTRTDRGWYCDNAGSVSFFPQAETCKVQSNRVFCDTMNS
LTLPSEVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSSEFDASISQVNEK
INQSLAFIRKSDELLSAIGGYIPEAPRDGQAYVRKDGEWVLLSTFL
SEQ ID NO:8 
MELLIHRSSAIFLTLAINALYLTSSQNITEEFYQSTCSAVSRGYFSACRTGWYHSVITIELSNIK
ETKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEVLAYVVQLPIYGCIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSSEFDASISQVNEKINQS
LAFIRRSDELLSAIGGYIPEAPRDGQAYVRKDGEWVLLSTFL
SEQ ID NO:9 
MELPILKTNAITTILAAVTLCFASSQNITEEFYQSTCSAVSKGYLCALRTGWYHSVITIELSNI
KENKCNGTDAKVKLIKQELDKYKNAVTELQLLMQSTPAANSRARRELPRFMNYTLNNTK
NTNVTLSKKRKRRFLGFLLGCGSAIASGIAVSKVLHLEGEVNKIKSALLSTNKAVVSLSNGV
SVLTSKVLDLKNYIDKQLLPIVNKQSCSISNIETVIEFQQKNNRLLEITREFSVNAGVTTPVST
YMLTNSELLSLINDMPITNDQKKLMSSNVQIVRQQSYSIMSIIKEEVLAYVVQLPLYGVIDCP
CWKLHTSPLCTTNTKEGSNICLTRTDRGWYCDNAGSVSFFPQAETCKVQSNRVFCDTMNS
LTLPSEVNLCNIDIFNPKYDCKIMTSKTDVSSSCITSLGAIVSCYGKTKCTASNKNRGIIKTFS
NGCDYVSNKGVDTVSVGNTLYYVNKQEGKSLYVKGEPIINFYDPLVFPSSEFDASISQVNEK
INQSLAFIRKSDELLSAIGGYIPEAPRDGQAYVRKDGEWVLLSTFL
SEQ ID NO:10 
MELLIHRSSAIFLTLAINALYLTSSQNITEEFYQSTCSAVSRGYFCALRTGWYHSVITIELSNIK
ETKCNGTDTKVKLIKQELDKYKNAVTELQLLMQNTPAANNRARREAPQYMNYTINTTKNL
NVSISKKRKRRFLGFLLGCGSAIASGIAVSKVLHLEGEVNKIKNALLSTNKAVVSLSNGVSV
LTSKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEVLAYVVQLPIYGVIDCPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSCITSLGAIVSCYGKTKCTASNKNRGIIKTFSNGCD
YVSNKGVDTVSVGNTLYYVNKLEGKNLYVKGEPIINYYDPLVFPSSEFDASISQVNEKINQS
LAFIRRSDELLSAIGGYIPEAPRDGQAYVRKDGEWVLLSTFL
SEQ ID NO:11 
MELPILKTNAITTILAAVTLCFASSQNITEEFYQSTCSAVSKGYLCALRTGWYHSVITIELSNI
KENKCNGTDAKVKLIKQELDKYKNAVTELQLLMQSTPAANSRARRELPRFMNYTLNNTK
NTNVTLSKKRKRRFLGFLLGVGSAIASGIAVSKVLHLEGEVNKIKSALLSTNKAVVSLSNGV
SVLTSKVLDLKNYIDKQLLPIVNKQSCSISNIETVIEFQQKNNRLLEITREFSVNAGVTTPVST
YMLTNSELLSLINDMPITNDQKKLMSSNVQIVRQQSYSIMSIIKEEVLAYVVQLPLYGVIDCP
CWKLHTSPLCTTNTKEGSNICLTRTDRGWYCDNAGSVSFFPQAETCKVQSNRVFCDTMNS
LTLPSEVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSSEFDASISQVNEK
INQSLAFIRKSDELLSAIGGYIPEAPRDGQAYVRKDGEWVLLSTFL
SEQ ID NO:12 
MELLIHRSSAIFLTLAINALYLTSSQNITEEFYQSTCSAVSRGYFCALRTGWYHSVITIELSNIK
ETKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEVLAYVVQLPIYGVIDCPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSSEFDASISQVNEKINQS
LAFIRRSDELLSAIGGYIPEAPRDGQAYVRKDGEWVLLSTFL
SEQ ID NO:13 MELPILKTNAITTILAAVTLCFASSQNITEEFYQSTCSAVSKGYLSALRTGWYHSVITIELSNIK 
ENKCNGTDAKVKLIKQELDKYKNAVTELQLLMQSTPCANSRARRELPRFMNYTLNNTKNT
NVTLSKKRKRRFLGFLLGVGSACASGIAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLSAIGGYIPEAPRDGQAYVRKDGEWVLLSTFL
SEQ ID NO: 14 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPCANNRARREAPQYMNYTINTTKNL
NVSISKKRKRRFLGFLLGVGSACASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLSAIGGYIPEAPRDGQAYVRKDGEWVLLSTFL
SEQ ID NO:15 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLSAIGGYIPEAPRDGQAYVRKDGEWVLLSTFL
SEQ ID NO:16 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPCANNRARREAPQYMNYTINTTKNL
NVSISKKRKRRFLGFLLGVGSAIASGCAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLSAIGGYIPEAPRDGQAYVRKDGEWVLLSTFL
SEQ ID NO:17 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPAACSRARRELPRFMNYTLNNTKNT
 
NVTLSKKRKRRFLGFLLGVCSAIASGIAVSKVLHLEGEVNKIKSALLSTNKAVVSLSNGVSV
LTIKVLDLKNYIDKQLLPIVNKQSCSISNIETVIEFQQKNNRLLEITREFSVNAGVTTPVSTYM
LTNSELLSLINDMPITNDQKKLMSSNVQIVRQQSYSIMSIIKEEILAYVVQLPLYGVIDTPCW
KLHTSPLCTTNTKEGSNICLTRTDRGWYCDNAGSVSFFPQAETCKVQSNRVFCDTMNSLTL
PSEVNLCNIDIFNPKYDCKIMTSKTDVSSSVITSLGAIVSCYGKTKCTASNKNRGIIKTFSNGC
DYVSNKGVDTVSVGNTLYYVNKQEGKSLYVKGEPIINFYDPLVFPSSEFDASISQVNEKINQ
SLAFIRKSDELLSAIGGYIPEAPRDGQAYVRKDGEWVLLSTFL
SEQ ID NO:18 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPAACNRARREAPQYMNYTINTTKNL
NVSISKKRKRRFLGFLLGVCSAIASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLSAIGGYIPEAPRDGQAYVRKDGEWVLLSTFL
SEQ ID NO: 19 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPAACSRARRELPRFMNYTLNNTKNT
NVTLSKKRKRRFLGFLLGVGCAIASGIAVSKVLHLEGEVNKIKSALLSTNKAVVSLSNGVSV
LTIKVLDLKNYIDKQLLPIVNKQSCSISNIETVIEFQQKNNRLLEITREFSVNAGVTTPVSTYM
LTNSELLSLINDMPITNDQKKLMSSNVQIVRQQSYSIMSIIKEEILAYVVQLPLYGVIDTPCW
KLHTSPLCTTNTKEGSNICLTRTDRGWYCDNAGSVSFFPQAETCKVQSNRVFCDTMNSLTL
PSEVNLCNIDIFNPKYDCKIMTSKTDVSSSVITSLGAIVSCYGKTKCTASNKNRGIIKTFSNGC
DYVSNKGVDTVSVGNTLYYVNKQEGKSLYVKGEPIINFYDPLVFPSSEFDASISQVNEKINQ
SLAFIRKSDELLSAIGGYIPEAPRDGQAYVRKDGEWVLLSTFL
SEQ ID NO:20 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPAACNRARREAPQYMNYTINTTKNL
NVSISKKRKRRFLGFLLGVGCAIASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLSAIGGYIPEAPRDGQAYVRKDGEWVLLSTFL
SEQ ID NO:21 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPAANCRARRELPRFMNYTLNNTKN
TNVTLSKKRKRRFLGFLLGVGSCIASGIAVSKVLHLEGEVNKIKSALLSTNKAVVSLSNGVS
 
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLSAIGGYIPEAPRDGQAYVRKDGEWVLLSTFL
SEQ ID NO:22 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPAANCRARREAPQYMNYTINTTKNL
NVSISKKRKRRFLGFLLGVGSCIASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLSAIGGYIPEAPRDGQAYVRKDGEWVLLSTFL
SEQ ID NO:23 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPAANCRARRELPRFMNYTLNNTKN
TNVTLSKKRKRRFLGFLLGVCSAIASGIAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLSAIGGYIPEAPRDGQAYVRKDGEWVLLSTFL
SEQ ID NO:24 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPAANCRARREAPQYMNYTINTTKNL
NVSISKKRKRRFLGFLLGVCSAIASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLSAIGGYIPEAPRDGQAYVRKDGEWVLLSTFL
SEQ ID NO:25 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPAANCRARRELPRFMNYTLNNTKN
TNVTLSKKRKRRFLGFLLGVGCAIASGIAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
 
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLSAIGGYIPEAPRDGQAYVRKDGEWVLLSTFL
SEQ ID NO:26 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPAANCRARREAPQYMNYTINTTKNL
NVSISKKRKRRFLGFLLGVGCAIASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLSAIGGYIPEAPRDGQAYVRKDGEWVLLSTFL
SEQ ID NO:27 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTCAANSRARRELPRFMNYTLNNTKN
TNVTLSKKRKRRFLGFLLGVGSAIASGCAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLSAIGGYIPEAPRDGQAYVRKDGEWVLLSTFL
SEQ ID NO:28 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTCAANNRARREAPQYMNYTINTTKN
LNVSISKKRKRRFLGFLLGVGSAIASGCAVSKVLHLEGEVNKIKNALLSTNKAVVSLSNGVS
VLTIKVLDLKNYINNQLLPIVNQQSCRISNIETVIEFQQKNSRLLEITREFSVNAGVTTPLSTY
MLTNSELLSLINDMPITNDQKKLMSSNVQIVRQQSYSIMSIIKEEILAYVVQLPIYGVIDTPC
WKLHTSPLCTTNIKEGSNICLTRTDRGWYCDNAGSVSFFPQADTCKVQSNRVFCDTMNSLT
LPSEVSLCNTDIFNSKYDCKIMTSKTDISSSVITSLGAIVSCYGKTKCTASNKNRGIIKTFSNG
CDYVSNKGVDTVSVGNTLYYVNKLEGKNLYVKGEPIINYYDPLVFPSSEFDASISQVNEKIN
QSLAFIRRSDELLSAIGGYIPEAPRDGQAYVRKDGEWVLLSTFL
SEQ ID NO:29 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPACNSRARRELPRFMNYTLNNTKNT
NVTLSKKRKRRFLGFLLGVGCAIASGIAVSKVLHLEGEVNKIKSALLSTNKAVVSLSNGVSV
LTIKVLDLKNYIDKQLLPIVNKQSCSISNIETVIEFQQKNNRLLEITREFSVNAGVTTPVSTYM
LTNSELLSLINDMPITNDQKKLMSSNVQIVRQQSYSIMSIIKEEILAYVVQLPLYGVIDTPCW
 
KLHTSPLCTTNTKEGSNICLTRTDRGWYCDNAGSVSFFPQAETCKVQSNRVFCDTMNSLTL
PSEVNLCNIDIFNPKYDCKIMTSKTDVSSSVITSLGAIVSCYGKTKCTASNKNRGIIKTFSNGC
DYVSNKGVDTVSVGNTLYYVNKQEGKSLYVKGEPIINFYDPLVFPSSEFDASISQVNEKINQ
SLAFIRKSDELLSAIGGYIPEAPRDGQAYVRKDGEWVLLSTFL
SEQ ID NO:30 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPACNNRARREAPQYMNYTINTTKNL
NVSISKKRKRRFLGFLLGVGCAIASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLSAIGGYIPEAPRDGQAYVRKDGEWVLLSTFL
SEQ ID NO:31 
MELPILKTNAITTILAAVTLCFASSQNITEEFYQSTCSAVSKGYLSALRTGWYTSVITIELSNIK
INKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCSISPIETVIEFQQKNNRLLEITREFSVNAGVTTPVSTYM
LTNSELLSLINDMPITNDQKKLMSSNVQIVRQQSYSIMSIIKEEVLAYVVQLPLYGVIDTPCW
KLHTSPLCTTNTKEGSNICLTRTDRGWYCDNAGSVSFFPQAETCKVQSNRVFCDTMNSLTL
PSEVNLCNIDIFNPKYDCKIMTSKTDVSSSVITSLGAIVSCYGKTKCTASNKNRGIIKTFSNGC
DYVSNKGVDTVSVGNTLYYVNKQEGKSLYVKGEPIINFYDPLVFPSQEFDASISQVNEKINQ
SLAFIRKSDELLSAIGGYIPEAPRDGQAYVRKDGEWVLLSTFL
SEQ ID NO:32 
MELLIHRSSAIFLTLAINALYLTSSQNITEEFYQSTCSAVSRGYFSALRTGWYTSVITIELSNIKI
TKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCRISPIETVIEFQQKNSRLLEITREFSVNAGVTTPLSTYM
LTNSELLSLINDMPITNDQKKLMSSNVQIVRQQSYSIMSIIKEEVLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSQEFDASISQVNEKINQS
LAFIRRSDELLSAIGGYIPEAPRDGQAYVRKDGEWVLLSTFL
SEQ ID NO:33 
MELPILKTNAITTILAAVTLCFASSQNITEEFYQSTCSAVSKGYLSALRTGWYTSVITIELSNIK
INKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCSISPIETVIEFQQKNNRLLEITREFSVNAGVTTPVSTYM
LTNSELLSLINDMPITNDQKKLMSSNVQIVRQQSYSIMSIIKEEVLAYVVQLPLYGVIDTPCW
KLHTSPLCTTNTKEGSNICLTRTDRGWYCDNAGSVSFFPQAETCKVQSNRVFCDTMNSLTL
 
PSEVNLCNIDIFNPKYDCKIMTSKTDVSSSVITSLGAIVSCYGKTKCTASNKNRGIIKTFSNGC
DYVSNKGVDTVSVGNTLYYVNKQEGKSLYVKGEPIINFYDPLVFPSDTFDASISQVNEKINQ
SLAFIRKSDELLSAIGGYIPEAPRDGQAYVRKDGEWVLLSTFL
SEQ ID NO:34 
MELLIHRSSAIFLTLAINALYLTSSQNITEEFYQSTCSAVSRGYFSALRTGWYTSVITIELSNIKI
TKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCRISPIETVIEFQQKNSRLLEITREFSVNAGVTTPLSTYM
LTNSELLSLINDMPITNDQKKLMSSNVQIVRQQSYSIMSIIKEEVLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDTFDASISQVNEKINQS
LAFIRRSDELLSAIGGYIPEAPRDGQAYVRKDGEWVLLSTFL
SEQ ID NO:35 
MELPILKTNAITTILAAVTLCFASSQNITEEFYQSTCSAVSKGYLSALRTGWYTSVITIELSNIK
EIKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCSISPIETVIEFQQKNNRLLEITREFSVNAGVTTPVSTYM
LTNSELLSLINDMPITNDQKKLMSSNVQIVRQQSYSIMSIIKEEVLAYVVQLPLYGVIDTPCW
KLHTSPLCTTNTKEGSNICLTRTDRGWYCDNAGSVSFFPQAETCKVQSNRVFCDTMNSLTL
PSEVNLCNIDIFNPKYDCKIMTSKTDVSSSVITSLGAIVSCYGKTKCTASNKNRGIIKTFSNGC
DYVSNKGVDTVSVGNTLYYVNKQEGKSLYVKGEPIINFYDPLVFPSDQFDASISQVNEKINQ
SLAFIRKSDELLSAIGGYIPEAPRDGQAYVRKDGEWVLLSTFL
SEQ ID NO:36 
MELLIHRSSAIFLTLAINALYLTSSQNITEEFYQSTCSAVSRGYFSALRTGWYTSVITIELSNIK
EIKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCRISPIETVIEFQQKNSRLLEITREFSVNAGVTTPLSTYM
LTNSELLSLINDMPITNDQKKLMSSNVQIVRQQSYSIMSIIKEEVLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDQFDASISQVNEKINQS
LAFIRRSDELLSAIGGYIPEAPRDGQAYVRKDGEWVLLSTFL
SEQ ID NO:37 
MELPILKTNAITTILAAVTLCFASSQNITEEFYQSTCSAVSKGYLSALRTGWYTSVITIELSNIK
INKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCPISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
 
CDYVSNKGVDTVSVGNTLYYVNKQEGKSLYVKGEPIINFYDPLVFPSQEFDASISQVNEKIN
QSLAFIRKSDELLSAIGGYIPEAPRDGQAYVRKDGEWVLLSTFL
SEQ ID NO:38 
MELLIHRSSAIFLTLAINALYLTSSQNITEEFYQSTCSAVSRGYFSALRTGWYTSVITIELSNIKI
TKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCPISNIETVIEFQQKNSRLLEITREFSVNAGVTTPLSTYM
LTNSELLSLINDMPITNDQKKLMSSNVQIVRQQSYSIMSIIKEEVLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSQEFDASISQVNEKINQS
LAFIRRSDELLSAIGGYIPEAPRDGQAYVRKDGEWVLLSTFL
SEQ ID NO:39 
MELPILKTNAITTILAAVTLCFASSQNITEEFYQSTCSAVSKGYLSALRTGWYTSVITIELSNIK
INKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCPISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDTFDASISQVNEKIN
QSLAFIRKSDELLSAIGGYIPEAPRDGQAYVRKDGEWVLLSTFL
SEQ ID NO:40 
MELLIHRSSAIFLTLAINALYLTSSQNITEEFYQSTCSAVSRGYFSALRTGWYTSVITIELSNIKI
TKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCPISNIETVIEFQQKNSRLLEITREFSVNAGVTTPLSTYM
LTNSELLSLINDMPITNDQKKLMSSNVQIVRQQSYSIMSIIKEEVLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDTFDASISQVNEKINQS
LAFIRRSDELLSAIGGYIPEAPRDGQAYVRKDGEWVLLSTFL
SEQ ID NO:41 
MELPILKTNAITTILAAVTLCFASSQNITEEFYQSTCSAVSKGYLSALRTGWYTSVITIELSNIK
EIKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCPISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDQFDASISQVNEKIN
 QSLAFIRKSDELLSAIGGYIPEAPRDGQAYVRKDGEWVLLSTFL
SEQ ID NO:42 
MELLIHRSSAIFLTLAINALYLTSSQNITEEFYQSTCSAVSRGYFSALRTGWYTSVITIELSNIK
EIKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCPISNIETVIEFQQKNSRLLEITREFSVNAGVTTPLSTYM
LTNSELLSLINDMPITNDQKKLMSSNVQIVRQQSYSIMSIIKEEVLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDQFDASISQVNEKINQS
LAFIRRSDELLSAIGGYIPEAPRDGQAYVRKDGEWVLLSTFL
SEQ ID NO:43 
MELPILKTNAITTILAAVTLCFASSQNITEEFYQSTCSAVSKGYLSALRTGWYTSVITIELSNIK
EIKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCSIPNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDQFDASISQVNEKIN
QSLAFIRKSDELLSAIGGYIPEAPRDGQAYVRKDGEWVLLSTFL
SEQ ID NO:44 
MELLIHRSSAIFLTLAINALYLTSSQNITEEFYQSTCSAVSRGYFSALRTGWYTSVITIELSNIK
EIKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCRIPNIETVIEFQQKNSRLLEITREFSVNAGVTTPLSTYM
LTNSELLSLINDMPITNDQKKLMSSNVQIVRQQSYSIMSIIKEEVLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDQFDASISQVNEKINQS
LAFIRRSDELLSAIGGYIPEAPRDGQAYVRKDGEWVLLSTFL
SEQ ID NO:45 
MELPILKTNAITTILAAVTLCFASSQNITEEFYQSTCSAVSKGYLSALRTGWYTSVITIELSNIK
INKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCSISNPETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDTFDASISQVNEKIN
QSLAFIRKSDELLSAIGGYIPEAPRDGQAYVRKDGEWVLLSTFL
SEQ ID NO:46 
MELLIHRSSAIFLTLAINALYLTSSQNITEEFYQSTCSAVSRGYFSALRTGWYTSVITIELSNIKI
TKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCRISNPETVIEFQQKNSRLLEITREFSVNAGVTTPLSTY
MLTNSELLSLINDMPITNDQKKLMSSNVQIVRQQSYSIMSIIKEEVLAYVVQLPIYGVIDTPC
WKLHTSPLCTTNIKEGSNICLTRTDRGWYCDNAGSVSFFPQADTCKVQSNRVFCDTMNSLT
LPSEVSLCNTDIFNSKYDCKIMTSKTDISSSVITSLGAIVSCYGKTKCTASNKNRGIIKTFSNG
CDYVSNKGVDTVSVGNTLYYVNKLEGKNLYVKGEPIINYYDPLVFPSDTFDASISQVNEKIN
QSLAFIRRSDELLSAIGGYIPEAPRDGQAYVRKDGEWVLLSTFL
SEQ ID NO:47 
MELPILKTNAITTILAAVTLCFASSQNITEEFYQSTCSAVSKGYLSALRTGWYTSVITIELSNIK
EIKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCSISNPETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDQFDASISQVNEKIN
QSLAFIRKSDELLSAIGGYIPEAPRDGQAYVRKDGEWVLLSTFL
SEQ ID NO:48 
MELLIHRSSAIFLTLAINALYLTSSQNITEEFYQSTCSAVSRGYFSALRTGWYTSVITIELSNIK
EIKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCRISNPETVIEFQQKNSRLLEITREFSVNAGVTTPLSTY
MLTNSELLSLINDMPITNDQKKLMSSNVQIVRQQSYSIMSIIKEEVLAYVVQLPIYGVIDTPC
WKLHTSPLCTTNIKEGSNICLTRTDRGWYCDNAGSVSFFPQADTCKVQSNRVFCDTMNSLT
LPSEVSLCNTDIFNSKYDCKIMTSKTDISSSVITSLGAIVSCYGKTKCTASNKNRGIIKTFSNG
CDYVSNKGVDTVSVGNTLYYVNKLEGKNLYVKGEPIINYYDPLVFPSDQFDASISQVNEKI
NQSLAFIRRSDELLSAIGGYIPEAPRDGQAYVRKDGEWVLLSTFL
SEQ ID NO:49 
MELPILKTNAITTILAAVTLCFASSQNITEEFYQSTCSAVSKGYLSALRTGWYTSVITIELSNIK
INKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCSISNPETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSQEFDASISQVNEKIN
QSLAFIRKSDELLSAIGGYIPEAPRDGQAYVRKDGEWVLLSTFL
SEQ ID NO:50 
MELLIHRSSAIFLTLAINALYLTSSQNITEEFYQSTCSAVSRGYFSALRTGWYTSVITIELSNIKI
TKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCRISNPETVIEFQQKNSRLLEITREFSVNAGVTTPLSTY
MLTNSELLSLINDMPITNDQKKLMSSNVQIVRQQSYSIMSIIKEEVLAYVVQLPIYGVIDTPC
WKLHTSPLCTTNIKEGSNICLTRTDRGWYCDNAGSVSFFPQADTCKVQSNRVFCDTMNSLT
LPSEVSLCNTDIFNSKYDCKIMTSKTDISSSVITSLGAIVSCYGKTKCTASNKNRGIIKTFSNG
CDYVSNKGVDTVSVGNTLYYVNKLEGKNLYVKGEPIINYYDPLVFPSQEFDASISQVNEKIN
QSLAFIRRSDELLSAIGGYIPEAPRDGQAYVRKDGEWVLLSTFL
SEQ ID NO:51 
MELPILKTNAITTILAAVTLCFASSQNITEEFYQSTCSAVSKGYLSALRTGWYTSVITIELSNIK
INKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCPISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDTFDASISQVNEKIN
QSLAFIRKSDELLHNVNAGKSTTNIMITTIIIVIIVILLALIAVGLLLY
SEQ ID NO:52 
MELLIHRSSAIFLTLAINALYLTSSQNITEEFYQSTCSAVSRGYFSALRTGWYTSVITIELSNIKI
TKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCPISNIETVIEFQQKNSRLLEITREFSVNAGVTTPLSTYM
LTNSELLSLINDMPITNDQKKLMSSNVQIVRQQSYSIMSIIKEEVLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDTFDASISQVNEKINQS
LAFIRRSDELLHNVNTGKSTTNIMITAIIIVIIVVLLSLIAIGLLLY
SEQ ID NO:53 
MELPILKTNAITTILAAVTLCFASSQNITEEFYQSTCSAVSKGYLSALRTGWYTSVITIELSNIK
INKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCPISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSQEFDASISQVNEKIN
QSLAFIRKSDELLHNVNAGKSTTNIMITTIIIVIIVILLALIAVGLLLY
SEQ ID NO:54 
MELLIHRSSAIFLTLAINALYLTSSQNITEEFYQSTCSAVSRGYFSALRTGWYTSVITIELSNIKI
TKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCPISNIETVIEFQQKNSRLLEITREFSVNAGVTTPLSTYM
LTNSELLSLINDMPITNDQKKLMSSNVQIVRQQSYSIMSIIKEEVLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSQEFDASISQVNEKINQS
LAFIRRSDELLHNVNTGKSTTNIMITAIIIVIIVVLLSLIAIGLLLY
SEQ ID NO:55 
MELPILKTNAITTILAAVTLCFASSQNITEEFYQSTCSAVSKGYLSALRTGWYTSVITIELSNIK
INKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTSKVLDLKNYIDKQLLPIVNKQSCPISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:56 
MELLIHRSSAIFLTLAINALYLTSSQNITEEFYQSTCSAVSRGYFSALRTGWYTSVITIELSNIKI
TKCNGTDTKVKLIKQELDKYKNAVTELQLLMQNTPCANNRARREAPQYMNYTINTTKNL
NVSISKKRKRRFLGFLLGVGSAIASGCAVSKVLHLEGEVNKIKNALLSTNKAVVSLSNGVSV
LTSKVLDLKNYINNQLLPIVNQQSCPISNIETVIEFQQKNSRLLEITREFSVNAGVTTPLSTYM
LTNSELLSLINDMPITNDQKKLMSSNVQIVRQQSYSIMSIIKEEVLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDEFDASISQVNEKINQS
LAFIRRSDELLHNVNTGKSTTNIMITAIIIVIIVVLLSLIAIGLLLY
SEQ ID NO:57 
MELPILKTNAITTILAAVTLCFASSQNITEEFYQSTCSAVSKGYLSALRTGWYTSVITIELSNIK
INKCNGTDAKVKLIKQELDKYKNAVTELQLLMQSTCAANSRARRELPRFMNYTLNNTKNT
NVTLSKKRKRRFLGFLLGVGSAIASGCAVSKVLHLEGEVNKIKSALLSTNKAVVSLSNGVS
VLTSKVLDLKNYIDKQLLPIVNKQSCPISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:58 MELLIHRSSAIFLTLAINALYLTSSQNITEEFYQSTCSAVSRGYFSALRTGWYTSVITIELSNIKI 
TKCNGTDTKVKLIKQELDKYKNAVTELQLLMQNTCAANNRARREAPQYMNYTINTTKNL
NVSISKKRKRRFLGFLLGVGSAIASGCAVSKVLHLEGEVNKIKNALLSTNKAVVSLSNGVSV
LTSKVLDLKNYINNQLLPIVNQQSCPISNIETVIEFQQKNSRLLEITREFSVNAGVTTPLSTYM
LTNSELLSLINDMPITNDQKKLMSSNVQIVRQQSYSIMSIIKEEVLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDEFDASISQVNEKINQS
LAFIRRSDELLHNVNTGKSTTNIMITAIIIVIIVVLLSLIAIGLLLY
SEQ ID NO:59 
MELPILKTNAITTILAAVTLCFASSQNITEEFYQSTCSAVSKGYLSALRTGWYTSVITIELSNIK
INKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCPISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:60 
MELLIHRSSAIFLTLAINALYLTSSQNITEEFYQSTCSAVSRGYFSALRTGWYTSVITIELSNIKI
TKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCPISNIETVIEFQQKNSRLLEITREFSVNAGVTTPLSTYM
LTNSELLSLINDMPITNDQKKLMSSNVQIVRQQSYSIMSIIKEEVLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDEFDASISQVNEKINQS
LAFIRRSDELLHNVNTGKSTTNIMITAIIIVIIVVLLSLIAIGLLLY
SEQ ID NO:61 
MELPILKTNAITTILAAVTLCFASSQNITEEFYQSTCSAVSKGYLSALRTGWYVSVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTMKVLDLKNYIDKQLLPIVNKQSCSISNIETVIEFQQKNNRLLEITREFSVNAGVTTPVST
YMLTNSELLSLINDMPITNDQKKLMSSNVQIVRQQSYSIMSIIKEELLAYVVQLPLYGVIDTP
CWKLHTSPLCTTNTKEGSNICLTRTDRGWYCDNAGSVSFFPQAETCKVQSNRVFCDTMNS
LTLPSEVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSDEFDASISQVNE
KINQSLAFIRKSDELLHNVNAGKSTTNIMITTIIIVIIVILLALIAVGLLLY
SEQ ID NO:62 
MELLIHRSSAIFLTLAINALYLTSSQNITEEFYQSTCSAVSRGYFSALRTGWYVSVITIELSNIK
ETKCNGTDTKVKLIKQELDKYKNAVTELQLLMQNTPCANNRARREAPQYMNYTINTTKNL
 
NVSISKKRKRRFLGFLLGVGSAIASGCAVSKVLHLEGEVNKIKNALLSTNKAVVSLSNGVSV
LTMKVLDLKNYINNQLLPIVNQQSCRISNIETVIEFQQKNSRLLEITREFSVNAGVTTPLSTY
MLTNSELLSLINDMPITNDQKKLMSSNVQIVRQQSYSIMSIIKEELLAYVVQLPIYGVIDTPC
WKLHTSPLCTTNIKEGSNICLTRTDRGWYCDNAGSVSFFPQADTCKVQSNRVFCDTMNSLT
LPSEVSLCNTDIFNSKYDCKIMTSKTDISSSVITSLGAIVSCYGKTKCTASNKNRGIIKTFSNG
CDYVSNKGVDTVSVGNTLYYVNKLEGKNLYVKGEPIINYYDPLVFPSDEFDASISQVNEKIN
QSLAFIRRSDELLHNVNTGKSTTNIMITAIIIVIIVVLLSLIAIGLLLY
SEQ ID NO:63 
MELPILKTNAITTILAAVTLCFASSQNITEEFYQSTCSAVSKGYLSALRTGWYFSVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTMKVLDLKNYIDKQLLPIVNKQSCSISNIETVIEFQQKNNRLLEITREFSVNAGVTTPVST
YMLTNSELLSLINDMPITNDQKKLMSSNVQIVRQQSYSIMSIIKEELLAYVVQLPLYGVIDTP
CWKLHTSPLCTTNTKEGSNICLTRTDRGWYCDNAGSVSFFPQAETCKVQSNRVFCDTMNS
LTLPSEVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSDEFDASISQVNE
KINQSLAFIRKSDELLHNVNAGKSTTNIMITTIIIVIIVILLALIAVGLLLY
SEQ ID NO:64 
MELLIHRSSAIFLTLAINALYLTSSQNITEEFYQSTCSAVSRGYFSALRTGWYFSVITIELSNIK
ETKCNGTDTKVKLIKQELDKYKNAVTELQLLMQNTPCANNRARREAPQYMNYTINTTKNL
NVSISKKRKRRFLGFLLGVGSAIASGCAVSKVLHLEGEVNKIKNALLSTNKAVVSLSNGVSV
LTMKVLDLKNYINNQLLPIVNQQSCRISNIETVIEFQQKNSRLLEITREFSVNAGVTTPLSTY
MLTNSELLSLINDMPITNDQKKLMSSNVQIVRQQSYSIMSIIKEELLAYVVQLPIYGVIDTPC
WKLHTSPLCTTNIKEGSNICLTRTDRGWYCDNAGSVSFFPQADTCKVQSNRVFCDTMNSLT
LPSEVSLCNTDIFNSKYDCKIMTSKTDISSSVITSLGAIVSCYGKTKCTASNKNRGIIKTFSNG
CDYVSNKGVDTVSVGNTLYYVNKLEGKNLYVKGEPIINYYDPLVFPSDEFDASISQVNEKIN
QSLAFIRRSDELLHNVNTGKSTTNIMITAIIIVIIVVLLSLIAIGLLLY
SEQ ID NO:65 
MELPILKTNAITTILAAVTLCFASSQNITEEFYQSTCSAVSKGYLSALRTGWYCSVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTMKVLDLKNYIDKQLLPIVNKQSCSISNIETVIEFQQKNNRLLEITREFSVNAGVTTPVST
YMLTNSELLSLINDMPITNDQKKLMSSNVQIVRQQSYSIMSIIKEELLAYVVQLPLYGVIDTP
CWKLHTSPLCTTNTKEGSNICLTRTDRGWYCDNAGSVSFFPQAETCKVQSNRVFCDTMNS
LTLPSEVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSDEFDASISQVNE
KINQSLAFIRKSDELLHNVNAGKSTTNIMITTIIIVIIVILLALIAVGLLLY
SEQ ID NO:66 
MELLIHRSSAIFLTLAINALYLTSSQNITEEFYQSTCSAVSRGYFSALRTGWYCSVITIELSNIK
ETKCNGTDTKVKLIKQELDKYKNAVTELQLLMQNTPCANNRARREAPQYMNYTINTTKNL
NVSISKKRKRRFLGFLLGVGSAIASGCAVSKVLHLEGEVNKIKNALLSTNKAVVSLSNGVSV
 
LTMKVLDLKNYINNQLLPIVNQQSCRISNIETVIEFQQKNSRLLEITREFSVNAGVTTPLSTY
MLTNSELLSLINDMPITNDQKKLMSSNVQIVRQQSYSIMSIIKEELLAYVVQLPIYGVIDTPC
WKLHTSPLCTTNIKEGSNICLTRTDRGWYCDNAGSVSFFPQADTCKVQSNRVFCDTMNSLT
LPSEVSLCNTDIFNSKYDCKIMTSKTDISSSVITSLGAIVSCYGKTKCTASNKNRGIIKTFSNG
CDYVSNKGVDTVSVGNTLYYVNKLEGKNLYVKGEPIINYYDPLVFPSDEFDASISQVNEKIN
QSLAFIRRSDELLHNVNTGKSTTNIMITAIIIVIIVVLLSLIAIGLLLY
SEQ ID NO:67 
MELPILKTNAITTILAAVTLCFASSQNITEEFYQSTCSAVSKGYLSALRTGWYASVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTMKVLDLKNYIDKQLLPIVNKQSCSISNIETVIEFQQKNNRLLEITREFSVNAGVTTPVST
YMLTNSELLSLINDMPITNDQKKLMSSNVQIVRQQSYSIMSIIKEELLAYVVQLPLYGVIDTP
CWKLHTSPLCTTNTKEGSNICLTRTDRGWYCDNAGSVSFFPQAETCKVQSNRVFCDTMNS
LTLPSEVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSDEFDASISQVNE
KINQSLAFIRKSDELLHNVNAGKSTTNIMITTIIIVIIVILLALIAVGLLLY
SEQ ID NO:68 
MELLIHRSSAIFLTLAINALYLTSSQNITEEFYQSTCSAVSRGYFSALRTGWYASVITIELSNIK
ETKCNGTDTKVKLIKQELDKYKNAVTELQLLMQNTPCANNRARREAPQYMNYTINTTKNL
NVSISKKRKRRFLGFLLGVGSAIASGCAVSKVLHLEGEVNKIKNALLSTNKAVVSLSNGVSV
LTMKVLDLKNYINNQLLPIVNQQSCRISNIETVIEFQQKNSRLLEITREFSVNAGVTTPLSTY
MLTNSELLSLINDMPITNDQKKLMSSNVQIVRQQSYSIMSIIKEELLAYVVQLPIYGVIDTPC
WKLHTSPLCTTNIKEGSNICLTRTDRGWYCDNAGSVSFFPQADTCKVQSNRVFCDTMNSLT
LPSEVSLCNTDIFNSKYDCKIMTSKTDISSSVITSLGAIVSCYGKTKCTASNKNRGIIKTFSNG
CDYVSNKGVDTVSVGNTLYYVNKLEGKNLYVKGEPIINYYDPLVFPSDEFDASISQVNEKIN
QSLAFIRRSDELLHNVNTGKSTTNIMITAIIIVIIVVLLSLIAIGLLLY
SEQ ID NO:69 
MELPILKTNAITTILAAVTLCFASSQNITEEFYQSTCSAVSKGYLSALRTGWYVSVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTFKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:70 
MELLIHRSSAIFLTLAINALYLTSSQNITEEFYQSTCSAVSRGYFSALRTGWYVSVITIELSNIK
ETKCNGTDTKVKLIKQELDKYKNAVTELQLLMQNTPCANNRARREAPQYMNYTINTTKNL
NVSISKKRKRRFLGFLLGVGSAIASGCAVSKVLHLEGEVNKIKNALLSTNKAVVSLSNGVSV
LTFKVLDLKNYINNQLLPIVNQQSCRISNIETVIEFQQKNSRLLEITREFSVNAGVTTPLSTYM
 
LTNSELLSLINDMPITNDQKKLMSSNVQIVRQQSYSIMSIIKEELLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDEFDASISQVNEKINQS
LAFIRRSDELLHNVNTGKSTTNIMITAIIIVIIVVLLSLIAIGLLLY
SEQ ID NO:71 
MELPILKTNAITTILAAVTLCFASSQNITEEFYQSTCSAVSKGYLSALRTGWYFSVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTFKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:72 
MELLIHRSSAIFLTLAINALYLTSSQNITEEFYQSTCSAVSRGYFSALRTGWYFSVITIELSNIK
ETKCNGTDTKVKLIKQELDKYKNAVTELQLLMQNTPCANNRARREAPQYMNYTINTTKNL
NVSISKKRKRRFLGFLLGVGSAIASGCAVSKVLHLEGEVNKIKNALLSTNKAVVSLSNGVSV
LTFKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEELLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDEFDASISQVNEKINQS
LAFIRRSDELLHNVNTGKSTTNIMITAIIIVIIVVLLSLIAIGLLLY
SEQ ID NO:73 
MELPILKTNAITTILAAVTLCFASSQNITEEFYQSTCSAVSKGYLSALRTGWYCSVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTFKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:74 
MELLIHRSSAIFLTLAINALYLTSSQNITEEFYQSTCSAVSRGYFSALRTGWYCSVITIELSNIK
ETKCNGTDTKVKLIKQELDKYKNAVTELQLLMQNTPCANNRARREAPQYMNYTINTTKNL
NVSISKKRKRRFLGFLLGVGSAIASGCAVSKVLHLEGEVNKIKNALLSTNKAVVSLSNGVSV
LTFKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEELLAYVVQLPIYGVIDTPCW
 
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDEFDASISQVNEKINQS
LAFIRRSDELLHNVNTGKSTTNIMITAIIIVIIVVLLSLIAIGLLLY
SEQ ID NO:75 
MELPILKTNAITTILAAVTLCFASSQNITEEFYQSTCSAVSKGYLSALRTGWYASVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTFKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:76 
MELLIHRSSAIFLTLAINALYLTSSQNITEEFYQSTCSAVSRGYFSALRTGWYASVITIELSNIK
ETKCNGTDTKVKLIKQELDKYKNAVTELQLLMQNTPCANNRARREAPQYMNYTINTTKNL
NVSISKKRKRRFLGFLLGVGSAIASGCAVSKVLHLEGEVNKIKNALLSTNKAVVSLSNGVSV
LTFKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEELLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDEFDASISQVNEKINQS
LAFIRRSDELLHNVNTGKSTTNIMITAIIIVIIVVLLSLIAIGLLLY
SEQ ID NO:77 
MELPILKTNAITTILAAVTLCFASSQNITEEFYQSTCSAVSKGYLSALRTGWYVSVITIELSNIK
ENKCNGTDAKVKLIKQELDKYKNAVTELQLLMQSTCAANSRARRELPRFMNYTLNNTKN
TNVTLSKKRKRRFLGFLLGVGSAIASGCAVSKVLHLEGEVNKIKSALLSTNKAVVSLSNGVS
VLTMKVLDLKNYIDKQLLPIVNKQSCSISNIETVIEFQQKNNRLLEITREFSVNAGVTTPVST
YMLTNSELLSLINDMPITNDQKKLMSSNVQIVRQQSYSIMSIIKEELLAYVVQLPLYGVIDTP
CWKLHTSPLCTTNTKEGSNICLTRTDRGWYCDNAGSVSFFPQAETCKVQSNRVFCDTMNS
LTLPSEVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSDEFDASISQVNE
KINQSLAFIRKSDELLHNVNAGKSTTNIMITTIIIVIIVILLALIAVGLLLY
SEQ ID NO:78 
MELLIHRSSAIFLTLAINALYLTSSQNITEEFYQSTCSAVSRGYFSALRTGWYVSVITIELSNIK
ETKCNGTDTKVKLIKQELDKYKNAVTELQLLMQNTCAANNRARREAPQYMNYTINTTKN
LNVSISKKRKRRFLGFLLGVGSAIASGCAVSKVLHLEGEVNKIKNALLSTNKAVVSLSNGVS
VLTMKVLDLKNYINNQLLPIVNQQSCRISNIETVIEFQQKNSRLLEITREFSVNAGVTTPLST
YMLTNSELLSLINDMPITNDQKKLMSSNVQIVRQQSYSIMSIIKEELLAYVVQLPIYGVIDTP
CWKLHTSPLCTTNIKEGSNICLTRTDRGWYCDNAGSVSFFPQADTCKVQSNRVFCDTMNSL
 
TLPSEVSLCNTDIFNSKYDCKIMTSKTDISSSVITSLGAIVSCYGKTKCTASNKNRGIIKTFSN
GCDYVSNKGVDTVSVGNTLYYVNKLEGKNLYVKGEPIINYYDPLVFPSDEFDASISQVNEKI
NQSLAFIRRSDELLHNVNTGKSTTNIMITAIIIVIIVVLLSLIAIGLLLY
SEQ ID NO:79 
MELPILKTNAITTILAAVTLCFASSQNITEEFYQSTCSAVSKGYLSALRTGWYFSVITIELSNIK
ENKCNGTDAKVKLIKQELDKYKNAVTELQLLMQSTCAANSRARRELPRFMNYTLNNTKN
TNVTLSKKRKRRFLGFLLGVGSAIASGCAVSKVLHLEGEVNKIKSALLSTNKAVVSLSNGVS
VLTMKVLDLKNYIDKQLLPIVNKQSCSISNIETVIEFQQKNNRLLEITREFSVNAGVTTPVST
YMLTNSELLSLINDMPITNDQKKLMSSNVQIVRQQSYSIMSIIKEELLAYVVQLPLYGVIDTP
CWKLHTSPLCTTNTKEGSNICLTRTDRGWYCDNAGSVSFFPQAETCKVQSNRVFCDTMNS
LTLPSEVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSDEFDASISQVNE
KINQSLAFIRKSDELLHNVNAGKSTTNIMITTIIIVIIVILLALIAVGLLLY
SEQ ID NO:80 
MELLIHRSSAIFLTLAINALYLTSSQNITEEFYQSTCSAVSRGYFSALRTGWYFSVITIELSNIK
ETKCNGTDTKVKLIKQELDKYKNAVTELQLLMQNTCAANNRARREAPQYMNYTINTTKN
LNVSISKKRKRRFLGFLLGVGSAIASGCAVSKVLHLEGEVNKIKNALLSTNKAVVSLSNGVS
VLTMKVLDLKNYINNQLLPIVNQQSCRISNIETVIEFQQKNSRLLEITREFSVNAGVTTPLST
YMLTNSELLSLINDMPITNDQKKLMSSNVQIVRQQSYSIMSIIKEELLAYVVQLPIYGVIDTP
CWKLHTSPLCTTNIKEGSNICLTRTDRGWYCDNAGSVSFFPQADTCKVQSNRVFCDTMNSL
TLPSEVSLCNTDIFNSKYDCKIMTSKTDISSSVITSLGAIVSCYGKTKCTASNKNRGIIKTFSN
GCDYVSNKGVDTVSVGNTLYYVNKLEGKNLYVKGEPIINYYDPLVFPSDEFDASISQVNEKI
NQSLAFIRRSDELLHNVNTGKSTTNIMITAIIIVIIVVLLSLIAIGLLLY
SEQ ID NO:81 
MELPILKTNAITTILAAVTLCFASSQNITEEFYQSTCSAVSKGYLSALRTGWYCSVITIELSNIK
ENKCNGTDAKVKLIKQELDKYKNAVTELQLLMQSTCAANSRARRELPRFMNYTLNNTKN
TNVTLSKKRKRRFLGFLLGVGSAIASGCAVSKVLHLEGEVNKIKSALLSTNKAVVSLSNGVS
VLTMKVLDLKNYIDKQLLPIVNKQSCSISNIETVIEFQQKNNRLLEITREFSVNAGVTTPVST
YMLTNSELLSLINDMPITNDQKKLMSSNVQIVRQQSYSIMSIIKEELLAYVVQLPLYGVIDTP
CWKLHTSPLCTTNTKEGSNICLTRTDRGWYCDNAGSVSFFPQAETCKVQSNRVFCDTMNS
LTLPSEVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSDEFDASISQVNE
KINQSLAFIRKSDELLHNVNAGKSTTNIMITTIIIVIIVILLALIAVGLLLY
SEQ ID NO:82 
MELLIHRSSAIFLTLAINALYLTSSQNITEEFYQSTCSAVSRGYFSALRTGWYCSVITIELSNIK
ETKCNGTDTKVKLIKQELDKYKNAVTELQLLMQNTCAANNRARREAPQYMNYTINTTKN
LNVSISKKRKRRFLGFLLGVGSAIASGCAVSKVLHLEGEVNKIKNALLSTNKAVVSLSNGVS
VLTMKVLDLKNYINNQLLPIVNQQSCRISNIETVIEFQQKNSRLLEITREFSVNAGVTTPLST
YMLTNSELLSLINDMPITNDQKKLMSSNVQIVRQQSYSIMSIIKEELLAYVVQLPIYGVIDTP
CWKLHTSPLCTTNIKEGSNICLTRTDRGWYCDNAGSVSFFPQADTCKVQSNRVFCDTMNSL
TLPSEVSLCNTDIFNSKYDCKIMTSKTDISSSVITSLGAIVSCYGKTKCTASNKNRGIIKTFSN
 
GCDYVSNKGVDTVSVGNTLYYVNKLEGKNLYVKGEPIINYYDPLVFPSDEFDASISQVNEKI
NQSLAFIRRSDELLHNVNTGKSTTNIMITAIIIVIIVVLLSLIAIGLLLY
SEQ ID NO:83 
MELPILKTNAITTILAAVTLCFASSQNITEEFYQSTCSAVSKGYLSALRTGWYASVITIELSNIK
ENKCNGTDAKVKLIKQELDKYKNAVTELQLLMQSTCAANSRARRELPRFMNYTLNNTKN
TNVTLSKKRKRRFLGFLLGVGSAIASGCAVSKVLHLEGEVNKIKSALLSTNKAVVSLSNGVS
VLTMKVLDLKNYIDKQLLPIVNKQSCSISNIETVIEFQQKNNRLLEITREFSVNAGVTTPVST
YMLTNSELLSLINDMPITNDQKKLMSSNVQIVRQQSYSIMSIIKEELLAYVVQLPLYGVIDTP
CWKLHTSPLCTTNTKEGSNICLTRTDRGWYCDNAGSVSFFPQAETCKVQSNRVFCDTMNS
LTLPSEVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSDEFDASISQVNE
KINQSLAFIRKSDELLHNVNAGKSTTNIMITTIIIVIIVILLALIAVGLLLY
SEQ ID NO:84 
MELLIHRSSAIFLTLAINALYLTSSQNITEEFYQSTCSAVSRGYFSALRTGWYASVITIELSNIK
ETKCNGTDTKVKLIKQELDKYKNAVTELQLLMQNTCAANNRARREAPQYMNYTINTTKN
LNVSISKKRKRRFLGFLLGVGSAIASGCAVSKVLHLEGEVNKIKNALLSTNKAVVSLSNGVS
VLTMKVLDLKNYINNQLLPIVNQQSCRISNIETVIEFQQKNSRLLEITREFSVNAGVTTPLST
YMLTNSELLSLINDMPITNDQKKLMSSNVQIVRQQSYSIMSIIKEELLAYVVQLPIYGVIDTP
CWKLHTSPLCTTNIKEGSNICLTRTDRGWYCDNAGSVSFFPQADTCKVQSNRVFCDTMNSL
TLPSEVSLCNTDIFNSKYDCKIMTSKTDISSSVITSLGAIVSCYGKTKCTASNKNRGIIKTFSN
GCDYVSNKGVDTVSVGNTLYYVNKLEGKNLYVKGEPIINYYDPLVFPSDEFDASISQVNEKI
NQSLAFIRRSDELLHNVNTGKSTTNIMITAIIIVIIVVLLSLIAIGLLLY
SEQ ID NO:85 
MELPILKTNAITTILAAVTLCFASSQNITEEFYQSTCSAVSKGYLSALRTGWYVSVITIELSNIK
ENKCNGTDAKVKLIKQELDKYKNAVTELQLLMQSTCAANSRARRELPRFMNYTLNNTKN
TNVTLSKKRKRRFLGFLLGVGSAIASGCAVSKVLHLEGEVNKIKSALLSTNKAVVSLSNGVS
VLTFKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:86 
MELLIHRSSAIFLTLAINALYLTSSQNITEEFYQSTCSAVSRGYFSALRTGWYVSVITIELSNIK
ETKCNGTDTKVKLIKQELDKYKNAVTELQLLMQNTCAANNRARREAPQYMNYTINTTKN
LNVSISKKRKRRFLGFLLGVGSAIASGCAVSKVLHLEGEVNKIKNALLSTNKAVVSLSNGVS
VLTFKVLDLKNYINNQLLPIVNQQSCRISNIETVIEFQQKNSRLLEITREFSVNAGVTTPLSTY
MLTNSELLSLINDMPITNDQKKLMSSNVQIVRQQSYSIMSIIKEELLAYVVQLPIYGVIDTPC
WKLHTSPLCTTNIKEGSNICLTRTDRGWYCDNAGSVSFFPQADTCKVQSNRVFCDTMNSLT
LPSEVSLCNTDIFNSKYDCKIMTSKTDISSSVITSLGAIVSCYGKTKCTASNKNRGIIKTFSNG
CDYVSNKGVDTVSVGNTLYYVNKLEGKNLYVKGEPIINYYDPLVFPSDEFDASISQVNEKIN
 QSLAFIRRSDELLHNVNTGKSTTNIMITAIIIVIIVVLLSLIAIGLLLY
SEQ ID NO:87 
MELPILKTNAITTILAAVTLCFASSQNITEEFYQSTCSAVSKGYLSALRTGWYFSVITIELSNIK
ENKCNGTDAKVKLIKQELDKYKNAVTELQLLMQSTCAANSRARRELPRFMNYTLNNTKN
TNVTLSKKRKRRFLGFLLGVGSAIASGCAVSKVLHLEGEVNKIKSALLSTNKAVVSLSNGVS
VLTFKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:88 
MELLIHRSSAIFLTLAINALYLTSSQNITEEFYQSTCSAVSRGYFSALRTGWYFSVITIELSNIK
ETKCNGTDTKVKLIKQELDKYKNAVTELQLLMQNTCAANNRARREAPQYMNYTINTTKN
LNVSISKKRKRRFLGFLLGVGSAIASGCAVSKVLHLEGEVNKIKNALLSTNKAVVSLSNGVS
VLTFKVLDLKNYINNQLLPIVNQQSCRISNIETVIEFQQKNSRLLEITREFSVNAGVTTPLSTY
MLTNSELLSLINDMPITNDQKKLMSSNVQIVRQQSYSIMSIIKEELLAYVVQLPIYGVIDTPC
WKLHTSPLCTTNIKEGSNICLTRTDRGWYCDNAGSVSFFPQADTCKVQSNRVFCDTMNSLT
LPSEVSLCNTDIFNSKYDCKIMTSKTDISSSVITSLGAIVSCYGKTKCTASNKNRGIIKTFSNG
CDYVSNKGVDTVSVGNTLYYVNKLEGKNLYVKGEPIINYYDPLVFPSDEFDASISQVNEKIN
QSLAFIRRSDELLHNVNTGKSTTNIMITAIIIVIIVVLLSLIAIGLLLY
SEQ ID NO:89 
MELPILKTNAITTILAAVTLCFASSQNITEEFYQSTCSAVSKGYLSALRTGWYCSVITIELSNIK
ENKCNGTDAKVKLIKQELDKYKNAVTELQLLMQSTCAANSRARRELPRFMNYTLNNTKN
TNVTLSKKRKRRFLGFLLGVGSAIASGCAVSKVLHLEGEVNKIKSALLSTNKAVVSLSNGVS
VLTFKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:90 
MELLIHRSSAIFLTLAINALYLTSSQNITEEFYQSTCSAVSRGYFSALRTGWYCSVITIELSNIK
ETKCNGTDTKVKLIKQELDKYKNAVTELQLLMQNTCAANNRARREAPQYMNYTINTTKN
LNVSISKKRKRRFLGFLLGVGSAIASGCAVSKVLHLEGEVNKIKNALLSTNKAVVSLSNGVS
VLTFKVLDLKNYINNQLLPIVNQQSCRISNIETVIEFQQKNSRLLEITREFSVNAGVTTPLSTY
MLTNSELLSLINDMPITNDQKKLMSSNVQIVRQQSYSIMSIIKEELLAYVVQLPIYGVIDTPC
WKLHTSPLCTTNIKEGSNICLTRTDRGWYCDNAGSVSFFPQADTCKVQSNRVFCDTMNSLT
LPSEVSLCNTDIFNSKYDCKIMTSKTDISSSVITSLGAIVSCYGKTKCTASNKNRGIIKTFSNG
CDYVSNKGVDTVSVGNTLYYVNKLEGKNLYVKGEPIINYYDPLVFPSDEFDASISQVNEKIN
QSLAFIRRSDELLHNVNTGKSTTNIMITAIIIVIIVVLLSLIAIGLLLY
SEQ ID NO:91 
MELPILKTNAITTILAAVTLCFASSQNITEEFYQSTCSAVSKGYLSALRTGWYASVITIELSNIK
ENKCNGTDAKVKLIKQELDKYKNAVTELQLLMQSTCAANSRARRELPRFMNYTLNNTKN
TNVTLSKKRKRRFLGFLLGVGSAIASGCAVSKVLHLEGEVNKIKSALLSTNKAVVSLSNGVS
VLTFKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:92 
MELLIHRSSAIFLTLAINALYLTSSQNITEEFYQSTCSAVSRGYFSALRTGWYASVITIELSNIK
ETKCNGTDTKVKLIKQELDKYKNAVTELQLLMQNTCAANNRARREAPQYMNYTINTTKN
LNVSISKKRKRRFLGFLLGVGSAIASGCAVSKVLHLEGEVNKIKNALLSTNKAVVSLSNGVS
VLTFKVLDLKNYINNQLLPIVNQQSCRISNIETVIEFQQKNSRLLEITREFSVNAGVTTPLSTY
MLTNSELLSLINDMPITNDQKKLMSSNVQIVRQQSYSIMSIIKEELLAYVVQLPIYGVIDTPC
WKLHTSPLCTTNIKEGSNICLTRTDRGWYCDNAGSVSFFPQADTCKVQSNRVFCDTMNSLT
LPSEVSLCNTDIFNSKYDCKIMTSKTDISSSVITSLGAIVSCYGKTKCTASNKNRGIIKTFSNG
CDYVSNKGVDTVSVGNTLYYVNKLEGKNLYVKGEPIINYYDPLVFPSDEFDASISQVNEKIN
QSLAFIRRSDELLHNVNTGKSTTNIMITAIIIVIIVVLLSLIAIGLLLY
SEQ ID NO:93 
MELPILKTNAITTILAAVTLCFASSQNITEEFYQSTCSAVSKGYLSALRTGWYVSVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:94 
MELLIHRSSAIFLTLAINALYLTSSQNITEEFYQSTCSAVSRGYFSALRTGWYVSVITIELSNIK
ETKCNGTDTKVKLIKQELDKYKNAVTELQLLMQNTPCANNRARREAPQYMNYTINTTKNL
NVSISKKRKRRFLGFLLGVGSAIASGCAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEELLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDEFDASISQVNEKINQS
LAFIRRSDELLHNVNTGKSTTNIMITAIIIVIIVVLLSLIAIGLLLY
SEQ ID NO:95 
MELPILKTNAITTILAAVTLCFASSQNITEEFYQSTCSAVSKGYLSALRTGWYFSVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:96 
MELLIHRSSAIFLTLAINALYLTSSQNITEEFYQSTCSAVSRGYFSALRTGWYFSVITIELSNIK
ETKCNGTDTKVKLIKQELDKYKNAVTELQLLMQNTPCANNRARREAPQYMNYTINTTKNL
NVSISKKRKRRFLGFLLGVGSAIASGCAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEELLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDEFDASISQVNEKINQS
LAFIRRSDELLHNVNTGKSTTNIMITAIIIVIIVVLLSLIAIGLLLY
SEQ ID NO:97 
MELPILKTNAITTILAAVTLCFASSQNITEEFYQSTCSAVSKGYLSALRTGWYCSVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:98 
MELLIHRSSAIFLTLAINALYLTSSQNITEEFYQSTCSAVSRGYFSALRTGWYCSVITIELSNIK
ETKCNGTDTKVKLIKQELDKYKNAVTELQLLMQNTPCANNRARREAPQYMNYTINTTKNL
NVSISKKRKRRFLGFLLGVGSAIASGCAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEELLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDEFDASISQVNEKINQS
LAFIRRSDELLHNVNTGKSTTNIMITAIIIVIIVVLLSLIAIGLLLY
SEQ ID NO:99 
MELPILKTNAITTILAAVTLCFASSQNITEEFYQSTCSAVSKGYLSALRTGWYASVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:100 
MELLIHRSSAIFLTLAINALYLTSSQNITEEFYQSTCSAVSRGYFSALRTGWYASVITIELSNIK
ETKCNGTDTKVKLIKQELDKYKNAVTELQLLMQNTPCANNRARREAPQYMNYTINTTKNL
NVSISKKRKRRFLGFLLGVGSAIASGCAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEELLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDEFDASISQVNEKINQS
LAFIRRSDELLHNVNTGKSTTNIMITAIIIVIIVVLLSLIAIGLLLY
SEQ ID NO:101 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPACNSRARRELPRFMNYTLNNTKNT
NVTLSKKRKRRFLGFLLGVGSACASGIAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLSAIGGYIPEAPRDGQAYVRKDGEWVLLSTFL
SEQ ID NO: 102 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPACNNRARREAPQYMNYTINTTKNL
NVSISKKRKRRFLGFLLGVGSACASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLSAIGGYIPEAPRDGQAYVRKDGEWVLLSTFL
SEQ ID NO: 103 MELPILKTNAITTILAAVTLCFASSQNITEEFYQSTCSAVSKGYLSALRTGWYHSVITIELSNIK 
ENKCNGTDAKVKLIKQELDKYKNAVTELQLLMQSTPACNSRARRELPRFMNYTLNNTKNT
NVTLSKKRKRRFLGFLLGVGSACASGIAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLHNVNAGKSTTNIMITTIIIVIIVILLALIAVGLLLY
SEQ ID NO:104 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPACNNRARREAPQYMNYTINTTKNL
NVSISKKRKRRFLGFLLGVGSACASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLHNVNTGKSTTNIMITAIIIVIIVVLLSLIAIGLLLY
SEQ ID NO:105 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTTGCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGATGCGGAAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTCTAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCGTGTGCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCTGCATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
 
CGTGAAGGGCGAGCCCATCATCAACTTCTACGACCCTCTGGTGTTCCCCAGCAGCGAGT
TTGATGCCAGCATCTCCCAAGTGAACGAGAAGATCAACCAGAGCCTGGCCTTCATCAGA
AAGTCCGATGAGCTGCTGAGCGCCATCGGCGGCTATATCCCTGAAGCTCCTAGAGATGG
ACAGGCCTATGTGCGGAAGGATGGCGAATGGGTGCTGCTGTCTACCTTCCTGTGA
SEQ ID NO:106 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTTGCCTGAGAACCGGCTGGTATCACAGCGTGATCACCATC
GAGCTGAGCAACATCAAAGAAACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGATGCGGAAGCGCCATTGCCTCTGGAATTGCCGTGTCTAAG
GTGCTGCACCTGGAAGGCGAAGTGAACAAGATCAAGAATGCCCTGCTGAGCACCAACA
AGGCCGTGGTGTCTCTGTCTAATGGCGTGTCCGTGCTGACCAGCAAGGTGCTGGACCTG
AAGAACTACATCAACAACCAGCTGCTGCCCATCGTGAACCAGCAGAGCTGCCGGATCA
GCAACATCGAGACAGTGATCGAGTTCCAGCAGAAGAACAGCAGACTGCTGGAAATCAC
CCGCGAGTTTTCCGTGAATGCCGGCGTGACAACACCCCTGAGCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGTCCTACAGCATCATGAGCATTATCA
AAGAAGAGGTGCTGGCCTACGTGGTGCAGCTGCCTATCTACGGCGTGTGCGATACCCCT
TGCTGGAAGCTGCACACAAGCCCTCTGTGCACCACCAATATCAAAGAGGGCTCCAACAT
CTGCCTGACCAGAACCGATAGAGGCTGGTACTGCGATAATGCCGGCAGCGTCAGCTTCT
TCCCACAAGCCGATACCTGCAAGGTGCAGAGCAACAGAGTGTTCTGCGATACCATGAAC
AGCCTGACACTGCCCAGCGAGGTGTCCCTGTGCAACACCGACATCTTCAACTCTAAGTA
CGACTGCAAGATCATGACCTCCAAGACCGACATCAGCTCCTCCTGCATCACATCTCTGG
GCGCCATCGTGTCCTGCTACGGCAAGACAAAGTGTACCGCCAGCAACAAGAACCGGGG
CATCATCAAGACCTTCAGCAACGGCTGCGACTACGTGTCCAACAAAGGCGTGGACACC
GTGTCTGTGGGCAACACCCTGTACTACGTGAACAAGCTGGAAGGCAAGAATCTGTACGT
GAAGGGCGAGCCCATCATCAACTACTACGACCCTCTGGTGTTCCCCAGCAGCGAGTTTG
ATGCCAGCATCAGCCAAGTGAACGAGAAGATCAACCAGAGCCTGGCCTTCATCCGCAG
ATCCGATGAACTGCTGTCTGCCATCGGCGGCTATATCCCCGAAGCTCCTAGAGATGGACA
GGCCTATGTGCGGAAGGATGGCGAATGGGTGCTGCTGTCTACCTTCCTGTGA
SEQ ID NO: 107 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTTGCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGAATTGCCGTGTCCAAG
GTGCTGCATCTGGAAGGCGAAGTGAACAAGATCAAGAGCGCCCTGCTGAGCACCAACA
 
AGGCCGTGGTTTCTCTGAGCAATGGCGTGTCCGTGCTGACCTCTAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCGTGTGCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCAGCGAGT
TTGATGCCAGCATCTCCCAAGTGAACGAGAAGATCAACCAGAGCCTGGCCTTCATCAGA
AAGTCCGATGAGCTGCTGAGCGCCATCGGCGGCTATATCCCTGAAGCTCCTAGAGATGG
ACAGGCCTATGTGCGGAAGGATGGCGAATGGGTGCTGCTGTCTACCTTCCTGTGA
SEQ ID NO: 108 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTTGCCTGAGAACCGGCTGGTATCACAGCGTGATCACCATC
GAGCTGAGCAACATCAAAGAAACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGGTGCTGGCCTACGTGGTGCAGCTGCCTATCTACGGCGTGTGCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
 
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGA
SEQ ID NO:109 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCTGCAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGATGCGGAAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTCTAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCTGCATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCTGCATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCAGCGAGT
TTGATGCCAGCATCTCCCAAGTGAACGAGAAGATCAACCAGAGCCTGGCCTTCATCAGA
AAGTCCGATGAGCTGCTGAGCGCCATCGGCGGCTATATCCCTGAAGCTCCTAGAGATGG
ACAGGCCTATGTGCGGAAGGATGGCGAATGGGTGCTGCTGTCTACCTTCCTGTGA
SEQ ID NO:110 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCTGCAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGATGCGGAAGCGCCATTGCCTCTGGAATTGCCGTGTCTAA
GGTGCTGCACCTGGAAGGCGAAGTGAACAAGATCAAGAATGCCCTGCTGAGCACCAAC
 
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGGTGCTGGCCTACGTGGTGCAGCTGCCTATCTACGGCTGCATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCTGCATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGA
SEQ ID NO:111 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCTGCAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTCTAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCTGCATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
 
CGTGAAGGGCGAGCCCATCATCAACTTCTACGACCCTCTGGTGTTCCCCAGCAGCGAGT
TTGATGCCAGCATCTCCCAAGTGAACGAGAAGATCAACCAGAGCCTGGCCTTCATCAGA
AAGTCCGATGAGCTGCTGAGCGCCATCGGCGGCTATATCCCTGAAGCTCCTAGAGATGG
ACAGGCCTATGTGCGGAAGGATGGCGAATGGGTGCTGCTGTCTACCTTCCTGTGA
SEQ ID NO:112 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCTGCAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGGTGCTGGCCTACGTGGTGCAGCTGCCTATCTACGGCTGCATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGA
SEQ ID NO: 113 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTGCGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGATGCGGAAGCGCCATTGCCTCTGGAATTGCCGTGTCCAAG
GTGCTGCATCTGGAAGGCGAAGTGAACAAGATCAAGAGCGCCCTGCTGAGCACCAACA
 
AGGCCGTGGTTTCTCTGAGCAATGGCGTGTCCGTGCTGACCTCTAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCGTGATCGATTGCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCTGCATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCAGCGAGT
TTGATGCCAGCATCTCCCAAGTGAACGAGAAGATCAACCAGAGCCTGGCCTTCATCAGA
AAGTCCGATGAGCTGCTGAGCGCCATCGGCGGCTATATCCCTGAAGCTCCTAGAGATGG
ACAGGCCTATGTGCGGAAGGATGGCGAATGGGTGCTGCTGTCTACCTTCCTGTGA
SEQ ID NO:114 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTGCGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGATGCGGAAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGGTGCTGGCCTACGTGGTGCAGCTGCCTATCTACGGCGTGATCGATTGCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCTGCATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
 
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGA
SEQ ID NO:115 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTGCGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTCTAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCGTGATCGATTGCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCAGCGAGT
TTGATGCCAGCATCTCCCAAGTGAACGAGAAGATCAACCAGAGCCTGGCCTTCATCAGA
AAGTCCGATGAGCTGCTGAGCGCCATCGGCGGCTATATCCCTGAAGCTCCTAGAGATGG
ACAGGCCTATGTGCGGAAGGATGGCGAATGGGTGCTGCTGTCTACCTTCCTGTGA
SEQ ID NO:116 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTGCGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
 
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGGTGCTGGCCTACGTGGTGCAGCTGCCTATCTACGGCGTGATCGATTGCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGA
SEQ ID NO:117 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCTGCGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
 
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO:118 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTTGCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCTGCGCCTCTGGAATTGCCGTGTCTA
AGGTGCTGCACCTGGAAGGCGAAGTGAACAAGATCAAGAATGCCCTGCTGAGCACCAA
CAAGGCCGTGGTGTCTCTGTCTAATGGCGTGTCCGTGCTGACCATCAAGGTGCTGGACC
TGAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO:119 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
 
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO: 120 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTTGCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
 
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO:121 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCTGCAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGTGCAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO:122 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCGCCTGCAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGTGCAGCGCCATTGCCTCTGGAATTGCCGTGTCTAA
GGTGCTGCACCTGGAAGGCGAAGTGAACAAGATCAAGAATGCCCTGCTGAGCACCAAC
 
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO: 123 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCTGCAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGATGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
 
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO: 124 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCGCCTGCAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGATGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO:125 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATTGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCTGCATTGCCTCTGGAATTGCCGTGTCCAAG
GTGCTGCATCTGGAAGGCGAAGTGAACAAGATCAAGAGCGCCCTGCTGAGCACCAACA
 
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO: 126 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCGCCAACTGCCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCTGCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
 
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO:127 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATTGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGTGCAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO:128 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCGCCAACTGCCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGTGCAGCGCCATTGCCTCTGGAATTGCCGTGTCTAA
GGTGCTGCACCTGGAAGGCGAAGTGAACAAGATCAAGAATGCCCTGCTGAGCACCAAC
 
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO: 129 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATTGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGATGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
 
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO: 130 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCGCCAACTGCCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGATGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO:131 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCTGCGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
 
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO: 132 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCTGCGCCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
 
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO:133 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCTGCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGATGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO: 134 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCTGCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGATGCGCCATTGCCTCTGGAATTGCCGTGTCTAA
GGTGCTGCACCTGGAAGGCGAAGTGAACAAGATCAAGAATGCCCTGCTGAGCACCAAC
 
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO: 135 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAATCAACAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTGCCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGAATTGCCGTGTCCAAGG
TGCTGCATCTGGAAGGCGAAGTGAACAAGATCAAGAGCGCCCTGCTGAGCACCAACAA
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCAGCATCAG
CCCCATCGAGACAGTGATCGAGTTCCAGCAGAAGAACAACCGGCTGCTGGAAATCACC
CGCGAGTTTTCTGTGAATGCCGGCGTGACCACACCTGTGTCCACCTACATGCTGACCAA
CAGCGAGCTGCTGTCCCTGATCAACGACATGCCCATCACCAACGACCAGAAAAAGCTG
ATGAGCAGCAACGTGCAGATCGTGCGGCAGCAGAGCTACTCCATCATGAGCATTATCAA
AGAAGAGGTGCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
 
GTGAAGGGCGAGCCCATCATCAACTTCTACGACCCTCTGGTGTTCCCCAGCCAGGAGTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO: 136 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAATCACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCGGATC
AGCCCCATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGGTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCCAGGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO:137 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAATCAACAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTGCCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGAATTGCCGTGTCCAAGG
TGCTGCATCTGGAAGGCGAAGTGAACAAGATCAAGAGCGCCCTGCTGAGCACCAACAA
 
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCAGCATCAG
CCCCATCGAGACAGTGATCGAGTTCCAGCAGAAGAACAACCGGCTGCTGGAAATCACC
CGCGAGTTTTCTGTGAATGCCGGCGTGACCACACCTGTGTCCACCTACATGCTGACCAA
CAGCGAGCTGCTGTCCCTGATCAACGACATGCCCATCACCAACGACCAGAAAAAGCTG
ATGAGCAGCAACGTGCAGATCGTGCGGCAGCAGAGCTACTCCATCATGAGCATTATCAA
AGAAGAGGTGCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCGACACCTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO: 138 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAATCACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCGGATC
AGCCCCATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGGTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
 
TGAAGGGCGAGCCCATCATCAACTACTACGACCCTCTGGTGTTCCCCAGCGACACCTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO:139 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAGAAATCAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTGCCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGAATTGCCGTGTCCAAGG
TGCTGCATCTGGAAGGCGAAGTGAACAAGATCAAGAGCGCCCTGCTGAGCACCAACAA
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCAGCATCAG
CCCCATCGAGACAGTGATCGAGTTCCAGCAGAAGAACAACCGGCTGCTGGAAATCACC
CGCGAGTTTTCTGTGAATGCCGGCGTGACCACACCTGTGTCCACCTACATGCTGACCAA
CAGCGAGCTGCTGTCCCTGATCAACGACATGCCCATCACCAACGACCAGAAAAAGCTG
ATGAGCAGCAACGTGCAGATCGTGCGGCAGCAGAGCTACTCCATCATGAGCATTATCAA
AGAAGAGGTGCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCGACCAGTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO: 140 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAGAAATCAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
 
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCGGATC
AGCCCCATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGGTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACCAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO:141 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAATCAACAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTGCCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGAATTGCCGTGTCCAAGG
TGCTGCATCTGGAAGGCGAAGTGAACAAGATCAAGAGCGCCCTGCTGAGCACCAACAA
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCCCCATCAGC
AACATCGAGACAGTGATCGAGTTCCAGCAGAAGAACAACCGGCTGCTGGAAATCACCC
GCGAGTTTTCTGTGAATGCCGGCGTGACCACACCTGTGTCCACCTACATGCTGACCAAC
AGCGAGCTGCTGTCCCTGATCAACGACATGCCCATCACCAACGACCAGAAAAAGCTGAT
GAGCAGCAACGTGCAGATCGTGCGGCAGCAGAGCTACTCCATCATGAGCATTATCAAAG
AAGAGGTGCTGGCCTACGTGGTGCAGCTGCCTCTGTATGGCGTGATCGATACCCCTTGCT
GGAAGCTGCACACAAGCCCTCTGTGCACCACCAACACCAAAGAGGGCTCCAACATCTG
CCTGACCAGAACCGATAGAGGCTGGTACTGCGATAATGCCGGCAGCGTCAGCTTCTTCC
CACAAGCCGAGACATGCAAGGTGCAGAGCAACAGAGTGTTCTGCGATACCATGAACAG
CCTGACACTGCCCTCCGAAGTGAATCTGTGCAATATCGACATCTTCAACCCGAAGTACG
ACTGCAAGATCATGACCAGCAAGACCGACGTGTCAAGCTCCGTGATCACATCTCTGGGC
GCCATCGTGTCCTGCTACGGCAAGACAAAGTGCACCGCCAGCAACAAGAACCGGGGCA
TCATCAAGACCTTCAGCAACGGCTGCGACTACGTGTCCAACAAAGGCGTGGACACCGT
GTCTGTGGGCAACACCCTGTACTACGTGAACAAGCAAGAAGGCAAGAGCCTGTACGTG
 
AAGGGCGAGCCCATCATCAACTTCTACGACCCTCTGGTGTTCCCCAGCCAGGAGTTTGA
TGCCAGCATCTCCCAAGTGAACGAGAAGATCAACCAGAGCCTGGCCTTCATCAGAAAG
TCCGATGAGCTGCTGAGCGCCATCGGCGGCTATATCCCTGAAGCTCCTAGAGATGGACA
GGCCTATGTGCGGAAGGATGGCGAATGGGTGCTGCTGTCTACCTTCCTGTGATGA
SEQ ID NO: 142 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAATCACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCCCATCA
GCAACATCGAGACAGTGATCGAGTTCCAGCAGAAGAACAGCAGACTGCTGGAAATCAC
CCGCGAGTTTTCCGTGAATGCCGGCGTGACAACACCCCTGAGCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGTCCTACAGCATCATGAGCATTATCA
AAGAAGAGGTGCTGGCCTACGTGGTGCAGCTGCCTATCTACGGCGTGATCGATACCCCT
TGCTGGAAGCTGCACACAAGCCCTCTGTGCACCACCAATATCAAAGAGGGCTCCAACAT
CTGCCTGACCAGAACCGATAGAGGCTGGTACTGCGATAATGCCGGCAGCGTCAGCTTCT
TCCCACAAGCCGATACCTGCAAGGTGCAGAGCAACAGAGTGTTCTGCGATACCATGAAC
AGCCTGACACTGCCCAGCGAGGTGTCCCTGTGCAACACCGACATCTTCAACTCTAAGTA
CGACTGCAAGATCATGACCTCCAAGACCGACATCAGCTCCTCCGTGATCACATCTCTGG
GCGCCATCGTGTCCTGCTACGGCAAGACAAAGTGTACCGCCAGCAACAAGAACCGGGG
CATCATCAAGACCTTCAGCAACGGCTGCGACTACGTGTCCAACAAAGGCGTGGACACC
GTGTCTGTGGGCAACACCCTGTACTACGTGAACAAGCTGGAAGGCAAGAATCTGTACGT
GAAGGGCGAGCCCATCATCAACTACTACGACCCTCTGGTGTTCCCCAGCCAGGAGTTTG
ATGCCAGCATCAGCCAAGTGAACGAGAAGATCAACCAGAGCCTGGCCTTCATCCGCAG
ATCCGATGAACTGCTGTCTGCCATCGGCGGCTATATCCCCGAAGCTCCTAGAGATGGACA
GGCCTATGTGCGGAAGGATGGCGAATGGGTGCTGCTGTCTACCTTCCTGTGATGA
SEQ ID NO: 143 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAATCAACAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTGCCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGAATTGCCGTGTCCAAGG
TGCTGCATCTGGAAGGCGAAGTGAACAAGATCAAGAGCGCCCTGCTGAGCACCAACAA
 
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCCCCATCAGC
AACATCGAGACAGTGATCGAGTTCCAGCAGAAGAACAACCGGCTGCTGGAAATCACCC
GCGAGTTTTCTGTGAATGCCGGCGTGACCACACCTGTGTCCACCTACATGCTGACCAAC
AGCGAGCTGCTGTCCCTGATCAACGACATGCCCATCACCAACGACCAGAAAAAGCTGAT
GAGCAGCAACGTGCAGATCGTGCGGCAGCAGAGCTACTCCATCATGAGCATTATCAAAG
AAGAGGTGCTGGCCTACGTGGTGCAGCTGCCTCTGTATGGCGTGATCGATACCCCTTGCT
GGAAGCTGCACACAAGCCCTCTGTGCACCACCAACACCAAAGAGGGCTCCAACATCTG
CCTGACCAGAACCGATAGAGGCTGGTACTGCGATAATGCCGGCAGCGTCAGCTTCTTCC
CACAAGCCGAGACATGCAAGGTGCAGAGCAACAGAGTGTTCTGCGATACCATGAACAG
CCTGACACTGCCCTCCGAAGTGAATCTGTGCAATATCGACATCTTCAACCCGAAGTACG
ACTGCAAGATCATGACCAGCAAGACCGACGTGTCAAGCTCCGTGATCACATCTCTGGGC
GCCATCGTGTCCTGCTACGGCAAGACAAAGTGCACCGCCAGCAACAAGAACCGGGGCA
TCATCAAGACCTTCAGCAACGGCTGCGACTACGTGTCCAACAAAGGCGTGGACACCGT
GTCTGTGGGCAACACCCTGTACTACGTGAACAAGCAAGAAGGCAAGAGCCTGTACGTG
AAGGGCGAGCCCATCATCAACTTCTACGACCCTCTGGTGTTCCCCAGCGACACCTTTGA
TGCCAGCATCTCCCAAGTGAACGAGAAGATCAACCAGAGCCTGGCCTTCATCAGAAAG
TCCGATGAGCTGCTGAGCGCCATCGGCGGCTATATCCCTGAAGCTCCTAGAGATGGACA
GGCCTATGTGCGGAAGGATGGCGAATGGGTGCTGCTGTCTACCTTCCTGTGATGA
SEQ ID NO: 144 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAATCACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCCCATCA
GCAACATCGAGACAGTGATCGAGTTCCAGCAGAAGAACAGCAGACTGCTGGAAATCAC
CCGCGAGTTTTCCGTGAATGCCGGCGTGACAACACCCCTGAGCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGTCCTACAGCATCATGAGCATTATCA
AAGAAGAGGTGCTGGCCTACGTGGTGCAGCTGCCTATCTACGGCGTGATCGATACCCCT
TGCTGGAAGCTGCACACAAGCCCTCTGTGCACCACCAATATCAAAGAGGGCTCCAACAT
CTGCCTGACCAGAACCGATAGAGGCTGGTACTGCGATAATGCCGGCAGCGTCAGCTTCT
TCCCACAAGCCGATACCTGCAAGGTGCAGAGCAACAGAGTGTTCTGCGATACCATGAAC
AGCCTGACACTGCCCAGCGAGGTGTCCCTGTGCAACACCGACATCTTCAACTCTAAGTA
CGACTGCAAGATCATGACCTCCAAGACCGACATCAGCTCCTCCGTGATCACATCTCTGG
GCGCCATCGTGTCCTGCTACGGCAAGACAAAGTGTACCGCCAGCAACAAGAACCGGGG
CATCATCAAGACCTTCAGCAACGGCTGCGACTACGTGTCCAACAAAGGCGTGGACACC
GTGTCTGTGGGCAACACCCTGTACTACGTGAACAAGCTGGAAGGCAAGAATCTGTACGT
 
GAAGGGCGAGCCCATCATCAACTACTACGACCCTCTGGTGTTCCCCAGCGACACCTTTG
ATGCCAGCATCAGCCAAGTGAACGAGAAGATCAACCAGAGCCTGGCCTTCATCCGCAG
ATCCGATGAACTGCTGTCTGCCATCGGCGGCTATATCCCCGAAGCTCCTAGAGATGGACA
GGCCTATGTGCGGAAGGATGGCGAATGGGTGCTGCTGTCTACCTTCCTGTGATGA
SEQ ID NO: 145 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAGAAATCAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTGCCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGAATTGCCGTGTCCAAGG
TGCTGCATCTGGAAGGCGAAGTGAACAAGATCAAGAGCGCCCTGCTGAGCACCAACAA
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCCCCATCAGC
AACATCGAGACAGTGATCGAGTTCCAGCAGAAGAACAACCGGCTGCTGGAAATCACCC
GCGAGTTTTCTGTGAATGCCGGCGTGACCACACCTGTGTCCACCTACATGCTGACCAAC
AGCGAGCTGCTGTCCCTGATCAACGACATGCCCATCACCAACGACCAGAAAAAGCTGAT
GAGCAGCAACGTGCAGATCGTGCGGCAGCAGAGCTACTCCATCATGAGCATTATCAAAG
AAGAGGTGCTGGCCTACGTGGTGCAGCTGCCTCTGTATGGCGTGATCGATACCCCTTGCT
GGAAGCTGCACACAAGCCCTCTGTGCACCACCAACACCAAAGAGGGCTCCAACATCTG
CCTGACCAGAACCGATAGAGGCTGGTACTGCGATAATGCCGGCAGCGTCAGCTTCTTCC
CACAAGCCGAGACATGCAAGGTGCAGAGCAACAGAGTGTTCTGCGATACCATGAACAG
CCTGACACTGCCCTCCGAAGTGAATCTGTGCAATATCGACATCTTCAACCCGAAGTACG
ACTGCAAGATCATGACCAGCAAGACCGACGTGTCAAGCTCCGTGATCACATCTCTGGGC
GCCATCGTGTCCTGCTACGGCAAGACAAAGTGCACCGCCAGCAACAAGAACCGGGGCA
TCATCAAGACCTTCAGCAACGGCTGCGACTACGTGTCCAACAAAGGCGTGGACACCGT
GTCTGTGGGCAACACCCTGTACTACGTGAACAAGCAAGAAGGCAAGAGCCTGTACGTG
AAGGGCGAGCCCATCATCAACTTCTACGACCCTCTGGTGTTCCCCAGCGACCAGTTTGA
TGCCAGCATCTCCCAAGTGAACGAGAAGATCAACCAGAGCCTGGCCTTCATCAGAAAG
TCCGATGAGCTGCTGAGCGCCATCGGCGGCTATATCCCTGAAGCTCCTAGAGATGGACA
GGCCTATGTGCGGAAGGATGGCGAATGGGTGCTGCTGTCTACCTTCCTGTGATGA
SEQ ID NO: 146 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAGAAATCAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
 
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCCCATCA
GCAACATCGAGACAGTGATCGAGTTCCAGCAGAAGAACAGCAGACTGCTGGAAATCAC
CCGCGAGTTTTCCGTGAATGCCGGCGTGACAACACCCCTGAGCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGTCCTACAGCATCATGAGCATTATCA
AAGAAGAGGTGCTGGCCTACGTGGTGCAGCTGCCTATCTACGGCGTGATCGATACCCCT
TGCTGGAAGCTGCACACAAGCCCTCTGTGCACCACCAATATCAAAGAGGGCTCCAACAT
CTGCCTGACCAGAACCGATAGAGGCTGGTACTGCGATAATGCCGGCAGCGTCAGCTTCT
TCCCACAAGCCGATACCTGCAAGGTGCAGAGCAACAGAGTGTTCTGCGATACCATGAAC
AGCCTGACACTGCCCAGCGAGGTGTCCCTGTGCAACACCGACATCTTCAACTCTAAGTA
CGACTGCAAGATCATGACCTCCAAGACCGACATCAGCTCCTCCGTGATCACATCTCTGG
GCGCCATCGTGTCCTGCTACGGCAAGACAAAGTGTACCGCCAGCAACAAGAACCGGGG
CATCATCAAGACCTTCAGCAACGGCTGCGACTACGTGTCCAACAAAGGCGTGGACACC
GTGTCTGTGGGCAACACCCTGTACTACGTGAACAAGCTGGAAGGCAAGAATCTGTACGT
GAAGGGCGAGCCCATCATCAACTACTACGACCCTCTGGTGTTCCCCAGCGACCAGTTTG
ATGCCAGCATCAGCCAAGTGAACGAGAAGATCAACCAGAGCCTGGCCTTCATCCGCAG
ATCCGATGAACTGCTGTCTGCCATCGGCGGCTATATCCCCGAAGCTCCTAGAGATGGACA
GGCCTATGTGCGGAAGGATGGCGAATGGGTGCTGCTGTCTACCTTCCTGTGATGA
SEQ ID NO:147 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAGAAATCAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTGCCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGAATTGCCGTGTCCAAGG
TGCTGCATCTGGAAGGCGAAGTGAACAAGATCAAGAGCGCCCTGCTGAGCACCAACAA
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCAGCATCCCC
AACATCGAGACAGTGATCGAGTTCCAGCAGAAGAACAACCGGCTGCTGGAAATCACCC
GCGAGTTTTCTGTGAATGCCGGCGTGACCACACCTGTGTCCACCTACATGCTGACCAAC
AGCGAGCTGCTGTCCCTGATCAACGACATGCCCATCACCAACGACCAGAAAAAGCTGAT
GAGCAGCAACGTGCAGATCGTGCGGCAGCAGAGCTACTCCATCATGAGCATTATCAAAG
AAGAGGTGCTGGCCTACGTGGTGCAGCTGCCTCTGTATGGCGTGATCGATACCCCTTGCT
GGAAGCTGCACACAAGCCCTCTGTGCACCACCAACACCAAAGAGGGCTCCAACATCTG
CCTGACCAGAACCGATAGAGGCTGGTACTGCGATAATGCCGGCAGCGTCAGCTTCTTCC
CACAAGCCGAGACATGCAAGGTGCAGAGCAACAGAGTGTTCTGCGATACCATGAACAG
CCTGACACTGCCCTCCGAAGTGAATCTGTGCAATATCGACATCTTCAACCCGAAGTACG
ACTGCAAGATCATGACCAGCAAGACCGACGTGTCAAGCTCCGTGATCACATCTCTGGGC
GCCATCGTGTCCTGCTACGGCAAGACAAAGTGCACCGCCAGCAACAAGAACCGGGGCA
TCATCAAGACCTTCAGCAACGGCTGCGACTACGTGTCCAACAAAGGCGTGGACACCGT
GTCTGTGGGCAACACCCTGTACTACGTGAACAAGCAAGAAGGCAAGAGCCTGTACGTG
 
AAGGGCGAGCCCATCATCAACTTCTACGACCCTCTGGTGTTCCCCAGCGACCAGTTTGA
TGCCAGCATCTCCCAAGTGAACGAGAAGATCAACCAGAGCCTGGCCTTCATCAGAAAG
TCCGATGAGCTGCTGAGCGCCATCGGCGGCTATATCCCTGAAGCTCCTAGAGATGGACA
GGCCTATGTGCGGAAGGATGGCGAATGGGTGCTGCTGTCTACCTTCCTGTGATGA
SEQ ID NO:148 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAGAAATCAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCGGATC
CCCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGGTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACCAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO: 149 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAATCAACAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTGCCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGAATTGCCGTGTCCAAGG
TGCTGCATCTGGAAGGCGAAGTGAACAAGATCAAGAGCGCCCTGCTGAGCACCAACAA
 
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCAGCATCAG
CAACCCCGAGACAGTGATCGAGTTCCAGCAGAAGAACAACCGGCTGCTGGAAATCACC
CGCGAGTTTTCTGTGAATGCCGGCGTGACCACACCTGTGTCCACCTACATGCTGACCAA
CAGCGAGCTGCTGTCCCTGATCAACGACATGCCCATCACCAACGACCAGAAAAAGCTG
ATGAGCAGCAACGTGCAGATCGTGCGGCAGCAGAGCTACTCCATCATGAGCATTATCAA
AGAAGAGGTGCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCGACACCTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO: 150 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAATCACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCGGATC
AGCAACCCCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGGTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
 
TGAAGGGCGAGCCCATCATCAACTACTACGACCCTCTGGTGTTCCCCAGCGACACCTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO:151 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAGAAATCAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTGCCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGAATTGCCGTGTCCAAGG
TGCTGCATCTGGAAGGCGAAGTGAACAAGATCAAGAGCGCCCTGCTGAGCACCAACAA
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCAGCATCAG
CAACCCCGAGACAGTGATCGAGTTCCAGCAGAAGAACAACCGGCTGCTGGAAATCACC
CGCGAGTTTTCTGTGAATGCCGGCGTGACCACACCTGTGTCCACCTACATGCTGACCAA
CAGCGAGCTGCTGTCCCTGATCAACGACATGCCCATCACCAACGACCAGAAAAAGCTG
ATGAGCAGCAACGTGCAGATCGTGCGGCAGCAGAGCTACTCCATCATGAGCATTATCAA
AGAAGAGGTGCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCGACCAGTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO: 152 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAGAAATCAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
 
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCGGATC
AGCAACCCCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGGTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACCAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO: 153 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAATCAACAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTGCCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGAATTGCCGTGTCCAAGG
TGCTGCATCTGGAAGGCGAAGTGAACAAGATCAAGAGCGCCCTGCTGAGCACCAACAA
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCAGCATCAG
CAACCCCGAGACAGTGATCGAGTTCCAGCAGAAGAACAACCGGCTGCTGGAAATCACC
CGCGAGTTTTCTGTGAATGCCGGCGTGACCACACCTGTGTCCACCTACATGCTGACCAA
CAGCGAGCTGCTGTCCCTGATCAACGACATGCCCATCACCAACGACCAGAAAAAGCTG
ATGAGCAGCAACGTGCAGATCGTGCGGCAGCAGAGCTACTCCATCATGAGCATTATCAA
AGAAGAGGTGCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
 
GTGAAGGGCGAGCCCATCATCAACTTCTACGACCCTCTGGTGTTCCCCAGCCAGGAGTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO:154 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAATCACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCGGATC
AGCAACCCCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGGTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCCAGGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO: 155 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAATCAACAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTGCCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGAATTGCCGTGTCCAAGG
TGCTGCATCTGGAAGGCGAAGTGAACAAGATCAAGAGCGCCCTGCTGAGCACCAACAA
 
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCCCCATCAGC
AACATCGAGACAGTGATCGAGTTCCAGCAGAAGAACAACCGGCTGCTGGAAATCACCC
GCGAGTTTTCTGTGAATGCCGGCGTGACCACACCTGTGTCCACCTACATGCTGACCAAC
AGCGAGCTGCTGTCCCTGATCAACGACATGCCCATCACCAACGACCAGAAAAAGCTGAT
GAGCAGCAACGTGCAGATCGTGCGGCAGCAGAGCTACTCCATCATGAGCATTATCAAAG
AAGAGGTGCTGGCCTACGTGGTGCAGCTGCCTCTGTATGGCGTGATCGATACCCCTTGCT
GGAAGCTGCACACAAGCCCTCTGTGCACCACCAACACCAAAGAGGGCTCCAACATCTG
CCTGACCAGAACCGATAGAGGCTGGTACTGCGATAATGCCGGCAGCGTCAGCTTCTTCC
CACAAGCCGAGACATGCAAGGTGCAGAGCAACAGAGTGTTCTGCGATACCATGAACAG
CCTGACACTGCCCTCCGAAGTGAATCTGTGCAATATCGACATCTTCAACCCGAAGTACG
ACTGCAAGATCATGACCAGCAAGACCGACGTGTCAAGCTCCGTGATCACATCTCTGGGC
GCCATCGTGTCCTGCTACGGCAAGACAAAGTGCACCGCCAGCAACAAGAACCGGGGCA
TCATCAAGACCTTCAGCAACGGCTGCGACTACGTGTCCAACAAAGGCGTGGACACCGT
GTCTGTGGGCAACACCCTGTACTACGTGAACAAGCAAGAAGGCAAGAGCCTGTACGTG
AAGGGCGAGCCCATCATCAACTTCTACGACCCTCTGGTGTTCCCCAGCGACACCTTTGA
TGCCAGCATCTCCCAAGTGAACGAGAAGATCAACCAGAGCCTGGCCTTCATCAGAAAG
TCCGATGAGCTGCTGCACAACGTGAACGCCGGCAAGTCCACCACCAATATCATGATCAC
CACGATCATCATCGTGATCATCGTCATCCTGCTGGCTCTGATCGCTGTGGGCCTGCTGCT
GTATTGATGA
SEQ ID NO: 156 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAATCACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCCCATCA
GCAACATCGAGACAGTGATCGAGTTCCAGCAGAAGAACAGCAGACTGCTGGAAATCAC
CCGCGAGTTTTCCGTGAATGCCGGCGTGACAACACCCCTGAGCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGTCCTACAGCATCATGAGCATTATCA
AAGAAGAGGTGCTGGCCTACGTGGTGCAGCTGCCTATCTACGGCGTGATCGATACCCCT
TGCTGGAAGCTGCACACAAGCCCTCTGTGCACCACCAATATCAAAGAGGGCTCCAACAT
CTGCCTGACCAGAACCGATAGAGGCTGGTACTGCGATAATGCCGGCAGCGTCAGCTTCT
TCCCACAAGCCGATACCTGCAAGGTGCAGAGCAACAGAGTGTTCTGCGATACCATGAAC
AGCCTGACACTGCCCAGCGAGGTGTCCCTGTGCAACACCGACATCTTCAACTCTAAGTA
CGACTGCAAGATCATGACCTCCAAGACCGACATCAGCTCCTCCGTGATCACATCTCTGG
GCGCCATCGTGTCCTGCTACGGCAAGACAAAGTGTACCGCCAGCAACAAGAACCGGGG
CATCATCAAGACCTTCAGCAACGGCTGCGACTACGTGTCCAACAAAGGCGTGGACACC
 
GTGTCTGTGGGCAACACCCTGTACTACGTGAACAAGCTGGAAGGCAAGAATCTGTACGT
GAAGGGCGAGCCCATCATCAACTACTACGACCCTCTGGTGTTCCCCAGCGACACCTTTG
ATGCCAGCATCAGCCAAGTGAACGAGAAGATCAACCAGAGCCTGGCCTTCATCCGCAG
ATCCGATGAACTGCTGCACAATGTGAACACCGGCAAGAGCACCACAAACATCATGATCA
CCGCCATCATCATCGTGATCATCGTCGTGCTGCTGAGCCTGATCGCTATCGGCCTGCTGCT
GTACTGATGA
SEQ ID NO: 157 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAATCAACAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTGCCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGAATTGCCGTGTCCAAGG
TGCTGCATCTGGAAGGCGAAGTGAACAAGATCAAGAGCGCCCTGCTGAGCACCAACAA
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCCCCATCAGC
AACATCGAGACAGTGATCGAGTTCCAGCAGAAGAACAACCGGCTGCTGGAAATCACCC
GCGAGTTTTCTGTGAATGCCGGCGTGACCACACCTGTGTCCACCTACATGCTGACCAAC
AGCGAGCTGCTGTCCCTGATCAACGACATGCCCATCACCAACGACCAGAAAAAGCTGAT
GAGCAGCAACGTGCAGATCGTGCGGCAGCAGAGCTACTCCATCATGAGCATTATCAAAG
AAGAGGTGCTGGCCTACGTGGTGCAGCTGCCTCTGTATGGCGTGATCGATACCCCTTGCT
GGAAGCTGCACACAAGCCCTCTGTGCACCACCAACACCAAAGAGGGCTCCAACATCTG
CCTGACCAGAACCGATAGAGGCTGGTACTGCGATAATGCCGGCAGCGTCAGCTTCTTCC
CACAAGCCGAGACATGCAAGGTGCAGAGCAACAGAGTGTTCTGCGATACCATGAACAG
CCTGACACTGCCCTCCGAAGTGAATCTGTGCAATATCGACATCTTCAACCCGAAGTACG
ACTGCAAGATCATGACCAGCAAGACCGACGTGTCAAGCTCCGTGATCACATCTCTGGGC
GCCATCGTGTCCTGCTACGGCAAGACAAAGTGCACCGCCAGCAACAAGAACCGGGGCA
TCATCAAGACCTTCAGCAACGGCTGCGACTACGTGTCCAACAAAGGCGTGGACACCGT
GTCTGTGGGCAACACCCTGTACTACGTGAACAAGCAAGAAGGCAAGAGCCTGTACGTG
AAGGGCGAGCCCATCATCAACTTCTACGACCCTCTGGTGTTCCCCAGCCAGGAGTTTGA
TGCCAGCATCTCCCAAGTGAACGAGAAGATCAACCAGAGCCTGGCCTTCATCAGAAAG
TCCGATGAGCTGCTGCACAACGTGAACGCCGGCAAGTCCACCACCAATATCATGATCAC
CACGATCATCATCGTGATCATCGTCATCCTGCTGGCTCTGATCGCTGTGGGCCTGCTGCT
GTATTGATGA
SEQ ID NO:158 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAATCACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
 
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCCCATCA
GCAACATCGAGACAGTGATCGAGTTCCAGCAGAAGAACAGCAGACTGCTGGAAATCAC
CCGCGAGTTTTCCGTGAATGCCGGCGTGACAACACCCCTGAGCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGTCCTACAGCATCATGAGCATTATCA
AAGAAGAGGTGCTGGCCTACGTGGTGCAGCTGCCTATCTACGGCGTGATCGATACCCCT
TGCTGGAAGCTGCACACAAGCCCTCTGTGCACCACCAATATCAAAGAGGGCTCCAACAT
CTGCCTGACCAGAACCGATAGAGGCTGGTACTGCGATAATGCCGGCAGCGTCAGCTTCT
TCCCACAAGCCGATACCTGCAAGGTGCAGAGCAACAGAGTGTTCTGCGATACCATGAAC
AGCCTGACACTGCCCAGCGAGGTGTCCCTGTGCAACACCGACATCTTCAACTCTAAGTA
CGACTGCAAGATCATGACCTCCAAGACCGACATCAGCTCCTCCGTGATCACATCTCTGG
GCGCCATCGTGTCCTGCTACGGCAAGACAAAGTGTACCGCCAGCAACAAGAACCGGGG
CATCATCAAGACCTTCAGCAACGGCTGCGACTACGTGTCCAACAAAGGCGTGGACACC
GTGTCTGTGGGCAACACCCTGTACTACGTGAACAAGCTGGAAGGCAAGAATCTGTACGT
GAAGGGCGAGCCCATCATCAACTACTACGACCCTCTGGTGTTCCCCAGCCAGGAGTTTG
ATGCCAGCATCAGCCAAGTGAACGAGAAGATCAACCAGAGCCTGGCCTTCATCCGCAG
ATCCGATGAACTGCTGCACAATGTGAACACCGGCAAGAGCACCACAAACATCATGATCA
CCGCCATCATCATCGTGATCATCGTCGTGCTGCTGAGCCTGATCGCTATCGGCCTGCTGCT
GTACTGATGA
SEQ ID NO: 159 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAATCAACAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTTGCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGATGCGCCGTGTCCAAGG
TGCTGCATCTGGAAGGCGAAGTGAACAAGATCAAGAGCGCCCTGCTGAGCACCAACAA
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCCCCATCAGC
AACATCGAGACAGTGATCGAGTTCCAGCAGAAGAACAACCGGCTGCTGGAAATCACCC
GCGAGTTTTCTGTGAATGCCGGCGTGACCACACCTGTGTCCACCTACATGCTGACCAAC
AGCGAGCTGCTGTCCCTGATCAACGACATGCCCATCACCAACGACCAGAAAAAGCTGAT
GAGCAGCAACGTGCAGATCGTGCGGCAGCAGAGCTACTCCATCATGAGCATTATCAAAG
AAGAGGTGCTGGCCTACGTGGTGCAGCTGCCTCTGTATGGCGTGATCGATACCCCTTGCT
GGAAGCTGCACACAAGCCCTCTGTGCACCACCAACACCAAAGAGGGCTCCAACATCTG
CCTGACCAGAACCGATAGAGGCTGGTACTGCGATAATGCCGGCAGCGTCAGCTTCTTCC
CACAAGCCGAGACATGCAAGGTGCAGAGCAACAGAGTGTTCTGCGATACCATGAACAG
CCTGACACTGCCCTCCGAAGTGAATCTGTGCAATATCGACATCTTCAACCCGAAGTACG
 
ACTGCAAGATCATGACCAGCAAGACCGACGTGTCAAGCTCCGTGATCACATCTCTGGGC
GCCATCGTGTCCTGCTACGGCAAGACAAAGTGCACCGCCAGCAACAAGAACCGGGGCA
TCATCAAGACCTTCAGCAACGGCTGCGACTACGTGTCCAACAAAGGCGTGGACACCGT
GTCTGTGGGCAACACCCTGTACTACGTGAACAAGCAAGAAGGCAAGAGCCTGTACGTG
AAGGGCGAGCCCATCATCAACTTCTACGACCCTCTGGTGTTCCCCAGCGACGAGTTTGA
TGCCAGCATCTCCCAAGTGAACGAGAAGATCAACCAGAGCCTGGCCTTCATCAGAAAG
TCCGATGAGCTGCTGCACAACGTGAACGCCGGCAAGTCCACCACCAATATCATGATCAC
CACGATCATCATCGTGATCATCGTCATCCTGCTGGCTCTGATCGCTGTGGGCCTGCTGCT
GTATTGATGA
SEQ ID NO:160 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAATCACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTTGCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCCCATCA
GCAACATCGAGACAGTGATCGAGTTCCAGCAGAAGAACAGCAGACTGCTGGAAATCAC
CCGCGAGTTTTCCGTGAATGCCGGCGTGACAACACCCCTGAGCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGTCCTACAGCATCATGAGCATTATCA
AAGAAGAGGTGCTGGCCTACGTGGTGCAGCTGCCTATCTACGGCGTGATCGATACCCCT
TGCTGGAAGCTGCACACAAGCCCTCTGTGCACCACCAATATCAAAGAGGGCTCCAACAT
CTGCCTGACCAGAACCGATAGAGGCTGGTACTGCGATAATGCCGGCAGCGTCAGCTTCT
TCCCACAAGCCGATACCTGCAAGGTGCAGAGCAACAGAGTGTTCTGCGATACCATGAAC
AGCCTGACACTGCCCAGCGAGGTGTCCCTGTGCAACACCGACATCTTCAACTCTAAGTA
CGACTGCAAGATCATGACCTCCAAGACCGACATCAGCTCCTCCGTGATCACATCTCTGG
GCGCCATCGTGTCCTGCTACGGCAAGACAAAGTGTACCGCCAGCAACAAGAACCGGGG
CATCATCAAGACCTTCAGCAACGGCTGCGACTACGTGTCCAACAAAGGCGTGGACACC
GTGTCTGTGGGCAACACCCTGTACTACGTGAACAAGCTGGAAGGCAAGAATCTGTACGT
GAAGGGCGAGCCCATCATCAACTACTACGACCCTCTGGTGTTCCCCAGCGACGAGTTTG
ATGCCAGCATCAGCCAAGTGAACGAGAAGATCAACCAGAGCCTGGCCTTCATCCGCAG
ATCCGATGAACTGCTGCACAATGTGAACACCGGCAAGAGCACCACAAACATCATGATCA
CCGCCATCATCATCGTGATCATCGTCGTGCTGCTGAGCCTGATCGCTATCGGCCTGCTGCT
GTACTGATGA
SEQ ID NO:161 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
 
AGCTGAGCAACATCAAAATCAACAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCTGCGCCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGATGCGCCGTGTCCAAGG
TGCTGCATCTGGAAGGCGAAGTGAACAAGATCAAGAGCGCCCTGCTGAGCACCAACAA
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCCCCATCAGC
AACATCGAGACAGTGATCGAGTTCCAGCAGAAGAACAACCGGCTGCTGGAAATCACCC
GCGAGTTTTCTGTGAATGCCGGCGTGACCACACCTGTGTCCACCTACATGCTGACCAAC
AGCGAGCTGCTGTCCCTGATCAACGACATGCCCATCACCAACGACCAGAAAAAGCTGAT
GAGCAGCAACGTGCAGATCGTGCGGCAGCAGAGCTACTCCATCATGAGCATTATCAAAG
AAGAGGTGCTGGCCTACGTGGTGCAGCTGCCTCTGTATGGCGTGATCGATACCCCTTGCT
GGAAGCTGCACACAAGCCCTCTGTGCACCACCAACACCAAAGAGGGCTCCAACATCTG
CCTGACCAGAACCGATAGAGGCTGGTACTGCGATAATGCCGGCAGCGTCAGCTTCTTCC
CACAAGCCGAGACATGCAAGGTGCAGAGCAACAGAGTGTTCTGCGATACCATGAACAG
CCTGACACTGCCCTCCGAAGTGAATCTGTGCAATATCGACATCTTCAACCCGAAGTACG
ACTGCAAGATCATGACCAGCAAGACCGACGTGTCAAGCTCCGTGATCACATCTCTGGGC
GCCATCGTGTCCTGCTACGGCAAGACAAAGTGCACCGCCAGCAACAAGAACCGGGGCA
TCATCAAGACCTTCAGCAACGGCTGCGACTACGTGTCCAACAAAGGCGTGGACACCGT
GTCTGTGGGCAACACCCTGTACTACGTGAACAAGCAAGAAGGCAAGAGCCTGTACGTG
AAGGGCGAGCCCATCATCAACTTCTACGACCCTCTGGTGTTCCCCAGCGACGAGTTTGA
TGCCAGCATCTCCCAAGTGAACGAGAAGATCAACCAGAGCCTGGCCTTCATCAGAAAG
TCCGATGAGCTGCTGCACAACGTGAACGCCGGCAAGTCCACCACCAATATCATGATCAC
CACGATCATCATCGTGATCATCGTCATCCTGCTGGCTCTGATCGCTGTGGGCCTGCTGCT
GTATTGATGA
SEQ ID NO: 162 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAATCACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCTGCGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCCCATCA
GCAACATCGAGACAGTGATCGAGTTCCAGCAGAAGAACAGCAGACTGCTGGAAATCAC
CCGCGAGTTTTCCGTGAATGCCGGCGTGACAACACCCCTGAGCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGTCCTACAGCATCATGAGCATTATCA
AAGAAGAGGTGCTGGCCTACGTGGTGCAGCTGCCTATCTACGGCGTGATCGATACCCCT
TGCTGGAAGCTGCACACAAGCCCTCTGTGCACCACCAATATCAAAGAGGGCTCCAACAT
 
CTGCCTGACCAGAACCGATAGAGGCTGGTACTGCGATAATGCCGGCAGCGTCAGCTTCT
TCCCACAAGCCGATACCTGCAAGGTGCAGAGCAACAGAGTGTTCTGCGATACCATGAAC
AGCCTGACACTGCCCAGCGAGGTGTCCCTGTGCAACACCGACATCTTCAACTCTAAGTA
CGACTGCAAGATCATGACCTCCAAGACCGACATCAGCTCCTCCGTGATCACATCTCTGG
GCGCCATCGTGTCCTGCTACGGCAAGACAAAGTGTACCGCCAGCAACAAGAACCGGGG
CATCATCAAGACCTTCAGCAACGGCTGCGACTACGTGTCCAACAAAGGCGTGGACACC
GTGTCTGTGGGCAACACCCTGTACTACGTGAACAAGCTGGAAGGCAAGAATCTGTACGT
GAAGGGCGAGCCCATCATCAACTACTACGACCCTCTGGTGTTCCCCAGCGACGAGTTTG
ATGCCAGCATCAGCCAAGTGAACGAGAAGATCAACCAGAGCCTGGCCTTCATCCGCAG
ATCCGATGAACTGCTGCACAATGTGAACACCGGCAAGAGCACCACAAACATCATGATCA
CCGCCATCATCATCGTGATCATCGTCGTGCTGCTGAGCCTGATCGCTATCGGCCTGCTGCT
GTACTGATGA
SEQ ID NO:163 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAATCAACAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTGCCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGAATTGCCGTGTCCAAGG
TGCTGCATCTGGAAGGCGAAGTGAACAAGATCAAGAGCGCCCTGCTGAGCACCAACAA
GGCCGTGGTTTCTCTGAGCAATGGCGTGTCCGTGCTGACCTCTAAGGTGCTGGACCTGA
AGAACTACATCGACAAACAGCTGCTGCCCATCGTCAACAAGCAGAGCTGCCCCATCAGC
AACATCGAGACAGTGATCGAGTTCCAGCAGAAGAACAACCGGCTGCTGGAAATCACCC
GCGAGTTTTCTGTGAATGCCGGCGTGACCACACCTGTGTCCACCTACATGCTGACCAAC
AGCGAGCTGCTGTCCCTGATCAACGACATGCCCATCACCAACGACCAGAAAAAGCTGAT
GAGCAGCAACGTGCAGATCGTGCGGCAGCAGAGCTACTCCATCATGAGCATTATCAAAG
AAGAGGTGCTGGCCTACGTGGTGCAGCTGCCTCTGTATGGCGTGATCGATACCCCTTGCT
GGAAGCTGCACACAAGCCCTCTGTGCACCACCAACACCAAAGAGGGCTCCAACATCTG
CCTGACCAGAACCGATAGAGGCTGGTACTGCGATAATGCCGGCAGCGTCAGCTTCTTCC
CACAAGCCGAGACATGCAAGGTGCAGAGCAACAGAGTGTTCTGCGATACCATGAACAG
CCTGACACTGCCCTCCGAAGTGAATCTGTGCAATATCGACATCTTCAACCCGAAGTACG
ACTGCAAGATCATGACCAGCAAGACCGACGTGTCAAGCTCCGTGATCACATCTCTGGGC
GCCATCGTGTCCTGCTACGGCAAGACAAAGTGCACCGCCAGCAACAAGAACCGGGGCA
TCATCAAGACCTTCAGCAACGGCTGCGACTACGTGTCCAACAAAGGCGTGGACACCGT
GTCTGTGGGCAACACCCTGTACTACGTGAACAAGCAAGAAGGCAAGAGCCTGTACGTG
AAGGGCGAGCCCATCATCAACTTCTACGACCCTCTGGTGTTCCCCAGCGACGAGTTTGA
TGCCAGCATCTCCCAAGTGAACGAGAAGATCAACCAGAGCCTGGCCTTCATCAGAAAG
TCCGATGAGCTGCTGCACAACGTGAACGCCGGCAAGTCCACCACCAATATCATGATCAC
CACGATCATCATCGTGATCATCGTCATCCTGCTGGCTCTGATCGCTGTGGGCCTGCTGCT
GTATTGATGA
SEQ ID NO: 164 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAATCACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCAGCAAGGTGCTGGACCT
GAAGAACTACATCAACAACCAGCTGCTGCCCATCGTGAACCAGCAGAGCTGCCCCATCA
GCAACATCGAGACAGTGATCGAGTTCCAGCAGAAGAACAGCAGACTGCTGGAAATCAC
CCGCGAGTTTTCCGTGAATGCCGGCGTGACAACACCCCTGAGCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGTCCTACAGCATCATGAGCATTATCA
AAGAAGAGGTGCTGGCCTACGTGGTGCAGCTGCCTATCTACGGCGTGATCGATACCCCT
TGCTGGAAGCTGCACACAAGCCCTCTGTGCACCACCAATATCAAAGAGGGCTCCAACAT
CTGCCTGACCAGAACCGATAGAGGCTGGTACTGCGATAATGCCGGCAGCGTCAGCTTCT
TCCCACAAGCCGATACCTGCAAGGTGCAGAGCAACAGAGTGTTCTGCGATACCATGAAC
AGCCTGACACTGCCCAGCGAGGTGTCCCTGTGCAACACCGACATCTTCAACTCTAAGTA
CGACTGCAAGATCATGACCTCCAAGACCGACATCAGCTCCTCCGTGATCACATCTCTGG
GCGCCATCGTGTCCTGCTACGGCAAGACAAAGTGTACCGCCAGCAACAAGAACCGGGG
CATCATCAAGACCTTCAGCAACGGCTGCGACTACGTGTCCAACAAAGGCGTGGACACC
GTGTCTGTGGGCAACACCCTGTACTACGTGAACAAGCTGGAAGGCAAGAATCTGTACGT
GAAGGGCGAGCCCATCATCAACTACTACGACCCTCTGGTGTTCCCCAGCGACGAGTTTG
ATGCCAGCATCAGCCAAGTGAACGAGAAGATCAACCAGAGCCTGGCCTTCATCCGCAG
ATCCGATGAACTGCTGCACAATGTGAACACCGGCAAGAGCACCACAAACATCATGATCA
CCGCCATCATCATCGTGATCATCGTCGTGCTGCTGAGCCTGATCGCTATCGGCCTGCTGCT
GTACTGATGA
SEQ ID NO:165 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATGTGAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATGAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
 
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:166 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATGTGAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTTGCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATGAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
 CTGTACTGATGA
SEQ ID NO:167 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATTTCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATGAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO: 168 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATTTCAGCGTGATCACCATC
GAGCTGAGCAACATCAAAGAAACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTTGCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATGAAGGTGCTGGACCT
GAAGAACTACATCAACAACCAGCTGCTGCCCATCGTGAACCAGCAGAGCTGCCGGATC
 
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:169 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATTGCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATGAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
 
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO: 170 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATTGCAGCGTGATCACCATC
GAGCTGAGCAACATCAAAGAAACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTTGCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATGAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:171 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATGCCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
 
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATGAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO: 172 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATGCCAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTTGCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATGAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
 
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:173 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATGTGAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTTCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO: 174 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATGTGAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
 
CAGAATACCCCTTGCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCTTCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO: 175 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATTTCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTTCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
 
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:176 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATTTCAGCGTGATCACCATC
GAGCTGAGCAACATCAAAGAAACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTTGCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCTTCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:177 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
 
TGTCTAAGGGCTATCTGTCTGCCCTGAGAACCGGCTGGTATtgcAGCGTGATCACCATCGA
GCTGAGCAACATCAAAGAAAACAAGTGCAACGGCACCGACGCCAAAGTGAAGCTGAT
CAAGCAAGAGCTGGACAAGTACAAGAACGCCGTGACCGAACTGCAGCTGCTGATGCAG
TCTACCCCTTGCGCCAATAGCAGAGCCAGACGGGAACTGCCTAGATTCATGAACTACAC
CCTGAACAACACCAAGAACACCAACGTGACCCTGAGCAAGAAGCGGAAGCGGAGATT
CCTGGGCTTTCTGCTCGGAGTGGGAAGCGCCATTGCCTCTGGATGCGCCGTGTCCAAGG
TGCTGCATCTGGAAGGCGAAGTGAACAAGATCAAGAGCGCCCTGCTGAGCACCAACAA
GGCCGTGGTTTCTCTGAGCAATGGCGTGTCCGTGCTGACCTTCAAGGTGCTGGACCTGA
AGAACTACATCGACAAACAGCTGCTGCCCATCGTCAACAAGCAGAGCTGCAGCATCAG
CAACATCGAGACAGTGATCGAGTTCCAGCAGAAGAACAACCGGCTGCTGGAAATCACC
CGCGAGTTTTCTGTGAATGCCGGCGTGACCACACCTGTGTCCACCTACATGCTGACCAA
CAGCGAGCTGCTGTCCCTGATCAACGACATGCCCATCACCAACGACCAGAAAAAGCTG
ATGAGCAGCAACGTGCAGATCGTGCGGCAGCAGAGCTACTCCATCATGAGCATTATCAA
AGAAGAGCTGCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCGACGAGTT
TGATGCCAGCATCTCCCAAGTGAACGAGAAGATCAACCAGAGCCTGGCCTTCATCAGAA
AGTCCGATGAGCTGCTGCACAACGTGAACGCCGGCAAGTCCACCACCAATATCATGATC
ACCACGATCATCATCGTGATCATCGTCATCCTGCTGGCTCTGATCGCTGTGGGCCTGCTG
CTGTATTGATGA
SEQ ID NO: 178 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATTGCAGCGTGATCACCATC
GAGCTGAGCAACATCAAAGAAACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTTGCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCTTCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
 
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:179 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATGCCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTTCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:180 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATGCCAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTTGCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCTTCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:181 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATGTGAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCTGCGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATGAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
 
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO: 182 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATGTGAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCTGCGCCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATGAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
 
ACCGCCATCATCATCGTGATCATCGTCGTGCTGCTGAGCCTGATCGCTATCGGCCTGCTG
CTGTACTGATGA
SEQ ID NO:183 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATTTCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCTGCGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATGAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO: 184 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATTTCAGCGTGATCACCATC
GAGCTGAGCAACATCAAAGAAACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCTGCGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATGAAGGTGCTGGACCT
 
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO: 185 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATTGCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCTGCGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATGAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
 
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:186 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATTGCAGCGTGATCACCATC
GAGCTGAGCAACATCAAAGAAACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCTGCGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATGAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO: 187 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATGCCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCTGCGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
 
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATGAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO: 188 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATGCCAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCTGCGCCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATGAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
 
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:189 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATGTGAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCTGCGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTTCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:190 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATGTGAGCGTGATCACCAT
CGAGCTGAGCAACATCAAAGAAACAAAGTGCAACGGCACCGACACCAAAGTGAAGCT
 
GATCAAGCAAGAGCTGGACAAGTACAAGAACGCCGTGACCGAACTGCAGCTGCTGATG
CAGAATACCTGCGCCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCTTCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCΛΛCΛCCCTGTΛCTΛCGTGΛΛCΛΛGCTGGΛΛGGCΛΛGΛΛTCTGTΛCG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ΛCCGCCΛTCΛTCΛTCGTGΛTCΛTCGTCGTGCTGCTGΛGCCTGΛTCGCTΛTCGGCCTGCTG
CTGTACTGATGA
SEQ ID NO:191 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATTTCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCTGCGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTTCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
 
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:192 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATTTCAGCGTGATCACCATC
GAGCTGAGCAACATCAAAGAAACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCTGCGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCTTCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO: 193 ATGGAACTGCCCATCCTGAAAACAAACGCCATCACCACCATCCTGGCCGCCGTGACACT 
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATTGCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCTGCGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTTCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:194 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATTGCAGCGTGATCACCATC
GAGCTGAGCAACATCAAAGAAACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCTGCGCCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCTTCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
 
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:195 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATGCCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCTGCGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTTCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:196 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATGCCAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCTGCGCCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCTTCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO: 197 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATGTGAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
 
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:198 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATGTGAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTTGCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
 
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:199 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATTTCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:200 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATTTCAGCGTGATCACCATC
GAGCTGAGCAACATCAAAGAAACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTTGCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
 
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:201 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATTGCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
 
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:202 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATTGCAGCGTGATCACCATC
GAGCTGAGCAACATCAAAGAAACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTTGCGCCAACAACCGGGCCAGAAGAGAAGCCCCTCAGTACATGAACTA
CACCATCAACACCACCAAGAACCTGAACGTGTCCATCAGCAAGAAGCGGAAGCGGAGA
TTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:203 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATGCCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
 
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:204 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATGCCAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTTGCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGCTGCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
 
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCGACGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:205 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCTGCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCTGCGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGAGCGCCATCGGCGGCTATATCCCTGAAGCTCCTAGAGATGGA
CAGGCCTATGTGCGGAAGGATGGCGAATGGGTGCTGCTGTCTACCTTCCTGTGATGA
SEQ ID NO:206 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
 
GATCAAGCAAGAGCTGGACAAGTACAAGAACGCCGTGACCGAACTGCAGCTGCTGATG
CAGAATACCCCTGCCTGCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCTGCGCCTCTGGAATTGCCGTGTCTA
AGGTGCTGCACCTGGAAGGCGAAGTGAACAAGATCAAGAATGCCCTGCTGAGCACCAA
CAAGGCCGTGGTGTCTCTGTCTAATGGCGTGTCCGTGCTGACCATCAAGGTGCTGGACC
TGAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGTCTGCCATCGGCGGCTATATCCCCGAAGCTCCTAGAGATGGAC
AGGCCTATGTGCGGAAGGATGGCGAATGGGTGCTGCTGTCTACCTTCCTGTGATGA
SEQ ID NO:207 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCTGCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCTGCGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
 
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGCACAACGTGAACGCCGGCAAGTCCACCACCAATATCATGATC
ACCACGATCATCATCGTGATCATCGTCATCCTGCTGGCTCTGATCGCTGTGGGCCTGCTG
CTGTATTGATGA
SEQ ID NO:208 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCTGCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCTGCGCCTCTGGAATTGCCGTGTCTA
AGGTGCTGCACCTGGAAGGCGAAGTGAACAAGATCAAGAATGCCCTGCTGAGCACCAA
CAAGGCCGTGGTGTCTCTGTCTAATGGCGTGTCCGTGCTGACCATCAAGGTGCTGGACC
TGAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:209 
TAATACGACTCACTATAGGACAGATCGCCTGGAGACGCCATCCACGCTGTTTTGACCTCC
ATAGAAGACACCGGGACCGATCCAGCCTCCGCGGCCGGGAACGGTGCATTGGAACGCG
 GATTCCCCGTGCCAAGAGTGACGTAAGTACCGCAGTCACCGTCCTTGACACG
SEQ ID NO:210 
TCTTCACCCAGTCTGTCTCTTAGAATTTATTTCCCACTGACACAAACAAGCCATTTTGTCT
GCTTGTATAACCC
SEQ ID NO:211 
AAAAAAAAAAAAGAAAAAAAAAAAAAAAAAAGAAAGAAAAAAAAAAAAAAAAAAA
AAAAAAAAAAAAAAAAAAAAAAAAAAAAAAAAAAAAAAAAAAAAAAAAAAAAAA
SEQ ID NO:216 
MELPILKTNAITTILAAVTLCFASSQNITEEFYQSTCSAVSKGYLSALRTGWYTSVITIELSNIK
ENKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLYCKARSTPVTLSKDQLS
GINNIAFSN
SEQ ID NO:217 
MELLIHRSSAIFLTLAINALYLTSSQNITEEFYQSTCSAVSRGYFSALRTGWYTSVITIELSNIK
ETKCNGTDTKVKLIKQELDKYKNAVTELQLLMQNTPAANNRARREAPQYMNYTINTTKNL
NVSISKKRKRRFLGFLLGVGSAIASGIAVSKVLHLEGEVNKIKNALLSTNKAVVSLSNGVSV
LTSKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEVLAYVVQLPIYGVIDTPCW
KLHTSPLCTTNIKEGSNICLTRTDRGWYCDNAGSVSFFPQADTCKVQSNRVFCDTMNSLTLP
SEVSLCNTDIFNSKYDCKIMTSKTDISSSVITSLGAIVSCYGKTKCTASNKNRGIIKTFSNGCD
YVSNKGVDTVSVGNTLYYVNKLEGKNLYVKGEPIINYYDPLVFPSDEFDASISQVNEKINQS
LAFIRRSDELLHNVNTGKSTTNIMITAIIIVIIVVLLSLIAIGLLLYCKAKNTPVTLSKDQLSGIN
NIAFSK
SEQ ID NO:218 
MELLILKANAITTILTAVTFCFASGQNITEEFYQSTCSAVSKGYLSALRTGWYTSVITIELSNIK
ENKCNGTDAKVKLIKQELDKYKNAVTELQLLMQSTPATGSGSAICSGVAVCKVLHLEGEVN
KIKSALLSTNKAVVSLSNGVSVLTFKVLDLKNYIDKQLLPILNKQSCSISNIETVIEFQQKNN
RLLEITREFSVNAGVTTPVSTYMLTNSELLSLINDMPITNDQKKLMSNNVQIVRQQSYSIMCI
IKEEVLAYVVQLPLYGVIDTPCWKLHTSPLCTTNTKEGSNICLTRTDRGWYCDNAGSVSFFP
QAETCKVQSNRVFCDTMNSRTLPSEVNLCNVDIFNPKYDCKIMTSKTDVSSSVITSLGAIVS
CYGKTKCTASNKNRGIIKTFSNGCDYVSNKGVDTVSVGNTLYCVNKQEGKSLYVKGEPIIN
FYDPLVFPSDEFDASISQVNEKINQSLAFIRKSDELLHNVNAGKSTTNIMITTIIIVIIVILLSLIA
VGLLLY
SEQ ID NO:219 
MELPILKTNAITTILAAVTLCFASSQNITEEFYQSTCSAVSKGYLSALRTGWYTSVITIELSNIK
ENKCNGTDAKVKLIKQELDKYKNAVTELQLLMQSTPACNSRARRELPRFMNYTLNNTKNT
NVTLSKKRKRRFLGFLLGVGSACASGIAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLHNVNAGKSTTNIMITTIIIVIIVILLALIAVGLLLY
SEQ ID NO:220 
MELLIHRSSAIFLTLAINALYLTSSQNITEEFYQSTCSAVSRGYFSALRTGWYTSVITIELSNIK
ETKCNGTDTKVKLIKQELDKYKNAVTELQLLMQNTPACNNRARREAPQYMNYTINTTKNL
NVSISKKRKRRFLGFLLGVGSACASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLHNVNTGKSTTNIMITAIIIVIIVVLLSLIAIGLLLY
SEQ ID NO:221 
ATGGAGCTGCTGATCCTGAAGGCCAACGCCATCACGACCATCCTGACCGCCGTGACCTT
CTGCTTCGCCAGCGGGCAGAACATCACCGAGGAGTTCTACCAGTCCACCTGCTCCGCCG
TGAGCAAGGGCTACCTGTCTGCCCTGAGAACCGGCTGGTACACCAGCGTGATCACCATC
GAGCTGTCCAACATCAAGGAGAACAAGTGCAACGGCACCGACGCCAAGGTGAAGCTG
ATCAAGCAGGAGCTGGACAAGTACAAGAACGCAGTGACCGAGCTGCAGCTGCTGATGC
AGAGCACACCAGCCACCGGTAGCGGGTCCGCCATTTGCTCCGGCGTGGCCGTGTGCAA
GGTGCTGCACCTGGAGGGCGAGGTGAACAAGATCAAGAGCGCCCTGCTCTCCACCAAC
AAGGCCGTGGTGAGCCTGAGCAACGGGGTGAGCGTGCTGACCTTCAAGGTGCTGGACC
TGAAGAACTACATCGACAAGCAGCTGCTGCCTATCCTGAACAAGCAGAGCTGCAGCATC
AGCAACATCGAGACAGTGATCGAGTTCCAGCAGAAGAACAACCGGCTGCTGGAGATCA
CCAGGGAGTTCAGCGTGAACGCAGGGGTGACCACACCCGTGTCCACCTACATGCTGAC
CAACTCCGAGCTGCTGAGCCTGATCAACGATATGCCCATCACCAACGACCAGAAGAAGC
TGATGAGCAACAACGTGCAGATCGTGCGGCAGCAGTCCTACTCCATCATGTGCATCATC
AAGGAGGAGGTGCTGGCCTACGTGGTGCAGCTGCCCCTGTACGGCGTGATCGACACCC
CTTGCTGGAAGCTGCACACCAGCCCTCTGTGCACCACCAACACGAAGGAGGGCAGCAA
TATCTGCCTGACCCGGACCGACAGGGGCTGGTACTGCGACAACGCCGGCAGCGTGTCCT
TCTTTCCCCAGGCCGAGACATGCAAGGTGCAGTCCAACAGGGTGTTCTGCGACACCATG
AACTCTCGCACCCTGCCCAGCGAGGTGAACCTGTGCAACGTGGACATCTTCAACCCCA
AGTACGACTGCAAGATCATGACCTCCAAGACCGACGTGTCCTCTAGCGTTATCACCTCCC
TGGGCGCCATCGTGAGCTGCTACGGCAAGACCAAGTGCACCGCCAGCAACAAGAACAG
GGGCATCATCAAGACCTTCAGCAACGGGTGCGACTACGTGTCCAACAAGGGCGTGGAC
ACCGTGTCCGTGGGCAACACCCTGTACTGCGTGAACAAGCAGGAGGGCAAGAGCCTGT
 
ACGTGAAGGGCGAGCCCATCATCAACTTCTACGACCCTCTGGTGTTCCCCAGCGACGAG
TTCGACGCCAGCATCTCCCAGGTGAACGAGAAGATCAACCAGAGCCTGGCCTTCATCCG
CAAGAGCGACGAGCTGCTGCACAACGTGAACGCCGGCAAGAGCACCACAAACATCATG
ATCACCACCATCATCATCGTGATAATCGTGATCCTGCTGTCCCTGATCGCTGTGGGCCTGC
TGCTGTACTGA
SEQ ID NO:222 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCTGCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCTGCGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGCACAACGTGAACGCCGGCAAGTCCACCACCAATATCATGATC
ACCACGATCATCATCGTGATCATCGTCATCCTGCTGGCTCTGATCGCTGTGGGCCTGCTG
CTGTATTGA
SEQ ID NO:223 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATACCAGCGTGATCACCATC
GAGCTGAGCAACATCAAAGAAACAAAGTGCAACGGCACCGACACCAAAGTGAAGCTG
ATCAAGCAAGAGCTGGACAAGTACAAGAACGCCGTGACCGAACTGCAGCTGCTGATGC
AGAATACCCCTGCCTGCAACAACCGGGCCAGAAGAGAAGCCCCTCAGTACATGAACTA
CACCATCAACACCACCAAGAACCTGAACGTGTCCATCAGCAAGAAGCGGAAGCGGAGA
 
TTCCTGGGCTTTCTGCTCGGAGTGGGAAGCGCCTGCGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGA
SEQ ID NO:224 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPCANSRARRELPRFMNYTLNNTKNT
NVTLSKKRKRRFLGFLLGVGSACASGIAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLHNVNAGKSTTNIMITTIIIVIIVILLALIAVGLLLY
SEQ ID NO:225 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPCANNRARREAPQYMNYTINTTKNL
NVSISKKRKRRFLGFLLGVGSACASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLHNVNTGKSTTNIMITAIIIVIIVVLLSLIAIGLLLY
SEQ ID NO:226 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLHNVNAGKSTTNIMITTIIIVIIVILLALIAVGLLLY
SEQ ID NO:227 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPCANNRARREAPQYMNYTINTTKNL
NVSISKKRKRRFLGFLLGVGSAIASGCAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLHNVNTGKSTTNIMITAIIIVIIVVLLSLIAIGLLLY
SEQ ID NO:228 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPAACSRARRELPRFMNYTLNNTKNT
NVTLSKKRKRRFLGFLLGVCSAIASGIAVSKVLHLEGEVNKIKSALLSTNKAVVSLSNGVSV
LTIKVLDLKNYIDKQLLPIVNKQSCSISNIETVIEFQQKNNRLLEITREFSVNAGVTTPVSTYM
LTNSELLSLINDMPITNDQKKLMSSNVQIVRQQSYSIMSIIKEEILAYVVQLPLYGVIDTPCW
KLHTSPLCTTNTKEGSNICLTRTDRGWYCDNAGSVSFFPQAETCKVQSNRVFCDTMNSLTL
PSEVNLCNIDIFNPKYDCKIMTSKTDVSSSVITSLGAIVSCYGKTKCTASNKNRGIIKTFSNGC
DYVSNKGVDTVSVGNTLYYVNKQEGKSLYVKGEPIINFYDPLVFPSSEFDASISQVNEKINQ
SLAFIRKSDELLHNVNAGKSTTNIMITTIIIVIIVILLALIAVGLLLY
SEQ ID NO:229 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPAACNRARREAPQYMNYTINTTKNL
NVSISKKRKRRFLGFLLGVCSAIASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLHNVNTGKSTTNIMITAIIIVIIVVLLSLIAIGLLLY
SEQ ID NO:230 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPAACSRARRELPRFMNYTLNNTKNT
NVTLSKKRKRRFLGFLLGVGCAIASGIAVSKVLHLEGEVNKIKSALLSTNKAVVSLSNGVSV
LTIKVLDLKNYIDKQLLPIVNKQSCSISNIETVIEFQQKNNRLLEITREFSVNAGVTTPVSTYM
LTNSELLSLINDMPITNDQKKLMSSNVQIVRQQSYSIMSIIKEEILAYVVQLPLYGVIDTPCW
KLHTSPLCTTNTKEGSNICLTRTDRGWYCDNAGSVSFFPQAETCKVQSNRVFCDTMNSLTL
PSEVNLCNIDIFNPKYDCKIMTSKTDVSSSVITSLGAIVSCYGKTKCTASNKNRGIIKTFSNGC
DYVSNKGVDTVSVGNTLYYVNKQEGKSLYVKGEPIINFYDPLVFPSSEFDASISQVNEKINQ
SLAFIRKSDELLHNVNAGKSTTNIMITTIIIVIIVILLALIAVGLLLY
SEQ ID NO:231 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPAACNRARREAPQYMNYTINTTKNL
NVSISKKRKRRFLGFLLGVGCAIASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLHNVNTGKSTTNIMITAIIIVIIVVLLSLIAIGLLLY
SEQ ID NO:232 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPAANCRARRELPRFMNYTLNNTKN
TNVTLSKKRKRRFLGFLLGVGSCIASGIAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLHNVNAGKSTTNIMITTIIIVIIVILLALIAVGLLLY
SEQ ID NO:233 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPAANCRARREAPQYMNYTINTTKNL
NVSISKKRKRRFLGFLLGVGSCIASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLHNVNTGKSTTNIMITAIIIVIIVVLLSLIAIGLLLY
SEQ ID NO:234 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPAANCRARRELPRFMNYTLNNTKN
TNVTLSKKRKRRFLGFLLGVCSAIASGIAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLHNVNAGKSTTNIMITTIIIVIIVILLALIAVGLLLY
SEQ ID NO:235 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPAANCRARREAPQYMNYTINTTKNL
NVSISKKRKRRFLGFLLGVCSAIASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLHNVNTGKSTTNIMITAIIIVIIVVLLSLIAIGLLLY
SEQ ID NO:236 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPAANCRARRELPRFMNYTLNNTKN
TNVTLSKKRKRRFLGFLLGVGCAIASGIAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLHNVNAGKSTTNIMITTIIIVIIVILLALIAVGLLLY
SEQ ID NO:237 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPAANCRARREAPQYMNYTINTTKNL
NVSISKKRKRRFLGFLLGVGCAIASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLHNVNTGKSTTNIMITAIIIVIIVVLLSLIAIGLLLY
SEQ ID NO:238 MELPILKTNAITTILAAVTLCFASSQNITEEFYQSTCSAVSKGYLSALRTGWYHSVITIELSNIK 
ENKCNGTDAKVKLIKQELDKYKNAVTELQLLMQSTCAANSRARRELPRFMNYTLNNTKN
TNVTLSKKRKRRFLGFLLGVGSAIASGCAVSKVLHLEGEVNKIKSALLSTNKAVVSLSNGVS
VLTIKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEILAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSSEFDASISQVNEKIN
QSLAFIRKSDELLHNVNAGKSTTNIMITTIIIVIIVILLALIAVGLLLY
SEQ ID NO:239 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTCAANNRARREAPQYMNYTINTTKN
LNVSISKKRKRRFLGFLLGVGSAIASGCAVSKVLHLEGEVNKIKNALLSTNKAVVSLSNGVS
VLTIKVLDLKNYINNQLLPIVNQQSCRISNIETVIEFQQKNSRLLEITREFSVNAGVTTPLSTY
MLTNSELLSLINDMPITNDQKKLMSSNVQIVRQQSYSIMSIIKEEILAYVVQLPIYGVIDTPC
WKLHTSPLCTTNIKEGSNICLTRTDRGWYCDNAGSVSFFPQADTCKVQSNRVFCDTMNSLT
LPSEVSLCNTDIFNSKYDCKIMTSKTDISSSVITSLGAIVSCYGKTKCTASNKNRGIIKTFSNG
CDYVSNKGVDTVSVGNTLYYVNKLEGKNLYVKGEPIINYYDPLVFPSSEFDASISQVNEKIN
QSLAFIRRSDELLHNVNTGKSTTNIMITAIIIVIIVVLLSLIAIGLLLY
SEQ ID NO:240 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPACNNRARREAPQYMNYTINTTKNL
NVSISKKRKRRFLGFLLGVGCAIASGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLHNVNTGKSTTNIMITAIIIVIIVVLLSLIAIGLLLY
SEQ ID NO:241 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPACNSRARRELPRFMNYTLNNTKNT
NVTLSKKRKRRFLGFLLGVGCAIASGIAVSKVLHLEGEVNKIKSALLSTNKAVVSLSNGVSV
LTIKVLDLKNYIDKQLLPIVNKQSCSISNIETVIEFQQKNNRLLEITREFSVNAGVTTPVSTYM
LTNSELLSLINDMPITNDQKKLMSSNVQIVRQQSYSIMSIIKEEILAYVVQLPLYGVIDTPCW
KLHTSPLCTTNTKEGSNICLTRTDRGWYCDNAGSVSFFPQAETCKVQSNRVFCDTMNSLTL
PSEVNLCNIDIFNPKYDCKIMTSKTDVSSSVITSLGAIVSCYGKTKCTASNKNRGIIKTFSNGC
DYVSNKGVDTVSVGNTLYYVNKQEGKSLYVKGEPIINFYDPLVFPSSEFDASISQVNEKINQ
SLAFIRKSDELLHNVNAGKSTTNIMITTIIIVIIVILLALIAVGLLLY
SEQ ID NO:242 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPCANNRARREAPQYMNYTINTTKNL
 
NVSISKKRKRRFLGFLLGVGSAIASGICVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLHNVNTGKSTTNIMITAIIIVIIVVLLSLIAIGLLLY
SEQ ID NO:243 
MELLIHRSSAIFLTLAINALYLTSSQNITEEFYQSTCSAVSRGYFSALRTGWYHSVITIELSNIK
ETKCNGTDTKVKLIKQELDKYKNAVTELQLLMQNTPCANNRARREAPQYMNYTINTTKNL
NVSISKKRKRRFLGFLLGVGSAIACGIAVSKVLHLEGEVNKIKNALLSTNKAVVSLSNGVSV
LTIKVLDLKNYINNQLLPIVNQQSCRISNIETVIEFQQKNSRLLEITREFSVNAGVTTPLSTYM
LTNSELLSLINDMPITNDQKKLMSSNVQIVRQQSYSIMSIIKEEILAYVVQLPIYGVIDTPCWK
LHTSPLCTTNIKEGSNICLTRTDRGWYCDNAGSVSFFPQADTCKVQSNRVFCDTMNSLTLPS
EVSLCNTDIFNSKYDCKIMTSKTDISSSVITSLGAIVSCYGKTKCTASNKNRGIIKTFSNGCDY
VSNKGVDTVSVGNTLYYVNKLEGKNLYVKGEPIINYYDPLVFPSSEFDASISQVNEKINQSL
AFIRRSDELLHNVNTGKSTTNIMITAIIIVIIVVLLSLIAIGLLLY
SEQ ID NO:244 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGICVSKVLHLEGEVNKIKSALLSTNKAVVSLSNGVSV
LTIKVLDLKNYIDKQLLPIVNKQSCSISNIETVIEFQQKNNRLLEITREFSVNAGVTTPVSTYM
LTNSELLSLINDMPITNDQKKLMSSNVQIVRQQSYSIMSIIKEEILAYVVQLPLYGVIDTPCW
KLHTSPLCTTNTKEGSNICLTRTDRGWYCDNAGSVSFFPQAETCKVQSNRVFCDTMNSLTL
PSEVNLCNIDIFNPKYDCKIMTSKTDVSSSVITSLGAIVSCYGKTKCTASNKNRGIIKTFSNGC
DYVSNKGVDTVSVGNTLYYVNKQEGKSLYVKGEPIINFYDPLVFPSSEFDASISQVNEKINQ
SLAFIRKSDELLHNVNAGKSTTNIMITTIIIVIIVILLALIAVGLLLY
SEQ ID NO:245 
MELPILKTNAITTILAAVTLCFASSQNITEEFYQSTCSAVSKGYLSALRTGWYHSVITIELSNIK
ENKCNGTDAKVKLIKQELDKYKNAVTELQLLMQSTPCANSRARRELPRFMNYTLNNTKNT
NVTLSKKRKRRFLGFLLGVGSAIACGIAVSKVLHLEGEVNKIKSALLSTNKAVVSLSNGVSV
LTIKVLDLKNYIDKQLLPIVNKQSCSISNIETVIEFQQKNNRLLEITREFSVNAGVTTPVSTYM
LTNSELLSLINDMPITNDQKKLMSSNVQIVRQQSYSIMSIIKEEILAYVVQLPLYGVIDTPCW
KLHTSPLCTTNTKEGSNICLTRTDRGWYCDNAGSVSFFPQAETCKVQSNRVFCDTMNSLTL
PSEVNLCNIDIFNPKYDCKIMTSKTDVSSSVITSLGAIVSCYGKTKCTASNKNRGIIKTFSNGC
DYVSNKGVDTVSVGNTLYYVNKQEGKSLYVKGEPIINFYDPLVFPSSEFDASISQVNEKINQ
SLAFIRKSDELLHNVNAGKSTTNIMITTIIIVIIVILLALIAVGLLLY
SEQ ID NO:246 
MELPILKTNAITTILAAVTLCFASSQNITEEFYQSTCSAVSKGYLSALRTGWYASVITIELSNIK
ENKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
 
LTSKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:247 
MELPILKTNAITTILAAVTLCFASSQNITEEFYQSTCSAVSKGYLSALRTGWYTSVITIELSNIK
ENKCNGTDAKVKLIKQELDKYKNAVTELQLLMQSTCAANSRARRELPRFMNYTLNNTKN
TNVTLSKKRKRRFLGFLLGVGSAIASGCAVSKVLHLEGEVNKIKSALLSTNKAVVSLSNGVS
VLTSKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:248 
MELPILKTNAITTILAAVTLCFASSQNITEEFYQSTCSAVSKGYLSALRTGWYTSVITIELSNIK
ENKCNGTDAKVKLIKQELDKYKNAVTELQLLMQSTPACNSRARRELPRFMNYTLNNTKNT
NVTLSKKRKRRFLGFLLGVGSACASGIAVSKVLHLEGEVNKIKSALLSTNKAVVSLSNGVS
VLTSKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:249 
MELPILKTNAITTILAAVTLCFASSQNITEEFYQSTCSAVSKGYLSALRTGWYTSVITIELSNIK
ENKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTMKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:250 
MELPILKTNAITTILAAVTLCFASSQNITEEFYQSTCSAVSKGYLSALRTGWYTSVITIELSNIK
ENKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTIKVLDLKNYIDKQLLPIVNKQSCSISNIETVIEFQQKNNRLLEITREFSVNAGVTTPVSTYM
 
LTNSELLSLINDMPITNDQKKLMSSNVQIVRQQSYSIMSIIKEEVLAYVVQLPLYGVIDTPCW
KLHTSPLCTTNTKEGSNICLTRTDRGWYCDNAGSVSFFPQAETCKVQSNRVFCDTMNSLTL
PSEVNLCNIDIFNPKYDCKIMTSKTDVSSSVITSLGAIVSCYGKTKCTASNKNRGIIKTFSNGC
DYVSNKGVDTVSVGNTLYYVNKQEGKSLYVKGEPIINFYDPLVFPSDEFDASISQVNEKINQ
SLAFIRKSDELLHNVNAGKSTTNIMITTIIIVIIVILLALIAVGLLLY
SEQ ID NO:251 
MELPILKTNAITTILAAVTLCFASSQNITEEFYQSTCSAVSKGYLSALRTGWYTSVITIELSNIK
ENKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:252 
MELPILKTNAITTILAAVTLCFASSQNITEEFYQSTCSAVSKGYLSALRTGWYASVITIELSNIK
ENKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTMKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:253 
MELPILKTNAITTILAAVTLCFASSQNITEEFYQSTCSAVSKGYLSALRTGWYASVITIELSNIK
ENKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTSKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:254 
MELPILKTNAITTILAAVTLCFASSQNITEEFYQSTCSAVSKGYLSALRTGWYTSVITIELSNIK
ENKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTMKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
 
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:255 
MELPILKTNAITTILAAVTLCFASSQNITEEFYQSTCSAVSKGYLSALRTGWYASVITIELSNIK
ENKCNGTDAKVKLIKQELDKYKNAVTELQLLMQSTPAANSRARRELPRFMNYTLNNTKNT
NVTLSKKRKRRFLGFLLGVGSAIASGIAVSKVLHLEGEVNKIKSALLSTNKAVVSLSNGVSV
LTMKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEELLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:256 
MELPILKTNAITTILAAVTLCFASSQNITEEFYQSTCSAVSKGYLSALRTGWYTSVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGCAVSKVLHLEGEVNKIKSALLSTNKAVVSLSNGVS
VLTSKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:257 
MELPILKTNAITTILAAVTLCFASSQNITEEFYQSTCSAVSKGYLSALRTGWYTSVITIELSNIK
ENKCNGTDAKVKLIKQELDKYKNAVTELQLLMQSTPCANSRARRELPRFMNYTLNNTKNT
NVTLSKKRKRRFLGFLLGVGSAIASGICVSKVLHLEGEVNKIKSALLSTNKAVVSLSNGVSV
LTSKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:258 
MELPILKTNAITTILAAVTLCFASSQNITEEFYQSTCSAVSKGYLSALRTGWYTSVITIELSNIK
ENKCNGTDAKVKLIKQELDKYKNAVTELQLLMQSTPCANSRARRELPRFMNYTLNNTKNT
NVTLSKKRKRRFLGFLLGVGSAIACGIAVSKVLHLEGEVNKIKSALLSTNKAVVSLSNGVSV
LTSKVLDLKNYIDKQLLPIVNKQSCSISNIETVIEFQQKNNRLLEITREFSVNAGVTTPVSTY
MLTNSELLSLINDMPITNDQKKLMSSNVQIVRQQSYSIMSIIKEEVLAYVVQLPLYGVIDTPC
WKLHTSPLCTTNTKEGSNICLTRTDRGWYCDNAGSVSFFPQAETCKVQSNRVFCDTMNSLT
 
LPSEVNLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLY
SEQ ID NO:259 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCTGCGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGCACAACGTGAACGCCGGCAAGTCCACCACCAATATCATGATC
ACCACGATCATCATCGTGATCATCGTCATCCTGCTGGCTCTGATCGCTGTGGGCCTGCTG
CTGTATTGATGA
SEQ ID NO:260 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTTGCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCTGCGCCTCTGGAATTGCCGTGTCTA
AGGTGCTGCACCTGGAAGGCGAAGTGAACAAGATCAAGAATGCCCTGCTGAGCACCAA
 
CAAGGCCGTGGTGTCTCTGTCTAATGGCGTGTCCGTGCTGACCATCAAGGTGCTGGACC
TGAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:261 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
 
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGCACAACGTGAACGCCGGCAAGTCCACCACCAATATCATGATC
ACCACGATCATCATCGTGATCATCGTCATCCTGCTGGCTCTGATCGCTGTGGGCCTGCTG
CTGTATTGATGA
SEQ ID NO:262 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTTGCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:263 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCTGCAGCAGAGCCAGACGGGAACTGCCTAGATTCATGAACTACA
 
CCCTGAACAACACCAAGAACACCAACGTGACCCTGAGCAAGAAGCGGAAGCGGAGAT
TCCTGGGCTTTCTGCTCGGAGTGTGCAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGCACAACGTGAACGCCGGCAAGTCCACCACCAATATCATGATC
ACCACGATCATCATCGTGATCATCGTCATCCTGCTGGCTCTGATCGCTGTGGGCCTGCTG
CTGTATTGATGA
SEQ ID NO:264 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCGCCTGCAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGTGCAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
 
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:265 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCTGCAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGATGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGCACAACGTGAACGCCGGCAAGTCCACCACCAATATCATGATC
ACCACGATCATCATCGTGATCATCGTCATCCTGCTGGCTCTGATCGCTGTGGGCCTGCTG
CTGTATTGATGA
SEQ ID NO:266 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
 
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCGCCTGCAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGATGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:267 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATTGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCTGCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
 
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGCACAACGTGAACGCCGGCAAGTCCACCACCAATATCATGATC
ACCACGATCATCATCGTGATCATCGTCATCCTGCTGGCTCTGATCGCTGTGGGCCTGCTG
CTGTATTGATGA
SEQ ID NO:268 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCGCCAACTGCCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCTGCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:269 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATTGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGTGCAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGCACAACGTGAACGCCGGCAAGTCCACCACCAATATCATGATC
ACCACGATCATCATCGTGATCATCGTCATCCTGCTGGCTCTGATCGCTGTGGGCCTGCTG
CTGTATTGATGA
SEQ ID NO:270 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCGCCAACTGCCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGTGCAGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
 
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:271 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATTGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGATGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGCACAACGTGAACGCCGGCAAGTCCACCACCAATATCATGATC
ACCACGATCATCATCGTGATCATCGTCATCCTGCTGGCTCTGATCGCTGTGGGCCTGCTG
 CTGTATTGATGA
SEQ ID NO:272 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCGCCAACTGCCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGATGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:273 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCTGCGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
 
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGCACAACGTGAACGCCGGCAAGTCCACCACCAATATCATGATC
ACCACGATCATCATCGTGATCATCGTCATCCTGCTGGCTCTGATCGCTGTGGGCCTGCTG
CTGTATTGATGA
SEQ ID NO:274 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCTGCGCCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGATGCGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
 
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:275 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTGCCTGCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGATGCGCCATTGCCTCTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:276 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCTGCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGATGCGCCATTGCCTCTGGAATTGCCGTGTCCAAG
 
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGCACAACGTGAACGCCGGCAAGTCCACCACCAATATCATGATC
ACCACGATCATCATCGTGATCATCGTCATCCTGCTGGCTCTGATCGCTGTGGGCCTGCTG
CTGTATTGATGA
SEQ ID NO:277 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTTGCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTCTGGAATTTGTGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
 
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:278 
ATGGAACTGCTGATTCACAGAAGCAGCGCCATCTTTCTGACCCTGGCCATCAATGCCCT
GTACCTGACCAGCAGCCAGAACATCACCGAAGAGTTCTACCAGAGCACCTGTAGCGCC
GTGTCCAGAGGCTACTTTTCTGCCCTGAGAACCGGCTGGTATCACAGCGTGATCACCAT
CGAGCTGAGCAACATCAAAGAAACAAAGTGCAACGGCACCGACACCAAAGTGAAGCT
GATCAAGCAAGAGCTGGACAAGTACAAGAACGCCGTGACCGAACTGCAGCTGCTGATG
CAGAATACCCCTTGCGCCAACAACCGGGCCAGAAGAGAAGCCCCTCAGTACATGAACT
ACACCATCAACACCACCAAGAACCTGAACGTGTCCATCAGCAAGAAGCGGAAGCGGAG
ATTCCTGGGCTTTCTGCTCGGAGTGGGAAGCGCCATTGCCTGTGGAATTGCCGTGTCTAA
GGTGCTGCACCTGGAAGGCGAAGTGAACAAGATCAAGAATGCCCTGCTGAGCACCAAC
AAGGCCGTGGTGTCTCTGTCTAATGGCGTGTCCGTGCTGACCATCAAGGTGCTGGACCT
GAAGAACTACATCAACAACCAGCTGCTGCCCATCGTGAACCAGCAGAGCTGCCGGATC
AGCAACATCGAGACAGTGATCGAGTTCCAGCAGAAGAACAGCAGACTGCTGGAAATCA
CCCGCGAGTTTTCCGTGAATGCCGGCGTGACAACACCCCTGAGCACCTACATGCTGACC
AACAGCGAGCTGCTGTCCCTGATCAACGACATGCCCATCACCAACGACCAGAAAAAGC
TGATGAGCAGCAACGTGCAGATCGTGCGGCAGCAGTCCTACAGCATCATGAGCATTATC
AAAGAAGAGATCCTGGCCTACGTGGTGCAGCTGCCTATCTACGGCGTGATCGATACCCC
TTGCTGGAAGCTGCACACAAGCCCTCTGTGCACCACCAATATCAAAGAGGGCTCCAACA
TCTGCCTGACCAGAACCGATAGAGGCTGGTACTGCGATAATGCCGGCAGCGTCAGCTTC
TTCCCACAAGCCGATACCTGCAAGGTGCAGAGCAACAGAGTGTTCTGCGATACCATGAA
CAGCCTGACACTGCCCAGCGAGGTGTCCCTGTGCAACACCGACATCTTCAACTCTAAGT
ACGACTGCAAGATCATGACCTCCAAGACCGACATCAGCTCCTCCGTGATCACATCTCTG
GGCGCCATCGTGTCCTGCTACGGCAAGACAAAGTGTACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCTGGAAGGCAAGAATCTGTACG
TGAAGGGCGAGCCCATCATCAACTACTACGACCCTCTGGTGTTCCCCAGCAGCGAGTTT
GATGCCAGCATCAGCCAAGTGAACGAGAAGATCAACCAGAGCCTGGCCTTCATCCGCA
GATCCGATGAACTGCTGCACAATGTGAACACCGGCAAGAGCACCACAAACATCATGATC
ACCGCCATCATCATCGTGATCATCGTCGTGCTGCTGAGCCTGATCGCTATCGGCCTGCTG
CTGTACTGATGA
SEQ ID NO:279 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
 
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGAATTGTGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGΛΛGGGCGΛGCCCΛTCΛTCΛΛCTTCTΛCGΛCCCTCTGGTGTTCCCCΛGCΛGCGΛGTT
TGATGCCAGCATCTCCCAAGTGAACGAGAAGATCAACCAGAGCCTGGCCTTCATCAGAA
AGTCCGATGAGCTGCTGCACAACGTGAACGCCGGCAAGTCCACCACCAATATCATGATC
ACCACGATCATCATCGTGATCATCGTCATCCTGCTGGCTCTGATCGCTGTGGGCCTGCTG
CTGTATTGATGA
SEQ ID NO:280 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATCACAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTGTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGATCCTGGCCTACGTGGTGCAGCTGCCTCTGTATGGCGTGATCGATACCCCTT
GCTGGAAGCTGCACACAAGCCCTCTGTGCACCACCAACACCAAAGAGGGCTCCAACAT
CTGCCTGACCAGAACCGATAGAGGCTGGTACTGCGATAATGCCGGCAGCGTCAGCTTCT
TCCCACAAGCCGAGACATGCAAGGTGCAGAGCAACAGAGTGTTCTGCGATACCATGAA
 
CAGCCTGACACTGCCCTCCGAAGTGAATCTGTGCAATATCGACATCTTCAACCCGAAGT
ACGACTGCAAGATCATGACCAGCAAGACCGACGTGTCAAGCTCCGTGATCACATCTCTG
GGCGCCATCGTGTCCTGCTACGGCAAGACAAAGTGCACCGCCAGCAACAAGAACCGGG
GCATCATCAAGACCTTCAGCAACGGCTGCGACTACGTGTCCAACAAAGGCGTGGACAC
CGTGTCTGTGGGCAACACCCTGTACTACGTGAACAAGCAAGAAGGCAAGAGCCTGTAC
GTGAAGGGCGAGCCCATCATCAACTTCTACGACCCTCTGGTGTTCCCCAGCAGCGAGTT
TGATGCCAGCATCTCCCAAGTGAACGAGAAGATCAACCAGAGCCTGGCCTTCATCAGAA
AGTCCGATGAGCTGCTGCACAACGTGAACGCCGGCAAGTCCACCACCAATATCATGATC
ACCACGATCATCATCGTGATCATCGTCATCCTGCTGGCTCTGATCGCTGTGGGCCTGCTG
CTGTATTGATGA
SEQ ID NO:281 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATGCCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTCTAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:282 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
 
TGTCTAAGGGCTATCTGTCTGCCCTGAGAACCGGCTGGTATACCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCTGCGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTCTAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:283 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCTGCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCTGCGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTCTAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCGTGATCGATACCCCT
 
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:284 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATGAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:285 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATCAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:286 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTCTAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
 
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:287 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATGCCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATGAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
 
CACCACGATCATCATCGTGATCATCGTCATCCTGCTGGCTCTGATCGCTGTGGGCCTGCT
GCTGTATTGATGA
SEQ ID NO:288 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATGCCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTCTAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:289 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATGAAGGTGCTGGACCTG
 
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:290 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATGCCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTGCCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCATGAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGCTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
 
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:291 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTCTGGATGCGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTCTAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:292 
ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
 
TCCTGGGCTTTCTGCTCGGAGTGGGAAGCGCCATTGCCTCTGGAATTTGCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTCTAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT
GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA
SEQ ID NO:293

ATGGAACTGCCCATCCTGAAAACAAACGCCATCACCACCATCCTGGCCGCCGTGACACT
GTGTTTTGCCAGCAGCCAGAACATCACCGAAGAGTTCTACCAGAGCACCTGTAGCGCCG
TGTCTAAGGGCTATCTGTCTGCCCTGAGAACCGGCTGGTATACCAGCGTGATCACCATCG
AGCTGAGCAACATCAAAGAAAACAAGTGCAACGGCACCGACGCCAAAGTGAAGCTGA
TCAAGCAAGAGCTGGACAAGTACAAGAACGCCGTGACCGAACTGCAGCTGCTGATGCA
GTCTACCCCTTGCGCCAATAGCAGAGCCAGACGGGAACTGCCTAGATTCATGAACTACA
CCCTGAACAACACCAAGAACACCAACGTGACCCTGAGCAAGAAGCGGAAGCGGAGAT
TCCTGGGCTTTCTGCTCGGAGTGGGAAGCGCCATTGCCTGCGGAATTGCCGTGTCCAAG
GTGCTGCATCTGGAAGGCGAAGTGAACAAGATCAAGAGCGCCCTGCTGAGCACCAACA
AGGCCGTGGTTTCTCTGAGCAATGGCGTGTCCGTGCTGACCTCTAAGGTGCTGGACCTG
AAGAACTACATCGACAAACAGCTGCTGCCCATCGTCAACAAGCAGAGCTGCAGCATCA
GCAACATCGAGACAGTGATCGAGTTCCAGCAGAAGAACAACCGGCTGCTGGAAATCAC
CCGCGAGTTTTCTGTGAATGCCGGCGTGACCACACCTGTGTCCACCTACATGCTGACCA
ACAGCGAGCTGCTGTCCCTGATCAACGACATGCCCATCACCAACGACCAGAAAAAGCT
GATGAGCAGCAACGTGCAGATCGTGCGGCAGCAGAGCTACTCCATCATGAGCATTATCA
AAGAAGAGGTGCTGGCCTACGTGGTGCAGCTGCCTCTGTATGGCGTGATCGATACCCCT
TGCTGGAAGCTGCACACAAGCCCTCTGTGCACCACCAACACCAAAGAGGGCTCCAACA
TCTGCCTGACCAGAACCGATAGAGGCTGGTACTGCGATAATGCCGGCAGCGTCAGCTTC
TTCCCACAAGCCGAGACATGCAAGGTGCAGAGCAACAGAGTGTTCTGCGATACCATGA
ACAGCCTGACACTGCCCTCCGAAGTGAATCTGTGCAATATCGACATCTTCAACCCGAAG
TACGACTGCAAGATCATGACCAGCAAGACCGACGTGTCAAGCTCCGTGATCACATCTCT

GGGCGCCATCGTGTCCTGCTACGGCAAGACAAAGTGCACCGCCAGCAACAAGAACCGG
GGCATCATCAAGACCTTCAGCAACGGCTGCGACTACGTGTCCAACAAAGGCGTGGACA
CCGTGTCTGTGGGCAACACCCTGTACTACGTGAACAAGCAAGAAGGCAAGAGCCTGTA
CGTGAAGGGCGAGCCCATCATCAACTTCTACGACCCTCTGGTGTTCCCCAGCGACGAGT
TTGATGCCAGCATCTCCCAAGTGAACGAGAAGATCAACCAGAGCCTGGCCTTCATCAGA
AAGTCCGATGAGCTGCTGCACAACGTGAACGCCGGCAAGTCCACCACCAATATCATGAT
CACCACGATCATCATCGTGATCATCGTCATCCTGCTGGCTCTGATCGCTGTGGGCCTGCT
GCTGTATTGATGA

## Claims

1. A nucleic acid comprising a polynucleotide encoding a mutant of RSV F protein comprising an F1 polypeptide and an F2 polypeptide, and comprising, as compared to a wild-type RSV F protein, one or more of the following mutations: disulfide bond mutations, cavity filling mutation and electrostatic mutation.

2. The nucleic acid according to claim 1, wherein the disulfide bond mutations comprise one or more of the follows: S46C and T311C, A47C and I309C, L48C and V308C, S55C and L188C, V56C and T189C, V56C and V300C, I57C and S190C, I57C and Y299C, T58C and K191C, I59C and L193C, I59C and L297C, E60C and D194C, P101C and I152C, A102C and I148C, A102C and I152C, T103C and I146C, T103C and I148C, N104C and G145C, N104C and S146C, N105C and G145C, N105C and S146C, N105C and A147C, V144C and V406C, and S146C and N460C;
preferably, the disulfide bond mutations comprise one or more of the follows: A47C and I309C, I57C and Y299C, P101C and I152C, A102C and I148C, A102C and I152C, T103C and I146C, N104C and G145C, N104C and S146C, N105C and G145C, N105C and S146C, and N105C and A147C.

3. The nucleic acid according to any one of claims 1-2, wherein the cavity filling mutation comprises one or more of the following (1) to (3):
(1) the substitution of T at position 54 by F, W, V, C, A, H, I, Y, L or M; preferably, the substitution of T at position 54 by F, W or V;
(2) the substitution of S at position 190 by V, I, M, F, L, W, Y, H, R, E, A, Q, G, P, T, C, D, N or K; and
(3) the substitution of V at position 296 by I, L, Y, H, F, or M.

4. The nucleic acid according to any one of claims 1-3, wherein the electrostatic mutation comprises one or more of the follows:
(1) the substitution of D at position 486 by Q, S, H, N, T, P, C, L, I, F, or W; and
(2) the substitution of V at position 487 by Q, T, N, I, K, P, R, S, Y, D, F, L, H, or C.

5. The nucleic acid according to any one of claims 1-4, wherein the mutations of the mutant comprise disulfide bond mutations and at least one of cavity filling mutation and electrostatic mutation; preferably, the mutations of the mutant comprise cavity filling mutation and disulfide bond mutations.

6. The nucleic acid according to any one of claims 1-5, wherein the mutations of the mutant are selected from the following (1) to (20):
(1) A102C, 1152C, T54V, S190M and V296L;
(2) A102C, 1152C, T54F, S190M and V296L;
(3) A102C, 1152C, T54C, S190M and V296L;
(4) A102C, 1152C, T54A, S190M and V296L;
(5) A102C, I152C, T54V, S190F and V296L;
(6) A102C, I152C, T54F, S190F and V296L;
(7) A102C, 1152C, T54C, S190F and V296L;
(8) A102C, 1152C, T54A, S190F and V296L;
(9) P101C, 1152C, T54V, S190M and V296L;
(10) P101C, 1152C, T54F, S190M and V296L;
(11) P101C, 1152C, T54C, S190M and V296L;
(12) P101C, 1152C, T54A, S190M and V296L;
(13) P101C, I152C, T54V, S190F and V296L;
(14) P101C, 1152C, T54F, S190F and V296L;
(15) P101C, I152C, T54C, S190F and V296L;
(16) P101C, I152C, T54A, S190F and V296L;
(17) A102C, 1152C, T54V, S190I and V296L;
(18) A102C, 1152C, T54F, S190I and V296L;
(19) A102C, 1152C, T54C, S190I and V296L; and
(20) A102C, 1152C, T54A, S190I and V296L.

7. The nucleic acid according to claim 6, wherein the mutant is selected from one of the following (1) to (40):
(1) a mutant comprising the amino acid sequence as shown in SEQ ID NO: 61 or 62;
(2) a mutant comprising mutations A102C, I152C, T54V, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 61 or 62;
(3) a mutant comprising the amino acid sequence as shown in SEQ ID NO: 63 or 64;
(4) a mutant comprising mutations A102C, I152C, T54F, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 63 or 64;
(5) a mutant comprising the amino acid sequence as shown in SEQ ID NO: 65 or 66;
(6) a mutant comprising mutations A102C, I152C, T54C, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 65 or 66;
(7) a mutant comprising the amino acid sequence as shown in SEQ ID NO: 67 or 68;
(8) a mutant comprising mutations A102C, I152C, T54A, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 67 or 68;
(9) a mutant comprising the amino acid sequence as shown in SEQ ID NO: 69 or 70;
(10) a mutant comprising mutations A102C, 1152C, T54V, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 69 or 70;
(11) a mutant comprising the amino acid sequence as shown in SEQ ID NO: 71 or 72;
(12) a mutant comprising mutations A102C, 1152C, T54F, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 71 or 72;
(13) a mutant comprising the amino acid sequence as shown in SEQ ID NO: 73 or 74;
(14) a mutant comprising mutations A102C, 1152C, T54C, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 73 or 74;
(15) a mutant comprising the amino acid sequence as shown in SEQ ID NO: 75 or 76;
(16) a mutant comprising mutations A102C, 1152C, T54A, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 75 or 76;
(17) a mutant comprising the amino acid sequence as shown in SEQ ID NO: 77 or 78;
(18) a mutant comprising mutations P101C, 1152C, T54V, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 77 or 78;
(19) a mutant comprising the amino acid sequence as shown in SEQ ID NO: 79 or 80;
(20) a mutant comprising mutations P101C, 1152C, T54F, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 79 or 80;
(21) a mutant comprising the amino acid sequence as shown in SEQ ID NO: 81 or 82;
(22) a mutant comprising mutations P101C, I152C, T54C, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 81 or 82;
(23) a mutant comprising the amino acid sequence as shown in SEQ ID NO: 83 or 84;
(24) a mutant comprising mutations P101C, I152C, T54A, S190M and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 83 or 84;
(25) a mutant comprising the amino acid sequence as shown in SEQ ID NO: 85 or 86;
(26) a mutant comprising mutations P101C, 1152C, T54V, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 85 or 86;
(27) a mutant comprising the amino acid sequence as shown in SEQ ID NO: 87 or 88;
(28) a mutant comprising mutations P101C, 1152C, T54F, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 87 or 88;
(29) a mutant comprising the amino acid sequence as shown in SEQ ID NO: 89 or 90;
(30) a mutant comprising mutations P101C, I152C, T54C, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 89 or 90;
(31) a mutant comprising the amino acid sequence as shown in SEQ ID NO: 91 or 92;
(32) a mutant comprising mutations P101C, I152C, T54A, S190F and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 91 or 92;
(33) a mutant comprising the amino acid sequence as shown in SEQ ID NO: 93 or 94;
(34) a mutant comprising mutations A102C, I152C, T54V, S190I and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 93 or 94;
(35) a mutant comprising the amino acid sequence as shown in SEQ ID NO: 95 or 96;
(36) a mutant comprising mutations A102C, I152C, T54F, S190I and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 95 or 96;
(37) a mutant comprising the amino acid sequence as shown in SEQ ID NO: 97 or 98;
(38) a mutant comprising mutations A102C, I152C, T54C, S190I and V296L and comprising an amino acid sequence at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 97 or 98;
(39) a mutant comprising the amino acid sequence as shown in SEQ ID NO: 99 or 100; and
(40) a mutant comprising mutations A102C, I152C, T54A, S190I and V296L and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identical to the amino acid sequence as shown in SEQ ID NO: 99 or 100.

8. A nucleic acid, comprising a polynucleotide encoding a mutant of RSV F protein comprising an F1 polypeptide and an F2 polypeptide, and comprising, as compared to a wild-type RSV F protein, one or more mutations of the following (1) to (8):
(1) the substitution of K or E at position 66 by I, L, M, F or V;
(2) the substitution of N at position 67 by I;
(3) the substitution of S at position 213 by P;
(4) the substitution of S at position 215 by P;
(5) the substitution of N at position 216 by P;
(6) the substitution of I at position 217 by P, F, Y or W;
(7) the substitution of D at position 486 by Q, S, H, N, T, P, C, L, I, F, or W; and
(8) the substitution of V at position 487 by Q, T, N, I, K, P, R, S, Y, D, F, L, H, or C.

9. The nucleic acid according to claim 8, wherein the mutations of the mutant comprise the following (1) to (3):
(1) one of the following mutations: the substitution of S at position 213 by P, the substitution of S at position 215 by P, the substitution of N at position 216 by P, and the substitution of I at position 217 by P, F, Y or W;
(2) one of the following mutations: the substitution of K or E at position 66 by I, L, M, F or V and the substitution of N at position 67 by I; and
(3) one of the following mutations: the substitution of D at position 486 by Q, S, H, N, T, P, C, L, I, F or W and the substitution of V at position 487 by Q, T, N, I, K, P, R, S, Y, D, F, L, H or C.

10. The nucleic acid according to any one of claims 1-9, wherein the mutations of the mutant are selected from one group of the following (1) to (13):
(1) I217P, E66I and D486Q;
(2) I217P, N67I and E487Q;
(3) I217P, E66I and E487T;
(4) S215P, N67I and E487Q;
(5) S213P, N67I and E487Q;
(6) S213P, E66I and E487T;
(7) S213P, E66I and D486Q;
(8) N216P, N67I and E487Q;
(9) N216P, E66I and E487T;
(10) N216P, E66I and D486Q;
(11) A102C, 1152C, S213P and E66I;
(12) P101C, I152C, S213P and E66I; and
(13) S213P and E66I.

11. The nucleic acid according to claim 10, wherein the mutant is selected from one of the following (1) to (30):
(1) a mutant comprising the amino acid sequence as shown in SEQ ID NO: 31 or 32;
(2) a mutant comprising mutations N216P, E66I and D486Q and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 31 or 32;
(3) a mutant comprising the amino acid sequence as shown in SEQ ID NO: 33 or 34;
(4) a mutant comprising mutations N216P, E66I, and E487T and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 33 or 34;
(5) a mutant comprising the amino acid sequence as shown in SEQ ID NO: 35 or 36;
(6) a mutant comprising mutations N216P, N67I, and E487Q and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 35 or 36;
(7) a mutant comprising the amino acid sequence as shown in SEQ ID NO: 37 or 38;
(8) a mutant comprising mutations S213P, E66I, and D486Q and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 37 or 38;
(9) a mutant comprising the amino acid sequence as shown in SEQ ID NO: 39 or 40;
(10) a mutant comprising mutations S213P, E66I, and E487T and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 39 or 40;
(11) a mutant comprising the amino acid sequence as shown in SEQ ID NO: 41 or 42;
(12) a mutant comprising mutations S213P, N67I and E487Q and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 41 or 42;
(13) a mutant comprising the amino acid sequence as shown in SEQ ID NO: 43 or 44;
(14) a mutant comprising mutations S215P, N67I and E487Q and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 43or 44;
(15) a mutant comprising the amino acid sequence as shown in SEQ ID NO: 45 or 46;
(16) a mutant comprising mutations I217P, E66I, and E487T and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 45 or 46;
(17) a mutant comprising the amino acid sequence as shown in SEQ ID NO: 47 or 48;
(18) a mutant comprising mutations I217P, N67I, and E487Q and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 47 or 48;
(19) a mutant comprising the amino acid sequence as shown in SEQ ID NO: 49 or 50;
(20) a mutant comprising mutations I217P, E66I and D486Q and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 49 or 50;
(21) a mutant comprising the amino acid sequence as shown in SEQ ID NO: 51 or 52;
(22) a mutant comprising mutations S213P, E66I and E487T and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 51 or 52;
(23) a mutant comprising the amino acid sequence as shown in SEQ ID NO: 53 or 54;
(24) a mutant comprising mutations S213P, E66I, and D486Q and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 53 or 54;
(25) a mutant comprising the amino acid sequence as shown in SEQ ID NO: 55 or 56;
(26) a mutant comprising mutations A102C, I152C, S213P and E66I and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 55or 56;
(27) a mutant comprising the amino acid sequence as shown in SEQ ID NO: 57 or 58;
(28) a mutant comprising mutations P101C, I152C, S213P and E66I and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 57 or 58;
(29) a mutant comprising the amino acid sequence as shown in SEQ ID NO: 59 or 60; and
(30) a mutant comprising mutations S213P and E66I and comprising an amino acid sequence at least 85%, 88%, 90%, 91%, 92%, 93%, 94%, 95%, 95.5%, 96%, 96.5%, 97%, 97.5%, 98%, 98.5%, 99%, or 99.5% identical to the amino acid sequence as shown in SEQ ID NO: 59 or 60.

12. The nucleic acid according to any one of claims 1-11, wherein the wild-type RSV is subtype A or subtype B;
preferably, the wild-type RSV is wild-type hRSV;
preferably, the wild-type hRSV is strain A2, strain Ontario or strain Buenos Aires.

13. The nucleic acid according to any one of claims 1-12, wherein the nucleic acid is RNA; preferably, the RNA is mRNA; more preferably, the mRNA comprises at least one of 5'-cap structure, 5'-UTR, 3'-UTR and poly(A) tail;
or, the nucleic acid is DNA; preferably, the DNA can be transcribed into RNA.

14. The nucleic acid according to claim 13, wherein the DNA sequence corresponding to the 5'-UTR is shown in SEQ ID NO: 209;
and/or, the DNA sequence corresponding to the 3'-UTR is shown in SEQ ID NO: 210;
and/or, the nucleotides constituting the poly(A) tail comprise at least 20, at least 40, at least 80, at least 100 or at least 120 A nucleotides; preferably, the nucleotides constituting the poly(A) tail comprise at least 20, at least 40, at least 80, at least 100 or at least 120 consecutive A nucleotides; preferably, the nucleotides constituting the poly(A) tail comprise one or more nucleotides other than the A nucleotide; more preferably, the DNA sequence corresponding to the poly(A) tail is shown in SEQ ID NO: 211.

15. The nucleic acid according to any one of claims 1-14, comprising a modified nucleotide;
preferably, the nucleic acid comprises a modified nucleoside; preferably, the modified nucleoside comprises at least one of a modified uridine, a modified cytidine, a modified adenosine, and a modified guanosine.

16. The nucleic acid of any one of claims 1-15, comprising a polynucleotide encoding one or more mutants of RSV F protein.

17. The nucleic acid according to any one of claims 1-16, further comprising a polynucleotide encoding a protein or polypeptide other than the mutant of RSV F protein.

18. The nucleic acid according to any one of claims 1-17, further comprising one or more of a polynucleotide encoding the transmembrane domain of RSV F protein, a polynucleotide encoding a pep27 polypeptide, a polynucleotide encoding a signal peptide, and a polynucleotide encoding a trimerization domain.

19. A genetic engineering vector comprising the nucleic acid according to any one of claims 1-18, or a polynucleotide that can be transcribed into the nucleic acid according to any one of claims 1-18.

20. A host cell comprising the genetic engineering vector of claim 19.

21. A mutant of RSV F protein encoded by the nucleic acid according to any one of claims 1-18;
preferably, the mutant is a trimer.

22. A delivery carrier comprising
a nucleic acid composition comprising a first nucleic acid, which is the nucleic acid according to any one of claims 1-18, or a first genetic engineering vector, which is the genetic engineering vector according to claim 19;
or, a plurality of first nucleic acids, which are the nucleic acids according to any one of claims 1-18 or a plurality of first genetic engineering vectors, which are the genetic engineering vector according to claim 19, and the plurality of first nucleic acids are independent from each other or the plurality of first genetic engineering vectors are independent from each other.

23. The delivery carrier according to claim 22, wherein the nucleic acid composition further comprises a second nucleic acid comprising a polynucleotide encoding a protein or polypeptide other than the mutant of RSV F protein, or a second vector comprising a polynucleotide encoding a protein or polypeptide other than the mutant of RSV F protein;
and/or, the first nucleic acid and the second nucleic acid are RNAs; more preferably, the first nucleic acid and the second nucleic acid are mRNAs.

24. The delivery carrier according to any one of claims 22-23, wherein the delivery carrier is a lipid nanoparticle (LNP), a cationic liposome, a cationic proteins or a lipopolyplex (LPP);
preferably, the delivery carrier is a lipid nanoparticle comprising a cationic lipid comprising the following compound (IV), or a pharmaceutically acceptable salt or stereoisomer thereof: 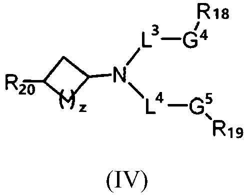 where:
L³ and L⁴ are the same or different, and are each independently C1-C12 alkylene, C2-C12 alkenylene or C2-C12 alkynylene; preferably L³ and L⁴ are the same or different, and are each independently C3-C10 alkylene, C3-C10 alkenylene or C3-C10 alkynylene; further preferably, L³ and L⁴ are the same or different, and are each independently C3-C10 alkylene; most preferably, L³ and L⁴ are the same or different, and are each independently C5-C8 alkylene;
G⁴ and G⁵ are the same or different, and are each independently -O-(C=O)-, -(C=O)-O-, - C(=O)-, -O-, -C(=O)-S- or -S-C(=O)-; preferably, G⁴ and G⁵ are the same or different, and are each independently -O-(C=O)-, -(C=O)-O-, -C(=O)- or -O-; most preferably, G⁴ and G⁵ are the same or different, and are each independently selected from -O-(C=O)- or -(C=O)-O-;
R₁₈ and R₁₉ are the same or different, and are each independently C5-C27 alkyl or C5-C27 alkenyl containing one or more double bonds; preferably, R₁₈ and R₁₉ are the same or different, and are each independently C8-C20 alkyl or C8-C20 alkenyl containing one or more double bonds; further preferably, R₁₈ and R₁₉ are the same or different, and are each independently C9-C17 alkyl or C9-C18 alkenyl containing one or two double bonds; most preferably, R₁₈ and R₁₉ are the same or different, and are each independently 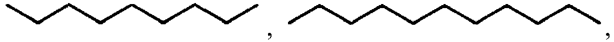 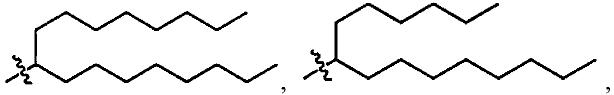 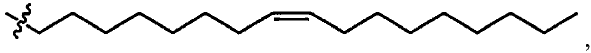
or 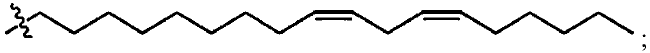
R₂₀ is halogen, hydroxyl, cyano, C1-C6 alkyl, nitro, C1-C6 alkoxy, C1-C6 alkylcarbonyloxy, C1-C6 alkoxycarbonyl, C1-C6 alkylaminocarbonyl or C1-C6 alkylcarbonylamino; preferably, R₂₀ is halogen, hydroxyl, cyano, C1-C6 alkoxy, C1-C6 alkylcarbonyloxy, C1-C6 alkoxycarbonyl, C1-C6 alkylaminocarbonyl or C1-C6 alkylcarbonylamino; further preferably, R₂₀ is halogen, hydroxyl, cyano, C1-C4 alkoxy, C1-C4 alkylcarbonyloxy, C1-C4 alkoxycarbonyl, C1-C4 alkylaminocarbonyl or C1-C4 alkylcarbonylamino; most preferably, R₂₀ is fluorine, hydroxyl, cyano, methoxy, acetoxy, methoxycarbonyl, butylaminocarbonyl or acetamido;
z is 1, 2 or 3.

25. The delivery carrier according to claim 24, wherein the cationic lipid is the following compound (IV-1), or a pharmaceutically acceptable salt or stereoisomer thereof: 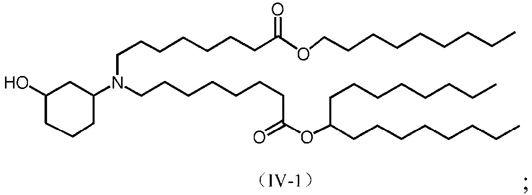
or the cationic lipid is the following compound, or a pharmaceutically acceptable salt thereof: 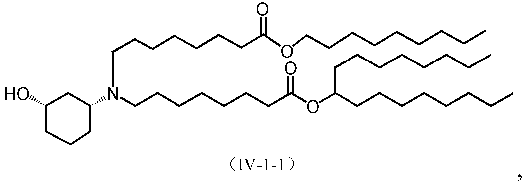 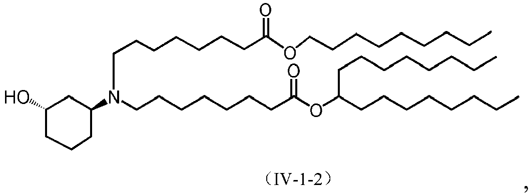 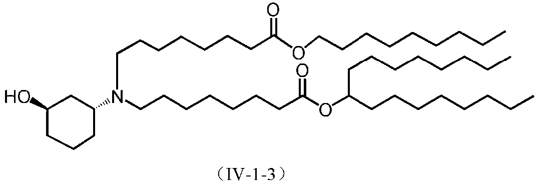
or

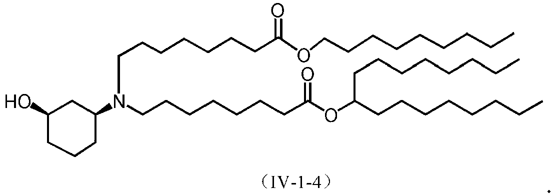

26. A pharmaceutical composition comprising the nucleic acid of any one of claims 1-18, the genetic engineering vector of claim 19, the host cell of claim 20, the mutant of RSV F protein of claim 21, or the delivery carrier of any one of claims 22-25, and a pharmaceutically acceptable carrier.

27. The pharmaceutical composition of claim 26, comprising a plurality of said delivery carriers;
or the pharmaceutical composition comprises a plurality of said nucleic acids.

28. Use of the nucleic acid according to any one of claims 1-18, the genetic engineering vector according to claim 19, the host cell according to claim 20, the mutant of RSV F protein according to claim 21, the delivery carrier according to any one of claims 22-25 or the pharmaceutical composition according to any one of claims 26-27 in the preparation of a medicament;
preferably, the medicament is used for preventing or treating the infection of RSV or a disease caused by the infection of RSV;
preferably, the medicament is a vaccine.

29. A vaccine comprising the nucleic acid of any one of claims 1-18, the genetic engineering vector of claim 19, the mutant of RSV F protein of claim 21, or the delivery carrier of any one of claims 22-25.
